(19) Europäisches Patentamt / European Patent Office / Office européen des brevets

(11) **EP 2 935 613 B1**

(12) **EUROPEAN PATENT SPECIFICATION**

(45) Date of publication and mention
of the grant of the patent:
**27.05.2020 Bulletin 2020/22**

(21) Application number: **13864616.1**

(22) Date of filing: **19.12.2013**

(51) Int Cl.:
*G01N 33/543* (2006.01)        *C12Q 1/24* (2006.01)
*B01L 3/00* (2006.01)          *G01N 33/569* (2006.01)

(86) International application number:
**PCT/US2013/076649**

(87) International publication number:
**WO 2014/100456 (26.06.2014 Gazette 2014/26)**

(54) **TARGET CAPTURE SYSTEM**

**ZIELERFASSUNGSSYSTEM**

**SYSTÈME DE CAPTURE DE CIBLE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:
19.12.2012  US 201261739644 P
19.12.2012  US 201261739511 P
19.12.2012  US 201261739575 P
19.12.2012  US 201261739618 P
19.12.2012  US 201261739577 P
19.12.2012  US 201261739616 P
19.12.2012  US 201261739647 P
19.12.2012  US 201261739612 P
19.12.2012  US 201261739567 P

(43) Date of publication of application:
**28.10.2015 Bulletin 2015/44**

(73) Proprietor: **DNAE Group Holdings Limited
London
W12 7SB (GB)**

(72) Inventors:
• **CLARIZIA, Lisa-jo, Ann**
  **Albuquerque, NM 87106 (US)**
• **ADAMS, Eddie, W.**
  **Albuquerque, NM 87108 (US)**
• **DRYGA, Sergey, A.**
  **Rio Rancho, NM 87144 (US)**
• **NORVELL, Meghan, E.**
  **Albuquerque, NM 87110 (US)**

• **DYKES, Colin**
  **Albuquerque, NM 87110 (US)**
• **BARR, Alexandra**
  **Albuquerque, NM 87112 (US)**
• **SITDIKOV, Ravil, A.**
  **Albuquerque, NM 87120 (US)**
• **TORRANCE, Magdalena, A.**
  **Albuquerque, NM 87114 (US)**
• **ALEY, David, K.**
  **Albuquerque, NM 87110 (US)**
• **SMITH, Erik, J.**
  **Los Lunas, NM 87031 (US)**
• **ESCH, Victor, C.**
  **Albuquerque, NM 87111 (US)**
• **MACEMON, James, H.**
  **Albuquerque, NM 87111 (US)**
• **VANDERVEST, Jaclyn**
  **Albuquerque, NM 87120 (US)**

(74) Representative: **Graham Watt & Co LLP
St. Botolph's House
7-9 St. Botolph's Road
Sevenoaks TN13 3AJ (GB)**

(56) References cited:
EP-A2- 2 261 650        WO-A1-2011/019874
US-A1- 2006 233 712     US-A1- 2011 098 623
US-A1- 2011 137 018     US-A1- 2011 262 893
US-A1- 2011 262 925     US-A1- 2012 045 828
US-B2- 6 867 021

**Description**

Related Application

The present application claims the benefit of and priority to each of U.S. provisional patent application serial number 61/739,647, filed December 19, 2012; U.S. provisional patent application serial number 61/739,577, filed December 19, 2012; U.S. provisional patent application serial number 61/739,644, filed December 19, 2012; U.S. provisional patent application serial number 61/739,612, filed December 19, 2012; U.S. provisional patent application serial number 61/739,511, filed December 19, 2012; U.S. provisional patent application serial number 61/739,616, filed December 19, 2012; U.S. provisional patent application serial number 61/739,567, filed December 19, 2012; U.S. provisional patent application serial number 61/739,575, filed December 19, 2012; and U.S. provisional patent application serial number 61/739,618, filed December 19, 2012.

Technical Field

This invention generally relates to cartridges for separating targets from a sample and methods for separating targets from a sample with or without the use of cartridges of the invention.

Background

Many laboratory and clinical procedures involve processing a sample to separate a target from the sample for subsequent identification and analysis of the target. Such processes are commonly used to detect a wide range of targets, including biological entities such as cells, viruses, proteins, bacterium, nucleic acids, etc., and have applications in clinical diagnostics, biohazard screenings, and forensic analyses.

Often there is an immediate need to identify the target, whether to determine the proper course of treatment or to develop response protocol for biohazard threat. For example, blood-borne pathogens are a significant healthcare problem because a delayed or improper diagnosis can lead to sepsis. Sepsis, a severe inflammatory response to infection, is a leading cause of death in the United States and early detection of the blood-borne pathogens underlying the infection is crucial to preventing the onset of sepsis. With early detection, the pathogen's drug/antibiotic resistant profile can be obtained which allows the clinician to determine the appropriate anti-microbial therapy for a quicker and more effective treatment.

Pathogens during active blood-borne infection or after antibiotic treatment are typically present in minute levels per mL of body fluid. Several techniques have been developed for isolation of pathogens in a body fluid sample, which include molecular detection methods, antigen detection methods, and metabolite detection methods. These conventional methods often require culturing specimens, such as performing an incubation or enrichment step, in order to detect the low levels of pathogens. The incubation/enrichment period is intended to allow for the growth of bacteria and an increase in bacterial cell numbers to more readily aid in isolation and identification. In many cases, a series of two or three separate incubations is needed to isolate the target bacteria. Moreover, enrichment steps require a significant amount of time and can potentially compromise test sensitivity by killing some of the cells sought to be measured. In certain cases, a full week may be necessary to reach the desired levels of bacteria.

The above techniques can be carried out on microfluidic devices to separate the pathogen or target from the sample. However, current microfluidic devices require extensive sample preparation prior to introducing the sample into the device for subsequent isolation. Microfluidic devices by definition are designed to conduct reactions and process at a microfluidic scale. To isolate pathogens in a microfluidic device, it is necessary to incubate/enrich the sample to expand the levels of pathogens to increase the chance that the small volume of sample introduced into the microfluidic device contains a pathogen. Alternatively, a large volume of fluid suspected of containing a pathogen is introduced at an extremely slow pace through a tube or by piecemeal pipetting the sample into the microfluidic device. These methods are equally time consuming as incubation and can result in loss of clinically relevant sample (i.e. sample with sufficient levels of targets for capture). In addition, the transfer of large volume of fluid into the microfluidic device may expose the sample to contamination.

Currently, there is no effective device capable of rapidly and effectively isolating a target that is fast and sensitive in order to provide data critical for patient treatment and biohazard analysis in a clinically relevant time frame.

Summary

The invention provides a system (target capture system) that allows rapid isolation of a target analyte from a sample without the need for sample preparation or extensive manual operation. A target capture system of the invention concentrates essentially all of a clinically relevant portion of a sample (less than 1 mL) from an initial sample volume of about 10 mL. The ability to isolate small, clinically relevant, volumes of sample improves the efficiency in which nucleic acids can be extracted from a target for subsequent analysis.

The target capture systems generally include a cartridge and an instrument. The cartridge includes components such as channels, mixing chambers, and traps to process the sample for target isolation. The cartridge may interface with an instrument having one or more assemblies, such as mechanical, magnetic, pneumatic, and fluidic assemblies, that interact with the cartridge to assist/drive the processes performed on the cartridge. The target capture systems of the invention are integrated to perform several processes on a sample inputted into the cartridge to achieve a final result without user manipulation. The final result can be capture of live/whole target cells or isolation of nucleic acids from target cells within the sample.

In certain aspects, the processes performed by the target capture system include introducing a plurality of magnetic particles, in which a plurality of the particles include at least one captured moiety specific to a target, into a sample to form a mixture that includes sample and particles. The mixture is incubated to form at least one target/particle complex and a magnetic field is applied to isolate the magnetic particle/target complexes from the sample. The process starts at inputting a sample and ends at delivering a capture target (or nucleic acids of the target) into a container for further analysis.

Cartridges of the invention include a chamber for holding and releasing the magnetic particles into a sample to form a mixture and at least two magnetic traps for receiving the sample/magnetic particle mixture. The magnetic trap engages with a magnetic assembly to isolate the magnetic particles from the sample. The cartridge includes a first magnetic trap in communication with a second magnetic trap, in which both magnetic traps engage with a magnetic assembly to isolate magnetic particles from a sample. In one embodiment, the first magnetic trap isolates magnetic particles from the sample and the isolated magnetic particles are then transferred through the second magnetic trap. The second magnetic trap engages with a second magnetic assembly to isolate the plurality of magnetic particles from the first magnetic trap. A lysing mechanism operably associated with the second magnetic trap can be used to lyse at least one target cell bound to at least one of the magnetic particles within the second magnetic trap. The cartridge may further include a matrix for receiving lysate from the first magnetic trap and retaining nucleic acid from the lysate. In one embodiment, the matrix is an affinity column. The cartridge can further include a reaction chamber, such as a bubble mixer, in communication with the matrix for pre-treating the lysate.

In one embodiment, the cartridge is for use with an instrument and the instrument includes a first magnetic assembly, a second magnetic assembly, and a lysing mechanism. The first and second magnetic assemblies generate magnetic fields to isolate magnetic particles disposed within a sample against a surface of a corresponding magnetic trap. The lysing mechanism may perform cell lysis on targets bound to the magnetic particles. The lysing mechanism can be a sonication device. Lysis can be achieved by placing the sonication against a surface of a magnetic trap and generating sonic wave to invoke cell lysis on any targets bound to the magnetic particles within the magnetic trap. The instrument can also include a drive mechanism for driving and controlling movement of the sample, the magnetic particles, and any other fluids or substances into, within, and out of the cartridge. The drive mechanism can be pneumatic, mechanical, magnetic and/or fluidic.

For isolation and detection assays conducted on cartridges or chips, it is important to transfer the entire obtained sample from a collection device into the cartridge to increase the efficiency of isolation or detection. Especially in situations where there is little sample, which is often the case in forensic analysis, or where there is a small concentration of targets per mL of sample (e.g. 1 CFU/mL), which is often the case for pathogenic detection.

Accordingly, the cartridges of the invention include a cartridge/vessel interface designed to maximize the amount of sample transferred from the vessel and into the cartridge to avoid loss of clinically relevant sample within a sample collection vessel and/or during sample transfer. This ensures that substantially all clinically relevant sample is transferred into the cartridge and processed. The cartridge/vessel interface places the vessel containing the sample in two-way communication with the cartridge. The communication between the vessel and the cartridge can be pneumatic, fluidic, or both. In certain embodiments, the cartridge/vessel interface can include an input member and an output member. The output member introduces fluids, gases, and substances from the cartridge into the vessel and the input member transfers the sample and any introduced fluids, gases, and substances from vessel into the cartridge. Typically, the input and output members define a lumen and include a penetrating tip. The input and output members may be designed to penetrate and extend into the vessel.

The cartridge includes a cartridge/vessel interface, a chamber, and a magnetic trap. The cartridge/vessel interface couples a vessel containing a sample to the cartridge. The chamber is in communication with the cartridge/interface and can releasably hold fluid or substance for introducing the fluid or substance into the vessel. The magnetic trap is in communication with the vessel at the interface and receives the contents of the vessel. The vessel contents can include the sample and the fluid or substances introduced into the vessel from the chamber. In one embodiment, the chamber contains a plurality of magnetic particles to release into the vessel. The magnetic particles can include one or more binding moieties specific to a target within the sample. The sample and magnetic particles are transferred into the magnetic trap of the cartridge. The magnetic trap can engage with a magnetic assembly to isolate the magnetic particles from the sample.

A significant advantage of certain embodiments is that the cartridge includes both macrofluidic and microfluidic components so that the cartridge can process both macrofluidic and microfluidic volumes of fluid. This aspect of the invention accounts for the fact that a minute amount of target cells may be present in a sample having a macrofluidic volume which necessitates processing the entire macrofluidic volume in order to increase the likelihood that the target cell will be isolated. To isolate pathogens in a microfluidic device, the entire macrofluidic volume of sample would have to be transferred slowly or in a piecemeal fashion (e.g. via pipetting) into a microfluidic device at microfluidic rate, which undesirably takes a long amount of time and risks losing the target analyte of interest during the transfer. In certain aspects, the cartridge is designed to consolidate a sample of macrofluidic volume into a concentrated microfluidic volume of fluid that contains target cells of interest. The concentrated microfluidic volume is then processed at the microfluidic level.

In certain aspects, the target capture system includes a macrofluidic portion and a microfluidic portion. The macrofluidic portion includes a first magnetic trap and is configured to process a macro-scale volume of fluid including a sample, in which processing includes introducing a quantity of magnetic particles into the macro-scale volume of fluid. The microfluidic portion is in communication with the macrofluidic portion and includes a second magnetic trap. The microfluidic portion is configured to isolate a micro-scale volume of the macro-scale volume of fluid in which the micro-scale volume of fluid contains substantially the entire quantity of magnetic particles. In certain embodiments, the magnetic particles include a binding moiety specific to a target within the sample. Within the cartridge, the magnetic particles initially bind to targets in a macrofluidic volume of fluid and then the magnetic particles are concentrated into a micro-scale volume of fluid. This allows one to isolate targets initially present in a macro-scale volume into an easy to process micro-scale volume of fluid.

Magnetic particles suitable for use in the target capture system are conjugated to a capture moiety specific to a target analyte. In certain embodiments, the target capture system utilizes compositions that include a plurality of sets of magnetic particles conjugated to capture moieties to capture different targets of interest. For example, each set of the plurality of magnetic particles may be conjugated with capture moieties having different specificities for different pathogens. The capture moieties conjugated to the magnetic particles may be of the same class or different class. For example, one set of capture moieties may be antibodies specific to a first pathogen, and the other set of capture moieties may be other antibodies specific to a second pathogen. In another example, one set of capture moieties may be antibodies specific to a first pathogen, and the other set of capture moieties may be lectins specific to a second pathogen. In certain embodiments, each magnetic particle of a set is conjugated to at least two different sets of capture moieties specific to different pathogens. That is, one magnetic particle may have two or more capture moieties specific to different pathogens. The two or more capture moieties conjugated to a magnetic particle may be of the same class or different class.

Brief Description of the Drawings

FIG. 1 outlines the processing steps of the target capture system according to certain embodiments.

FIG. 2 depicts a schematic overview of a cartridge according to certain embodiments.

FIG. 3 depicts an external view of the cartridge according to certain embodiments.

FIG. 4 depicts the cartridge/vessel interface according to certain embodiments.

FIG 5 depicts the instrument of the target capture system according to certain embodiments.

FIG.6 depicts the instrument of the target capture system according to certain embodiments.

FIG. 7 depicts a schematic overview of a cartridge according to certain embodiments.

FIGS. 8-22 depict the processing steps for target capture within the cartridge of the target capture system according to certain embodiments.

FIG.23 provides one exemplary configuration of a flow cell and first and second sets of magnets for generating alternative magnetic fields.

FIG. 24A provides an exemplary process chart for implementation of methods of the invention for separation of bacteria from blood. FIG. 24B provides a magnified view of a target/magnetic particle complex.

FIG. 25 gives a diagram of methods for isolating different pathogens.

FIG. 26 illustrates inducing the expression of a common antigen by a viral preparation.

FIG. 27 shows using a viral preparation to biotinylate different unknown pathogens.

FIG. 28 illustrates phage display of a common antigen by different unknown pathogens.

FIG. 29 illustrates a fluidic cartridge according to certain embodiments.

FIG. 30 gives a perspective view of the cartridge shown in FIG. 29.

FIG. 31 gives a schematic diagram of the cartridge shown of FIG. 29

FIG. 32 illustrates detecting an analyte according to certain embodiments.

FIG. 33 shows embodiments of methods of detecting a target analyte.
FIG. 34 shows a device according to embodiments of the invention.
FIG. 35 gives a perspective view of the device shown in FIG. 34.

Detailed Description

The invention generally relates to target capture systems configured to carry out the processes necessary to isolate a target analyte from a sample without the need for sample preparation or manual operation. The target capture systems generally include a cartridge and an instrument. The cartridge includes components such as channels, reaction chamber, and reservoirs, etc. configured to perform processes for isolating a target from a sample. The cartridge interfaces with an instrument having one or more assemblies or subsystems, such as mechanical, magnetic, pneumatic, and fluidic assemblies, that interact with the cartridge to assist/drive the processes performed on the cartridge. The systems of the invention are fully integrated to perform several processes on a sample inputted into the cartridge to achieve a final result, such as live cell capture or isolated nucleic acids from a target cell, without user manipulation.

Various embodiments of the target capture system including the cartridge and the instrument and processes performed by the target capture system are described in detail below.

In certain aspects, the processes performed by the target capture systems generally include introducing a plurality of magnetic particles, in which each particle includes at least one binding moiety specific to a target, into a sample to form at least one target/particle complex and applying a magnetic field to isolate the magnetic particle/target complexes from the sample. The process starts at inputting a sample and ends at delivering a capture target (or nucleic acids of the target) into a container for further analysis.

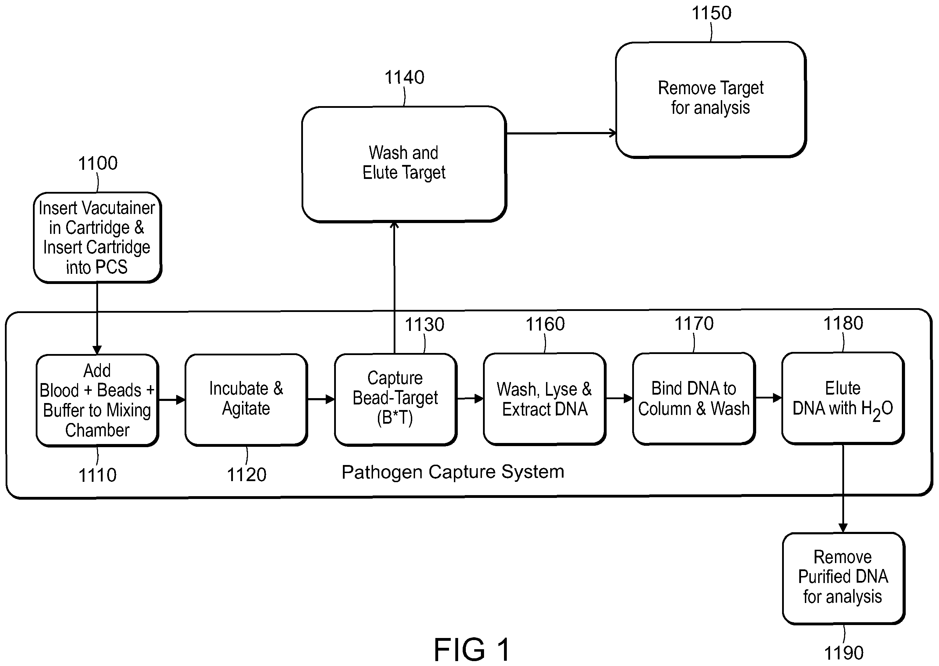

FIG. 1 outlines the processing steps for isolating a target using the target capture system according to one embodiment. In step 1100, a vessel is coupled to an interface on a cartridge to place the cartridge in direct communication with the vessel. The cartridge is then loaded into the instrument. The instrument is then activated to perform the following processes. In step 1110, magnetic capture particles from the cartridge are introduced into the vessel from the cartridge through the interface. The capture particles may include binding moieties specific to a target so that targets within the sample will bind to the particles. In certain aspects, the capture particles are disposed within a fluid, such as a buffer, to facilitate introduction of the capture particles and fluid in the vessel. The sample/capture particles/fluid mixture is transferred from the vessel into the cartridge. The fluid introduced into the vessel rinses any remaining sample from the vessel. In addition, air can be introduced into the vessel to force transfer of the vessel contents (sample/fluid/particles) into the cartridge. In step 1120, the sample/particle/fluid mixture is incubated and agitated within the cartridge to form target/magnetic field complexes. In step 1130, a magnetic field is applied to capture the target/magnetic field is applied to capture the target/magnetic particles on surface of a chamber/trap of the cartridge. As in steps 1140 and 1160, the surface of the magnetic trap is then washed with a wash solution that removes any unbound sample and/or removes the bound targets from the magnetic particles. In step 1150, the target capture system elutes the target/magnetic particles complexes or targets directly into a vial for subsequent analysis. This allows one to capture, for example, the whole cell/live cell for subsequent analysis. Alternatively and in step 1160, the target capture system can continue to process the capture target to extract and isolate nucleic acids from the target. In this embodiment, the magnetic particle/target complexes are subject to a cell lysis/nucleic acid extraction step after the wash to obtain nucleic acids from the target cell. In step 1170, the resulting lysate is driven through an affinity column for capturing nucleic acids from the target cells. In step 1180, the affinity column is then eluted to drive purified nucleic acids into a vial. The vial with the isolated targets can then be removed from the target capture system by an operator for further analysis as in step 1190.

In addition to the methods of target capture described herein, the target capture system may be utilized to isolate pathogens using other methods, including the methods described in co-owned U.S. publication nos. 2011/0263833, 2011/0262925, 2011/0262932, 2011/0262933, 2011/0262926, and 2011/0262927.

Sample

In certain aspects, the target capture system is designed to isolate targets from biological samples including, for example, blood, serum, plasma, buffy coat, saliva, wound exudates, pus, lung and other respiratory aspirates, nasal aspirates and washes, sinus drainage, bronchial lavage fluids, sputum, medial and inner ear aspirates, cyst aspirates, cerebral spinal fluid, stool, diarrheal fluid, urine, tears, mammary secretions, ovarian contents, ascites fluid, mucous, gastric fluid, gastrointestinal contents, urethral discharge, synovial fluid, peritoneal fluid, meconium, vaginal fluid or discharge, amniotic fluid, penile discharge, or the like may be tested. In addition, fluidic samples formed from swabs or lavages representative of mucosal secretions and epithelia are acceptable, for example mucosal swabs of the throat, tonsils, gingival, nasal passages, vagina, urethra, rectum, lower colon, and eyes, as are homogenates, lysates and digests of tissue specimens of all sorts. In addition to biological samples, samples of water, industrial discharges, food products, milk, air filtrates, and so forth are suitable for use with the target capture system. These include food, environmental and industrial samples. In certain embodiments, fluidization of a generally solid sample may be required and is a process that can readily be accomplished off-cartridge.

In certain aspects, the target capture system can process macro-scale and micro-scale volumes of fluid. Macro-scale volumes are considered volumes 1 mL and above and micro-scale volumes are considered volumes below 1 mL. The cartridge of the target capture system may be designed to directly couple to a vessel containing the sample. Vessels suitable for use with the target capture system can be macrofluidic vessles or microfluidic vessels. For example, the target capture system can process a sample from a vessel that contains fluid having a volume of 1 mL to 100 mL, preferably around 5 mL to 20 mL. Alternatively, the target capture system can process a sample from a vessel that contains a fluid having a volume of 10 to 500 $\mu$L. In one aspect, the cartridge of the target capture system is designed to couple to a 10 mL collection tube, such as a blood collection tube (e.g., VACUTAINER (test tube specifically designed for venipuncture, commercially available from Becton, Dickinson and company), . However, the invention is not limited to coupling with a VACUTAINER (test tube specifically designed for venipuncture, commercially available from Becton, Dickinson and company) and can coupled with any enclosed vessel with a top that is configured to directly couple to an interface on the cartridge. The vessel and vessel/cartridge interface are described in more detail hereinafter.

Targets

The target capture system of the invention can be used to isolate any target from the sample. The target refers to the substance in the sample that will be captured and isolated by the target capture system. The target may be bacteria, fungi, a protein, a cell (such as a cancer cell, a white blood cell a virally infected cell, or a fetal cell circulating in maternal circulation), a virus, a nucleic acid (e.g., DNA or RNA), a receptor, a ligand, a hormone, a drug, a chemical substance, or any molecule known in the art.

In certain embodiments, the target is a pathogenic bacterium, fungus or both. Exemplary fungal species that may be captured by methods of the invention include species from the Candida genus, Aspergillus genus, and Cryptococcus genus. In particular embodiments, the specific fungal species include C. albicans, C. glabarata, C. parasilosis, C. tropicalis, C. krusei, Cryptococcus neoformans, Cryptococcus gattii. Exemplary bacterial species that may be captured and isolated by methods of the invention include species from the following genera Escherichia, Listeria, Clostridium, Enterobacteriaceae, Mycobacterium, Shigella, Borrelia, Campylobacter, Bacillus, Salmonella, Enterococcus, Pneumococcus, Streptococcus, Staphylococcus, Acinetobacter, Strenotrophomonas, Pseudomanos, Neisseria, Haemophilus, Clostridium, and Enterococcus. The method may also be used to detect the mecA gene, which is a bacterial gene associated with antibiotic resistance. In addition, the specific species of pathogen selected for capture and isolation may be based on a certain phenotypic characteristic. Devices and methods of the invention may be used to isolate only gram positive bacteria from a sample, or, alternatively, used to isolate only gram negative bacteria from a sample. In certain embodiments, devices and methods of the invention are used to isolate both gram positive and gram negative bacteria from a sample.

Magnetic Particles

The target capture system uses magnetic particles to isolate a target from the sample. Any type of magnetic particles can be used in conjunction with the target capture system. Production of magnetic particles and particles for use with the invention are known in the art. See for example Giaever (U.S. 3,970,518), Senyi et al. (U.S. 4,230,685), Dodin et al. (U.S. 4,677,055), Whitehead et al. (U.S. 4,695,393), Benjamin et al. (U.S. 5,695,946), Giaever (U.S. 4,018,886), Rembaum (U.S. 4,267,234), Molday (U.S. 4,452,773), Whitehead et al. (U.S. 4,554,088), Forrest (U.S. 4,659,678), Liberti et al. (U.S. 5,186,827), Own et al. (U.S. 4,795,698), and Liberti et al (WO 91/02811).

Magnetic particles generally fall into two broad categories. The first category includes particles that are permanently magnetizable, or ferromagnetic; and the second category includes particles that demonstrate bulk magnetic behavior only when subjected to a magnetic field. The latter are referred to as magnetically responsive particles. Materials displaying magnetically responsive behavior are sometimes described as superparamagnetic. However, materials exhibiting bulk ferromagnetic properties, e.g., magnetic iron oxide, may be characterized as superparamagnetic when provided in crystals of about 30 nm or less in diameter. Larger crystals of ferromagnetic materials, by contrast, retain permanent magnet characteristics after exposure to a magnetic field and tend to aggregate thereafter due to strong particle-particle interaction. In certain embodiments, the particles are superparamagnetic particles. In certain embodiments, the magnetic particle is an iron containing magnetic particle. In other embodiments, the magnetic particle includes iron oxide or iron platinum.

In certain embodiments, the magnetic particles include at least about 10% superparamagnetic particles by weight, at least about 20% superparamagnetic particles by weight, at least about 30% superparamagnetic particles by weight, at least about 40% superparamagnetic particles by weight, at least about 50% superparamagnetic particles by weight, at least about 60% superparamagnetic particles by weight, at least about 70% superparamagnetic particles by weight, at least about 80% superparamagnetic particles by weight, at least about 90% superparamagnetic particles by weight, at least about 95% superparamagnetic particles by weight, or at least about 99% superparamagnetic particles by weight. In a particular embodiment, the magnetic particles include at least about 70% superparamagnetic particles by weight.

In certain embodiments, the superparamagnetic particles are less than 100 nm in diameter. In other embodiments, the superparamagnetic particles are about 150 nm in diameter, are about 200 nm in diameter, are about 250 nm in diameter, are about 300 nm in diameter, are about 350 nm in diameter, are about 400 nm in diameter, are about 500 nm in diameter, or are about 1000 nm in diameter. In a particular embodiment, the superparamagnetic particles are from about 100 nm to about 250 nm in diameter.

In certain embodiments, the particles are particles (e.g., nanoparticles) that incorporate magnetic materials, or magnetic materials that have been functionalized, or other configurations as are known in the art. In certain embodiments, nanoparticles may be used that include a polymer material that incorporates magnetic material(s), such as nanometal material(s). When those nanometal material(s) or crystal(s), such as $Fe_3O_4$, are superparamagnetic, they may provide advantageous properties, such as being capable of being magnetized by an external magnetic field, and demagnetized when the external magnetic field has been removed. This may be advantageous for facilitating sample transport into and away from an area where the sample is being processed without undue particle aggregation.

One or more or many different nanometal(s) may be employed, such as $Fe_3O_4$, FePt, or Fe, in a core-shell configuration to provide stability, and/or various others as may be known in the art. In many applications, it may be advantageous to have a nanometal having as high a saturated moment per volume as possible, as this may maximize gradient related forces, and/or may enhance a signal associated with the presence of the particles. It may also be advantageous to have the volumetric loading in a particle be as high as possible, for the same or similar reason(s). In order to maximize the moment provided by a magnetizable nanometal, a certain saturation field may be provided. For example, for $Fe_3O_4$ superparamagnetic particles, this field may be on the order of about 0.3T.

The size of the nanometal containing particle may be optimized for a particular application, for example, maximizing moment loaded upon a target, maximizing the number of particles on a target with an acceptable detectability, maximizing desired force-induced motion, and/or maximizing the difference in attached moment between the labeled target and non-specifically bound targets or particle aggregates or individual particles. While maximizing is referenced by example above, other optimizations or alterations are contemplated, such as minimizing or otherwise desirably affecting conditions.

In an exemplary embodiment, a polymer particle containing 80 wt% $Fe_3O_4$ superparamagnetic particles, or for example, 90 wt% or higher superparamagnetic particles, is produced by encapsulating superparamagnetic particles with a polymer coating to produce a particle having a diameter of about 250 nm.

Binding Moiety

Magnetic particles for use with the target capture system have a target-specific binding moiety (capture moiety) that allows for the particles to specifically bind the target of interest in the sample. The capture moiety may be any molecule known in the art and will depend on the target to be captured and isolated. In certain embodiments, the target capture system utilizes compositions that include a plurality of sets of magnetic particles conjugated to capture moieties specific to different targets of interest. Compositions of magnetic particles for isolating pathogens in heterogeneous samples are described in more detail in co-owned U.S. publication no. 2011/0263833 and 2011/0262925, as well co-owned U.S. provisional app. no. 61/739616, filed Dec. 19, 2012.

The one or more different sets of magnetic particles may be coupled to one or more classes of capture moieties. Exemplary classes of capture moieties include oligonucleotides (including nucleic acid probes), proteins, ligands, lectins, antibodies, aptamers, bacteriophages, host innate immunity biomarkers (e.g., CD14), host defense peptides (e.g., defensins), bacteriocins (e.g., pyocins), and receptors. The capture moiety may be specific to a certain species within a genus of pathogen, or the capture moiety may be generally specific to several species within or the entire genus of pathogen. A class of capture moieties may include one or more different types of that class, e.g. an antibody specific to one pathogen and an antibody specific to another pathogen. In addition, one set of magnetic particles may be conjugated to a class of capture moieties that are different from a class of capture moieties conjugated to another set. For example, one set of magnetic particles may be conjugated to an antibody specific to a pathogen and another set of magnetic particles may be conjugated to a lectin specific to a different pathogen. The classes and types of capture moieties utilized will depend on the target to be captured and isolated.

In certain embodiments, each magnetic particle of a set is conjugated to at least two different sets of capture moieties specific to different pathogens. That is, one magnetic particle may have two or more capture moieties specific to different pathogens. The two or more capture moieties conjugated to a magnetic particle may be of the same class or different class. For example, a magnetic particle may have two or more antibodies specific to different pathogens. In another example, a magnetic particle may be conjugated to an antibody specific to a pathogen and a lectin specific to a different pathogen.

Methods of Antibody Production

In particular embodiments, the capture moiety is an antibody, such as an antibody that binds a particular pathogen. General methodologies for antibody production, including criteria to be considered when choosing an animal for the production of antisera, are described in Harlow et al. (Antibodies, Cold Spring Harbor Laboratory, pp. 93-117, 1988). For example, an animal of suitable size such as goats, dogs, sheep, mice, or camels are immunized by administration of an amount of immunogen, such the target bacteria, effective to produce an immune response. An exemplary protocol is as follows. The animal is injected with 100 milligrams of antigen resuspended in adjuvant, for example Freund's complete adjuvant, dependent on the size of the animal, followed three weeks later with a subcutaneous injection of 100 micrograms to 100 milligrams of immunogen with adjuvant dependent on the size of the animal, for example Freund's incomplete adjuvant. Additional subcutaneous or intraperitoneal injections every two weeks with adjuvant, for example Freund's incomplete adjuvant, are administered until a suitable titer of antibody in the animal's blood is achieved. Exemplary titers include a titer of at least about 1:5000 or a titer of 1:100,000 or more, i.e., the dilution having a detectable activity. The antibodies are purified, for example, by affinity purification on columns containing protein G resin or target-specific affinity resin.

Polyclonal antibodies, monoclonal antibodies, or both can be conjugated to magnetic particles in accordance with compositions and methods of the invention. Polyclonal antibodies are antibodies that are secreted by different B cell lineages, and are a collection of immunoglobulin molecules that react against a specific antigen, each identifying a different eptitope. Thus, polyclonal antibodies recognize multiple epitopes on any one antigen. Polyclonal antibodies are useful in identifying homologous pathogens, and allow for general isolation and capture of a range of species. In contrast, monoclonal antibodies are constructive from one cell line, and recognize only one epitope on an antigen. Monoclonal antibodies are more specific, and typically only bind to the epitope of the specific target cell (e.g. less likely to bind to a range of species).

The technique of in vitro immunization of human lymphocytes is used to generate monoclonal antibodies. Techniques for in vitro immunization of human lymphocytes are well known to those skilled in the art. See, e.g., Inai, et al., Histochemistry, 99(5):335 362, May 1993; Mulder, et al., Hum. Immunol., 36(3):186 192, 1993; Harada, et al., J. Oral Pathol. Med., 22(4):145 152, 1993; Stauber, et al., J. Immunol. Methods, 161(2):157 168, 1993; and Venkateswaran, et al., Hybridoma, 11(6) 729 739, 1992. These techniques can be used to produce antigen-reactive monoclonal antibodies, including antigen-specific IgG, and IgM monoclonal antibodies.

Any antibody or fragment thereof having affinity and specific for the bacteria of interest is within the scope of the invention provided herein. Immunomagnetic particles against Salmonella are provided in Vermunt et al. (J. Appl. Bact. 72:112, 1992). Immunomagnetic particles against Staphylococcus aureus are provided in Johne et al. (J. Clin. Microbiol. 27:1631, 1989). Immunomagnetic particles against Listeria are provided in Skjerve et al. (Appl. Env. Microbiol. 56:3478, 1990). Immunomagnetic particles against Escherichia coli are provided in Lund et al. (J. Clin. Microbiol. 29:2259, 1991).

In certain embodiments, the target-specific binding moiety is a lectin. Lectins are sugar-binding proteins that are highly specific for their sugar moieties. Exemplary lectins that may be used as target-specific binding moieties include Concanavalin (ConA), Wheat Germ Extract WGA). Lectins that specifically bind to bacteria and fungi are known, see, e.g. Stoddart, R. W., and B. M. Herbertson. "The use of fluorescein-labelled lectins in the detection and identification of fungi pathogenic for man: a preliminary study." Journal of medical microbiology 11.3 (1978): 315-324; and U.S. Patent No. 5,004,699. In addition, other lectins that have pathogen-binding properties are shown in Table 1 below.

| Lectin | Source | Carbohydrate Specificity |
|--------|--------|--------------------------|
| AMA | Arum maculatum (AMA) from 'lords and ladies' flower | Mannose |
| ASA | Allium sativum (ASA) from garlic | Mannose |
| Con-A | Canavalia ensiformis (Con-A) from jack bean | α-D-Mannose, α-D-Glucose, branched mannose |
| GS-II | Griffonia simplicoflia (GS-II) from shrub GS | Terminal α- or β- N-Acetylglucosamine |
| HHA | Hippeastrum hybrid (HHA) from amaryllis | Mannose (int and term residues) |
| IRA | Iris hybric (IRA) from Dutch Iris | N-Acetyl-D-Galactosamine |
| LEA | Lycopersicon esculentum (LEA) from tomato | β(1,4)-linked N-Acetylglucosamine |
| LPA | Limulus polyphemus (LPA) from horseshoe crab | Sialic Acid (N-Acetylneuraminic acid) |
| MIA | Mangidera indica (MIA) from mango | Exact specificity unknown |
| PAA | Perseau americana (PAA) from avocado | Exact specificity unknown |
| WGA | Triticum vulgaris (WGA) from Wheat Germ | (GlcNAc-β-(1,4)-GlcNAc)1-4>β-GlcNAc>Neu5Ac |
| WGA-S | Succinyl Triticum vulgare (WGA-S) from wheat germ | (GlcNAc-β-(1,4)-GlcNAc)1-4>β-GlcNAc>Neu5Ac |

Table 1: Lectins with Carbohydrate Specificity

Capture moieties suitable for use in methods of the invention may also include a nucleic acid ligand (aptamer). A nucleic acid ligand (aptamer) is a nucleic acid macromolecule (e.g., DNA or RNA) that binds tightly to a specific molecular target. Like all nucleic acids, a particular nucleic acid ligand may be described by a linear sequence of nucleotides (A, U, T, C and G), typically 15-40 nucleotides long. In solution, the chain of nucleotides forms intramolecular interactions that fold the molecule into a complex three-dimensional shape. The shape of the nucleic acid ligand allows it to bind tightly against the surface of its target molecule. In addition to exhibiting remarkable specificity, nucleic acid ligands generally bind their targets with very high affinity, e.g., the majority of anti-protein nucleic acid ligands have equilibrium dissociation constants in the picomolar to low nanomolar range.

Nucleic acid ligands are generally discovered using an in vitro selection process referred to as SELEX (Systematic Evolution of Ligands by EXponential enrichment). See for example Gold et al. (U.S. patent number 5,270,163). SELEX is an iterative process used to identify a nucleic acid ligand to a chosen molecular target from a large pool of nucleic acids. The process relies on standard molecular biological techniques, using multiple rounds of selection, partitioning, and amplification of nucleic acid ligands to resolve the nucleic acid ligands with the highest affinity for a target molecule.

In addition, the capture moiety may be a nucleic acid probe, typically an oligonucleotide that specifically binds to a target nucleic acid sequence. A probe is generally a single-stranded nucleic acid sequence complementary to some degree to a nucleic acid sequence sought to be detected ("target sequence"). A probe may be labeled with a reporter group moiety such as a radioisotope, a fluorescent or chemiluminescent moiety, or with an enzyme or other ligand which can be used for detection. Standard techniques are used for nucleic acid and peptide synthesis. These molecular biological techniques may be performed according to conventional methods in the art and various general references (see generally, Sambrook et al. MOLECULAR CLONING: A LABORATORY MANUAL, 2d ed. (1989) Cold Spring Harbor Laboratory Press, Cold Spring Harbor, N.Y.,). Synthesis of complementary DNA is described in, for example, Nature Methods 2, 151 - 152 (2005) doi:10.1038/nmeth0205-151. Background descriptions of the use of nucleic acid hybridization to detect particular nucleic acid sequences are given in Kohne, U.S. Patent No. 4,851,330 issued July 25, 1989, and by Hogan et al., International Patent Application No. PCT/US87/03009, entitled "Nucleic Acid Probes for Detection and/or Quantitation of Non-Viral Organisms". Hogan et al., supra, describe methods for determining the presence of a non-viral organism or a group of non-viral organisms in a sample (e.g., sputum, urine, blood and tissue sections, food, soil and water).

Reporter phages may also be used as a capture moiety. Reporter phages, such as bacteriophages, are typically genetically modified phages used to introduce a gene of interest into a host pathogen. The reporter gene incorporates detectable codes for a fluorescent or substrate dependent colorimetric marker into host pathogen, which allows for subsequent pathogen detection. Bacteriophages used for pathogen detection are described in detail in Singh, Amit, et al. "Bacteriophage based probes for pathogen detection." Analyst 137.15 (2012): 3405-3421.; Dover, Jason E., et al. "Recent advances in peptide probe-based biosensors for detection of infectious agents." Journal of microbiological methods 78.1 (2009): 10-19.

Attaching Binding Moieties

Methods for attaching the target-specific binding moiety to the magnetic particle are known in the art. Coating magnetic particles with antibodies is well known in the art, see for example Harlow et al. (Antibodies, Cold Spring Harbor Laboratory, 1988), Hunter et al. (Immunoassays for Clinical Chemistry, pp. 147-162, eds., Churchill Livingston, Edinborough, 1983), and Stanley (Essentials in Immunology and Serology, Delmar, pp. 152-153, 2002). Such methodology can easily be modified by one of skill in the art to bind other types of target-specific binding moieties to the magnetic particles. Certain types of magnetic particles coated with a functional moiety are commercially available from Sigma-Aldrich (St. Louis, MO).

Since each set of particles is conjugated with capture moieties having different specificities for different pathogens, compositions used in methods of the invention may be provided such that each set of capture moiety conjugated particles is present at a concentration designed for detection of a specific pathogen in the sample. In certain embodiments, all of the sets are provided at the same concentration. Alternatively, the sets are provided at different concentrations. For example, compositions may be designed such that sets that bind gram positive bacteria are added to the sample at a concentration of $2 \times 10^9$ particles per/ml, while sets that bind gram negative bacteria are added to the sample at a concentration of $4 \times 10^9$ particles per/ml. Compositions used with methods of the invention are not affected by antibody cross-reactivity. However, in certain embodiments, sets are specifically designed such that there is no cross-reactivity between different antibodies and different sets.

The sets of magnetic particles may be mixed together to isolate certain fungi, bacteria, or both. These sets can be mixed together to isolate for example, E. coli and Listeria; or E. coli, Listeria, and Clostridium; or Mycobacterium, Campylobacter, Bacillus, Salmonella, and Staphylococcus, etc. One set may be specific to a certain bacterium and another set may be specific to a certain fungus. Any combination of sets may be used and compositions of the invention will vary depending on the suspected pathogen or pathogens to be isolated. In certain embodiments, compositions include two, three, four, five... 10, 20, etc. different sets of magnetic particles conjugated to different pathogens.

In preferred embodiments, sets of magnetic particles conjugated to capture moieties that are specific to different targets within multi-plex detection panel. For example, sets of modified magnetic particles may be chosen to isolate two or more certain pathogens. The two or more certain pathogens chosen may be causal of similar symptoms (e.g. digestive abnormalities), commonly found in the type of body fluid (e.g. stool), or pathogens common to a certain area (e.g. hospital setting). The following tables show exemplary panels of fungal (Table 2) targets and bacterial (Table 3) targets. "Sp" means a target species within a genus, and "Sp" means a target

TABLE 2: Fungal Panels-Compositions of the invention will include capture moieties bound to magnetic particles specific to each of the targets in the panel.

| Fungi Assay | Target 1 | Target 2 | Target 3 | Target 4 | Target 5 |
|---|---|---|---|---|---|
| Panel A | Species of Candida genus | C. albicans | C. glabrata | C. parapsilosis (or C. tropicalis) | N/A |
| Panel B | Species of Candida genus | C. albicans | C. glabrata | C. parapsilosis/C. tropicalis | C. krusei |
| Panel C | Species of Candida genus | C. albicans | C. glabrata/C. krusei | C. parapsilosis/C. tropicalis | Cryptococcus spp. |
| Panel D | Candida genus | C. albicans/C. parapsilosis/C. tropicalis | C. glabrata/C. krusei | Aspergillus spp. | Cryptococcus spp. |
| Panel E | Cryptococcus neoformans | C. albicans/C. parapsilosis/C. tropicalis | C. glabrata/C. krusei | Aspergillus spp. | Cryptococcus gattii |

TABLE 3: Bacteria Panels-Compositions of the invention will include capture moieties bound to magnetic particles specific to each of the targets in the panel.

| Bacteri a Assay | Target 1 | Target 2 | Target 3 | Target 4 | Target 5 |
|---|---|---|---|---|---|
| Panel A-Gram Positive Panel | S. aureus | CoNS | mecA | Streptococcus sp./Enterococco cus sp. | N/A |
| Panel B-Gram Negative Panel | Enterobacteriaceae | Pseudomonas aeruginosa | Acinetobacter sp. | Stenotrophomonas maltophilia (or other GNR such as Neisseria, Haemophilus) | N/A |
| Panel C | Staphylococcus sp. | CoNS | Enterobacteriaceae | Pseudomonas aeruginosa | N/A |
| Panel D | Staphylococcus sp. | Enterococcus sp./ Streptococcus sp. | E. coli | Pseudomonas aeruginosa | N/A |
| Panel E | Staphylococcus aureus | CoNS | Enterococcus sp./ Streptococcus sp. | Enterobacteriaceae | N/A |
| Panel F | Staphylococcus sp. | Enterococcus sp./ Streptococcus sp. | Enterobacteriaceae | Candida spp. | N/A |
| Panel G-Gram Positive Panel | S. aureus | CoNS | mecA | Streptococcus sp. | Enterococcocus sp. |
| Panel H-Gram Negative Panel | Enterobacteriaceae | Pseudomonas aeruginosa | Acinetobacter sp. | Stenotrophomonas maltophilia | Neisseria meningitidis |
| Panel I | Staphylococcus sp. | Enterococcus sp. | Streptococcus sp. | Enterobacteriaceae | Pseudomonas aeruginosa |
| Panel J | Staphylococcus sp. | Enterococcus sp. | Streptococcus sp. | E. coli | Pseudomonas aeruginosa |
| Panel K | Staphylococcus aureus | CoNS | Enterococcus sp./ Streptococcus sp. | Enterobacteriaceae | Pseudomonas aeruginosa |
| Panel L | Staphylococcus sp. | Enterococcus sp./ Streptococcus sp. | Enterobacteriaceae | Pseudomonas aeruginosa | Acinetobacter sp. |

In certain embodiments, compositions of the invention include at least one set magnetic particles conjugated to a capture moiety specific to an internal control (IC). For example, an IC detectable marker may be placed into a clinical sample suspected of containing a pathogen. For detection of the pathogen, a plurality of sets of magnetic particles are introduced to that clinical sample, in which at least one set is conjugated to a capture moiety designed to bind the IC detectable marker and one or more other sets are conjugated to capture moieties designed to bind to the suspected pathogens. When the assay is conducted to capture and isolate the pathogen, the presence or absence of the IC detectable marker indicates whether the assay properly separated the marker from the sample. That is, the introduction of a detectable marker allows one to determine whether the sets of magnetic particles conjugated to capture moieties are properly capturing or isolating the target pathogens that are present within the fluid. This is important to distinguish between a failed assay (i.e. failure to identify a target pathogen present in the sample) and a positive assay (i.e. positively determining the absence of a target pathogen). The presence of the marker indicates that the assay worked properly; and thus any detection of pathogen (or the absence of the pathogen) is accurate and not the result of a failed assay. Use of IC detectable markers is described in more detail in co-owned U.S. provisional application No. 61/739,577, filed December 18, 2012. The panels listed in Tables 2 and 3 above may include the addition of an IC detectable marker inserted into the sample, and the sets of magnetic particles would include sets conjugated to capture moieties specific to targets listed in the panel as well as the internal control.

Methods that utilize the internal control involve obtaining a sample suspected of containing a pathogen and introducing a detectable maker into the sample. An assay is conducted to detect the suspected pathogen and the detectable marker in the sample using a plurality of sets of magnetic particles (as described herein). Members of at least one set of magnetic particles are conjugated to binding entity specific to a pathogen, and members of at least one set of magnetic particles are conjugated to a binding entity specific to the detectable marker. After the conducting step, the presence or absence of the marker in the sample is determined. Based on the presence or absence of the detectable marker, a determination is made about the presence or absence of targets in the sample. The presence of the marker indicates that the assay worked properly; and thus any detection of pathogen (or the absence of pathogen) is accurate and not the result of a failed assay.

Any detectable marker may be used for an internal control. In certain embodiments, the IC detectable marker may be a microbe, including a viable or nonviable microbe. In addition, the IC detectable marker can be sufficiently similar to the target such that the assay performs functionally in the same manner for the detectable marker and the target. The detectable marker may be labeled or otherwise modified to allow for their differentiation from targets originally present in the sample. For example, but not by way of limitation, the detectable marker can be genetically modified so as to express a fluorescent protein, or alternatively, the detectable marker could be pre-stained with a persistent stain to allow their differentiation from microbes that are originally present in the fluid composition. In addition, the detectable marker may be chosen based a chromogen dye specific reaction to the presence of the detectable marker.

Reagents and Buffers

The target capture system can employ reagents and buffers for carrying the processes of the target capture system. In certain aspects, the cartridge of the target capture system includes reservoirs for storing the reagents and buffers. The cartridge also includes components such as channels, valves, etc. that provide a means for delivering the reagent and buffers within the cartridge. Accordingly, each of the reagents, buffers, and fluids described below can be stored within the cartridge and delivered into the sample to carry out the various processes of the target capture system.

In certain embodiments, a buffer solution is added to the sample along with the magnetic particles to facilitate binding of the particles to targets within the sample. The buffer can be stored within a reagent reservoir within the cartridge and introduced to the sample during processing. An exemplary buffer includes Tris(hydroximethyl)-aminomethane hydrochloride at a concentration of about 75mM. It has been found that the buffer composition, mixing parameters (speed, type of mixing, such as rotation, shaking etc., and temperature) influence binding. It is important to maintain osmolality of the final solution (e.g., blood + buffer) to maintain high label efficiency. In certain embodiments, buffers used in devices and methods of the invention are designed to prevent lysis of blood cells, facilitate efficient binding of targets with magnetic beads and to reduce formation of bead aggregates. It has been found that the buffer solution containing 300 mM NaCl, 75 mM Tris-HCl pH 8.0 and 0.1% Tween 20 meets these design goals.

Without being limited by any particular theory or mechanism of action, it is believed that sodium chloride is mainly responsible for maintaining osmolality of the solution and for the reduction of non-specific binding of magnetic bead through ionic interaction. Tris(hydroximethyl)-aminomethane hydrochloride is a well-established buffer compound frequently used in biology to maintain pH of a solution. It has been found that 75 mM concentration is beneficial and sufficient for high binding efficiency. Likewise, Tween 20 is widely used as a mild detergent to decrease nonspecific attachment due to hydrophobic interactions. Various assays use Tween 20 at concentrations ranging from 0.01% to 1%. The 0.1% concentration appears to be optimal for the efficient labeling of bacteria, while maintaining blood cells intact.

Additionally, devices and methods of the invention employ wash solutions to reduce particle aggregation and remove unwanted sample, non-specific target entities, and buffer. Exemplary solutions include heparin, Tris-HCl, Tris-borate-EDTA (TBE), Tris-acetate-EDTA (TAE), Tris-cacodylate, HEPES (4-(2-hydroxyethyl)-1-piperazineethanesulphonic acid), PBS (phosphate buffered saline), PIPES (piperazine-N,N'-bis(2-ethanesulfonic acid), MES (2-N-morpholino)ethanesulfonic acid), Tricine (N-(Tri(hydroximethyl)methyl)glycine), and similar buffering agents. In particular embodiments, the wash solution includes heparin. For embodiments in which the body fluid sample is blood, the heparin also reduces probability of clotting of blood components after magnetic capture. These wash solutions can be contained in one or more reagent reservoirs of the cartridge and are typically introduced into a magnetic trap flow through chamber during separation of the magnetic particles.

In certain embodiments, the target capture system includes introducing a solution to invoke cell lysis in capture target cells in order to release the nucleic acid. The solution can be any suitable lysis fluid/buffer capable of lysing the cells and/or particles of interest in the fluid sample. An example of a suitable lysis buffer is 100 mM Tris/HCl, 8 M GuSCN (pH 6.4).

In addition, the target capture system may also utilize an elutant fluid to elute purified nucleic acids from a nucleic acid extraction column. The eluant fluid can be any fluid suitable for eluting purified nucleic acids from the nucleic acid extraction unit. Examples of suitable elution fluids include water and 10 mM Tris/HCl, 1 mM EDTA Na.sub.2 (pH 8).

In addition, a reagent can be used that disrupts the interaction between the particle and the target cell, e.g. disrupts an antibody-antigen reaction. This reagent may be used after capture of the target/magnetic particle complexes to separate isolated whole target cells from the magnetic particles.

Cartridge

The target capture system of the invention includes a cartridge that is a single structure having one or more components (such as reagent reservoirs, magnetic traps, storage reservoirs, flow chambers, etc.) that are formed within the cartridge. These components can be connected via channels formed within the system. As such, there is no need for external tubing or other external attachments to connect the components of the cartridge.

A significant advantage of certain embodiments is that the cartridge includes both macrofluidic and microfluidic components and can process macrofluidic and microfluidic volumes of fluids to isolate a target. This aspect of the invention accounts for the fact that a minute amount of targets (such as pathogens) may be present in a sample having a macrofluidic volume which necessitates processing the entire macrofluidic volume in order to increase the likelihood that the target will be isolated. To isolate targets in a microfluidic device, the entire macrofluidic volume of sample would have to be transferred slowly or in a piecemeal fashion (e.g. via pipetting) into a microfluidic device at microfluidic rate, which undesirably takes a long amount of time and risks losing the target analyte of interest during the transfer. In certain aspects, the cartridge is designed to consolidate a sample of macrofluidic volume into a concentrated microfluidic volume of fluid that contains target cells of interest. The concentrated microfluidic volume is then processed at the microfluidic level.

Generally, microfluidics relates to small sample volumes and small channel pathways. For example, microfluidic volumes are normally below 1 mL, or on the microliter ($\mu$L) scale or smaller, for example, nL (nanoliters) and pL (picoliters). As used herein, microfluidic volumes relate to volumes less than 1 mL. In addition, microfluidics relates to small channel pathways on the micrometer scale. As used herein, microfluidic channels within systems of the invention refer to channels that have channel heights and/or widths equal to or less than 500 $\mu$m. See "Microfluidics and Nanofluidics: Theory and Selected Applications," Kleinstruer, C., John Wiley & Sons, 2013, which is incorporated by reference. The channel height or width is defined as the height or width of the path that the sample volume must pass through within the cartridge. Comparatively, macrofluidics volumes relate to volumes greater than the microliter ($\mu$L) scale, for example sample volumes on the milliliter (mL) scale. As used herein, macrofluidic volumes are volumes of 1 mL or greater. Macrofluidic channels within systems of the invention are channels having channel heights and/or widths of greater than 500 $\mu$m.

Other macrofluidic components are chambers, reservoirs, traps, mixers, etc. Such macrofluidic components are dimensioned to hold 1 mL or more of fluid. For example, the individual volume can range without limitation from about 10 to about 50 mL. Other microfluidic components are chambers, reservoirs, traps, mixers, etc. Such microfluidic components are dimensioned to hold less than 1 mL of fluid. For example, the individual volumes can range without limitation from about 1 $\mu$L to about 500 $\mu$L.

The cartridge includes channels to facilitate transportation of substances and fluids through into, within, and out of the cartridge. The channel generally will include characteristics that facilitate control over fluid transport, e.g., structural characteristics (an elongated indentation) and/or physical or chemical characteristics (e.g. lined with a solution or substance that prevents or reduces adherence aggregation of sample/particulates) and/or other characteristics that can exert a force (e.g., a containing force) on a sample or fluid. The channels can be independent, connected, and/or networked between components of the cartridge. Some (or all) of the channels may be of a particular size or less, for example, having a dimension perpendicular to the flow of fluid to achieve a desired fluid flow rate out of one component and into another. The channels can be designed to transfer macro and micro scales of fluid.

The channels of the cartridge can connect to and interconnect the components of the cartridge. The cartridge can include one or more of the following components: through holes, slides, foil caps, alignment features, liquid and lyophilized reagent storage chambers, reagent release chambers, pumps, metering chambers, lyophilized cake reconstitution chambers, ultrasonic chambers, joining and mixing chambers, mixing elements such as a mixing paddle and other mixing gear, membrane regions, filtration regions, venting elements, heating elements, magnetic traps/chambers, reaction chambers, waste chambers, membrane regions, thermal transfer regions, anodes, cathodes, and detection regions, drives, plugs, piercing blades, valve lines, valve structures, assembly features such as o-rings, instrument interface regions, cartridge/vessel interfaces, one or more needles associated with the sample interface, optical windows, thermal windows, and detection regions. These components can have macro- or micro-volumes.

The cartridge includes at least one inlet for introducing sample into the cartridge and at least one inlet for allowing the instrument to introduce air pressure, e.g., to drive fluid flow, or to introduce fluids into the cartridge. The cartridge further includes at least one outlet to deliver a final product to the operator, e.g. a captured target or nucleic acids of a captured target into a removable vial for further analysis. In preferred embodiments, the inlet and outlet are associated with the cartridge/vessel interface of the invention described in detail hereinafter.

In one embodiment, the cartridge further includes sensing elements to determine the stage of the processes performed within the cartridge. The sensing elements can be used to gauge the flow within the cartridge and the timing for when certain subsystems of the instrument interact with the cartridge. The sensing elements include, but are not limited to, optical sensors (e.g. for monitoring the stage of processing within the chamber), timers (e.g., for determining how long a sample is in a mixing chamber or in a reaction chamber); air displacement sensors (e.g. for determining the volume of fluid within one or more chambers); temperature sensors (e.g. for determining the temperature of a reaction), bubble sensor (e.g. for detecting air and/or volume of fluid within chambers and fluid flow; pressure sensors for determining, e.g., rate of fluid flow.

In certain aspects, fluids and substances are driven into, within, and out the cartridge via one or more drive mechanisms. The drive mechanisms can be located on the cartridge itself or located on an instrument in combination with the cartridge. The drive mechanisms provide a means for fluid control within the cartridge and allows for transport of fluid and substances within the cartridge. In addition, the drive mechanisms provide a means for transferring fluids and substances between the cartridge and the vessel at the cartridge/vessel interface. In one embodiment, the drive mechanism is a part of the instrument and is operably associated with the cartridge at one or more cartridge/instrument interface. The cartridge can include a filter at the cartridge/instrument interface to prevent unwanted particles from entering the cartridge from the drive mechanism or instrument. The filter also prevents sample and other fluids from exiting the cartridge at the cartridge/instrument interface. The drive mechanisms of the instrument are discussed in more detail hereinafter.

The cartridge (whether including macrofluidic components, microfluidic components, or both) can be fabricated using a variety of methods, including without limitation, computer numerical control (CNC) techniques, traditional lithographic techniques, soft lithography, laminate technologies, hot embossing patterns, die cutting, polymer molding, combinations thereof, etc. The cartridge can be fabricated from any etchable, machinable or moldable substrate. The term machining as used herein includes, without limitation printing, stamping cutting and laser ablating.

Suitable materials for the cartridge include but are not limited to non-elastomeric polymers, elastomeric polymers, fiberglass, Teflon, polystyrene and co-polymers of styrene and other materials, polypropylene, polyethylene, polybutylene, polycarbonate, polyurethanes, TEFLON (polytetrafluoroethylene, commercially available by the DuPont company), and derivatives thereof. Preferably, the cartridge and the cartridge components are formed primarily from plastic. Plastics are cost-efficient and allow for the cartridge to be economically manufactured at a large scale. As such, the cartridge can be designed as a single use, disposable cartridge.

There are some components of the cartridge that are not plastic, and these components can be formed from, for example, metals, silicon, quartz, and glass. These components include but are not limited to surfaces, glass ampoules, filters, assembly materials (such as screws and other fasteners), electrode pins, membrane, affinity columns, and collection vials.

The cartridge can also include thin film layers that form structures/interfaces (such as walls and valves) on the cartridge, interfaces between components within the cartridge, and interfaces between the cartridge and the instrument. In one aspect, the thin film layers are for bonding fabricated components together (such as CNC components and lithographic components), sealing components together, providing conduits between components, transferring stimulation between components (e.g. capable of transferring physical or mechanical stimulation from an assembly/system on the instrument to a chamber in the cartridge), supporting elements, covering the channel, functioning as a cap and/or frangible seal for reservoirs or chambers, and performing as a valve. The thin film can be elastomeric or non-elastomeric material. In certain aspects, the thin film is a polymer or thermoplastic polymer. Exemplary polymers or thermoplastic polymers can include, but are not limited to, polymers selected from the group consisting of polymethyl methacrylate (PMMA), polycarbonate (PC), polyvinylacetate (PVAc), polystyrene (PS), polypropylene, polyethylene, polymethyl methacrylate, poly(amides), poly(butylene), poly(pentadiene), polyvinyl chloride, polycarbonate, polybutylene terephthalate, polysulfone, polyimide, cellulose, cellulose acetate, ethylene-propylene copolymer, ethylene-butene-propylene terpolymer, polyoxazoline, polyethylene oxide, polypropylene oxide, polyvinylpyrrolidone, and combinations thereof. In addition, thin film can be an elastomer, polymer blend and copolymer selected from the group consisting of poly-dimethylsiloxane (PDMS), poly(isoprene), poly(butadiene), and combinations thereof. In some embodiments, the thin film includes rubber (including silicone) alone or in combination with a polymer.

In a preferred embodiment, the cartridge is pre-assembled prior to shipment to distributors/customers. The pre-assembled cartridge may also include one or more of reagents, capture particles (including magnetic particles), lysing beads, water, and other substances/fluids pre-loaded into one or more chambers or reservoirs formed within the cartridge. The pre-assembled cartridge may be partially pre-loaded, e.g. loaded with only a portion of the components necessary to isolate a target. If pre-assembled, the cartridge can include reagents and magnetic particles specific certain isolation assays and/or specific to certain target analytes. In addition, the cartridge can include magnetic particles with binding moieties specific to a plurality of different targets to provide for isolation of a target when the suspected target is not known. See co-pending and co-assigned U.S. application serial number 13/091506 that describes compositions for isolating a target sample from a heterogeneous sample. It is also contemplated that the cartridge is partially-assembled prior to shipment to distributors/customers to allow the individual customer to load the cartridge with reagents, beads, etc. that are tailored to the analysis/identification needs of the customer.

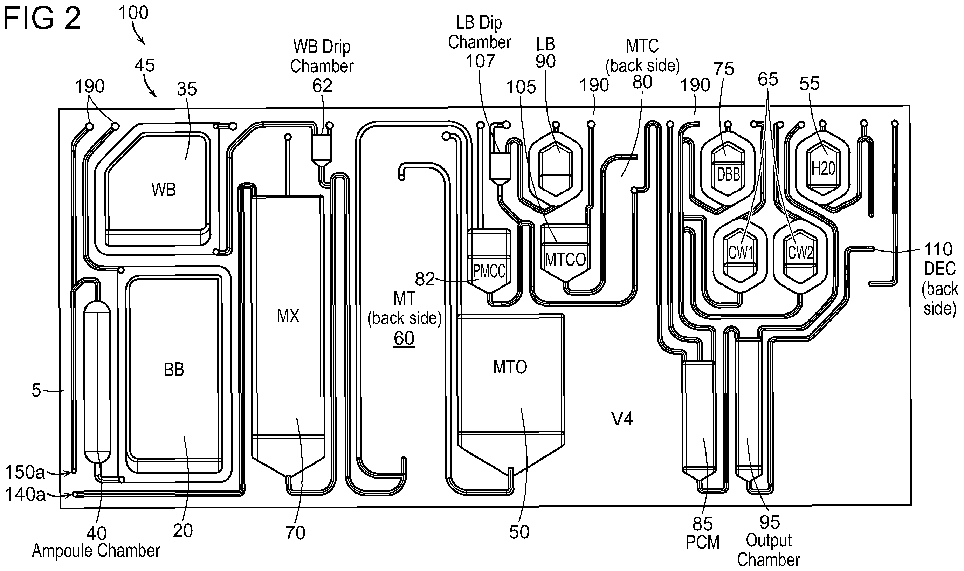

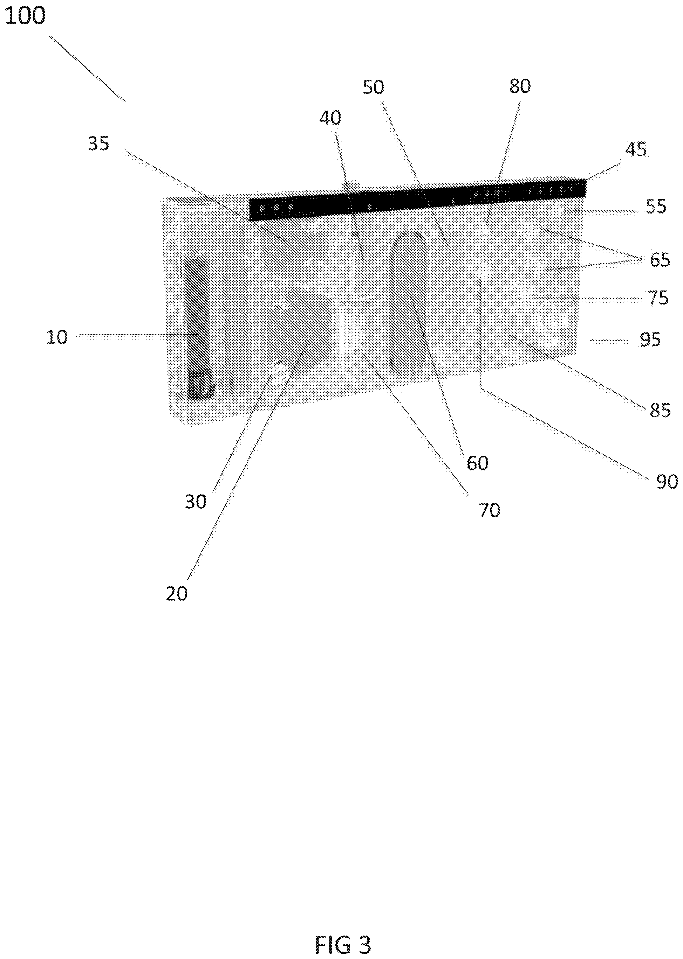

FIG. 2 depicts a schematic overview of a cartridge 100 according to the invention. FIG. 3 depicts the various chambers as described in the schematic overview of the cartridge in FIG. 2. The cartridge 100 as depicted in FIG. 2 includes both macrofluidic and microfluidic components. However, it is understood that the cartridge could be constructed with only macrofluidic or microfluidic components. Channels, valves, and other structures are included in the cartridge to facilitate and control communication between cartridge components.

As shown in FIG. 2, the cartridge 100 includes inlet port 140a and outlet port 150a. In inlet port 140a can introduce sample or other fluids into the cartridge and outlet port 150a allows for transfer of fluid/substances/pressure out of the cartridge and into a vessel 10 containing sample. Preferably, the inlet port 140a and outlet port 150a corresponds to the cartridge/vessel interface, which includes inlet member 140 and outlet member 150 (not shown). Inlet members 140 and outlet member 150 extend to into the vessel 10 containing sample coupled to the cartridge. The outlet port 150a allows fluids/substances to be transferred out of the cartridge 100 and into a sample vessel 10. The inlet port allows transfer of sample/fluids/substances from the vessel 10 to the cartridge 100. The inlet port 140a and outlet port 150a are associated with channels within the cartridge that direct fluid flow and can also be associated with one or more valves. The valves can be used to start, stop, increase, and/or decrease fluid into the inlet port 140a and out of the outlet port 150a.

Along the top of the cartridge 100 are several drive ports 190 at the instrument interface 45. The drive ports 190 connect various features on the cartridge 100 to the instrument's 200 drive mechanism. As shown in FIG. 2, a port 190 is in communication with a particle buffer reservoir 20. The particle buffer reservoir 20 can contain a buffer that promotes binding of particles and targets. The particle buffer reservoir 20 is in communication with a particle chamber 40 containing a plurality of magnetic particles. The plurality of magnetic particles may be used to isolated one or more targets of interest. As discussed above, the plurality of magnetic particles may include different sets of magnetic particles in which each set is conjugated to capture moiety specific to a different target of interest (e.g. for multiplex capture of targets). The particle chamber 40 is typically a glass ampoule. Glass ampoules are ideal because they are able to keep the magnetic particles in a constant environment (e.g. of a certain air pressure and/or humidity). The plurality of particles within the particle chamber 40 can include moieties specific to a target. The particle buffer reservoir 20 and the particle chamber 40 are in communication with the outlet port 150a. The particles and buffer can be transported by the drive mechanism out of the outlet port 150a and directly into the vessel 10 containing a sample.

The inlet port 140a is connected to a channel that directs the contents of the vessel (sample/particle/buffer) into a mixing chamber 170. The mixing chamber 70 can be used to incubate and agitate the sample/particle/buffer mixture. The mixing chamber 70 can include a mixing paddle disposed therein. A portion of the mixing paddle is operably associated with a mechanical drive on the instrument that causes the mixing paddle to move or rotate within the mixing chamber 70.

The mixing chamber 170 is in communication with a first magnetic trap 60 and a magnetic trap overflow 50. The first magnetic trap 60, as shown in FIG. 2, is a flow through chamber in which fluid can be driven into and out of the first magnetic trap 60. The first magnetic trap 60 can be a flow chamber having a planar or orthogonal inlet. In one embodiment, the first magnetic trap 60 has an orthogonal inlet because this inlet shape creates a uniform flow profile through the magnetic trap. The magnetic trap 60 is configured to engage with a magnet assembly of the instrument 200 to separate particles from the sample/buffer via application of a magnetic field. The magnetic assembly can include an array of bar magnets which are perpendicular to a flow of fluid within the first magnetic trap 60 and have a magnetic field sufficient to force the magnetic particles against a surface of the magnetic trap, thereby separating the magnetic particles. The surface of the first magnetic trap 60 may include binding moieties specific to the magnetic particles that, in addition to the magnetic field, aid in separating magnetic particles from the sample/buffer solution. The magnetic assembly that interacts with the first magnetic trap 60 may also include a swiper magnet that generates a magnetic field to release the magnets from the surface of the magnetic trap 60 after separation. The magnetic trap overflow chamber 50 is for receiving and transferring buffer/particle/sample solution to and from the first magnetic trap 60.

As further shown in FIG. 2, the first magnetic trap 60 is in communication with a wash buffer reservoir 35. A wash buffer drip chamber 62 between the first magnet trap 60 and the wash buffer reservoir 35 can control flow of buffer into the first magnetic trap 60. The wash buffer reservoir 35 may contain buffer to rinse extra sample/buffer from magnetic particles isolated in the first magnetic trap 60. In addition, the wash buffer can be used to transfer the separated magnetic particles to the second magnetic trap 70.

Optionally, a pre-magnetic trap chamber 82 is between the first magnetic trap 60 and the second magnetic trap 80 as shown in FIG. 2. The pre-magnetic trap chamber 82 can be used to control flow of particles and wash buffer from the first magnetic trap 60 to the second magnetic trap 80. In certain aspects, the first magnetic trap 60 is macrofluidic and the second magnetic trap 80 is microfluidic. In such aspect, the pre-magnetic trap chamber 82 acts an intermediate component to aid in transitioning from macro-to-micro by controlling fluid flow from the first magnetic trap 60 and the second magnetic trap 80.

The second magnetic trap 80 is configured to engage with a magnet of the instrument to further separate any remaining sample/buffer from the magnetic particles. The second magnetic trap 80 is a flow through chamber in communication with a second magnetic trap overflow chamber 105. The second magnetic trap overflow chamber 105 is used to store unwanted buffer/sample (waste) from the second magnetic trap 80. The second magnetic trap 80 is in communication with a lysis buffer reservoir 90 and optionally, a lysis buffer drip chamber 107 to control flow of lysis buffer into the second magnetic trap 80. The second magnetic trap 80 is also configured to engage with a sonication device of the instrument. In one embodiment, a wall of the second magnetic trap 80 that interfaces with the instrument has a certain thickness, such as 125 $\mu$M, that allows vibrations of the sonication device to invoke cell lysis on targets within the second magnetic trap. The wall interfacing the sonication device can be a Mylar film. The second magnetic trap 80 can optionally include binding moieties specific to the magnetic particles to assist in isolating the magnetic particles.

The second magnetic trap 80 is also in communication with a pre-column mixer 85, which receives the lysate from the second magnetic trap 80. The pre-column mixer 85 is in communication with to a nucleic acid binding buffer reservoir and in communication with a nucleic acid extraction column 110. An output chamber 95 can be included between the pre-column mixer 85 and the nucleic acid extraction member 110. Any nucleic acid extraction member 110 that retains extracted nucleic acid while allowing the other fluids such as lysis debris to flow through the member is suitable for use in the invention. The nucleic acid extraction member 110 can be a filter or a column, such as an affinity column. Examples of nucleic acid extraction members are described in, for example, United States Patent Publication No. 2011/0300609.

One or more column wash reservoirs 65 are connected to the nucleic acid extraction member 110 to direct unwanted sample/buffer/ect. from the column 110 to a waste reservoir. An elution reservoir 55 contains a buffer or fluid that is capable of eluting nucleic acids disposed within the nucleic acid extraction member. The fluid, such as water, is flushed from the elution reservoir 55 through extraction member 110 to elute purified nucleic acids into a collection vial (not shown).

In one embodiment, the particle chamber 40, the wash buffer 35, the mixing chamber 70, the first magnetic trap 60 and the magnetic trap overflow 50 of the cartridge 100 are all macrofluidic components designed to process a macrofluidic volume of fluid. Because these components are macrofluidic, the entire sample can be subject to the incubation, agitation, and the first magnetic separation step. After the magnetic particles are isolated in the first magnetic trap 60, a wash buffer flows through the first magnetic trap 60 to transport the separated particles to the second magnetic trap 80. The cartridge 100 components after the first magnetic trap 60 are microfluidic, including the second magnetic trap 60, magnetic trap overflow 105, pre-column mixer 80. The second magnetic trap 80 isolates substantially the entire quantity of magnetic particles within a microfluidic volume of fluid from the macrofluidic volume of fluid. The rate of fluid flow between the first and second magnetic traps can be adjusted to allow for the second magnetic trap 80 to isolate all of the magnetic particles. Thus, the macrofluidic volume of sample is concentrated into a microfluidic volume of concentrated clinically relevant sample. The concentrated microfluidic volume of fluid allows for more efficient nucleic acid extraction.

Cartridge and Vessel Interface

For isolation and detection assays conducted on cartridges or chips (whether microfluidic or macrofluidic), it is important to transfer the entire obtained sample from a collection device into the cartridge to increase the efficiency of isolation or detection. Especially in situations where there is little sample, which is often the case in forensic analysis, or when there is a small concentration of targets per mL of sample (e.g. 1 CFU/mL), which is often the case for pathogenic detection. Cartridges of the invention include a cartridge/vessel interface designed to maximize the amount of sample transferred into the vessel and the amount of sample subject to the cartridge processes to avoid loss of clinically relevant within a sample collection device during sample transfer. It is understood that the cartridge/vessel interface can be included on the cartridge of the target capture system and any other cartridge for processing a sample.

The cartridge/vessel interface may include one or more input and/or output members that enter a vessel containing sample to maximize the amount of sample that is transferred from the vessel containing sample into the cartridge for processing. In one embodiment, the cartridge/sample interface includes an inlet member and an outlet member to facilitate communication of fluids and substances out of the cartridge and into the sample vessel and to facilitate communication of fluids and substances (including the sample) out of the vessel and into the cartridge. The outlet member also provides for 1) introducing air to force the sample into the cartridge via the inlet port and/or the inlet member to maximize drainage; 2) introducing a fluid into the vessel to rinse the vessel container to transfer any remaining sample in the vessel into the cartridge; and 3) introducing fluids/substances necessary for cartridge processes directly to the entire sample to ensure the entirety of the sample engages with those fluids/substances. The input member provides for transferring the vessel contents into the cartridge for processing.

In certain embodiments, the fluid is introduced into the vessel at the same time the vessel contents (including sample and/or fluid) is transferred into the cartridge. Alternatively, the sample is at least partially transferred from the vessel into the cartridge prior to introducing the fluid from the cartridge into the vessel.

In one embodiment, both the inlet member and outlet member define a lumen and include a penetrating tip. For example, the inlet member and the outlet member can be hollow pins or needles. The inlet member and outlet member correspond with inlet and outlet ports on the cartridge. The input member and output member are designed to penetrate the vessel containing the sample to place the vessel (and thus the sample) in communication with the cartridge. The communication between the vessel and the cartridge through the input members and output members may be fluidic, pneumatic, or both. The input and output members can also act to couple the vessel to the cartridge and maintain the position of the vessel on the interface.

In certain embodiments, the input and output members are in communication with a drive mechanism. The drive mechanism can be a part of the cartridge itself or located on an instrument for use with the cartridge. The drive mechanism can apply air pressure or a vacuum force to facilitate transportation between the vessel and cartridge. For example, the drive mechanism can apply air pressure through a channel of the cartridge, out of the output member, and into the vessel to force the vessel contents to drain through the input member. In addition, the drive mechanism can apply a vacuum force to the input member to force the sample to drain into member.

The input member is in communication with one or more components of the cartridge (e.g. a mixing chamber, magnetic trap, storage reservoir, reagent reservoirs, ect.) that process fluids delivered from the vessel into the cartridge. The input member allows for fluids to transfer out of the vessel and into the cartridge for processing.

The output member is in communication with one or more components of the cartridge (e.g. storage reservoir, reagent reservoir, magnetic trap, etc.) to allow delivery of fluids, substances, and/or gases from the cartridge into the vessel container. In one embodiment, fluid from a reagent reservoir is driven through the output member and into the vessel to rinse sides of the vessel. The fluid may contain one or more substances. In one aspect, the fluid includes capture particles having binding moieties specific to one or more suspected targets within the sample. The fluid can be any fluid that does not interfere with the processes of the cartridge. In another embodiment, the fluid is an essential element of the cartridge processes. For example, the fluid can be a buffer that promotes a reaction within the sample, such as promoting target capture. In addition, fluids, substances, and/or gases may be subject to a reaction/process within the cartridge prior to being delivered into the vessel. For example, a buffer may be heated in the cartridge prior to introducing the buffer into the vessel.

In certain embodiments, one or more input members and one or more output members are inserted into the vessel to place the vessel in communication with the cartridge. This allows, for example, the vessel contents to be directed through one or more input member into one or more different channels in the cartridge for processing. In addition, one or more output members may be for delivering different fluids or reagents into the vessel.

The vessel for coupling to the cartridge interface can be an open or closed container. In one embodiment, the vessel is a collection tube, such as a vacutainer. Ideally, the vessel is enclosed, such as a collection tube enclosed by a stopper or a plug. The stopper or plug can be rubber, silicone, or polymeric material. For coupling the vessel to the sample, the vessel or the vessel plug is pressed against the input and output member until the input and output member are inserted into the vessel. The cartridge interface can also include a vessel holder to properly position the vessel onto the needles and a locking mechanism to lock the vessel in place while coupled to the cartridge. These features provide a snug fit of the cartridge and the vessel.

Vessels suitable for use with the cartridge can be of any volume size. For example, the vessels can range in volumes from 0.1 to 1 mL to 100 mL. In one embodiment, the vessel has a volume of 10 mL. The volume of the vessel may depend on the sample and the suspected target to be detected. That is, the vessel should be of a sufficient volume to contain an amount of sample fluid in which it is more likely than not that a suspected target is present.

In an embodiment, the output member is positioned within the vessel so that the output member delivers a fluid to the top of the vessel. This causes the fluid to run down at least one side of the vessel and rinse any sample that may have collected along the side of the vessel. The drive mechanism can be set to apply a pressure sufficient to deliver the fluid out of the output member so that it hits the top surface of the vessel. In addition, the input member is positioned within the vessel to promote drainage of the vessel contents. For example, the input member is level with or below the bottom of the vessel. In one embodiment, the vessel or the vessel plug is shaped to drain into the input member. For example, the vessel plug is conically-shaped.

In addition, the drive mechanism may provide sufficient pressure to release capture particles out of the output member and into the vessel. For example, pressure from the drive mechanism releases a buffer into a chamber having a plurality of capture particles disposed therein. The buffer/capture particles are then driven from the cartridge through the output member and directly into the vessel. The drive mechanism continues to force the buffer through the particle chamber and into the vessel until all of the capture particles are transferred into the vessel. At the same time, the input member may transfer the sample, buffer, and capture particles out of the vessel and into the cartridge. After substantially all the sample and capture particles are transferred into the cartridge, fluid or the buffer can continue to be introduced into the sample for an additional rinse. In another embodiment, the input member transfers at least a portion of the sample into the cartridge prior to introduction of the capture particles/buffer to provide space within the vessel.

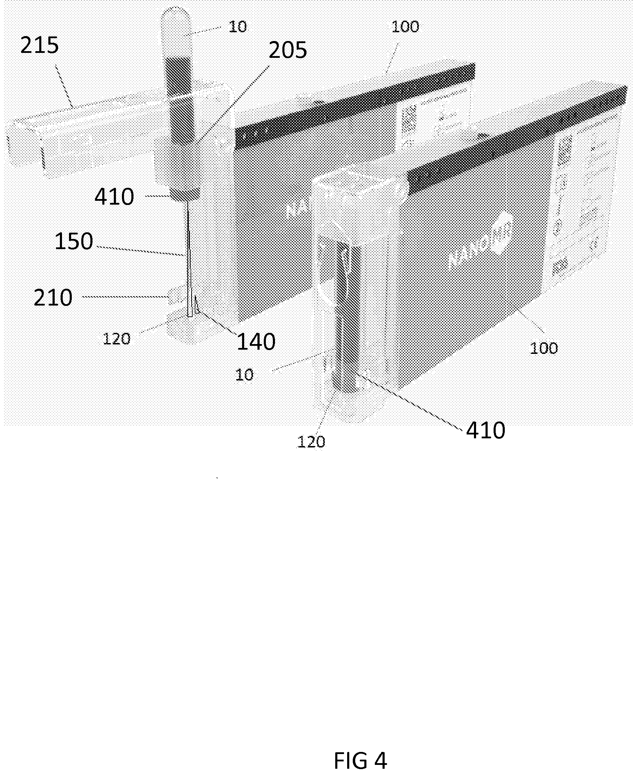

FIG. 4 highlights the vessel/cartridge interface 120 of the cartridge 100. The vessel/cartridge interface couples the vessel 10 to the cartridge 100 and provides communication (including fluidic and pneumatic communication) between the cartridge and the vessel. The vessel/cartridge interface includes output member 150 and input member 140. The output member 150 and input member 140 are hollow pins or needles that penetrate the vessel 10 to place the vessel 10 in communication with the cartridge 100. The input member 140 and output member 150 penetrate a stopper 410 coupled to the vessel. The vessel/cartridge interface 120 can include guide 205. For loading, the vessel 10 is pushed through the guide 205 to direct and align the vessel 10 onto the input member 140 and output member 150. One or more positioning arms 210 are designed to hold the vessel 10 in place once positioned onto the input member 140 and output member 150. In addition, the cartridge can include a vessel cover 215 that closes over the vessel 10 as coupled to the cartridge 100.

Instrument

In certain aspects, the cartridge interfaces with and is used in conjunction with an instrument. The instrument provides, for example, the pneumatic, fluidic, magnetic, mechanical, chemical functions, as necessary to process the sample within the cartridge. In one aspect, the cartridge is inserted into the instrument for processing and the instrument is turned on by an operator to activate sample processing. Once the cartridge is loaded into the instrument, the system does not require further manual technical operations on behalf of the operator.

In one embodiment, the instrument contains drive mechanisms that connect to the cartridge when inserted into the instrument. Any drive mechanism known in the art may be used with target capture system, including pneumatic drive mechanisms, hydraulic drive mechanisms, magnetic drive systems, and fluidic drive systems. The drive mechanism provides a means for fluid control within the cartridge and allows for transport of fluid and substances between chambers. The drive mechanism can be used to initiate and control fluid flow, open valves, form bubbles (e.g. for mixing) and to initiate mechanical/chemical processes within the cartridge.

The drive mechanism can also be operably associated with a controller so that the controller engages the drive mechanism at certain stages in the pathogen capture process. The controller may engage with one or more sensors to determine when and how to activate the drive mechanism during sample processing. In certain aspects, the controller is a computing system. In certain embodiments, drive mechanism is a pneumatic. The pneumatic drive mechanism can include pumps, electromechanical valves, pressure regulators, tubing, pneumatic manifolds, flow and pressure sensors. Pneumatic drive mechanisms use air pressure and air displacement to control the flow of fluids within the cartridge. In certain aspects, the pneumatic drive mechanism is coupled to electronic regulators. When coupled to an electronic regulator, the pneumatic mechanism may be an external compressor with a reservoir for pumping compressed nitrogen, argon or air.

The instrument also includes one or more magnetic assemblies. The magnetic assemblies engage with one or more magnetic traps (typically, flow-through chambers) of the cartridge. The magnetic assemblies can include permanent magnets, removable magnets, electromagnets, or the like, or combinations thereof. The magnet assemblies may have magnets of various shapes, and of varying strengths, depending on the application thereof. If the instrument includes electromagnets, i.e. magnets that produce a magnetic field upon introduction of an electric current, the instrument may also include a current generator to activate the electromagnets. Depending on the stage of processing, the magnetic assembly includes one or magnet that are positioned against the cartridge to facilitate capture of one or more magnetic particles on a surface of a magnetic trap. Alternatively, the electromagnets can be prepositioned next to the cartridge and activated by an electric current to facilitate capture of one or more magnetic particles against the surface of the trap.

The size and strength of the magnet(s) of the magnetic assembly should produce a magnetic field suffice to force the magnetic particles within the sample against a surface of the magnetic trap of the cartridge, either macrofluidic or mircofluidic. For example, the magnetic assembly can include 7 bar NdFeB magnets that can be positioned against a magnetic trap of the cartridge. In another example, the magnet assembly includes a magnet with a magnetic flux of about 0.6 T and a magnetic gradient of about 150 T/m. This magnet's high magnetic gradient of about 150 T/m is capable of isolating a plurality of magnetic particles (for example, 1000 magnetic particles) in on a surface with a micro-scale surface area.

The instrument can also include mechanical, electrical, and thermo-electrical systems. For instance, instrument can include mechanical mechanism for engaging with a paddle mixer disposed within in a mixing chamber of the cartridge. The instrument can also include pistons and plungers to activate one or more push valves located on the cartridge. In addition, the instrument can include a heating system designed to control the temperatures of one or more components of the cartridge. For example, the instrument can include a heating apparatus operably associated with the mixing chamber to heat the chamber and encourage binding of one or more magnetic particles with targets contained within the sample. The instrument may include a control processor or a computing system to activate other subsystems, such as the drive mechanism. The control processor can be keyed into sensors designed to track the process through the cartridge. This allows the control processor to activate certain substances based on the location of the fluid within the cartridge or based upon the stage of processing.

The instrument can also include a lysing mechanism for invoking lysis of cells within the sample. The lysing mechanism can include any sonication device that is well-known in the art. In certain embodiments, the sonication device is the VCX 750 Sonicator sold under the trademark VIBRA-CELL (sonicator, commercially available by Sonics & Materials, Inc.). Generally, the probe of the sonicator is placed into the liquid containing the targets to be lysed. Electrical energy from a power source is transmitted to a piezoelectric transducer within the sonicator converter, where it is changed to mechanical vibrations. The longitudinal vibrations from the converter are intensified by the probe, creating pressure waves in the liquid. These in turn produce microscopic bubbles, which expand during the negative pressure excursion and implode violently during the positive excursion. This phenomenon, referred to as cavitation, creates millions of shock waves and releases high levels of energy into the liquid, thereby lysing the target. In another embodiment, the sonication transducer may be brought in contact with a chamber holding captured complexes by way of a structural interface. The sonication transducer vibrates structural interface, such as a thin film between the magnetic trap and the transducer, until lysis is achieved. In either method, the appropriate intensity and period of sonication can be determined empirically by those skilled in the art.

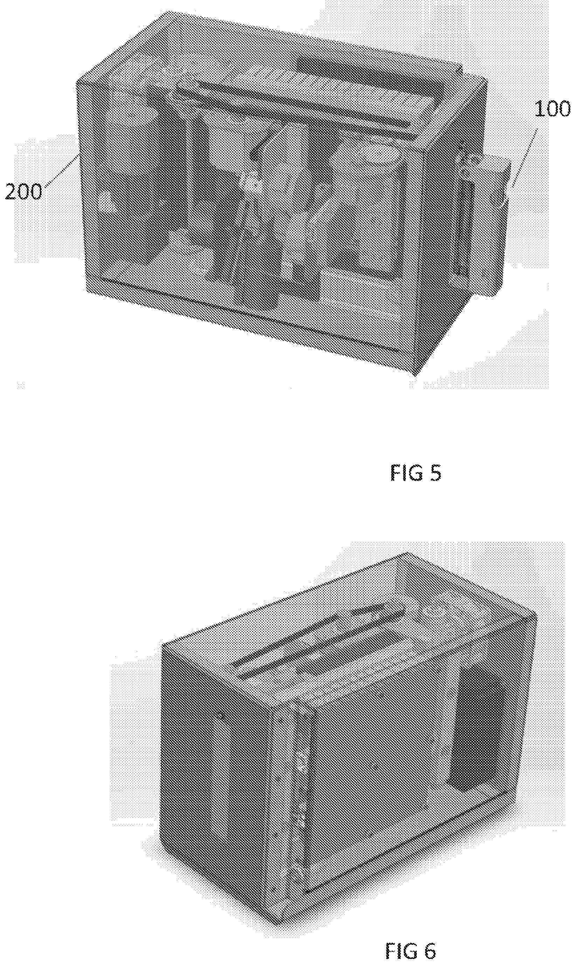

FIGS. 5 and 6 depict the instrument 200 of the target capture system for use with the cartridge 100. FIG. 5 depicts the cartridge 100 loaded into the instrument 200. FIG. 6 depicts the instrument 200 without a cartridge 100 loaded. The features of the instrument 200 are discussed in detail above.

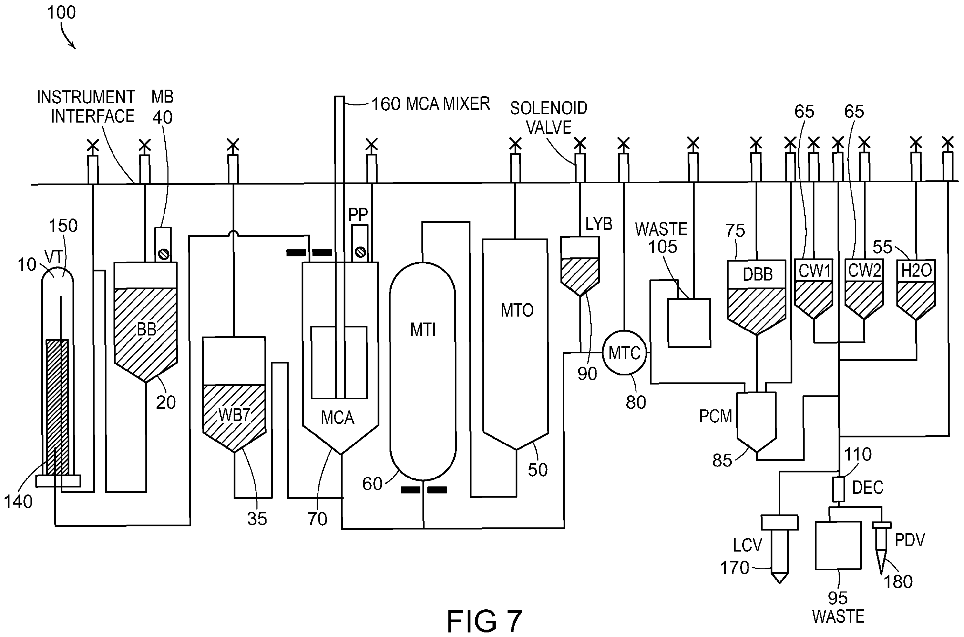

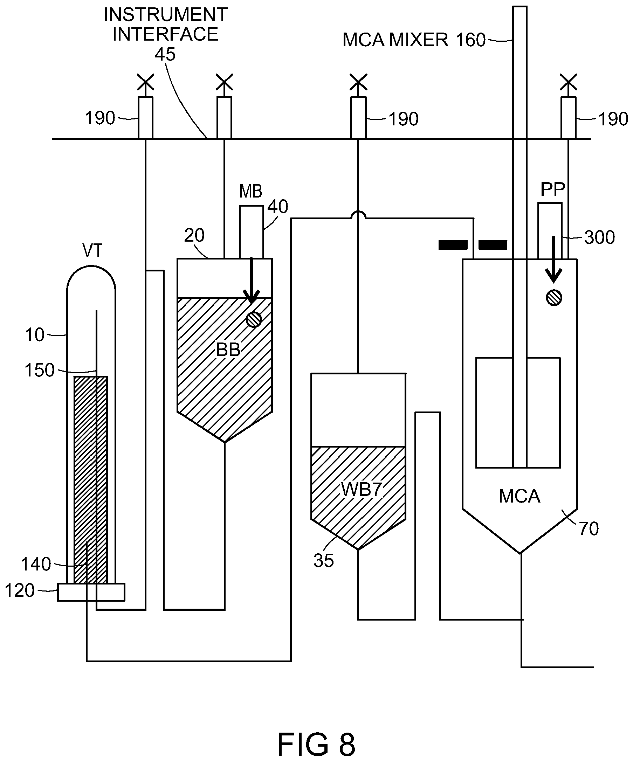

FIG. 7 depicts another schematic view of the cartridge 100 of the target capture system. FIGS. 8-23 depict the process of target capture within the target capture system as the sample is directed into and processed within the cartridge. The arrows within the figures indicate the path of fluid flow.

As shown in FIG. 8, a vessel 10 coupled to and in fluidic and pneumatic communication with the cartridge 100 via the output member 150 and input member 140. The vessel 10 is an enclosed collection tube containing a sample. In one embodiment, vessel contains 10 to 15 mL of blood. The cartridge 100 is placed within an instrument and connected to the instrument's 200 drive mechanism at the instrument interface 45 through interface ports 190. Once the vessel 10 is in communication with the cartridge 100, the user can activate the instrument to initiate the target capture process. The instrument 200 activates and empties a chamber containing magnetic particles 40 into a particle buffer reservoir 20. Each of the magnetic particles is conjugated to a moiety specific to at least one target. As discussed above, the plurality of magnetic particles in the chamber 40 may include different sets of magnetic particles in which each set is conjugated to capture moiety specific to a different target of interest (e.g. for multiplex capture of targets). The chamber containing magnetic particles 40 is typically a glass ampoule. A piston located on the instrument can apply pressure to the particle chamber 40 causing it to release the particles into the buffer reservoir 20. Alternatively and as shown in FIG. 2, the chamber containing the magnetic particles 40 is located between the buffer reservoir 20 and the vessel 10. In this configuration, the drive mechanism forces the buffer towards the particle chamber 40 with enough pressure to cause the buffer to break a seal on the particle chamber 40 and introduce the buffer into the particle chamber 40. In either embodiment, the result is having the buffer and particles in the same chamber/reservoir. Typically, the buffer/particle mixture is present in a 2:1 ratio to the initial volume of sample (e.g. buffer/particle mixture is about 20 mL to 30 mL for a sample of 10 mL to 15 mL). Any buffer suitable for promoting binding of magnetic particles (having binding moieties specific to targets) to targets is suitable for use in the cartridge 100. Once in the same chamber, a bubble can be introduced to ensure the magnetic particles are fully submersed in the buffer.

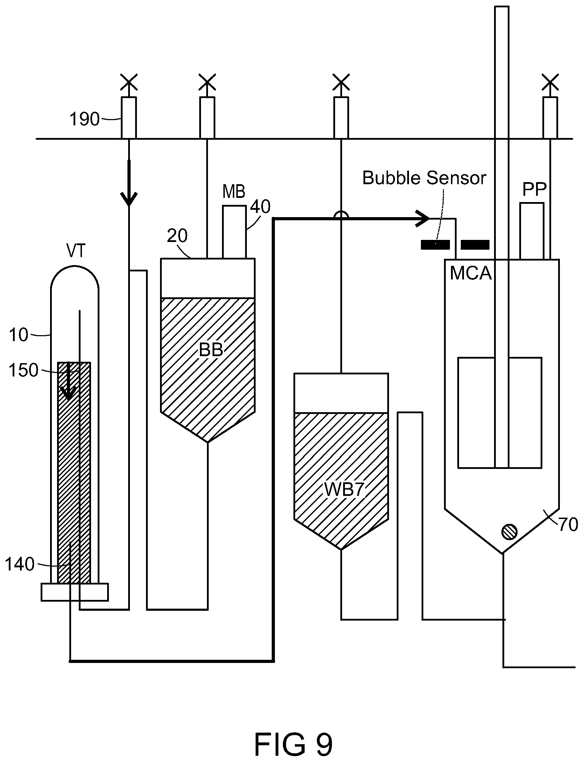

As shown in FIG. 9, prior to introducing the particle/buffer solution into the vessel, the drive mechanism of the instrument forces air into the vessel through output member 150. The air pressure causes at least a portion of the sample to flow through the input member 140 and into the cartridge 100. Within the cartridge 100, the sample is driven through a channel towards the mixing chamber 70. A bubble sensor prior to the inlet of the mixing chamber 70 and air displacement sensor monitor the amount of fluid flowing from the vessel to the mixing chamber 70. The instrument can alert an operator if the desired volume of fluid transferred is not as suspected. In certain embodiments, the drive mechanism forces the entire sample out of the vessel 10 and into the cartridge 100 prior to introducing the buffer/particle mixture into the vessel 10. Alternatively, the drive mechanism transfers none or a portion of the sample into the cartridge 100 prior to introducing the buffer/particle mixture into the vessel 10. In the addition, the step of introducing the particle/buffer mixture into the vessel 10 can be substantially concurrent with the step of transferring the sample and/or sample/particle/buffer mixture into the cartridge 100.

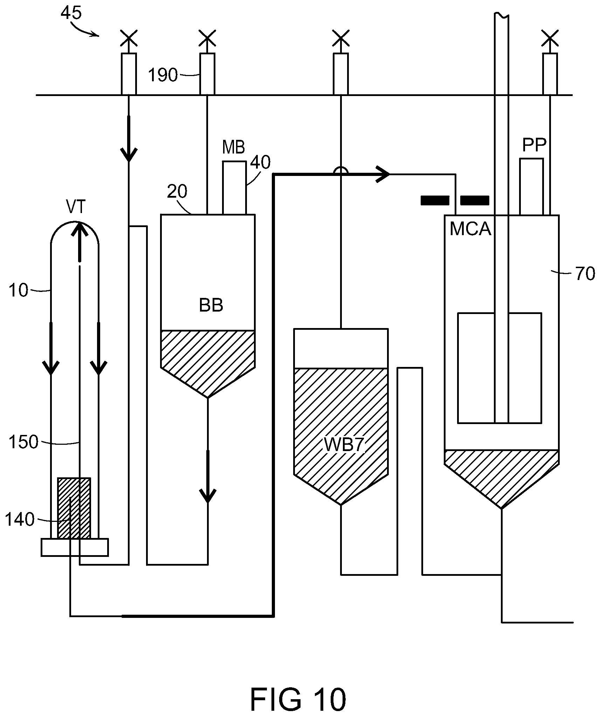

In FIG. 10, the buffer/particle mixture is transferred out of the particle buffer reservoir 20 and into the vessel 10 through output member 150 via air pressure from the drive mechanism. The buffer/particle mixture is transported into the vessel 10 with sufficient force to hit the top of the vessel 10 so that the buffer/particle mixture rinses down the sides of the vessel 10. This ensures any sample collected on the sides of the vessel 10 is introduced into the cartridge 100 through the input member 10. The sample/particle/buffer mixture is driven into the cartridge 100 through input member 140. In one embodiment, after substantially the entire sample and/or particles have been transferred into the cartridge, buffer is still introduced in to the cartridge to conduct an additional rinse. In certain embodiments, after transfer of the buffer into the vessel and draining of buffer/sample/particle mixture into the cartridge, the drive mechanism continues to force air into the vessel 10 to ensure any residual fluid is moved into cartridge 100. The process depicted in FIG. 10 increases the amount of initial sample that is introduced into the cartridge for processing. Because pathogens are often present in levels as 1 CFU/mL, the ability to transfer all of the initial sample fluid into the cartridge advantageously prevents the chance that sample containing the pathogen would remain in the vessel.

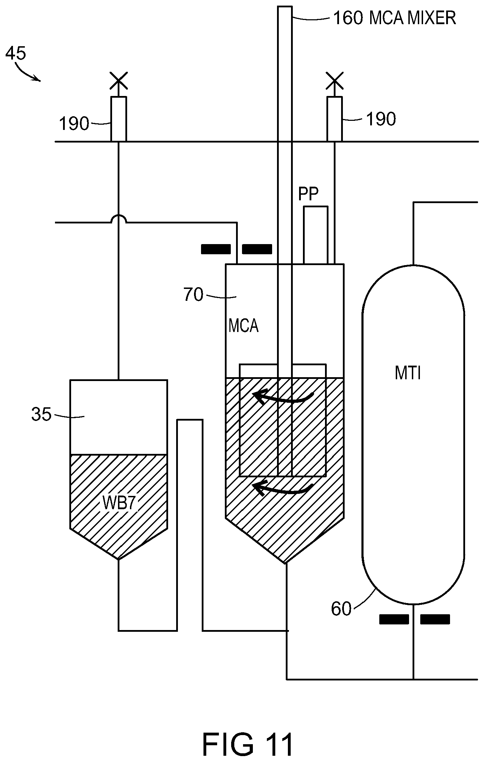

Once the sample/particle/buffer mixture is transferred from the vessel 10 to the mixing chamber 70 as shown in FIG. 11, the mixture is agitated and incubated in the mixing chamber 70. For agitation, the instrument 200 rotates the mixer paddle 170. In one embodiment, the instrument 200 heats the mixing chamber 70 to a temperature ideal for promoting binding of the magnetic particles and any targets present within the sample. The incubation/agitation process is to form target/particle complexes within the fluid. A temperature sensor can be included in the mixing chamber 70 to monitor temperature. The amount of time the sample/particle/buffer mixture is in the mixing chamber 70 can depend on a variety of factors. For example, incubation and agitation time will depend on the desired degree of binding between the pathogen and the compositions of the invention (e.g., the amount of moment that would be desirably attached to the pathogen), the amount of moment per target, the amount of time of mixing, the type of mixing, the reagents present to promote the binding and the binding chemistry system that is being employed. Incubation time can be anywhere from about 5 seconds to a few days. Exemplary incubation times range from about 10 seconds to about 2 hours. Binding occurs over a wide range of temperatures, generally between 15 °C and 40 °C

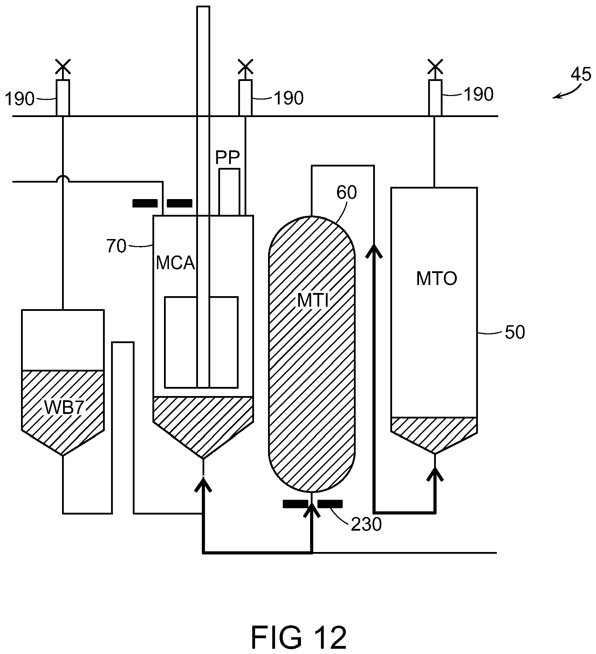

After incubation/agitation, the sample/particle/buffer mixture is cycled through the first magnetic trap 60, as shown in FIG. 12. During the cycling process, a magnetic assembly of the instrument engages with the first magnetic trap 60 to generate a magnetic field that captures the magnetic particles from the mixture cycled therethrough on a surface of the first magnetic trap 60. In one embodiment, the magnetic trap 60 has a flow path cross-section of 0.5 mm x 20 mm (h x w) and the magnetic assembly is an array of bar NdFeB magnets positioned perpendicular to the flow of fluid into the magnetic trap. For cycling, the drive mechanism uses air pressure to force the sample/particle/buffer mixture from the mixing chamber 70 through the first magnetic trap 60 and to the first magnetic trap overflow chamber 50. Once the fluid is passed through the magnetic trap 60 and into the overflow chamber 50, the fluid is then moved back through the first magnetic trap 60 and into the mixing chamber 70. The fluid can be cycled back and forth between the mixing chamber 70 and overflow chamber 50 multiple times and at different flow rates to ensure all magnetic particles are captured. In one embodiment, a bubble sensor 230 associated with the first magnetic trap is used to detect when fluid entering or exiting the magnetic trap is replaced with air. This alerts the instrument 200 that the fluid from the mixing chamber 70 has substantially transferred through the first magnetic trap 60 and into the magnetic trap overflow chamber 50. Once alerted, the fluid is moved back through the first magnetic trap 60. The bubble sensor 230 can be placed at the entrance of the first magnetic trap 60 (as shown in FIG. 12) or at the exit of the first magnetic trap. As an alternative to the bubble sensor, the cycling of fluid can be time controlled.

After the final cycle of fluid through the first magnetic trap 60, the remaining fluid (sample/buffer) separated from the captured magnetic particles is moved into the mixing chamber 70 and stored as waste. Alternatively, the remaining fluid can be transferred to a designated waste chamber.

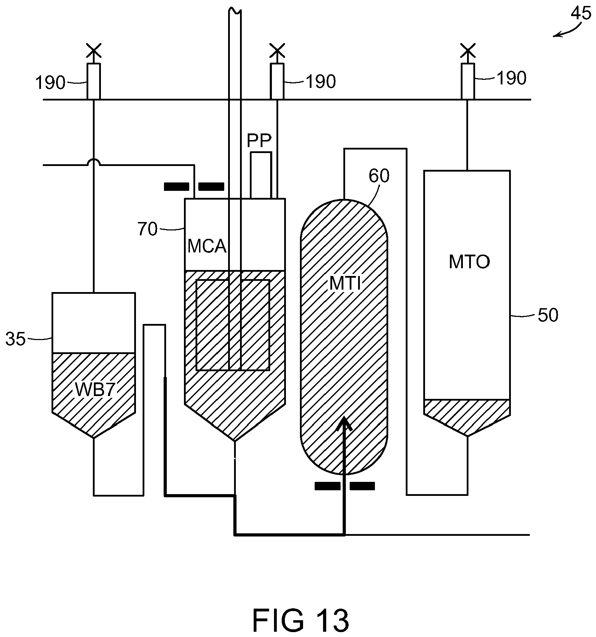

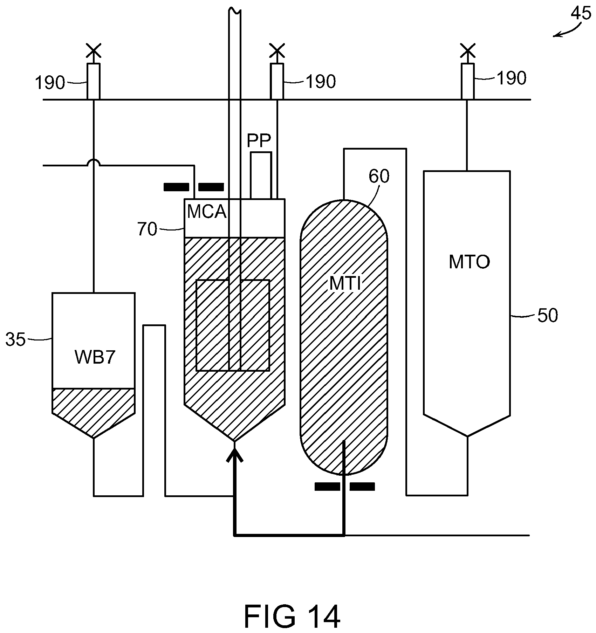

The captured particles within the first magnetic trap 60 are then subject to a wash process as shown in FIG. 13. During the wash process, the magnetic assembly is still engaged with the first magnetic trap 60. A wash solution from wash solution reservoir 35 is transferred into the first magnetic trap 60 until the first magnetic trap 60 is filled. As shown in FIG. 14, the wash solution is then moved out of the first magnetic trap 60, thereby washing/rinsing the particles captured on the surface of the first magnetic trap 60. The wash solution can be moved into the mixing chamber 70 and stored as waste or into a designated waste chamber. Pressure can used to avoid back flow of fluid into the first magnetic trap 60.

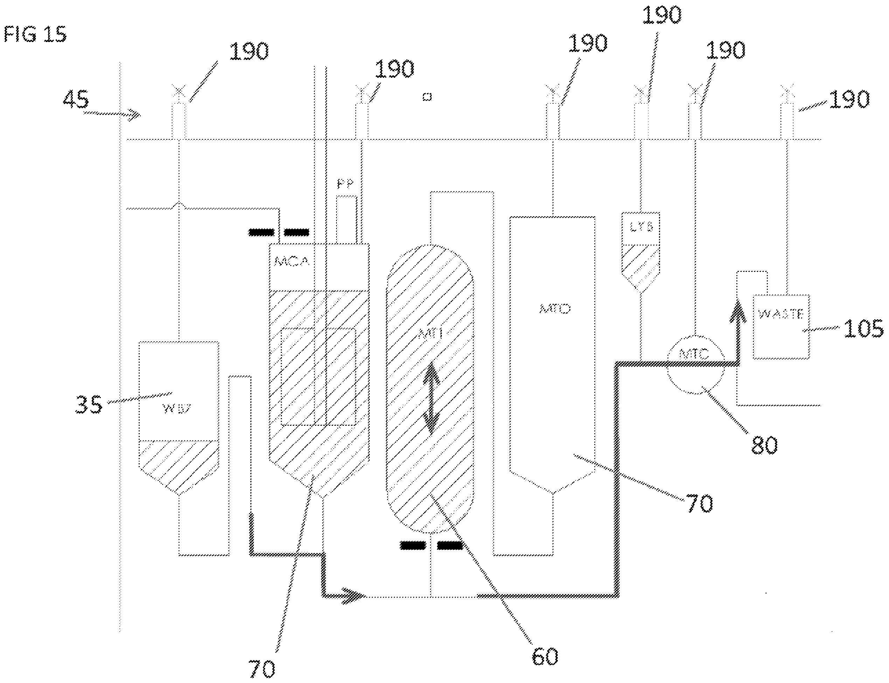

As shown in FIG. 15, the first magnetic trap 60 is further rinsed to suspend captured particles in a fluid and to move the magnetic particles into the second magnetic trap 80. The drive mechanism moves additional wash solution into the first magnetic trap 60, in which one opening of the magnetic trap is closed to prevent flow through. During introduction of wash solution to the first magnetic trap, the magnetic particles are released from the surface and suspended in the wash solution. To remove the magnetic particles from the surface of the first magnetic trap 60, the magnetic assembly is removed from against the first magnetic trap 60 or the magnetic assembly is no longer energized. In one embodiment, a swiper magnet engages an opposite side of the first magnetic trap 60 to encourage the particles to re-suspend. The fluid with the particles from the first magnetic trap 60 is then transported into the second magnetic trap 80. The second magnetic trap 80 is a flow through chamber. Prior to transfer of particles through the second magnetic trap 80, a magnetic assembly engages with the second magnetic trap 80. This magnetic assembly can be the same as or different from the magnetic assembly that engaged with the first magnetic trap 60. In one embodiment, the magnetic assembly is different and includes one or more magnets that emit a magnetic field capable of isolating the quantity of magnetic particles transferred from the first magnetic trap 60 within the second magnetic trap 80 having a microfluidic volume. For example, a second magnetic trap 80 having a volume 500 $\mu$L can engage with a magnet having a magnetic flux of 0.6T and a magnetic gradient of 150 T/m to isolate about 1000 particles (assuming 1000 particles were initially introduced into the sample, processed through the first magnetic trap 60, and transferred to the second magnetic trap 80).

The second magnetic trap 80, as engaged with the magnetic assembly, captures magnetic particles as the fluid flows from the first magnetic trap 60 through the second magnetic trap 80 and into a waste chamber 105. Pressure from the drive mechanism is applied to ensure all the fluid/magnetic particles are transferred into the magnetic trap and to prevent any fluid back flow. The rate of the fluid flow can be controlled to ensure all magnetic particles are capture while the fluid flows through the second magnetic trap 80. In one embodiment, the rate of fluid flow is 1 mL/min. In one aspect, the second magnetic trap 80 has a significantly smaller volume than the first magnetic trap 60 which allows the second magnetic trap 80 to concentrate the substantially the entire quantity of particles initially introduced into the sample into a small volume of fluid. The high concentration of particles in a small volume of fluid provides for easier downstream analysis of or processes performed on targets bound to those particles. That is, the target capture system is able to isolate the clinically relevant portion of a macrofluidic fluid volume in a microfluidic fluid volume. In one embodiment, the first magnetic trap 80 is macrofluidic (volume capacity above 1mL) and the second magnetic trap is microfluidic (volume capacity below 1000 $\mu$L). For example, the first magnetic trap 60 has a macrofluidic volume for processing 30 mL of fluid to initially capture magnetic particles disposed within the 30 mL of fluid and the second magnetic trap 80 has a microfluidic volume of 500 $\mu$L of less.

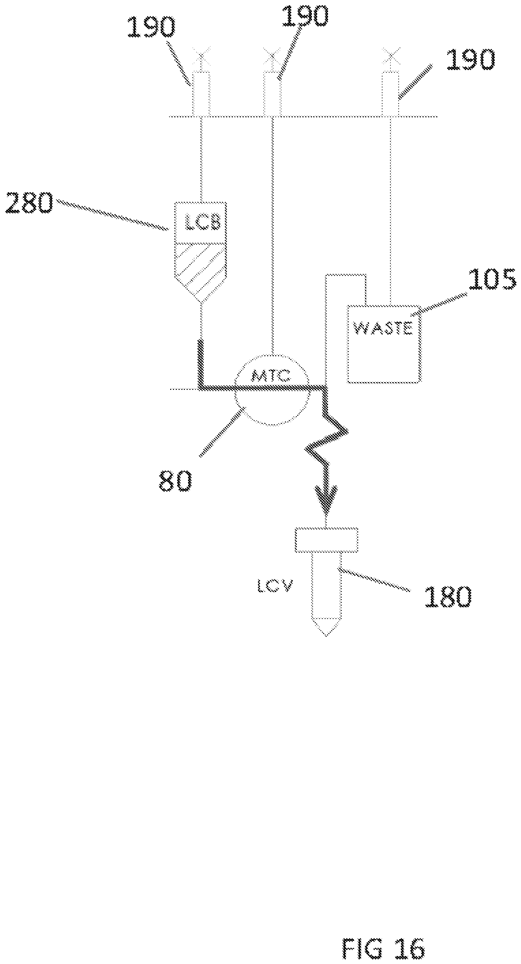

After the magnetic particles are concentrated in the second magnetic trap 80, the captured particles can be directed to a capture vial or subject to further processing. FIG. 16 shows the transfer of the captured particles from the second magnetic trap 80 to the capture vial 170. For transfer to the capture vial 170, the magnet assembly is disengaged from the second magnetic trap 80. Then, a target culture buffer from a reservoir 280 flushes the contents of the second magnetic trap 80 into the capture vial 170. This provides for direct capture of the particles and molecules attached thereto. Direct capture of particles from the second magnetic trap 80 can be used to capture whole live cell targets bound to the particles.

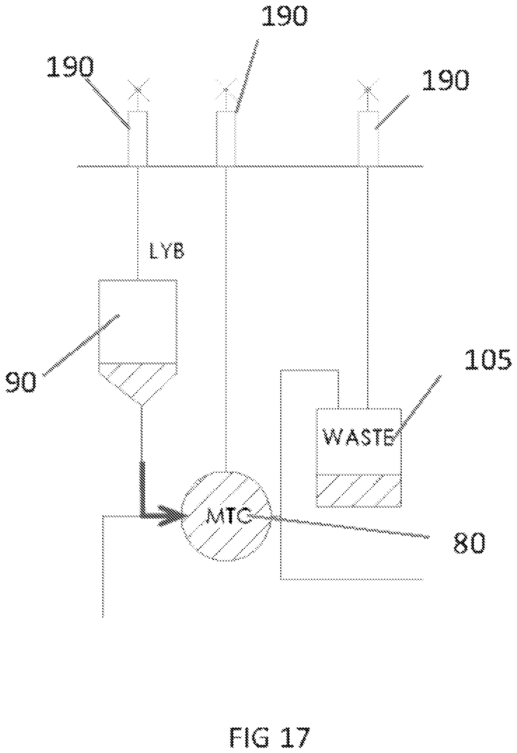

FIGS. 17-22 depict further processing of captured particles to obtain nucleic acids from any targets bound to the captured particles. First, the captured magnetic particles are subject to sonication to invoke cell lysis of target cells bound to the particles. This step allows lysis of target cells in the presence of the magnetic particles without pre-separation of the particles from the target. This step avoids potential loss of targets during the pre-separation step. As shown in FIG. 17, a fluid from reservoir 90 is transferred into the second magnetic trap 80. In one embodiment, the fluid is a lysing buffer or agent. The outlet port of the second magnetic trap 80 is closed to allow the cell lysis buffer to fill the second magnetic trap 80. Once filled, the magnetic assembly is disengaged from the second magnetic trap 80 to suspend the magnetic particles in the buffer. A sonicator probe (such as a VibraCell sonicator) of the instrument is positioned against a surface of the second magnetic trap 80. In one embodiment, the surface is a formed from a thin film, such as a Mylar (general purpose film, DuPont).film of 125 $\mu$M. The sonicator can be activated for different lengths and at different settings to achieve cell lysis. In one embodiment, the cartridge 100 further includes a reservoir of lysis bashing beads that can be transferred into the second magnetic trap 80 to assist with cell lysis.

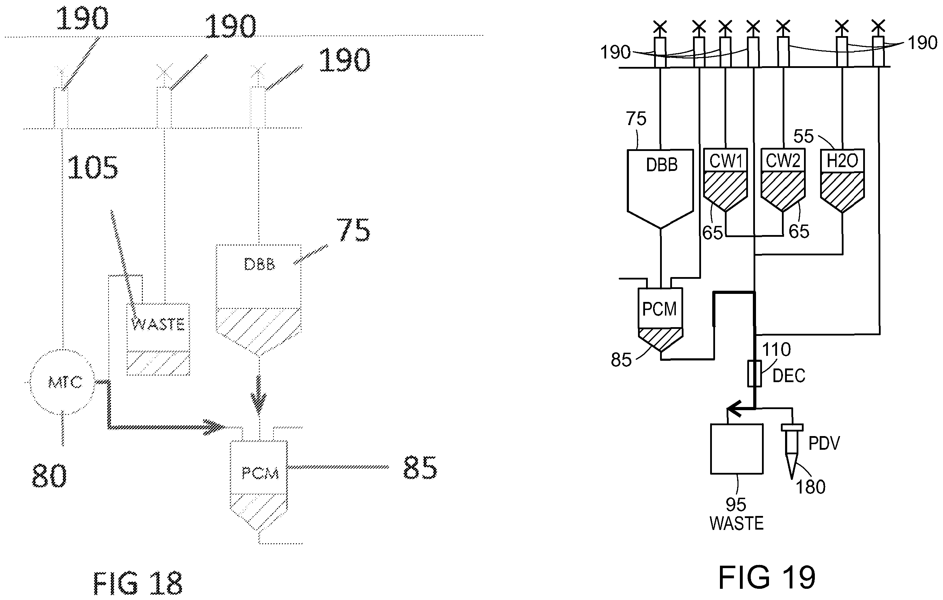

After lysis by sonication is complete, the lysate can be forced into a pre-column mixer 85 as shown in FIG. 18. A nucleic acid extraction buffer can also be introduced into the pre-column mixer 85 from reservoir 75. The lysate and the extraction buffer are mixed within the pre-column mixer 85. In one embodiment, the pre-column mixer 85 is a bubble mixer and the lysate/extraction buffer mixture is agitated via bubbling air into the mixer 85. FIG. 19 depicts the lysate/extraction buffer mixture is transferred from the pre-column mixer 85 through nucleic acid extraction matrix 110 and into a waste chamber 95. In one embodiment, the nucleic acid extraction matrix 110 is an affinity column. The nucleic acid extraction matrix 110 retains nucleic acids from the lysate/extraction buffer mixture.

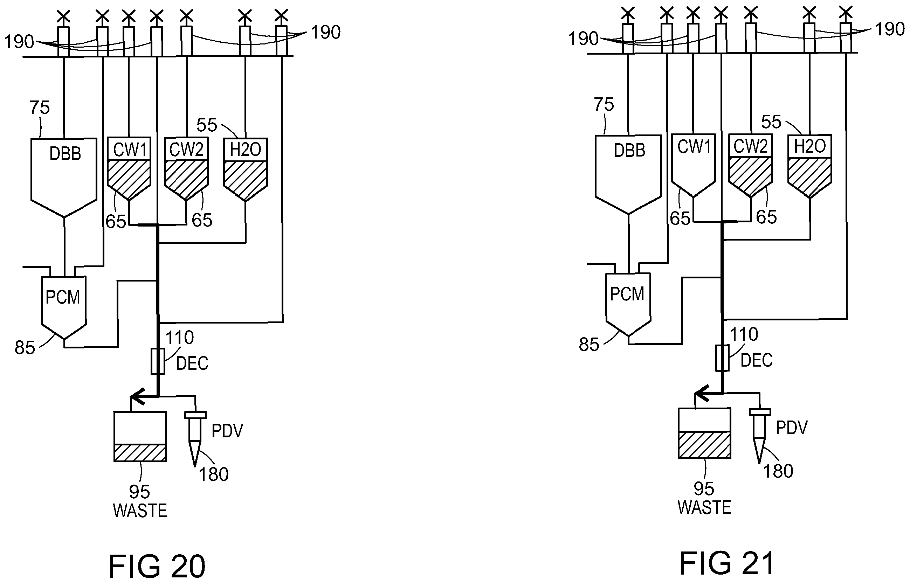

The nucleic acid extraction matrix 110 can then be subject to one or more washes. As shown in FIG. 20 and 21, the nucleic acid extraction matrix 95 is subject to two washes. Air pressure forces wash buffers from chambers 65 through the nucleic acid extraction member 110 and into waste chamber 95 to remove any unwanted particulates. In one embodiment, any remaining volatiles within the nucleic acid extraction matrix 110 are removed with air pressure from the drive mechanism.

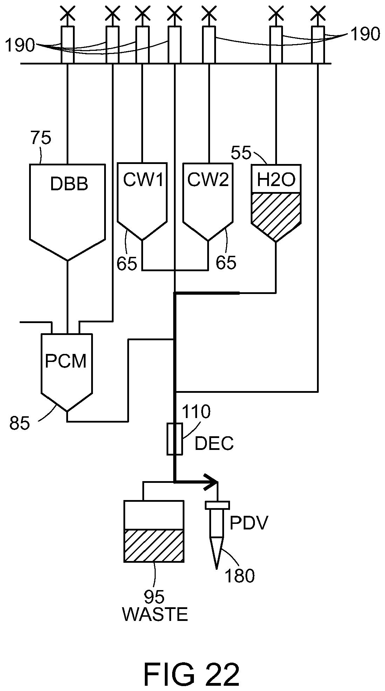

After the washes, the nucleic acid extraction matrix 110 can be eluted with a fluid from the elution reservoir 55. In one embodiment, the fluid is water. The drive mechanism uses high air pressure to force the fluid through the nucleic acid extraction matrix 110 into a nucleic acid capture vial 180 (See FIG. 22). This elution step transfers extracted nucleic acids into the vial. The capture vial 180 can be removed from the cartridge by the operator and subject to further analysis. In certain embodiments, the capture vial 180 may include one or more components required for subsequent analysis of the extracted nucleic acids. For example, the one or more components may include primer/probe sets for real-time PCR quantification. Preferably, the one or more components for subsequent analysis of the extracted nucleic acids are specific to same pathogens that the magnetic particles were designed to capture.

Detection of Targets

In particular embodiments, the isolated targets or the extracted nucleic acid from the captured targets, as isolated with the target capture system, may be analyzed by a multitude of technologies. These technologies include, for example, miniature NMR, Polymerase Chain Reaction (PCR), mass spectrometry, fluorescent labeling and visualization using microscopic observation, fluorescent *in situ* hybridization (FISH), growth-based antibiotic sensitivity tests, and variety of other methods that may be conducted with purified target without significant contamination from other sample components.

In one embodiment, isolated bacteria are eluted from the magnetic particles and are lysed with a chaotropic solution, and DNA is bound to DNA extraction resin. After washing of the resin, the bacterial DNA is eluted and used in quantitative RT-PCR to detect the presence of a specific species, and/or, subclasses of bacteria.

In another embodiment, captured bacteria is removed from the magnetic particles to which they are bound and the processed sample is mixed with fluorescent labeled antibodies specific to the bacteria or fluorescent Gram stain. After incubation, the reaction mixture is filtered through 0.2 $\mu$m to 1.0 $\mu$m filter to capture labeled bacteria while allowing majority of free particles and fluorescent labels to pass through the filter. Bacteria is visualized on the filter using microscopic techniques, e.g. direct microscopic observation, laser scanning or other automated methods of image capture. The presence of bacteria is detected through image analysis. After the positive detection by visual techniques, the bacteria can be further characterized using PCR or genomic methods.

Detection of bacteria of interest can be performed by use of nucleic acid probes following procedures which are known in the art. Suitable procedures for detection of bacteria using nucleic acid probes are described, for example, in Stackebrandt et al. (U.S. 5,089,386), King et al. (WO 90/08841), Foster et al. (WO 92/15883), and Cossart et al. (WO 89/06699), each of which is hereby incorporated by reference.

A suitable nucleic acid probe assay generally includes sample treatment and lysis, hybridization with selected probe(s), hybrid capture, and detection. Lysis of the bacteria is necessary to release the nucleic acid for the probes. The nucleic acid target molecules are released by treatment with any of a number of lysis agents, including alkali (such as NaOH), guanidine salts (such as guanidine thiocyanate), enzymes (such as lysozyme, mutanolysin and proteinase K), and detergents. Lysis of the bacteria, therefore, releases both DNA and RNA, particularly ribosomal RNA and chromosomal DNA both of which can be utilized as the target molecules with appropriate selection of a suitable probe. Use of rRNA as the target molecule(s), may be advantageous because rRNAs constitute a significant component of cellular mass, thereby providing an abundance of target molecules. The use of rRNA probes also enhances specificity for the bacteria of interest, that is, positive detection without undesirable cross-reactivity which can lead to false positives or false detection.

Hybridization includes addition of the specific nucleic acid probes. In general, hybridization is the procedure by which two partially or completely complementary nucleic acids are combined, under defined reaction conditions, in an anti-parallel fashion to form specific and stable hydrogen bonds. The selection or stringency of the hybridization/reaction conditions is defined by the length and base composition of the probe/target duplex, as well as by the level and geometry of mis-pairing between the two nucleic acid strands. Stringency is also governed by such reaction parameters as temperature, types and concentrations of denaturing agents present and the type and concentration of ionic species present in the hybridization solution.

The hybridization phase of the nucleic acid probe assay is performed with a single selected probe or with a combination of two, three or more probes. Probes are selected having sequences which are homologous to unique nucleic acid sequences of the target organism. In general, a first capture probe is utilized to capture formed hybrid molecules. The hybrid molecule is then detected by use of antibody reaction or by use of a second detector probe which may be labelled with a radioisotope (such as phosphorus-32) or a fluorescent label (such as fluorescein) or chemiluminescent label.

Detection of pathogen of interest can also be performed by use of PCR techniques. A suitable PCR technique is described, for example, in Verhoef et al. (WO 92/08805). Such protocols may be applied directly to the pathogen captured on the magnetic particles. The pathogen is combined with a lysis buffer and collected nucleic acid target molecules are then utilized as the template for the PCR reaction.

In certain embodiments, nucleic acids derived from the captured pathogen are analyzed in a multiplex reaction in order to rapidly detect two or more pathogens present in the sample. Any multiplex reaction known in the art may be used, such as multiplex ELISA, multiplex sequencing, multiplex probe hybridization, etc. In certain embodiments, multiplex reactions may involve the amplification and quantification of two or more targets in the same reaction volume. Typical multiplex reactions involve PCR, qPCR (real-time PCR), and sequencing.

Multiplex PCR refers to the use of more than one primer pair in a single reaction vessel in order to amplify more than one target sequence. In the case of real-time PCR, more than one primer pair/probe set is utilized. Multiplex PCR allows for simultaneous detection of different targets in the same reaction. The target sequences may be identified by an identifiable label (e.g. fluorescent probe) or by subsequent sequencing. Multiplex real-time PCR uses multiple probe-based assays, in which each assay has a specific probe labeled with a unique fluorescent dye, resulting in different observed colors for each assay. Real-time PCR instruments can discriminate between the fluorescence generated from different dyes. Different probes are labeled with different dyes that each have unique emission spectra. Spectral signals are collected with discrete optics, passed through a series of filter sets, and collected by an array of detectors. Spectral overlap between dyes is corrected by using pure dye spectra to deconvolute the experimental data by matrix algebra. An overview of real-time PCR techniques is described in detail in Elnifro, Elfath M., et al. "Multiplex PCR: optimization and application in diagnostic virology." Clinical Microbiology Reviews 13.4 (2000): 559-570. Multiplex PCR reaction techniques are described in U.S. Publication Nos. 20130059762, and 2005/0026144 as well as Carroll, N. M., E. E. Jaeger, et al. (2000). "Detection of and discrimination between gram-positive and gram-negative bacteria in intraocular samples by using nested PCR." J Clin Microbiol 38(5): 1753-1757, and Klaschik, S., L. E. Lehmann, et al. (2002). "Real-time PCR for detection and differentiation of gram-positive and gram-negative bacteria." J Clin Microbiol 40(11): 4304-4307.

Multiplex sequencing involves the simultaneous sequencing of multiple target sequences in a single sequencing run. Multiplex sequencing allows for differentiation between target sequences of different pathogens in a sample and differentiation between nucleic acid sequences of pooled samples (e.g. differentiate between two or more patient samples). In order to differentiate between target sequences, one or more different barcodes may be introduced to the nucleic acid of a sample. Early multiplex sequencing is described in more detail in G.M. Church in U.S. Patent Number 4,942,124 and further by G.M. Church and S. Kieffer-Higgins in U.S. Patent Number 5,149,625. Multiplex sequencing of multiple samples involves sequencing of a plurality of template nucleic acid molecules from different samples at the same time on the same platform by attaching a unique oligonucleotide sequence (i.e., a bar code) to the template nucleic acid molecules from different samples prior to pooling and sequencing of the template molecules. The bar code allows for template nucleic acid sequences from different samples to be differentiated from each other. Once bar coded, template molecules from different samples may be pooled and sequenced at the same time on the same platform. Because the bar code on each template molecule is sequenced as part of the sequencing reaction, the bar code is a component of the sequence data, and thus the sequence data for different samples is always associated with the sample from which it originated. Due to the association in the sequence data, the sequence data from the pooled samples may be separated after sequencing has occurred and correlated back to the sample from which it originated.

Any sequencing technique (for singleplex and multiplex assays) may be utilized to identify isolated pathogens by their nucleic acid extracts. Suitable sequencing techniques include, for example, classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of separated molecules has more recently been demonstrated by sequential or single extension reactions using polymerases or ligases as well as by single or sequential differential hybridizations with libraries of probes.

For detection of the selected pathogen by use of antibodies, isolated pathogen are contacted with antibodies specific to the pathogen of interest. As noted above, either polyclonal or monoclonal antibodies can be utilized, but in either case have affinity for the particular pathgoen to be detected. These antibodies will adhere/bind to material from the specific target pathogen. With respect to labeling of the antibodies, these are labeled either directly or indirectly with labels used in other known immunoassays. Direct labels may include fluorescent, chemiluminescent, bioluminescent, radioactive, metallic, biotin or enzymatic molecules. Methods of combining these labels to antibodies or other macromolecules are well known to those in the art. Examples include the methods of Hijmans, W. et al. (1969), Clin. Exp. Immunol. 4, 457-, for fluorescein isothiocyanate, the method of Goding, J. W. (1976), J. Immunol. Meth. 13, 215-, for tetramethylrhodamine isothiocyanate, and the method of Ingrall, E. (1980), Meth. in Enzymol. 70, 419-439 for enzymes.

These detector antibodies may also be labeled indirectly. In this case the actual detection molecule is attached to a secondary antibody or other molecule with binding affinity for the anti-bacteria cell surface antibody. If a secondary antibody is used it is preferably a general antibody to a class of antibody (IgG and IgM) from the animal species used to raise the anti-bacteria cell surface antibodies. For example, the second antibody may be conjugated to an enzyme, either alkaline phosphatase or to peroxidase. To detect the label, after the bacteria of interest is contacted with the second antibody and washed, the isolated component of the sample is immersed in a solution containing a chromogenic substrate for either alkaline phosphatase or peroxidase. A chromogenic substrate is a compound that can be cleaved by an enzyme to result in the production of some type of detectable signal which only appears when the substrate is cleaved from the base molecule. The chromogenic substrate is colorless, until it reacts with the enzyme, at which time an intensely colored product is made. Thus, material from the bacteria colonies adhered to the membrane sheet will become an intense blue/purple/black color, or brown/red while material from other colonies will remain colorless. Examples of detection molecules include fluorescent substances, such as 4-methylumbelliferyl phosphate, and chromogenic substances, such as 4-nitrophenylphosphate, 3,3',5,5'-tetramethylbenzidine and 2,2'-azino-di-[3-ethelbenz-thiazoliane sulfonate (6)]. In addition to alkaline phosphatase and peroxidase, other useful enzymes include β-galactosidase, β-glucuronidase, α-glucosidase, β-glucosidase, α-mannosidase, galactose oxidase, glucose oxidase and hexokinase.

Detection of bacteria of interest using NMR may be accomplished as follows. In the use of NMR as a detection methodology, in which a sample is delivered to a detector coil centered in a magnet, the target of interest, such as a magnetically labeled bacterium, may be delivered by a fluid medium, such as a fluid substantially composed of water. In such a case, the magnetically labeled target may go from a region of very low magnetic field to a region of high magnetic field, for example, a field produced by an about 1 to about 2 Tesla magnet. In this manner, the sample may traverse a magnetic gradient, on the way into the magnet and on the way out of the magnet. As may be seen via equations 1 and 2 below, the target may experience a force pulling into the magnet in the direction of sample flow on the way into the magnet, and a force into the magnet in the opposite direction of flow on the way out of the magnet. The target may experience a retaining force trapping the target in the magnet if flow is not sufficient to overcome the gradient force.

$$\text{m dot (del B)} = F \qquad\qquad \text{Equation 1}$$

$$v_t = -F/(6*p*n*r) \qquad\qquad \text{Equation 2}$$

where n is the viscosity, r is the particle diameter, F is the vector force, B is the vector field, and m is the vector moment of the particle

The detection method is based on a miniature NMR detector tuned to the magnetic resonance of water. When the sample is magnetically homogenous (no bound targets), the NMR signal from water is clearly detectable and strong. The presence of magnetic material in the detector coil disturbs the magnetic field, resulting in reduction in water signal. One of the primary benefits of this detection method is that there is no magnetic background in biological samples which significantly reduces the requirements for stringency of sample processing. In addition, since the detected signal is generated by water, there is a built-in signal amplification which allows for the detection of a single labeled bacterium. NMR detection is described in further detail in co-pending and co-assigned U.S. application serial number 13/091,506.

Sample Entry

In this particular embodiment, cartridges of the invention are used for isolating a target from a sample utilizing a vessel in communication with the cartridge. Methods are presented for maximizing the amount of sample transferred from a collection vessel into a cartridge, including microfluidic cartridges. Discussions related to cartridge, cartridge and vessel interface, sample, magnetic particles, targets, binding moiety, reagents and buffers, instrument, can be found above in the related sections. As stated above, the target capture system is to isolate a target from a sample. In particular methods of the invention, a sample is introduced into a cartridge for processing. The details of the cartridge and the cartridge/vessel interface are described above in the related sections. In a preferred embodiment, a vessel containing a sample is coupled to a cartridge for processing the sample, wherein the cartridge includes an interface for providing communication between the sample and the cartridge. Once coupled, a fluid from the cartridge is introduced into the vessel. A particular advantage of introducing a fluid from the cartridge and into the vessel is that the fluid is able to rinse the vessel to ensure full drainage of the sample into the cartridge. In addition, air may be introduced from the cartridge and into the vessel to force any remaining sample and fluid in the vessel into the cartridge. Moreover, the fluid may be essential to the processes of the cartridge, and introducing the fluid directly into the sample allows for the fluid to engage with the entire sample and facilitates mixing of the sample and fluid. The fluid and the sample may be transferred from the vessel and into the cartridge for processing.

Cartridges used in methods include a cartridge/vessel interface. The cartridge/vessel interface places the vessel containing the sample in two-way communication with the cartridge. The communication between the vessel and the cartridge can be pneumatic, fluidic, or both. In certain embodiments, the cartridge/vessel interface can include an input member and an output member. The output member introduces fluids, gases, and substances from the cartridge into the vessel and the input member transfers the sample and any introduced fluids, gases, and substances from vessel into the cartridge. Typically, the input and output members define a lumen and include a penetrating tip. The input and output members may be designed to penetrate and extend into the vessel.

In a preferred embodiment, a drive mechanism operably coupled to the cartridge may provide the force or pressure to drive the fluid, sample, substances or gas between the cartridge and vessel. The drive mechanism can be a part of the cartridge or a part of an instrument operably associated with the cartridge. In one embodiment, the output member is positioned within the vessel such that when the drive mechanism transfers the fluid from the cartridge via the output member and into the vessel, the fluid hits a top portion of the vessel and rinses down at least one side of the vessel. The input member may be positioned to maximize drainage of the vessel contents, e.g. positioned even with or below the bottom of the vessel.

Any cartridge for processing a sample is suitable for use with the methods of the invention and can include the cartridge/vessel interface of the invention. The cartridge may be a microfluidic or macrofluidic device and the cartridge may be for use in conjunction with an instrument. The cartridge may process a sample to isolate or detect a target within the sample. In certain embodiments, the cartridge/vessel interface is part of a target capture system for isolating a target from a sample using magnetic particles having binding moieties specific to a target and a plurality of magnetic traps.

Preferably, the vessel is enclosed except for the communication with the cartridge. In this embodiment, fluid is introduced to a top portion of the vessel such that the fluid rinses down at least one side of the vessel. The fluid is able to rinse sample that may have collected or aggregated on the sides of the vessel to ensure it is transferred from the vessel and into the cartridge. In addition, air, or any other gas, can be introduced into the vessel to force transfer of any remaining sample, fluid, or both in the vessel into the cartridge.

Any fluid that does not interfere with cartridge processing is suitable for use. Preferably, the fluid is chosen because the fluid is essential to the cartridge processes. In certain embodiments, the fluid contains a plurality of capture particles, in which each particle is conjugated to a binding moiety specific to a target. The fluid may be chosen because the fluid facilitates binding of one or more particles to a target within the sample. In one aspect, the fluid is a buffer. By introducing capture particles into the sample, methods of the invention ensure that the entirety of the sample is exposed to potential capture events. In direct contrast, when delivering the sample into the cartridge and then exposing the sample to capture particles, only the sample transferred into the cartridge is exposed to the particles for capture. In one embodiment, the capture particles are magnetic particles and processing may include exposing the magnetic particles to a magnetic field to separate the magnetic particles from the rest of the sample. Once the particles are captured, methods described above can be used, in any combination, to lyse, amplify, sequence, detect, classify, etc., methods for which are discussed below.

Isolation and Characterization of Pathogens

Certain aspects of the invention provide methods for identification of the genes expressed by a pathogen at an earlier stage of infection than possible with other techniques. Identification of genes expressed by a pathogen during active blood-borne infection has several benefits and uses. Determining which genes are expressed by a pathogen helps to identify novel targets for antimicrobial therapy and may elucidate the pathophysiology of infection by the target pathogen. In addition, the gene expression profiles of pathogens allow for identification of transcripts encoding for surface antigens, which can be used for antibody generation. The antibodies can be used for diagnostic purposes, e.g. identification of pathogens, or for immunological purposes, e.g. rational vaccine design. In certain embodiments, such methods may be carried out with the above described cartridges. In other embodiments, such methods are conducted without the need for the above described cartridges.

In certain aspects, a cDNA library of the pathogen isolated during active-blood borne infection is constructed. The cDNA library can be used in antibody screening efforts to identify those proteins most strongly identified by a particular antiserum. Knowing which proteins contribute to antibody responses allows one to generate tailored 'rational' vaccines against a smaller subset of antigens. For example, restricting a vaccine to a protein antigen will favor a T-cell response over a humoral B-cell response.

Methods of the invention can be conducted using the above described cartridges. Further described are methods performed without the cartridges. Such methods may involve introducing magnetic particles to a biological sample (e.g., a tissue or body fluid sample). The sample is incubated to allow the particles to bind to pathogens in the sample, and a magnetic field is applied to capture pathogen/magnetic particle complexes on a surface.

Optionally, the surface can be washed with a wash solution that reduces particle aggregation, thereby isolating pathogen/magnetic particle complexes. A particular advantage of compositions of the disclosure is for capture and isolation of bacteria and fungi directly from blood samples at low concentrations that are present in many clinical samples (as low as 1 CFU/mL of bacteria in a blood sample). Preferably, the magnetic particles comprise a pathogen binding element that has one or more magnetic particles attached to it.

In certain aspects, methods of the invention involve obtaining a heterogeneous sample including a pathogen, exposing the sample to a cocktail including a plurality of sets of magnetic particles, members of each set being conjugated to an antibody specific for a pathogen, and separating particle bound pathogen from other components in the sample. Methods of the invention may further involve characterizing the pathogen. Characterizing may include identifying the pathogen by any technique known in the art. Exemplary techniques include sequencing nucleic acid derived from the pathogen or amplifying the nucleic acid.

The antibodies conjugated to the particles may be either monoclonal or polyclonal antibodies. Methods of the invention may be used to isolate pathogen from heterogeneous sample. In particular embodiments, the heterogeneous sample is a blood sample.

Since each set of particles is conjugated with antibodies have different specificities for different pathogens, compositions of the invention may be provided such that each set of antibody conjugated particles is present at a concentration designed for detection of a specific pathogen in the sample. In certain embodiments, all of the sets are provided at the same concentration. Alternatively, the sets are provided at different concentrations.

To facilitate detection of the different sets of pathogen/magnetic particle complexes the particles may be differently labeled. Any detectable label may be used with compositions of the invention, such as fluorescent labels, radiolabels, enzymatic labels, and others. In particular embodiments, the detectable label is an optically-detectable label, such as a fluorescent label. Exemplary fluorescent labels include Cy3, Cy5, Atto, cyanine, rhodamine, fluorescien, coumarin, BODIPY, alexa, and conjugated multi-dyes.

Methods of the invention may be used to isolate only gram positive bacteria from a sample. Alternatively, methods of the invention may be used to isolate only gram negative bacteria from a sample. In certain embodiments, methods of the invention are used to isolate both gram positive and gram negative bacteria from a sample. In still other embodiments, methods isolate specific pathogen from a sample. Exemplary bacterial species that may be captured and isolated by methods of the invention include E. coli, Listeria, Clostridium, Mycobacterium, Shigella, Borrelia, Campylobacter, Bacillus, Salmonella, Staphylococcus, Enterococcus, Pneumococcus, Streptococcus, and a combination thereof.

Methods of the invention are not limited to isolating pathogens from a body fluid. Methods of the invention may be designed to isolate other types of target analytes, such as fungi, protein, a cell, a virus, a nucleic acid, a receptor, a ligand, or any molecule known in the art.

Compositions used in methods of the invention may use any type of magnetic particle, which are described above in the section entitled "Magnetic Particles." Another aspect of the invention provides methods for isolating pathogen from a heterogeneous sample, that involve labeling pathogen from a biological sample with a cocktail including a plurality of sets of magnetic particles, members of each set being conjugated to an antibody specific for a pathogen, exposing the sample to a magnetic field to isolate pathogen conjugated to the particles, and isolating particle bound pathogen from other components of the sample. Methods of the invention may further involve eluting pathogens from the particles.

Methods of the invention may further involve characterizing the pathogen. Characterizing may include identifying the pathogen by any technique known in the art. Exemplary techniques include conducting an assay to determine the expression profile of nucleic acid derived from the pathogen or amplifying the nucleic acid. For example, after capture, the pathogen is lysed and messenger RNA transcripts are converted into cDNA. The cDNA is then hybridized to DNA microarrays to obtain both the identity of the transcript and the relative expression level. Such microarray analysis is useful for studying the physiology of the pathogen as exposed to, for example, human blood and the human immune system. The generated cDNA can also be sub-cloned into a bacterial expression vector to construct a cDNA library.

In certain aspects, methods of the invention involve introducing magnetic particles including a target-specific binding moiety to a body fluid sample in order to create a mixture, incubating the mixture to allow the particles to bind to a target, applying a magnetic field to capture target/magnetic particle complexes on a surface, thereby isolating target/magnetic particle complexes. Methods of the invention may further involve washing the mixture in a wash solution that reduces particle aggregation. Certain fundamental technologies and principles are associated with binding magnetic materials to target entities and subsequently separating by use of magnet fields and gradients. Such fundamental technologies and principles are known in the art and have been previously described, such as those described in Janeway (Immunobiology, 6th edition, Garland Science Publishing), the content of which is incorporated by reference herein in its entirety.

Methods of the invention can be conducted with any type of sample, and exemplary types of samples are described above. The sample may be mixed with magnetic particles having a particular magnetic moment and also including a target-specific binding moiety to generate a mixture that is allowed to incubate such that the particles bind to a target in the sample, such as a bacterium in a blood sample. The mixture is allowed to incubate for a sufficient time to allow for the particles to bind to the target. The process of binding the magnetic particles to the targets associates a magnetic moment with the targets, and thus allows the targets to be manipulated through forces generated by magnetic fields upon the attached magnetic moment.

In general, incubation time will depend on the desired degree of binding between the target and the magnetic particles (e.g., the amount of moment that would be desirably attached to the target), the amount of moment per target, the amount of time of mixing, the type of mixing, the reagents present to promote the binding and the binding chemistry system that is being employed. Incubation time can be anywhere from about 5 seconds to a few days. Exemplary incubation times range from about 10 seconds to about 2 hours. Binding occurs over a wide range of temperatures, generally between 15 °C and 40 °C.

Methods of the invention are performed with magnetic particles having a magnetic moment that allows for isolation of as low as 1 CFU/mL of bacteria in the sample. Production of magnetic particles is shown for example in Giaever (U.S. 3,970,518), Senyi et al. (U.S. 4,230,685), Dodin et al. (U.S. 4,677,055), Whitehead et al. (U.S. 4,695,393), Benjamin et al. (U.S. 5,695,946), Giaever (U.S. 4,018,886), Rembaum (U.S. 4,267,234), Molday (U.S. 4,452,773), Whitehead et al. (U.S. 4,554,088), Forrest (U.S. 4,659,678), Liberti et al. (U.S. 5,186,827), Own et al. (U.S. 4,795,698), and Liberti et al. (WO 91/02811).

In particular embodiments, the target-specific binding moiety is an antibody, such as an antibody that binds a particular bacterium. General methodologies for antibody production are described above in the section entitled "Methods of Antibody Production." Methods for attaching the target-specific binding moiety to the magnetic particle are known in the art, and are summarized in the section entitled "Binding Moiety". After binding of the magnetic particles to the target in the mixture to form target/magnetic particle complexes, a magnetic field is applied to the mixture to capture the complexes on a surface. Components of the mixture that are not bound to magnetic particles will not be affected by the magnetic field and will remain free in the mixture. Methods and apparatuses for separating target/magnetic particle complexes from other components of a mixture are known in the art. Certain apparatuses are described above. Other apparatuses are known in the art. For example, a steel mesh may be coupled to a magnet, a linear channel or channels may be configured with adjacent magnets, or quadrapole magnets with annular flow may be used. Other methods and apparatuses for separating target/magnetic particle complexes from other components of a mixture are shown in Rao et al. (U.S. 6,551,843), Liberti et al. (U.S. 5,622,831), Hatch et al. (U.S. 6,514,415), Benjamin et al. (U.S. 5,695,946), Liberti et al. (U.S. 5,186,827), Wang et al. (U.S. 5,541,072), Liberti et al. (U.S. 5,466,574), and Terstappen et al. (U.S. 6,623,983).

In certain embodiments, the magnetic capture is achieved at high efficiency by utilizing a flow-through capture cell with a number of strong rare earth bar magnets placed perpendicular to the flow of the sample. When using a flow chamber with flow path cross-section 0.5 mm x 20 mm (h x w) and 7 bar NdFeB magnets, the flow rate could be as high as 5 mL/min or more, while achieving capture efficiency close to 100%.

In certain embodiments, the presence of magnetic particles that are not bound to target analytes and non-specific target entities on the surface that includes the target/magnetic particle complexes interferes with the ability to successfully detect the target of interest. The magnetic capture of the resulting mix, and close contact of magnetic particles with each other and labeled targets, result in the formation of aggregates that are hard to dispense and which might be resistant or inadequate for subsequent processing or analysis steps. Further, with addition of excess magnetic particles to the sample, a large number of particles may accumulate in the areas of high gradients, and thus a magnetically bound target analyte may likely be in the body of the accumulation of particles as opposed to the desired location adjacent the functionalized surface where specific binding may occur. Ignoring intra-bead forces (those forces associated with the magnetic field distribution of the individual beads and the forces these fields and associated gradients have on other beads), the beads may accumulate into large amorphous piles. Such intra-label forces do occur, and thus the aggregates of beads tend to exist in chains and long linear aggregates that are aligned with the 'field lines' of the magnetic trap pieces.

In certain embodiments, methods of the invention address this problem by applying alternating magnetic fields to the sample as it flows through the channel. In such embodiment, the flow chamber can be lined with second moieties specific to the target/magnetic particle complex. Alternating magnetic fields are then used to bring the target/magnetic particle complex in close proximity to the second moiety lined flow chamber and apart from other components in the sample.

The frequency of the alternating magnetic field is selected such that the free magnetic nanoparticles cannot transverse the whole distance between top and bottom of the flow cell before the direction of the magnetic field is changed, causing nanoparticles to move in the opposite direction. Therefore, a majority of free nanoparticles will not come into close contact with active surfaces of the flow cell and will be washed away by liquid flow. Labeled target, due to higher magnetic moment, have higher velocity in the magnetic field and will reach a surface of the flow cell before change of the magnetic field, thus coming into close contact with the surface. This, in turn, will result in a specific binding event and result in a specific capture of the target analyte in the sample (such as a bacterium or other rare cell) to the surface coated with a second moiety. Components of the mixture that are not bound to magnetic particles will not be affected by the magnetic field and will remain free in the mixture.

The second target-specific moiety may be the same or different from the first target-specific moiety. The second moiety may be attached to the surface of the flow channel by methods described above relating to attaching first target-specific moieties to magnetic particles.

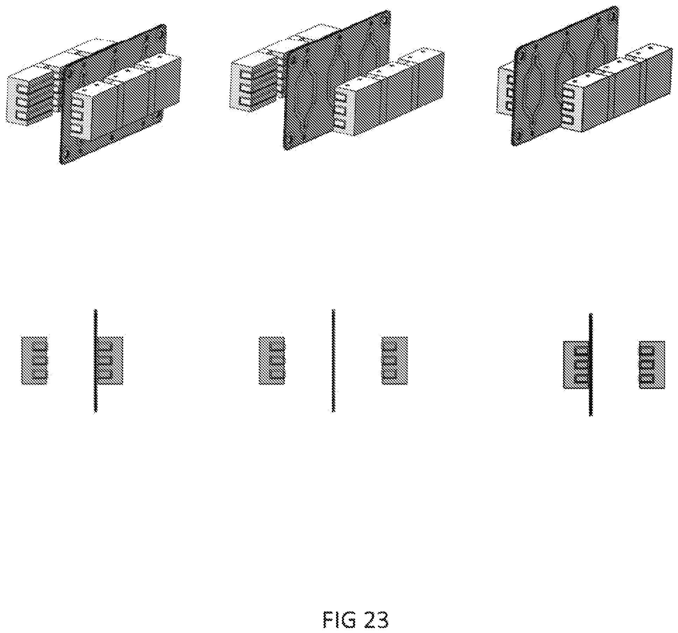

FIG. 23 provides one exemplary configuration of a flow cell and first and second sets of magnets for generating alternating magnetic fields. One of skill in the art will recognize that it is only an exemplary embodiment and that the separation can be achieved without using magnetic fields, as described in other embodiments throughout the application. This figure shows that the flow cell is positioned between the first and second sets of magnetics. Either movement of the flow cell or movement of the magnets brings the flow cell closer to one set of magnets and further from the other set of magnets. Subsequent movement brings the flow cell within proximity of the other set of magnets. Such movements generate alternating magnetic fields within the channels of the flow cell that are felt by the unbound magnetic particles and the target/magnetic particle complexes. In one embodiment, a flow cell may be about 15 mm wide and about 15 mm long, with a lead-in region and a lead-out section, and a height of about 0.5 mm (FIG. 23). A flow rate for such a cell may be about 100 μl/min, about 1 mL/min, about 10 mL/min, or from about 100 μL/min to about 10 mL/min or other ranges therein. A magnetic configuration may be an array of magnets, for example, an array of 7 bar magnets, or 5 bar magnets, or 3 bar magnets (FIG. 23). Magnets may be configured with alternating magnet poles facing one another, n-n, s-s, etc., with the pole face being normal to the array's rectangular face in this embodiment.

In a flowing system, successive encounters of unbound magnetic particles with a surface of a flow channel, without a resulting binding event, will allow the unbound magnetic particles to travel through the system and subsequently out of the cell. The cycling of the magnetic bar trap assemblies may be optimized based on the flow characteristics of the target(s) of interest. The expression of force on a magnetic moment and of terminal velocity for such target(s) is the following:

$$m \text{ dot } (\text{del } B) = F \qquad \text{Equation 1}$$

$$vt = -F/(6*p*n*r) \qquad \text{Equation 2}$$

where n is the viscosity, r is the bead diameter, F is the vector force, B is the vector field, and m is the vector moment of the bead.

A characteristic transit time across the height of the cell may be established. An efficient frequency of the alternating magnetic attractors, such that many surface interactions may be established prior to the exit from the flow cell, may be established. In certain embodiments, the transit time can be substantially different for the target of interest versus the unbound magnetic particles, or non-specific bound non-target. In such an embodiment, the target can be ensured to interact with surface a maximal amount of times, while the unbound magnetic particles or non-target can interact a minimal number of times, or not at all.

Because of the magnetizing characteristics of the particles, the unbound magnetic particles may form aggregates, which may be in the form of linear chains or clumps. This may be the case at high concentrations of beads. At all concentrations, the unbound magnetic particles may exhibit spatial poison statistics, and there is some probability that there will be a neighboring bead close enough to be captured by the forces associated with the magnetic field of the beads themselves. By using alternating magnetic fields, methods of the invention break up these linear aggregates, particularly when the spatial gradient field from the trap magnets is shifted faster than the unbound magnetic particles can move mutually to reorient to the new distribution of trap gradient. Particles organized in chains, with N-S axis co-aligned, may quickly be subjected to an external field that produces particle moments with the N-S poles shifted by 90°, and may produce very strong intra-particle repulsive forces. Transverse motion of the trap magnets serves this purpose in concert with, or as a discrete step in addition to, the alternation of the trapping magnets from one surface to the other.

In the optimization of the cycling timing of the trap magnets, the flow characteristics of the cell may be considered along with the spatial distribution of the gradient of the trap magnets. Flow characteristics may dictate the transport of the magnetic materials from entrance to exit of the cell, so that parabolic flow, plug flow, or any particular flow characteristic may be considered to facilitate obtaining desired deposition patterns and desired interactions with the surfaces of interest.

In certain embodiments, it may be desirable in various applications to maximize the encounters of the target/magnetic particle complexes with the functionalized surface of the channel, to minimize interference with the unbound magnetic particles, and/or to minimize adhesion of the unbound magnetic particles and non-specific materials to the surface. It may be advantageous to produce an array of pipes, or tubes, through which the flow of the sample materials may flow. By way of example, a 125 mm x 15 mm x 0.5 mm cell volume may be filled with tubes, longitudinally aligned with the cell flow direction, such that there is a great increase in the functionalized surface area and a limitation on the number of unbound magnetic particles that may interact and impede in the encounter of the target with the surface. Planar structures may be used for this purpose, in which the cell volume is constructed with multiple layers of smaller flow channels such that the surface area is increased and the number of unbound magnetic particles available to impede the target on its way to the surface is decreased. In this embodiment, the general approach of cycling the trap magnets is similar to that described above, but variables such as time constants, amplitudes and gradient field distributions, for example, are optimized for the particular situation. Similarly, in the case of transverse trap motion for the breaking-up of aggregates, the general approach is similar to that described above.

It may be desirable to shield the portion of the sample flow outside the trap cell from fringing magnetic field so that magnetic material does not have the opportunity to self-aggregate prior to entering the strong field and gradient zone of the trap. The magnetic materials and labeled target may also be trapped in flow tubes and other fluidic structures through magnetic forces in undesired areas. Shielding can be accomplished by the appropriate design of the trap magnets, for example, by managing the 'return path' of the field, and/or by using high permeability materials to capture and channel the field to minimize fringing field exposure.

The above described type of magnetic separation produces efficient capture of a target and the removal of a majority of the remaining components of a sample mixture. However, such a process may produce a sample that contains a percent of magnetic particles that are not bound to targets, as well as non-specific target entities. Non-specific target entities may for example be bound at a much lower efficiency, for example 1% of the surface area, while a target of interest might be loaded at 50% or nearly 100% of the available surface area or available antigenic cites. However, even 1 % loading may be sufficient to impart force necessary for trapping in a magnetic gradient flow cell or sample chamber.

The presence of magnetic particles that are not bound to targets and non-specific target entities on the surface that includes the target/magnetic particle complexes may interfere with the ability to successfully detect the target of interest. The magnetic capture of the resulting mix, and close contact of magnetic particles with each other and bound targets, result in the formation of aggregate that is hard to dispense and which might be resistant or inadequate for subsequent processing or analysis steps. In order to remove magnetic particles that are not bound to targets and non-specific target entities, methods of the invention may further involve washing the surface with a wash solution that reduces particle aggregation, thereby isolating target/magnetic particle complexes from the magnetic particles that are not bound to targets and non-specific target entities. The wash solution minimizes the formation of the aggregates.

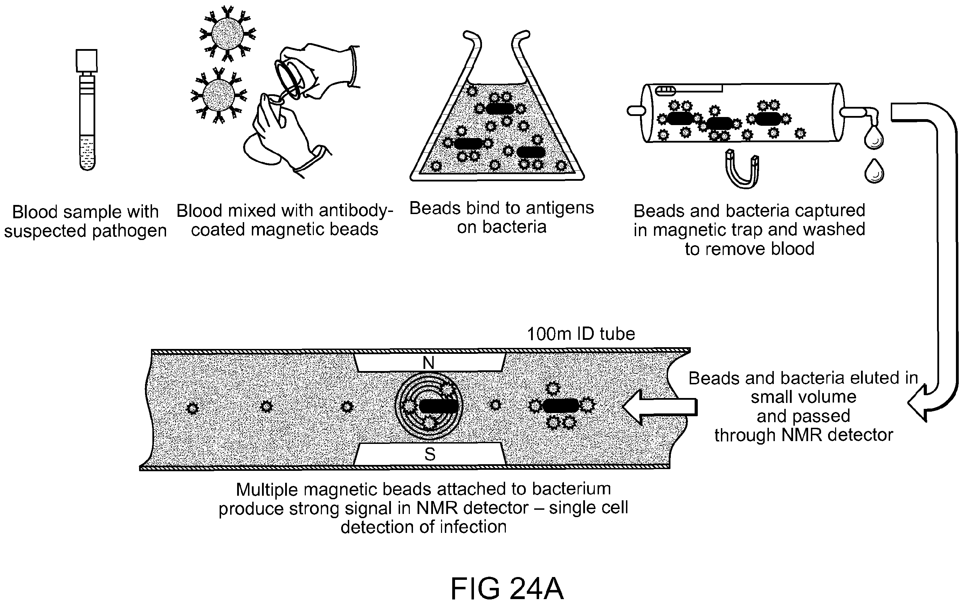

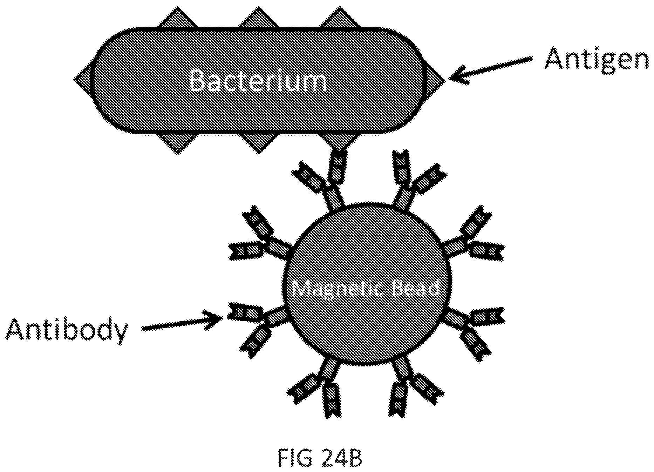

FIG. 24A provides an exemplary process chart for implementation of methods of the invention for separation of bacteria from blood. Sample is collected in sodium heparin tube by venipuncture, acceptable sample volume is 1 - 10 mL. Superparamagnetic particles having target-specific binding moieties are added to the sample, followed by incubation on a shaking incubator at 37°C for 30 -120 min. FIG. 24B provides an exemplary view of the target/magnetic particle complex, illustrating the binding between the two species.

Once the target/magnetic particle complexes are isolated, the target may be analyzed by a multitude of existing technologies, such as miniature NMR, Polymerase Chain Reaction (PCR), mass spectrometry, fluorescent labeling and visualization using microscopic observation, fluorescent in situ hybridization (FISH), growth-based antibiotic sensitivity tests, and variety of other methods that may be conducted with purified target without significant contamination from other sample components. These methods are described above in the preceding sections. In a preferred embodiment, the target is analyzed in order to identify the genes expressed by the target.

In certain embodiments, after targets have been obtained from the sample, it is preferable to lyse targets in order to isolate nucleic acids that can be found within the targets. Such targets may be a virus, a microorganism (e.g., bacteria or fungi) or cells (such as normal or abnormal cells, such as cancerous cells). Once the target/magnetic particle complexes have been captured, the process of lysis can be initiated. It will be appreciated by one skilled in the art that depending on the type of technique of analysis to be performed on the analyte, lysing can be applied to the method, or the process of lysing can be omitted. Lysing of the target/magnetic particle complexes occurs without disturbing the binding between the target and the magnetic particle, or that the target/magnetic particle complex is maintained during the lysing step. Lysis is described above.

The contents of the target are purified by standard methods of nucleic acid purification. Cellular extracts can be subjected to other steps to drive nucleic acid isolation toward completion by, e.g., differential precipitation, column chromatography, extraction with organic solvents and the like. Extracts then may be further treated, for example, by filtration and/or centrifugation and/or with chaotropic salts such as guanidinium isothiocyanate or urea or with organic solvents such as phenol and/or $HCCl_3$ to denature any contaminating and potentially interfering proteins. The nucleic acid can also be resuspended in a hydrating solution, such as an aqueous buffer. The nucleic acid can be suspended in, for example, water, Tris buffers, or other buffers.

Methods of detecting levels of gene products (e.g., RNA or protein) are known in the art. Commonly used methods known in the art for the quantification of mRNA expression in a sample include northern blotting and in situ hybridization (Parker & Barnes, Methods in Molecular Biology 106:247 283 (1999), the contents of which are incorporated by reference herein in their entirety); RNAse protection assays (Hod, Biotechniques 13:852 854 (1992), the contents of which are incorporated by reference herein in their entirety); and PCR-based methods, such as reverse transcription polymerase chain reaction (RT-PCR) (Weis et al., Trends in Genetics 8:263 264 (1992), the contents of which are incorporated by reference herein in their entirety). Alternatively, antibodies may be employed that can recognize specific duplexes, including RNA duplexes, DNA-RNA hybrid duplexes, or DNA-protein duplexes. Other methods known in the art for measuring gene expression (e.g., RNA or protein amounts) are shown in Yeatman et al. (U.S. patent application number 2006/0195269), the content of which is hereby incorporated by reference in its entirety.

In some embodiments, messenger RNA transcripts are converted into its complementary DNA sequence for analysis. Methods of the invention provide for converting RNA to cDNA. RNA can be converted to cDNA using any method known in the art. Generally, RNA to cDNA conversion steps include purifying messenger RNA (mRNA) using poly-A selection, performing reverse transcriptase and performing oligonucleotide-primed synthesis of cDNA.

RNA is usually converted into first strand cDNA enzymatically by reverse transcriptase (RT), a RNA-dependent DNA polymerase. Exemplary reverse transcriptase (RT) includes, but not limited to, the Moloney murine leukemia virus (M-MLV) RT as described in U.S. Pat. No. 4,943,531, a mutant form of M-MLV-RT lacking Rnase H activity as described in U.S. Pat. No. 5,405,776, human T-cell leukemia virus type I (HTLV-I) RT, bovine leukemia virus (BLV) RT, Rous sarcoma virus (RSV) RT, Avian Myeloblastosis Virus (AMV) RT and human immunodeficiency virus (HIV) RT. RTs suitable for this purpose may also be extracted from their natural hosts. Alternatively, RTs can be obtained commercially or isolated from host cells that express high levels of recombinant forms of the enzymes by methods known to those of skill in the art. The particular manner of obtaining the reverse transcriptase may be chosen based on factors such as convenience, cost, availability and the like.

Reverse transcriptase can extend the free end of an oligonucleotide (or a primer) that forms a stable base pairing with the target RNA molecule. Under most conditions, RT enzymes can produce cDNA molecules without the supply of exogenous primers. Alternatively, exogenous primers may be used. Two types of exogenous primers, random primers and specific primers, may be added to the reaction to facilitate the cDNA synthesis. Random primers, which have defined length but no defined sequence, can be used to prime the conversion of RNA to cDNA without discrimination of RNA species. The length of random primers is usually between 2 and 25 nucleotides (nt), but more often between 5 and 10 nt. The most commonly used is the random hexamers (6 nt). Specific primers, which have defined length and defined nucleotide sequence, may also be used to synthesize cDNA from a defined sub-population of RNA. An example of such specific primers is the oligo dT primers. Oligo dTs primers are a short sequence of deoxy-thymine nucleotides that are tagged as complementary primers which bind to the poly-A tail providing a free 3'-OH end, a characteristic of most messenger RNA in cells, which can be extended by reverse transcriptase to create the complementary DNA strand. The length of the oligo dT primers may be between 10 to 40 nt, between 15 and 25 nt, or about 18 nt. Another example of a specific primer is the gene-specific primer. A gene-specific primer may have a sequence complementary to that of a distinct RNA. Preferably, the gene-specific primer has a sequence substantially complementary to that of a distinct RNA. In some embodiments, a gene-specific primer primes the synthesis of cDNA from one unique sequence within a RNA molecule that corresponds to one single gene.

Exogenous primers can be synthesized according to conventional oligonucleotide chemistry methods, in which the nucleotide units may be: (A) solely nucleotides found in naturally occurring DNA and RNA, e.g., adenine, cytosine, guanine, thymine and uracil; or (B) solely nucleotide analogs that are capable of base pairing under hybridization conditions in the course of DNA synthesis such that they function as the nucleotides described in (A), e.g., inosine, xanthine, hypoxanthine, 1,2-diaminopurine and the like; or (C) any combination of the nucleotides described in both (A) and (B).

The buffer necessary for first strand cDNA synthesis may be purchased commercially from various sources, such as SuperArray, Promega, Invitrogen, Clontech, Amersham. These buffers have a pH ranging from 6 to 9, with 10-200 mM of Tris-HCl or HEPES. Other salts may include NaCl, KCl, MgCl2, Mg (OAc)2, MnCl2, Mn(OAc)2 etc., at concentrations ranging from 1-200 mM. Additional reagents such as reducing agents (DTT), detergents (TritonX-100), albumin and the like may be supplemented in the buffer. Chemical compound or polymers, such as DMSO, poly-lysine, betaine, and the like, may be added to the buffer to prevent RNA from forming secondary structures. Depending on the particular nature of the assay, a combination of the above mentioned reagents might be chosen to limit endogenous priming during reverse transcription.

Deoxyribonucleoside triphosphates (dNTPs) necessary for first strand cDNA synthesis through reverse transcription of RNAs may be purchased commercially from various sources, such as SuperArray, Promega, Invitrogen, Clontech, Amersham. In the reaction, dNTPs may include nucleotides that are commonly found in DNA, e.g. dATP, dGTP, dCTP dTTP and dUTP; analogs of above mentioned nucleotides that are less frequently found in nature, such as those with ribose moieties like inosine, xanthine, hypoxanthine; and combinations of the nucleotides commonly found in DNA and their analogs. The combination of nucleotide analogs may be helpful separating the newly synthesized DNA from the genomic DNA that may co-purify with the total RNA. Derivatives of inosine are an example commonly used for this purpose. Newly synthesized dITP-containing DNA has a lower melting temperature than the corresponding natural DNA, such as genomic DNA (Auer et al. Nucleic Acids Res. 1996;24:5021-5025, U.S. Pat. No. 5,618,703), and (Levy D D and Teebor G W. Nuc. Acids Res. 1991;19(12):3337-3343; Warren R A. Annu. Rev. Microbiol. 1980;34:137-158). Another unconventional nucleotide affecting the Tm of the newly synthesized DNA product is hydroxymethyl dUTP (HmdUTP), which occurs naturally in phage SP01 genomic DNA in place of dTTP. The melting temperature of HmdUTP-containing DNA is 10°C lower than normal DNA. (Levy D D and Teebor G W. Nuc. Acids Res. 1991; 19(12):3337-3343).

The resulting single stranded cDNA is converted into a double stranded DNA with the help of a DNA polymerase. However, for DNA polymerase to synthesize a complementary strand a free 3'-OH end is needed. This is provided by the single stranded cDNA itself by generating a hairpin loop at the 3' end by coiling on itself. The polymerase extends the 3'-OH end and later the loop at 3' end is opened by the scissoring action of an S1 nuclease.

In addition to the above described techniques for conversion of RNA into double stranded RNA, other methods of conversion are described in detail in Klickstein et al., 2001. Conversion of mRNA into Double-Stranded cDNA. Current Protocols in Molecular Biology. 29:5.5.1-5.5.14, the contents of which is incorporated by reference in its entirety. Klickstein describes two protocols for converting RNA into double stranded cDNA. One protocol describes a method for making blunt-ended cDNA that can then be ligated to linkers for subsequent cloning into a unique restriction site, and the other the other protocol is a variation that requires fewer enzymatic manipulations and allows construction of directional cDNA libraries.

In addition, kits are commercially available for conversion of RNA to cDNA from, for example, Illumina (San Diego, CA), Life Technologies (Foster City, CA), and Qiagen, (Valencia, CA). User Guides that describe in detail the protocol(s) to be followed are usually included in all these kits.

In certain embodiments, reverse transcriptase PCR (RT-PCR) is used to measure gene expression. RT-PCR is a quantitative method that can be used to compare mRNA levels in different sample populations to characterize patterns of gene expression, to discriminate between closely related mRNAs, and to analyze RNA structure.

The first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, Calif., USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TAQMAN PCR (gene expression assay, commercially available by Life Technologies company) typically utilizes the 5'-nuclease activity of Taq polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TAQMAN RT-PCR (gene expression assay, commercially available by Life Technologies company) can be performed using commercially available equipment, such as, for example, ABI PRISM 7700 SEQUENCE DETECTION SYSTEM (sequence detection system, commercially available from Perkin-Elmer-Applied Biosystems, Foster City, Calif., USA), or Lightcycler (Roche Molecular Biochemicals, Mannheim, Germany). In certain embodiments, the 5' nuclease procedure is run on a real-time quantitative PCR device such as the ABI PRISM 7700 SEQUENCE DETECTION SYSTEM (sequence detection system, commercially available from Perkin-Elmer-Applied Biosystems, Foster City, Calif., USA). The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle (Ct).

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin. For performing analysis on pre-implantation embryos and oocytes, Chuk is a gene that is used for normalization.

A more recent variation of the RT-PCR technique is the real time quantitative PCR, which measures PCR product accumulation through a dual-labeled fluorigenic probe (i.e., TAQMAN (gene expression assay, commercially available by Life Technologies company) probe). Real time PCR is compatible both with quantitative competitive PCR, in which internal competitor for each target sequence is used for normalization, and with quantitative comparative PCR using a normalization gene contained within the sample, or a housekeeping gene for RT-PCR. For further details see, e.g. Held et al., Genome Research 6:986 994 (1996).

In another embodiment, a MASSARRAY (DNA mass array, commercially available by Sequenom, Inc.) based gene expression profiling method is used to measure gene expression. In the MASSARRAY (DNA mass array, commercially available by Sequenom, Inc.) based gene expression profiling method, developed by Sequenom, Inc. (San Diego, Calif.) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry (MALDI-TOF MS) analysis. The cDNA present in the reaction is then quantified by analyzing the ratios of the peak areas in the mass spectrum generated. For further details see, e.g. Ding and Cantor, Proc. Natl. Acad. Sci. USA 100:3059 3064 (2003).

Further PCR-based techniques include, for example, differential display (Liang and Pardee, Science 257:967 971 (1992)); amplified fragment length polymorphism (iAFLP) (Kawamoto et al., Genome Res. 12:1305 1312 (1999)); BEADARRAY technology (microarray plateform, commercially available by Illumina Inc.) (Illumina, San Diego, Calif.; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex100 LabMAP system and multiple color-coded microspheres (Luminex Corp., Austin, Tex.) in a rapid assay for gene expression (Yang et al., Genome Res. 11:1888 1898 (2001)); and high coverage expression profiling (HiCEP) analysis (Fukumura et al., Nucl. Acids. Res. 31(16) e94 (2003)).

In certain embodiments, differential gene expression can also be identified, or confirmed using a microarray technique. In this method, polynucleotide sequences of interest (including cDNAs and oligonucleotides) are plated, or arrayed, on a microchip substrate. The arrayed sequences are then hybridized with specific DNA probes from cells or tissues of interest. Methods for making microarrays and determining gene product expression (e.g., RNA or protein) are shown in Yeatman et al. (U.S. patent application number 2006/0195269).

In a specific embodiment of the microarray technique, PCR amplified inserts of cDNA clones are applied to a substrate in a dense array, for example, at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pair-wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106 149 (1996). Microarray analysis can be performed by commercially available equipment, following manufacturer's protocols, such as by using the Affymetrix GenChip technology, or Incyte's microarray technology.

Alternatively, protein levels can be determined by constructing an antibody microarray in which binding sites comprise immobilized, preferably monoclonal, antibodies specific to a plurality of protein species encoded by the cell genome. Preferably, antibodies are present for a substantial fraction of the proteins of interest. Methods for making monoclonal antibodies are well known (see, e.g., Harlow and Lane, 1988, ANTIBODIES: A LABORATORY MANUAL, Cold Spring Harbor, N.Y., which is incorporated in its entirety for all purposes). In one embodiment, monoclonal antibodies are raised against synthetic peptide fragments designed based on genomic sequence of the cell. With such an antibody array, proteins from the cell are contacted to the array, and their binding is assayed with assays known in the art. Generally, the expression, and the level of expression, of proteins of diagnostic or prognostic interest can be detected through immunohistochemical staining of tissue slices or sections.

In other embodiments, Serial Analysis of Gene Expression (SAGE) is used to measure gene expression. Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484 487 (1995); and Velculescu et al., Cell 88:243 51 (1997).

In other embodiments Massively Parallel Signature Sequencing (MPSS) is used to measure gene expression. This method, described by Brenner et al., Nature Biotechnology 18:630 634 (2000), is a sequencing approach that combines non-gel-based signature sequencing with in vitro cloning of millions of templates on separate 5 $\mu$m diameter microbeads. First, a microbead library of DNA templates is constructed by in vitro cloning. This is followed by the assembly of a planar array of the template-containing microbeads in a flow cell at a high density (typically greater than 3 x 106 microbeads/cm2). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

Immunohistochemistry methods are also suitable for detecting the expression levels of the gene products of the present invention. Thus, antibodies (monoclonal or polyclonal) or antisera, such as polyclonal antisera, specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

In certain embodiments, a proteomics approach is used to measure gene expression. A proteome refers to the totality of the proteins present in a sample (e.g. tissue, organism, or cell culture) at a certain point of time. Proteomics includes, among other things, study of the global changes of protein expression in a sample (also referred to as expression proteomics). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

In some embodiments, mass spectrometry (MS) analysis can be used alone or in combination with other methods (e.g., immunoassays or RNA measuring assays) to determine the presence and/or quantity of the one or more biomarkers disclosed herein in a biological sample. In some embodiments, the MS analysis includes matrix-assisted laser desorption/ionization (MALDI) time-of-flight (TOF) MS analysis, such as for example direct-spot MALDI-TOF or liquid chromatography MALDI-TOF mass spectrometry analysis. In some embodiments, the MS analysis comprises electrospray ionization (ESI) MS, such as for example liquid chromatography (LC) ESI-MS. Mass analysis can be accomplished using commercially-available spectrometers. Methods for utilizing MS analysis, including MALDI-TOF MS and ESI-MS, to detect the presence and quantity of biomarker peptides in biological samples are known in the art. See for example U.S. Pat. Nos. 6,925,389; 6,989,100; and 6,890,763 for further guidance.

Methods of the invention also provide for generating a cDNA library from the total RNA extracted from the pathogen. Methods known in the art for cDNA library generation are suitable for use in methods of the invention, for example, use of a olgio-(DT) primed and directional cloned strategy for generating cDNA libraries. Similarly, methods for cDNA library screening to identify cDNA library clones representing genes of interest are also widely known, and include, for example, homology screening and DNA/protein interaction screens, and various forms of expression screening such as antibody-based immunoscreening, protein/protein interaction screening, and screenings based on functional assays. Methods and reagents for library construction and screening are available in a variety of sources, including but not limited to, Ausubel et al. (eds.), Current Protocols in Molecular Biology, Vol. 1-4, John Wiley & Sons, Inc., New York (1994) and Sambrook et al. (eds.), Molecular Cloning: A Laboratory Manual, Second Edition, Vol. 1-3, Cold Spring Harbor Laboratory Press, NY (1989).

In certain aspects, it may be preferable to detect the presence of the target prior to determining the expression profile of the target. Because the assay is capable of detecting a single target cell from a large volume of sample, it can be beneficial to know how much target there is for prior to expression analysis. For example, if there is only a small amount of target present, one may choose a more sensitive assay to determine the gene expression profile. In such embodiments, the following techniques may be used to determine the presence and/or amount of captured target within the sample that can be analyzed for gene expression.

In one embodiment, captured bacteria is removed from the magnetic particles to which they are bound and the processed sample is mixed with fluorescent labeled antibodies specific to the bacteria or fluorescent Gram stain. After incubation, the reaction mixture is filtered through 0.2 $\mu$m to 1.0 $\mu$m filter to capture labeled bacteria while allowing majority of free beads and fluorescent labels to pass through the filter. Bacteria is visualized on the filter using microscopic techniques, e.g. direct microscopic observation, laser scanning or other automated methods of image capture. The presence of bacteria is detected through image analysis. After the positive detection by visual techniques, the bacteria can be further characterized using PCR or genomic methods.

In a preferred embodiment, the target is analyzed in order to identify genes or fragments of nucleic acids expressed or contained within the target. DNA sequencing is well known in the art and has been described in detail in the preceding sections. In this embodiment, captured bacteria are lysed without first separating the bacteria from the magnetic particles. The lysate or analyte is then eluted from the magnetic particles and DNA contained within the lysate/analyte is bound to DNA extraction resin. After washing of the resin, the bacterial DNA is eluted and used in quantitative RT-PCR to detect the presence of a specific species, and/or, subclasses of bacteria, through nucleic acid identification or nucleic acid - nucleic acid comparison, such as DNA-DNA comparison, by means well known in the art. See for example IJSEM, Rademaker, et al., March 2000, 50:2, p. 665-677, which is incorporated by reference, and describes using repetitive sequence based (rep)-PCR and AFLP genomic fingerprinting to DNA-DNA hybridization studies to identify and classify known strains of microorganisms. Comparison analysis allows for the determination and identification of a known bacterium, pathogen, microorganism, microbe, prokaryote, or virus.

In another preferred embodiment, after the lysate/analyte is eluted, genetic variations or mutations within a species are identified by nucleic acid - nucleic acid comparison. It is well known in the art that pathogens, microorganisms, viruses, and bacteria mutate or can change genetically. Identifying these mutations is desired for further identification and determination of genetic closeness, along with determination of genetic distances between species. See for example J. Clin. Microbiol. December 2003 vol. 41 no. 12 5456-5465, which discloses guidelines for classification by using sequences of rRNA and protein-coding genes.

Furthermore, in another preferred embodiment, after the lysate/analyte is eluted nucleic acid quantification can be completed by means known in the art, as described in Appl. Environ. Microbiol. January 2002 vol. 68 no. 1 245-253, which discloses utilizing PCR and statistics to quantify bacterial populations. Quantification allows for not only identification, but allows for determination of the amount of bacterial or pathogenic species present in the sample. In particular applications, quantification is needed to determine the level of infection or contamination within a sample, and therefore, identification alone would be insufficient.

In another preferred embodiment, the captured target, such as a virus, could be lysed without first being eluted from the magnetic particles. The lysate/analyte is then eluted from the magnetic particles and DNA contained within the lysate/analyte is bound to DNA extraction resin. After washing of the resin, the DNA is eluted and used in quantitative RT-PCR to identify and quantify the presence of a virus in a sample. By known methods in the art, such as DNA microarrays (Wang et. al., PLOS, November 17, 2003, DOI: 10.1371/journal.pbio. 0000002). viral infections can be identified to determine which viruses are present in the sample. Furthermore, this embodiment can be used to identify whether the sample contains a pathogen or virus associated with a known infectious disease. In addition, the present invention can be employed for the means of classification of bacterial or viral species, including microorganisms and pathogens. This embodiment would be preferred for creating and establishing a classification system of the particular microorganisms or pathogens that are present in a particular group of samples.

In another preferred embodiment, after elution and sequencing of the nucleic acid as described above, mutations within a classification or species can be detected and identified. Known methods in the art may be employed, for example, J. Clin. Microbiol. January 1995 vol. 33 no. 1 248-250 (utilizes mismatch amplification mutation assay-multiplex PCR); PNAS July 1, 1984 vol. 81 no. 13 4154-4158 (utilizes electrophoresis for the identification of mutations); and PNAS May 23, 2006 vol. 103 no. 21 8107-8112 (utilizes PCR amplification and capillary sequencing). By identification of nucleic acids and particular genes, the level and degree of mutations of a microorganism, pathogen, virus, or bacteria can be determined if present in the sample. Identification of mutations within a species or classification allows for cataloging the changes in order to map evolutionary processes. This embodiment would allow for collection of data for understanding how mutation, phenotypic variation, and natural selection shape evolutionary processes. This method would allow for determination of whether a mutation within a species or classification exists, and the genetic closeness or similarities to other known mutations. For example, see Lancet, vol. 361, Issue 9371, 24 May 2003, pages 1779-1785, and discloses genome sequence analysis to indicate new strain differences in viruses to identify geographical origins and provide insights into vaccine developments. With detection and identification of mutations or varying differences, the point of origin of a microorganism can be determined, which can give information about the point of contamination, infection, or integration. See J. Clin. Microbiol. June 1999 vol. 37 no. 6 1661-1669, which describes employing electrophoresis in subtyping and strain classification. Determining the location based upon strain development or mutation can lead to information about point source contamination or point source infection.

In another embodiment, gene expression profiling can be employed to measure the activity of genes within the target. Gene expression profiling simultaneously compares the expression levels of numerous genes within a sample, or between two or more sample types. Identification of genes expressed by a pathogen during active blood borne infection is beneficial in identifying agents in antimicrobial therapy. See Wolk, et al., Eur. J. Immunol., 36: 1309-1323. doi: 10.1002/eji.200535503, which discloses identify agents that influence gene expression in development of antimicrobial therapy.

In another embodiment, a gene expression profile can be created to determine the genes expressed by the pathogen. The gene expression profiles of pathogens allow for identification of transcripts encoding for surface antigens, which can be used for antibody generation. See Molecular Microbiology, 44: 9-19. doi: 10.1046/j.1365-2958.2002.02813, which discloses identification of Mycobacterium tuberculosis genes that were determined to be expressed utilizing RT-PCR to identify variable surface antigens. Once antigens are identified, antibodies can be produced through known methods in the art. The antibodies can be used for diagnostic purposes, e.g. identification of pathogens, or for immunological purposes, e.g. rational vaccine design. See for example J Virol Methods. 2009 Dec; 162(1-2):194-202. doi: 10.1016/j.jviromet.2009.08.006, which discloses antibody production from an antigen for the purposes of diagnostic applications against the influenza virus H5. Furthermore, in another preferred embodiment, the antibodies can be produced in applications of pathology in identifying pathogens. See Methods Mol Biol. 2009;508:63-74. doi: 10.1007/978-1-59745-062-1_6, which discloses the use of monoclonal antibodies for the detection of diseases in plant pathology.

Furthermore, in another embodiment, the present invention can be utilized for immunological purposes, for example, rational vaccine design. Rationally designed vaccines are composed of antigens, delivery systems, and often adjuvants that elicit predictable immune responses against specific epitopes to protect against a particular pathogen. See for example PLOS DOI: 10.1371/journal.ppat. 100300, published November 8, 2012, and PLOS DOI: 10.1371/journal.ppat. 1002095, published June 16, 2011, which discloses development of vaccines based upon rational design.

In another embodiment, as described in the preceding paragraphs, the mRNA of a pathogen isolated during active-blood borne infection can be captured, eluted and analyzed for use in the creation of a cDNA library. See Bio-Techniques 38:451-458, March, 2005, which details the construction of a cDNA library from RNA. See Genetics November 1, 1992 vol. 132 no. 3 665-673, which details the use of a cDNA library for the identification of genes whose overexpression causes lethality in yeast. See also Büssow, Konrad, et al. Nucleic Acids Research 26.21 (1998): 5007-5008, which discloses a method for global protein expression and antibody screening utilizing a cDNA library. Knowing which proteins contribute to antibody responses allows one to generate tailored rational vaccines against a smaller subset of antigens. For example, restricting a vaccine to a protein antigen will favor a T-cell response over a humoral B-cell response.

It should be appreciated that with the above mentioned applications, data analysis can be accomplished by using any of a number of commercially available software packages available from Applied Math, Bio-Rad, BioSystematics, Media Cybernetics, or Scanalytics.

Target Detection

In other aspects, the invention provides methods for differentiating between negative and positive assays to increase the accuracy of target pathogen detection. When an assay in the current invention, it is important to be able to distinguish between a successful assay that correctly identifies the absence of a pathogen and a failed assay in which a pathogen was present but just not isolated. Because the failure to detect and treat blood-borne pathogens can result in significant health problems, there is a need to develop a method for rapid isolation of pathogens from a sample, such as a blood sample, that indicates whether the assay properly isolated suspected pathogens.

Methods of the invention involve obtaining a sample suspected of containing a target, e.g., pathogen. To decrease the reporting of a false negative result, a detectable marker is also added to the sample. An assay is conducted to detect the suspected pathogen using magnetic particles. The detectable marker's purpose is to be sufficiently similar to the pathogen such that the assay performs functionally in the same manner for the detectable marker and the pathogen. However, during the analysis of the captured complexes, the detectable marker can be distinguished from the pathogen. Therefore, this provides a quality assurance check on the assay to ensure that the assay is functioning properly or that the magnetic particles are correctly matched with the pathogen to be detected.

Methods of the invention account for the fact that, when isolating pathogens at such low pathogen levels, the difference between a failed assay (i.e. failure to identify a molecule present in the sample) and a positive assay (i.e. positively determining the presence of a molecule present) is often dependent upon the capture of a single target, e.g., pathogen species, meaning, that if a particular species is not captured, the assay reports a negative result. Methods of the invention provide for differentiating between negative and positive assays to increase the accuracy of target pathogen detection. In certain embodiments, such methods may be carried out with the above described cartridges. In other embodiments, such methods are conducted without the need for the above described cartridges.

Methods of the invention involve obtaining a sample suspected of containing a target, e.g., pathogen. Methods of the invention can be conducted with any type of sample, and exemplary types of samples are described above. To decrease the reporting of a false negative result, a detectable marker is also added to the sample. An assay is conducted to detect the suspected pathogen using magnetic particles. The detectable marker's purpose is to be sufficiently similar to the pathogen such that the assay performs functionally in the same manner for the detectable marker and the pathogen. However, during the analysis of the captured complexes, the detectable marker can be distinguished from the pathogen. Therefore, this provides a quality assurance check on the assay to ensure that the assay is functioning properly or that the magnetic particles are correctly matched with the pathogen to be detected.

In certain embodiments, at least two sets of magnetic particles are employed. In those embodiments, members of a first set of magnetic particles are coupled to a binding entity specific to the target, e.g., a pathogen, and members of a second set of magnetic particles are conjugated to a binding entity specific to the detectable marker. Alternatively, magnetic particles including more than one binding moiety (e.g, a binding moiety that is specific to the target and a binding moiety that is specific to the detectable marker) can be introduced into the sample to bind the target and the detectable marker. At the analysis step of the assay, once the presence or absence of the target is determined, the presence or absence of the detectable marker is also determined, either concurrently or subsequent to detection of the target. In some embodiments, analysis allows for the simultaneous detection of the detectable marker and the pathogen. Based on the presence or absence of the detectable marker, a determination is made about whether the assay properly functioned. Detection of the detectable marker indicates a properly executed assay.

The presence of the marker indicates that the assay worked properly; and thus any detection of pathogen (or the absence of pathogen) is accurate and not the result of a failed assay. If analysis of the isolated portion of the sample contains the detectable marker, it can be concluded that the assay properly functioned and the assay was successfully performed. However, if the isolated portion of the sample lacks the presence of the detectable marker, then the assay did not function properly and the results should not be relied upon. In a particular instance, a detectable marker can be introduced into the sample to bind with a plurality of magnetic particles to determine whether the magnetic particles employed in the assay bind properly.

Any assay for separating targets from a sample is useful according to methods of invention, including assays that utilize affinity columns and magnetic fields to isolate pathogens. In one embodiment, the assay includes introducing into a biological sample a cocktail including a plurality of sets of magnetic particles, wherein members of at least one set comprise a binding agent specific for a pathogen and members of at least one set comprise a binding agent specific to the a detectable marker. In another embodiment, magnetic particles including two or more binding moieties, are used. In such embodiments, a first binding moiety on the particle is specific to the target, e.g., pathogen, and a second binding moiety on the particle is specific to the detectable marker. In either embodiment, any pathogen present in the sample and the detectable marker bind to their respective moieties, either on the same particle or on different particles. The sample is exposed to a magnetic field to isolate bound particles (i.e., those that bind pathogen or the marker) from other components of the sample. Once isolated, the presence of the detectable marker is determined. The presence of the marker indicates that the assay worked. Thus, if no pathogen is detected, it is not the result of a failure of the assay to work, thereby eliminating a false negative result.

Any convenient method is useful for detecting the detectable marker and pathogen, including PCR, microarray hybridization, and sequencing. In certain aspects, the presence or absence of the detectable marker is determined prior to determining the presence or absence of the suspected pathogen target. Detecting the detectable marker prior to detecting any isolated pathogen reduces the time and costs associated with detecting targets in a failed assay.

In certain aspects, the detectable marker is a microbe, including a viable or nonviable microbe. The detectable marker can be sufficiently similar to the target such that the assay performs functionally in the same manner for the detectable marker and the target. The detectable marker may be labeled or otherwise modified to allow for their differentiation from targets originally present in the sample. For example, but not by way of limitation, the detectable marker can be genetically modified so as to express a fluorescent protein, or alternatively, the detectable marker could be pre-stained with a persistent stain to allow their differentiation from microbes that are originally present in the fluid composition. In addition, the detectable marker may be chosen based on a chromogen dye specific reaction to the presence of the detectable marker.

Binding entities attached to magnetic particles can be selected at the convenience of the user. In a preferred embodiment, antibodies conjugated to the magnetic particles are used and may be either monoclonal or polyclonal antibodies. Since each set of particles is conjugated with antibodies having different specificities for different pathogens and detectable markers. Compositions of the invention may be provided such that each set of antibody conjugated to a particle is present at a concentration designed for detection of a specific pathogen and specific detectable marker in the sample. In certain embodiments, all of the sets are provided at the same concentration. Alternatively, the sets are provided at different concentrations.

To facilitate detection of the different sets of pathogen/magnetic particle complexes and the detectable marker/magnetic particle complexes, the particles may be differently labeled. Any detectable label may be used with compositions of the invention, such as fluorescent labels, radiolabels, enzymatic labels, and others. In particular embodiments, the detectable label is an optically-detectable label, such as a fluorescent label. Exemplary fluorescent labels include Cy3, Cy5, Atto, cyanine, rhodamine, fluorescein, coumarin, BODIPY, alexa, and conjugated multi-dyes. In one aspect, both the detectable marker and the magnetic particle can be labeled to facilitate detection of the detectable marker.

Any type of magnetic material is suitable for use in methods of the invention. Magnetic particles have been described in detail in the section entitled, "Magnetic Particles." Accordingly, methods of the invention provide for differentiating between negative and positive assays to increase the reliability of target pathogen detection, i.e. providing an indication that the assay positively identified the presence or absence of a target analyte in a sample. Methods of the invention involve obtaining a sample suspect of containing targets, introducing a detectable marker into the sample, and conducting an assay using a plurality of magnetic particles to isolate targets and one or more detectable markers from the sample. The plurality of magnetic particles, can include different sets whereby at least one set of the magnetic particles is coupled to binding moieties (e.g., antibodies) specific to the suspected targets and at least one set of the magnetic particles is coupled to binding moieties (e.g., antibodies) specific to the detectable marker. In other embodiments, a plurality of magnetic particles include more than one binding moiety, (e.g., antibody) specific to the target and a binding moiety (e.g., antibody) specific to the detectable marker. After conducting the assay, methods of the invention provide for making a determination about the presence or absence of the targets in the sample based on the determination of the presence or absence of the detectable marker.

Presence of the detectable marker indicates that the magnetic particles are functioning properly and that all steps of the assay were performed accurately. A lack of the detectable marker indicates either malfunction of the magnetic particles, the employment of a non-binding magnetic particle, or error in execution of the assay. Because the detectable marker was placed into the sample, the subsequent determination of the presence or absence of the detectable marker indicates whether the assay properly bound the detectable marker to the magnetic particle or if the assay failed because it was unable to bind the detectable marker.

Aspects of the invention involve introducing one or more detectable markers into the sample suspected of containing a target or pathogen. Any object capable of isolation and detection from a sample using an assay can be used as the detectable marker. In one embodiment, the detectable marker that has similar properties to the suspected pathogen or targets that one desires to isolate or capture. This ensures that the assay is essentially functioning the same for both the suspected targets and the detectable marker. Any concentration of detectable markers can be introduced into the sample. For example, the concentration of detectable markers introduced is the same as an estimated amount of targets or pathogens suspected of being present in the sample. In another example, 1 to 10 detectable markers are introduced per mL of sample fluid.

Suitable detectable markers for use in the invention include a bacteria, fungi, a protein, a cell (such as a cancer cell, a white blood cell a virally infected cell, or a fetal cell circulating in maternal circulation), a virus, a nucleic acid (e.g., DNA or RNA), a receptor, a ligand, a hormone, a drug, a chemical substance, or any molecule known in the art. Preferably, the detectable marker does not have cross reactivity with the suspected target. In certain aspects, the detectable marker is a microbe, or microorganism. In one embodiment, the microbe is a nonviable microbe or viable mircrobe.

The detectable marker may be labeled or otherwise modified to allow for their differentiation from targets or pathogens originally present in the sample. For example, but not by way of limitation, the detectable marker could be genetically modified so as to express a fluorescent protein, or alternatively, the detectable marker could be pre-stained with a persistent stain to allow their differentiation from microbes that are originally present in the sample fluid. In addition, the detectable marker microbe may be chosen based on a specific-reaction to a chromogen.

In one aspect, the detectable marker is pre-stained using the FISH method, which is a method of fluorescence-staining a microbe by using a nucleic acid probe and targeting a nucleic acid in a cell. This method does not require the step of extracting a nucleic acid from a microbe, and directly adds a fluorescence-labeled nucleic acid probe to a pretreated microbe to make the probe hybridize to an rRNA or chromosome DNA of a nucleic acid in a microbial cell. In general, an rRNA of a nucleic acid in a microbial cell is used as a probe target. There are several thousand to several hundred thousand rRNA copies in a microbial cell, and hence there are probe targets equal in number to the rRNA copies. For this reason, a large amount of fluorescent dye bonded to the nucleic acid probe is accumulated in the target microbial cell. When the fluorescent dye used in this case is irradiated with proper excitation light, only the target microbial cell emits fluorescence without changing its shape to allow its observation under the epifluorescent microscope.

In another aspect, the detectable marker may be genetically modified or engineered to include a protein or other marker capable of being visualized. For example, the detectable marker can be modified with the GFP (Green Fluorescent Protein) gene or Luciferase reported gene. In this manner, the DTMO may be visualized non-invasively using appropriate UV or other suitable illumination and imaging conditions.

Alternatively, in another embodiment, the detectable marker is not pre-labeled or dyed prior to being introduced into the sample, but rather the detectable marker is labeled or differentiated from the target after the assay for detection. For example, the detectable marker can be contacted with antibodies specific to the detectable marker, and the antibody is labeled either directly or indirectly with labels used in known immunoassays.

In another example, the detectable marker is chosen based on a chromogen's reaction to the detectable marker, i.e. the chromogen undergoes a change in color based on the presence of the microbe. Any of a variety of different types of microbes may generally be detected by microbe-sensitive chomogen such as bacteria, fungi, viruses, mold, yeast, etc, and can detect microbes of various different shapes, cell arrangements, and compositions. Microbe-sensitive chromogens suitable for use in methods of the invention include, for example, any chromogen that undergoes a readily detectable change in color based on the chromogen's interaction with microbe.

Microbe specific chromogens suitable for use in the invention include a light absorbing chromophore that is a light-absorbing portion of the chromogen responsible for the chromogen's change of color. Common chromophores include azo groups (e.g., azo dyes), polyene groups (e.g., carotene dye), and carbonyl groups (e.g., anthraquinone dyes), and the chromophores may be connected to a conjugated system. Solvatochromic dyes are a class of chromogen that can be used to detect microbes. U.S. Patent Number 7,300,770 details various types and characteristics of microbe sensitive chromogens.

Suitable solvatochromic dyes may include, but are not limited to merocyanine dyes (e.g., mono-, di-, and tri-merocyanines), betaine dyes, such as 4-(2,4,6-triphenylpyridinium-1-yl)-2,6-diphenylphenolate (Reichardt's dye), 4-dicyanmethylene-2-methyl-6-(p-dimethylaminostyryl)-4H-pyran (DCM); 6-propionyl-2-(dimethylamino)naphthalene (PRODAN); 9-(diethylamino)-5H-benzo[a]phenox-azin-5-one (Nile Red); 4-(dicyanovinyl)julolidine (DCVJ); phenol blue; stilbazolium dyes; coumarin dyes; ketocyanine dyes; N,N-dimethyl-4-nitroaniline (NDMNA) and N-methyl-2-nitroaniline (NM2NA); Nile blue; 1-anilinonaphthalene-8-sulfonic acid (1,8-ANS), and dapoxylbutylsulfonamide (DBS) and other dapoxyl analogs.

Still other suitable dyes that may be used in the present invention include, but are not limited to, 4-[2-N-substituted-1,4-hydropyridin-4-ylidine)ethylidene]cyclohexa-2,5-di-en-1-one, red pyrazolone dyes, azomethine dyes, indoaniline dyes, and mixtures thereof, and merocyanine dyes (e.g., mono-, di-, and tri-merocyanines) are one example of a type of solvatochromic dye.

Methods of the invention provide for conducting an assay to isolate any suspected targets or pathogens and the detectable markers from the sample. To ensure the assay is properly working, the steps for isolating the target or pathogens and the detectable markers should be the same. Assays suitable for use in methods of the invention include, for example, affinity chromatography assays and immunomagnetic assays. Affinity chromatography assays utilizes differences in cell surface macromolecules by passing sample fluids containing the cells through what is known as an affinity column, wherein the affinity column is typically a porous membrane containing binding moieties specific to the target. Examples of affinity chromatography column assays for separating pathogens from sample include those described in Ashok Kumar, Akshay Srivastava. Cell separation using cryogel-based affinity chromatography, Nature protocols 2010; 5:1737-1747; Brewster J.D., Isolation and concentration of Salmonellae with an immunoaffinity column, J Microbiol Methods. 2003 Oct;55(1):287-93; Wang et al. Open-Tubular Capillary Cell Affinity Chromatography: Single and Tandem Blood Cell Separation, Anal. Chem. 2008, 80, 2118-2124.

In one aspect, the assay includes introducing a plurality of magnetic particles to the sample. The magnetic particles are divided into at least two sets. Particles of at least one set includes a pathogen specific binding moieties and particles of at least one other set include detectable marker specific binding moieties. Other sets can be used with binding moieties specific to different targets, such as a third set specific to a second target, a fourth set specific to a third target, a fifth set specific to a fourth target, etc. The sample is allowed to incubate to allow the magnetic particles to bind to the pathogens and the detectable markers. After incubation, the sample is subject to a magnetic field to capture pathogen/magnetic particles complexes and detectable marker/magnetic particles complexes on a surface. Optionally, after the complexes are bound, the surface is washed with a wash solution that reduces particle aggregation, thereby isolating the complexes.

In another aspect of the invention, the assay includes introducing a plurality of the magnetic particles into the sample, where a particle includes more than one binding moiety. That is, a single particle includes at least two binding moieties. In such embodiments, a first binding moiety on the particle is specific for binding to the target, e.g., pathogen, and a second binding moiety is specific for binding to the detectable marker. Such particles are further described, for example in Zeng, H. and Sun, S. (2008), Syntheses, Properties, and Potential Applications of Multicomponent Magnetic Nanoparticles. Adv. Funct. Mater., 18: 391-400. doi: 10.1002/adfm.200701211; see also J. Mater. Chem., 2004,14, 1336-1341, DOI: 10.1039/B315103D, accepted 13 Feb 2004.

In certain embodiments, the detectable marker and the pathogen have similar binding moieties such that a single binding moiety can bind to either the pathogen or the detectable marker.

In other embodiments, a magnetic particle may have two or more antibodies specific to different pathogens. In other embodiments, a magnetic particle may be conjugated to an antibody specific to a pathogen and a lectin specific to a different pathogen. Composition used in methods of the invention may use any type of magnetic particle. Production of magnetic particles and particles for use with the invention are known in the art, and have been described above.

In certain embodiments, the plurality of magnetic particles are divided into two or more sets, in which a first set of the particles has a target-specific binding moiety that allows for binding the target of interest in the sample, and a second set of the particles has a binding moiety that specifically binds of the detectable marker. In other embodiments, a plurality of magnetic particles include more than one binding moiety, e.g., a binding moiety (e.g., antibody) specific to the target and a binding moiety (e.g., antibody) specific to the detectable marker.

The target-specific moiety may be any molecule known in the art and will depend on the target to be captured and isolated. Exemplary target-specific binding moieties include nucleic acids, proteins, ligands, antibodies, aptamers, and receptors.

Methods for attaching the target-specific binding moiety to the magnetic particle are known in the art. Coating magnetic particles with antibodies is well known in the art; see for example Harlow et al. (Antibodies, Cold Spring Harbor Laboratory, 1988), Hunter et al. (Immunoassays for Clinical Chemistry, pp. 147-162, eds., Churchill Livingston, Edinborough, 1983), and Stanley (Essentials in Immunology and Serology, Delmar, pp. 152-153, 2002). Such methodology can easily be modified by one of skill in the art to bind other types of target-specific binding moieties to the magnetic particles. Certain types of magnetic particles coated with a functional moiety are commercially available from Sigma-Aldrich (St. Louis, MO).

Since each set of particles is conjugated with antibodies having different specificities for the suspected target or detectable marker, compositions used in methods of the invention may be provided such that each set of antibody conjugated particles is present at a concentration designed for detection of one or more different suspected targets and one or more detectable markers in the sample. In certain embodiments, sets for the suspected targets and detectable markers are provided at the same concentration. Alternatively, the sets are provided at different concentrations if, for example, there are more than one suspected targets in the sample. For example, compositions may be designed such that sets that bind gram positive bacteria are added to the sample at a concentration of 2 x 109 particles per/mL, while sets that bind gram negative bacteria are added to the sample at a concentration of 4 x 109 particles per/mL. Compositions used with methods of the invention are not affected by antibody cross-reactivity. However, in certain embodiments, sets are specifically designed such that there is no cross-reactivity between different antibodies and different sets.

Capture of a wide range of suspected pathogens or targets and the detectable marker simultaneously can be achieved by utilizing antibodies specific to target. Further, expanded reactivity can be achieved by mixing particles of different reactivity. The addition of high concentrations of non-specific particles does not interfere with the capture efficiency of target-specific particles.

In certain aspects, methods of the invention provide for differentially labeling the magnetic particles specific to the targets and the detectable marker for subsequent detection. Although the detectable marker can be detected on its own, the particle specific to the detectable marker is also labeled so that the detectable marker is subject to the same assay as the suspected pathogens. This is because aspects of the invention provide for conducting the same assay on the detectable markers as the suspected pathogens to ensure the assay functions essentially the same for both the detectable marker and the suspected pathogens. In this embodiment, the detectable marker can be detected on its own or through use of the detectable label attached to the magnetic particle.

To facilitate detection of the pathogen/magnetic particle complexes the particles may be differently labeled. Any detectable label may be used with compositions of the invention, such as fluorescent labels, radiolabels, enzymatic labels, and others. The detectable label may be directly or indirectly detectable. In certain embodiments, the exact label may be selected based, at least in part, on the particular type of detection method used. Exemplary detection methods include radioactive detection, optical absorbance detection, e.g., UV-visible absorbance detection, optical emission detection, e.g., fluorescence; phosphorescence or chemiluminescence; Raman scattering. Preferred labels include optically-detectable labels, such as fluorescent labels. Examples of fluorescent labels include, but are not limited to, 4-acetamido-4'-isothiocyanatostilbene-2,2'disulfonic acid; acridine and derivatives: acridine, acridine isothiocyanate; 5-(2'-aminoethyl)aminonaphthalene-1-sulfonic acid (EDANS); 4-amino-N-[3-vinylsulfonyl)phenyl]naphthalimide-3,5 disulfonate; N-(4-anilino-1-naphthyl)maleimide; anthranilamide; BODIPY; Brilliant Yellow; coumarin and derivatives; coumarin, 7-amino-4-methylcoumarin (AMC, Coumarin 120), 7-amino-4-trifluoromethylcoularin (Coumaran 151); cyanine dyes; cyanosine; 4',6-diaminidino-2-phenylindole (DAPI); 5'5"-dibromopyrogallol-sulfonaphthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino] naphthalene-1-sulfonyl chloride (DNS, dansylchloride); 4-dimethylaminophenylazophenyl-4'-isothiocyanate (DABITC); eosin and derivatives; eosin, eosin isothiocyanate, erythrosin and derivatives; erythrosin B, erythrosin, isothiocyanate; ethidium; fluorescein and derivatives; 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, QFITC, (XRITC); fluorescamine; IR144; IR1446; Malachite Green isothiocyanate; 4-methylumbelliferoneortho cresolphthalein; nitrotyrosine; pararosaniline; Phenol Red; B-phycoerythrin; o-phthaldialdehyde; pyrene and derivatives: pyrene, pyrene butyrate, succinimidyl 1-pyrene; butyrate quantum dots; Reactive Red 4 (Cibacron.TM. Brilliant Red 3B-A) rhodamine and derivatives: 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red); N,N,N',N'tetramethyl-6-carboxyrhodamine (TAMRA); tetramethyl rhodamine; tetramethyl rhodamine isothiocyanate (TRITC); riboflavin; rosolic acid; terbium chelate derivatives; Atto dyes, Cy3; Cy5; Cy5.5; Cy7; IRD 700; IRD 800; La Jolta Blue; phthalo cyanine; and naphthalo cyanine. Preferred fluorescent labels are cyanine-3 and cyanine-5. Labels other than fluorescent labels are contemplated by the invention, including other optically-detectable labels. Methods of linking fluorescent labels to magnetic particles or antibodies are known in the art.

The sample suspected of containing pathogens/targets and one or more detectable markers is then mixed with compositions as described above to generate a mixture that is allowed to incubate such that the plurality of magnetic particles bind to one or more suspected pathogens, if any, and one or more detectable markers in the sample. The mixture is allowed to incubate for a sufficient time to allow for the composition to bind to the pathogens and detectable markers. The process of binding the composition to the pathogen associates a magnetic moment with the pathogen, and thus allows the pathogen to be manipulated through forces generated by magnetic fields upon the attached magnetic moment.

In general, incubation time will depend on the desired degree of binding between the pathogen, detectable markers and the magnetic particles of the invention (e.g., the amount of moment that would be desirably attached to the pathogen), the amount of moment per target, the amount of time of mixing, the type of mixing, the reagents present to promote the binding and the binding chemistry system that is being employed. Incubation time can be anywhere from about 5 seconds to a few days. Exemplary incubation times range from about 10 seconds to about 2 hours. Binding occurs over a wide range of temperatures, generally between 15 °C and 40 °C.

In certain embodiments, a buffer solution is added to the sample along with the compositions of the invention. An exemplary buffer includes Tris(hydroximethyl)-aminomethane hydrochloride at a concentration of about 75mM. It has been found that the buffer composition, mixing parameters (speed, type of mixing, such as rotation, shaking etc., and temperature) influence binding. If labeled, it is important to maintain osmolality of the final solution (e.g., blood + buffer) to maintain high label efficiency. In certain embodiments, buffers used in methods of the invention are designed to prevent lysis of blood cells or any other cells, facilitate efficient binding of targets with magnetic particles and to reduce formation of particle aggregates. It has been found that the buffer solution containing 300 mM NaCl, 75 mM Tris-HCl pH 8.0 and 0.1% Tween 20 meets these design goals.

Without being limited by any particular theory or mechanism of action, it is believed that sodium chloride is mainly responsible for maintaining osmolality of the solution and for the reduction of non-specific binding of magnetic particle through ionic interaction. Tris(hydroximethyl)-aminomethane hydrochloride is a well-established buffer compound frequently used in biology to maintain pH of a solution. It has been found that 75 mM concentration is beneficial and sufficient for high binding efficiency. Likewise, Tween 20 is widely used as a mild detergent to decrease nonspecific attachment due to hydrophobic interactions. Various assays use Tween 20 at concentrations ranging from 0.01% to 1%. The 0.1% concentration appears to be optimal for the efficient labeling of bacteria, while maintaining blood cells intact.

Additional compounds can be used to modulate the capture efficiency by blocking or reducing non-specific interaction with blood components and either magnetic particles or pathogens. For example, chelating compounds, such as EDTA or EGTA, can be used to prevent or minimize interactions that are sensitive to the presence of $Ca^{2+}$ or $Mg^{2+}$ ions.

An alternative approach to achieve high binding efficiency while reducing time required for the binding step is to use static mixer, or other mixing devices that provide efficient mixing of viscous samples at high flow rates, such as at or around 5 mL/min. In one embodiment, the sample is mixed with binding buffer in ratio of, or about, 1:1, using a mixing interface connector. The diluted sample then flows through a mixing interface connector where it is mixed with target-specific nanoparticles. Additional mixing interface connectors providing mixing of sample and antigen-specific nanoparticles can be attached downstream to improve binding efficiency. The combined flow rate of the labeled sample is selected such that it is compatible with downstream processing.

After binding of the particles to the pathogen and detectable markers in the sample to form pathogen/magnetic particle complexes, a magnetic field is applied to the mixture to capture the complexes on a surface. Components of the mixture that are not bound to magnetic particles will not be affected by the magnetic field and will remain free in the mixture. Methods and apparatuses suitable for separating target/magnetic particle complexes and detectable marker/magnetic particles from other components of a mixture are known in the art. For example, a steel mesh may be coupled to a magnet, a linear channel or channels may be configured with adjacent magnets, or quadrapole magnets with annular flow may be used. Other methods and apparatuses for separating target/magnetic particle complexes from other components of a mixture are shown in Rao et al. (U.S. 6,551,843), Liberti et al. (U.S. 5,622,831), Hatch et al. (U.S. 6,514,415), Benjamin et al. (U.S. 5,695,946), Liberti et al. (U.S. 5,186,827), Wang et al. (U.S. 5,541,072), Liberti et al. (U.S. 5,466,574), and Terstappen et al. (U.S. 6,623,983).

In certain embodiments, the magnetic capture is achieved at high efficiency by utilizing a flow-through capture cell with a number of strong rare earth bar magnets placed perpendicular to the flow of the sample. When using a flow chamber with flow path cross-section 0.5 mm x 20 mm (h x w) and 7 bar NdFeB magnets, the flow rate could be as high as 5 mL/min or more, while achieving capture efficiency close to 100%. In one embodiment, alternating magnetic fields are applied to the complexes to encourage binding of the complexes to the surface. Additionally, binding moieties specific to the target/magnetic particle complex and detectable marker/magnetic particle complex can be used to further enhance binding of complexes to the surface. Methods of capture using alternating magnetic fields and surface binding moieties are described in co-pending and co-owned U.S. Patent Application Serial Number 12/855,147 (U.S. Patent Publication No. 2011-0262893).

The above described type of magnetic separation produces efficient capture of one or more targets and one more detectable markers and the removal of a majority of the remaining components of a sample mixture. However, such a process produces a sample that contains a very high percent of magnetic particles that are not bound to targets or detectable markers because the magnetic particles are typically added in excess, as well as non-specific target entities. Non-specific target entities may for example be bound at a much lower efficiency, for example 1% of the surface area, while a target of interest might be loaded at 50% or nearly 100% of the available surface area or available antigenic cites. However, even 1 % loading may be sufficient to impart force necessary for trapping in a magnetic gradient flow cell or sample chamber.

For example, in the case of immunomagnetic binding of bacteria or fungi in a blood sample, the sample may include: bound targets and detectable markers at a concentration of about 1/mL or a concentration less than about 106/mL; background particles at a concentration of about 107/mL to about 1010/mL; and non-specific targets at a concentration of about 10/mL to about 105/mL.

The presence of magnetic particles that are not bound to targets or detectable marker and non-specific target entities on the surface along with the target/magnetic particle complexes detectable marker/magnetic particle complexes interferes with the ability to successfully detect or isolate the target of interest. The magnetic capture of the resulting mix, and close contact of magnetic particles with each other and bound targets, result in the formation of aggregate that is hard to dispense and which might be resistant or inadequate for subsequent processing or analysis steps. In order to remove magnetic particles that are not bound to target analytes and non-specific target entities, the surface may be washed with a wash solution that reduces particle aggregation, thereby isolating target/magnetic particle complexes from the magnetic particles that are not bound to target analytes and non-specific target entities. The wash solution minimizes the formation of the aggregates.

Any wash solution that imparts a net negative charge to the magnetic particle that is not sufficient to disrupt interaction between the target-specific moiety of the magnetic particle and the target analyte may be used. The wash solutions are discussed in the preceding sections.

Once the assay is complete and the target/magnetic particle complexes and detectable marker complexes are isolated from the sample, methods of the invention provide for detecting the presence of the detectable marker in order to indicate whether the assay is properly working. Detection of the detectable marker can be performed before, simultaneously, or subsequent the detection of the suspected targets. If the detectable marker is positively detected, the assay is working properly and the detection of suspected targets is positively confirmed. In other words, detection of the detectable marker indicates proper functionality of the magnetic particle and proper execution of the assay. If the detectable marker is not detected, then the assay or the magnetic particles failed in capturing the target and detectable marker. These results should not be relied upon, and can prompt quality assurance investigations and remedies. In certain aspects, the detectable marker is detected prior to detection of the targets. This allows one to confirm that the assay is working prior to expending resources for detecting targets in what may result in a failed assay. In certain aspects, the detectable marker and the target are detected simultaneously, negating the need for two analysis steps.

The detectable marker and target may be analyzed by a multitude of existing technologies, such as miniature NMR, Polymerase Chain Reaction (PCR), mass spectrometry, fluorescent labeling and visualization using microscopic observation, fluorescent in situ hybridization (FISH), growth-based antibiotic sensitivity tests, and variety of other methods that may be conducted with purified target without significant contamination from other sample components.

In one embodiment, the detectable marker is detected using the microbe sensitive chromogens discussed in detail above. After the assay, the remaining contents, i.e. magnetically captured detectable markers and targets, are placed in a final collection tube. To determine if the assay worked properly, a chromogen specific to the detectable marker is placed in the tube in such an amount to undergo a detectable color change in the presence of the detectable marker. If the color appears, this color change indicates that the detectable marker has been properly captured, indicating that the assay and magnetic particles functioned properly. Then the contents can be analyzed for the presence or absence of targets in the sample. In another embodiment, chromogens specific to the target are used to detect the presence or absence of the target.

In another embodiment, isolated target and/or detectable marker are lysed with a chaotropic solution, and DNA is bound to DNA extraction resin. After washing of the resin, the DNA is eluted and used in quantitative RT-PCR to detect the presence of the target and/or detectable marker.

In certain embodiments, magnetic particles are introduced into the sample, where the magnetic particles contain at least one set to bind to the binding specific moiety of the target and at least one set to bind to the binding specific moiety of the detectable target. A magnetic field is applied to the sample, thereby capturing the magnetic particles onto a surface. After the assay, the presence of the detectable marker is determined for proper functioning of the assay.

In other embodiments, magnetic particles are introduced into the sample, where the magnetic particles are capable of binding to the target and to the detectable marker. Meaning, that each magnetic particle contains at least two different sites, wherein one site the target is bound and in a second site the detectable marker is bound. For example, each particle contains at least two types of binding moieties on each particle for binding the target and the detectable marker. After the assay, the presence of the detectable marker is determined for proper functioning of the assay.

In another embodiment, the detectable marker and target are removed from the magnetic particles to which they are bound and the processed sample is mixed with fluorescent labeled antibodies specific to each. After incubation, the reaction mixture is filtered through 0.2 $\mu$m to 1.0 $\mu$m filter to capture labeled detectable markers and targets while allowing majority of free beads and fluorescent labels to pass through the filter. The detectable markers and targets are visualized on the filter using microscopic techniques, e.g. direct microscopic observation, laser scanning or other automated methods of image capture. The presence of detectable markers and target is detected through image analysis.

After the results of the assay are confirmed based on the positive presence of the detectable marker, any targets isolated from the sample can be further characterized using PCR or genomic methods.

If the targets are bacteria, detection of the bacteria can be performed by use of nucleic acid probes following procedures which are known in the art. Suitable procedures for detection of bacteria using nucleic acid probes are described, for example, in Stackebrandt et al. (U.S. 5,089,386), King et al. (WO 90/08841), Foster et al. (WO 92/15883), and Cossart et al. (WO 9/06699). Nucleic acid probes are discussed in the preceding section entitled, "Dectection of Targets."

Detection of bacteria of interest can also be performed by use of PCR techniques. A suitable PCR technique is described, for example, in Verhoef et al. (WO 92/08805). Such protocols may be applied directly to the bacteria captured on the magnetic beads. The bacteria are combined with a lysis buffer and collected nucleic acid target molecules are then utilized as the template for the PCR reaction.

For detection of the selected bacteria by use of antibodies, isolated bacteria are contacted with antibodies specific to the bacteria of interest. As noted above, either polyclonal or monoclonal antibodies can be utilized, but in either case have affinity for the particular bacteria to be detected. These antibodies will adhere/bind to material from the specific target bacteria. With respect to labeling of the antibodies, these are labeled either directly or indirectly with labels used in other known immunoassays. Direct labels may include fluorescent, chemiluminescent, bioluminescent, radioactive, metallic, biotin or enzymatic molecules. Methods of combining these labels to antibodies or other macromolecules are well known to those in the art. Examples include the methods of Hijmans, W. et al. (1969), Clin. Exp. Immunol. 4, 457-, for fluorescein isothiocyanate, the method of Goding, J. W. (1976), J. Immunol. Meth. 13, 215-, for tetramethylrhodamine isothiocyanate, and the method of Ingrall, E. (1980), Meth. in Enzymol. 70, 419-439 for enzymes.

Methods For Amplifying Nucleic Acid From A Target

Alternative methods for detection of pathogens, particularly bacteria, have been described by others. Those methods include molecular detection methods, such as hybrid capture or polymerase chain reaction (PCR), which identify the pathogen from a sample by analyzing the nucleic acid from the pathogen. For PCR, a high starting concentration of purified nucleic acid is required. A general workflow to obtain the purified pathogen nucleic acid for the PCR reaction involves isolating pathogens from a biological sample, such as blood or urine, and lysing the isolated pathogen to release the pathogen nucleic acid. The lysis results in a mixture that includes free pathogen nucleic acid and debris from the lysed cells. That mixture is purified, e.g., by running the mixture through a column that retains the nucleic acid while allowing the lysis debris to flow through the column. The retained nucleic acid is then eluted from the column and the purified pathogen nucleic acid is subjected to PCR amplification.

A problem with nucleic acid purification processes is that pathogen nucleic acid is lost during the process. Certain aspects of the invention provide methods for amplifying nucleic acid in the presence of debris from a lysis procedure, i.e., amplification conducted without a prior purification step.

In a particular embodiment, methods of the invention are accomplished by introducing amplification reagents (e.g., primers, nucleotide triphosphates, polymerases, metal ions, buffers, etc.) into a sample vessel, as described above, including an isolated intact target. A lysis procedure is conducted in the presence of the amplification reagents, such that nucleic acid released from the lysed target is immediately exposed to the amplification reagents and an amplification reaction is conducted on the lysed target nucleic acid in the presence of the debris from the lysis procedure. Accordingly, all of the lysed nucleic acid is available to participate in the amplification reaction, and methods of the invention avoid nucleic acid loss that occurs using standard purification techniques. Additionally, nucleic acid degradation is avoided because the heat from the amplification reaction denatures/inactivates nucleases that cause nucleic acid degradation. In certain embodiments, such methods may be carried out with the above described cartridges. In other embodiments, such methods are conducted without the need for the above described cartridges.

In other embodiments, a sample which contains a target, as discussed above is assayed. Any assay known in the art may be used to capture/isolate the target from the sample. In certain embodiments, the assay uses magnetic particles and the assay involves introducing magnetic particles including a target-specific binding moiety to the sample in order to create a mixture, incubating the mixture to allow the particles to bind to the target in the sample, and applying a magnetic field to isolate target/magnetic particle complexes from the sample. The target-specific binding moiety will depend on the target to be captured. The moiety may be any capture moiety known in the art, such as an antibody, an aptamer, a nucleic acid, a protein, a receptor, a lectin, a phage or a ligand. In particular embodiments, the target-specific binding moiety is a lectin or an antibody. In certain embodiments, the antibody is specific for bacteria. In other embodiments, the antibody is specific for fungi.

In certain aspects, the processes performed includes introducing a plurality of magnetic particles, in which a plurality of the particles include at least one captured moiety specific to a target, into a sample to form a mixture that includes sample and particles. The mixture is incubated to form at least one target/particle complex and a magnetic field is applied to isolate the magnetic particle/target complexes from the sample. The process starts at inputting a sample and ends at delivering a capture target (or nucleic acids of the target) into a container for further analysis.

The isolation assay of the present invention may be performed with any type of magnetic particle, which have been described in the section entitled "Magnetic Particles." Once the target is isolated, reagents from an amplification reaction are added to the isolated target, the target is lysed to release nucleic acid from within the target, and the released nucleic acid is amplified in the presence of the debris (e.g., cellular components such as proteins and lipids) from the lysis. Any method known in the art may be used to lyse the target. Exemplary methods include mechanical methods, thermal methods, enzymatic methods, chemical methods, or a combination thereof. The lysis and the amplification reaction can occur simultaneously or sequentially and will depend on the lysis method employed. In particular embodiments, the target is lysed using thermal methods. Application of heat not only lyses the target, it also denatures the released double stranded nucleic acid into single stranded nucleic acid and initiates the amplification reaction. Another advantage of thermal lysis is that the heat facilitates denaturation/inactivation of nucleases that can cause degradation of nucleic acid. Any amplification reaction known in the art may be conducted on the released nucleic acid, and a discussion of amplication methods is presented above. Another aspect of the invention provides methods for amplifying nucleic acid from a target that involve obtaining a sample including a target, conducting an assay that isolates the target from the sample, lysing the target to release the nucleic acid, and amplifying the released nucleic acid such that the lysing and the amplifying occur in the same reaction vessel.

Another aspect of the invention provides methods for amplifying nucleic acid from a target that involve obtaining a sample including a target, conducting an assay that isolates the target from the sample, and applying heat to the isolated target to thereby lyse the target within a reaction vessel and initiate within the reaction vessel an amplification reaction of nucleic acid released from the target.

In certain embodiments, methods of the invention involve obtaining a sample including a target, conducting an assay that isolates the target from the sample, lysing the target to release the nucleic acid, and amplifying the released nucleic acid such that the amplifying occurs in the presence of debris from the lysing step. Accordingly, methods of the invention provide for amplification of a nucleic acid from a target without any intervening nucleic acid purification steps, i.e., direct amplification of a nucleic acid release from within a target.

In certain embodiments, magnetic beads including a capture moiety are used in an assay to isolate the target from the sample. Certain fundamental technologies and principles are associated with binding magnetic materials to targets and subsequently separating by use of magnet fields and gradients. Such fundamental technologies and principles are known in the art and have been previously described, such as those described in Janeway (Immunobiology, 6th edition, Garland Science Publishing).

For magnetic bead based isolation assays, the sample including the target of interest is mixed with magnetic particles having a particular magnetic moment and also including a target-specific binding moiety to generate a mixture that is allowed to incubate such that the particles bind to a target in the sample, such as a bacterium in a blood sample. The mixture is allowed to incubate for a sufficient time to allow for the particles to bind to the target analyte. The process of binding the magnetic particles to the target analytes associates a magnetic moment with the target analytes, and thus allows the target analytes to be manipulated through forces generated by magnetic fields upon the attached magnetic moment. Incubation with magnetic particles and isolation procedures and apparatuses are discussed above.

Magnetic particles for use with methods of the invention have a target-specific binding moiety that allows for the particles to specifically bind the target of interest in the sample. The target-specific moiety may be any molecule known in the art and will depend on the target to be captured and isolated. Exemplary target-specific binding moieties include nucleic acids (including nucleic acid probes), proteins, ligands, lectins, antibodies, aptamers, bactertiophages, host innate immunity biomarkers (e.g., CD14), host defense peptides (e.g., defensins), bacteriocins (e.g., pyocins), and receptors.

In particular embodiments, the target-specific binding moiety is an antibody, such as an antibody that binds a particular bacterium. General methodologies for antibody production are described above in the section entitled, "Methods of Antibody Production." Methods for attaching the target-specific binding moiety to the magnetic particle are discussed above. In some embodiments, the target specific binding moiety is a lectin, as discussed above.

After binding of the magnetic particles to the target in the mixture to form target/magnetic particle complexes, as discussed by methods in the above related sections, a magnetic field is applied to the mixture to capture the complexes on a surface. Components of the mixture that are not bound to magnetic particles will not be affected by the magnetic field and will remain free in the mixture. Methods and apparatuses for separating target/magnetic particle complexes from other components of a mixture are known in the art, and discussed above.

The above described type of magnetic separation produces efficient capture of a target analyte and the removal of a majority of the remaining components of a sample mixture. However, such a process may produce a sample that contains a percent of magnetic particles that are not bound to target analytes, as well as non-specific target entities. Non-specific target entities may for example be bound at a much lower efficiency, for example 1% of the surface area, while a target of interest might be loaded at 50% or nearly 100% of the available surface area or available antigenic cites. However, even 1 % loading may be sufficient to impart force necessary for trapping in a magnetic gradient flow cell or sample chamber.

The presence of magnetic particles that are not bound to target analytes and non-specific target entities on the surface that includes the target/magnetic particle complexes may interfere with the ability to successfully detect the target of interest. The magnetic capture of the resulting mix, and close contact of magnetic particles with each other and bound targets, result in the formation of aggregate that is hard to dispense and which might be resistant or inadequate for subsequent processing or analysis steps. In order to remove magnetic particles that are not bound to target analytes and non-specific target entities, methods of the invention may further involve washing the surface with a wash solution that reduces particle aggregation, thereby isolating target/magnetic particle complexes from the magnetic particles that are not bound to target analytes and non-specific target entities. The wash solution, discussed in the above related sections, minimizes the formation of the aggregates

Generally, amplification reagents (e.g., primers, nucleotide triphosphates, polymerases, metal ions, buffers, etc.) are then introduced into a vessel including the isolated intact target, and a lysis procedure is then conducted in the presence of the amplification reagents, such that nucleic acid is released from within the lysed target. However, the amplification reagents do not need to be added prior to the lysis procedure and in certain embodiments, the amplification reagents are added after the lysis procedure is conducted.Any lysis procedure known in the art may be used to lyse the target. Lysing methods are provided above. After lysing, amplification is carried out. Methods for amplification are discussed in the preceding sections. Any method known in the art may be used to detect the amplification products, and thus identify the target in the sample, for example identify a pathogen from a human tissue or body fluid sample, such as blood or urine. Exemplary methods for detecting amplification products involve running the amplification products through a gel using, for example polyacrylamide gel electrophoresis or capillary electrophoresis. In other embodiments, probe hybridization is carried out on the amplification products, methods for which are described above in the related sections.

In other embodiments, the QPCR reaction uses fluorescent Taqman methodology and an instrument capable of measuring fluorescence in real time (e.g., ABI Prism 7700 Sequence Detector). During amplification, fluorescent signal is generated in a TaqMan assay by the enzymatic degradation of the fluorescently labeled probe. The probe contains a dye and quencher that are maintained in close proximity to one another by being attached to the same probe. When in close proximity, the dye is quenched by fluorescence resonance energy transfer to the quencher. Certain probes are designed that hybridize to the wild-type of the target, and other probes are designed that hybridize to a variant of the wild-type of the target. Probes that hybridize to the wild-type of the target have a different fluorophore attached than probes that hybridize to a variant of the wild-type of the target. The probes that hybridize to a variant of the wild-type of the target are designed to specifically hybridize to a region in a PCR product that contains or is suspected to contain a single nucleotide polymorphism or small insertion or deletion.

During the PCR amplification, the amplicon is denatured allowing the probe and PCR primers to hybridize. The PCR primer is extended by Taq polymerase replicating the alternative strand. During the replication process the Taq polymerase encounters the probe which is also hybridized to the same strand and degrades it. This releases the dye and quencher from the probe which are then allowed to move away from each other. This eliminates the FRET between the two, allowing the dye to release its fluorescence. Through each cycle of cycling more fluorescence is released. The amount of fluorescence released depends on the efficiency of the PCR reaction and also the kinetics of the probe hybridization. If there is a single mismatch between the probe and the target sequence the probe will not hybridize as efficiently and thus a fewer number of probes are degraded during each round of PCR and thus less fluorescent signal is generated. This difference in fluorescence per droplet can be detected and counted. The efficiency of hybridization can be affected by such things as probe concentration, probe ratios between competing probes, and the number of mismatches present in the probe.

Sequencing

In other embodiments, sequencing is used to analyze the target, such as identifying a pathogen from a body fluid. Sequencing may be by any method known in the art. DNA sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of separated molecules has more recently been demonstrated by sequential or single extension reactions using polymerases or ligases as well as by single or sequential differential hybridizations with libraries of probes.

A sequencing technique that can be used in the methods of the provided invention includes, for example, Helicos True Single Molecule Sequencing (tSMS) (Harris T. D. et al. (2008) Science 320:106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm$^2$. The flow cell is then loaded into an instrument, e.g., HeliScope (sequencer, commercially available by Helicos Biosciences) sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step. Further description of tSMS is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

Another example of a DNA sequencing technique that can be used in the methods of the provided invention is SOLiD technology (Applied Biosystems). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

Another example of a DNA sequencing technique that can be used in the methods of the provided invention is Ion Torrent sequencing (U.S. patent application numbers 2009/0026082, 2009/0127589, 2010/0035252, 2010/0137143, 2010/0188073, 2010/0197507, 2010/0282617, 2010/0300559), 2010/0300895, 2010/0301398, and 2010/0304982). In Ion Torrent sequencing, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to a surface and is attached at a resolution such that the fragments are individually resolvable. Addition of one or more nucleotides releases a proton (H+), which signal detected and recorded in a sequencing instrument. The signal strength is proportional to the number of nucleotides incorporated.

Another example of a sequencing technology that can be used in the methods of the provided invention is Illumina sequencing. Illumina sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

Another example of a sequencing technology that can be used in the methods of the provided invention includes the single molecule, real-time (SMRT) technology of Pacific Biosciences. In SMRT, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

Another example of a sequencing technique that can be used in the methods of the provided invention is nanopore sequencing (Soni G V and Meller A. (2007) Clin Chem 53: 1996-2001). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

Another example of a sequencing technique that can be used in the methods of the provided invention involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be used in the methods of the provided invention involves using an electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

Methods For Raising Antibodies

Development of antibodies that efficiently and reliably capture particular pathogen species from biological matrices, such as blood, is complicated by the large number of strain types, often with markedly different antigenic properties (serotypes), which can exist within a species. Antibodies raised against a single index strain of species may not cross-react with other isolates, or strains of the same species. Accordingly, the development of an antibody that can be used against a wide variety of strains is problematic.

One possible solution would be to inoculate against all, or nearly all, of the known different strain-types and combine the antibodies. This, however, would require inoculation of hundreds of animals with individual pathogen strains. Combining isolates within the inoculation medium may reduce the number of animals required, however, a large number of animals is still necessary to cover combinations of all the known serotypes.

Another strategy would be to determine the minimal subset of strains that would represent most, or all, of the antigenic variations across a species. For some species, data regarding the known antigenic variation across the species is available, as well as the relative abundance of the antigenic variants, i.e., the serotypes, in different diseases or environments. However, such information does not exist for most species, indicating a need for alternate methods of developing antibodies that can be used across antigenic variants.

The present disclosure provides methods for employing genotyping to assess genomic variations across different species as a means of selecting a minimal subset of isolates to be used as immunogens to generate antibodies that interact with most, or all, of the clinically-important strains of each species. Using genomic information, a minimal subset of strains to represent most, or all, of the antigenic variation across a species is determined. Certain aspects of the invention provide methods for producing one or more antibodies that bind to a subset of a strain within a group.

In certain embodiments, antibodies are generated using genotyping to survey the extent of genetic variation within a species. The provided method then groups genomically-similar isolates together, such that one isolate from a group of closely related strains may be used to represent the entire species within the group. The groupings then serve as guides for selection of a minimal subset of strains representative of the spectrum of genomic variation existing within each species. An isolate from each group is then used in the production of antibodies, where based upon the genetic similarities within a group, the selected isolate would produce an immunological response in antibody production to cover the identified strains with a group. Therefore, by having several groups of genetically similar strains and selecting one isolate from each group, a panel of isolates is selected that would be used in antibody production.

Rather than assessing antigenic variation, methods of the present disclosure involve the use of genotyping to assess genomic variation across different species as a means of selecting a minimal subset of isolates to be used as antigens for the generation of antibodies able to interact with most, or all, of the clinically-important strains of each species. Accordingly, the present methods may facilitate the generation of broad specificity antibodies across most, or all, antigenic variants within clinically or industrially important microbial species and further facilitate the development of new antibody-based analytical methods.

In certain aspects, the disclosure provides a method for generating antibodies against a species of pathogen. The method involves obtaining a nucleic acid from a plurality of pathogens within the same species and comparing the obtained nucleic acids for genetic similarities. The method further includes grouping the pathogens based upon genomic similarities and then identifying the smallest subset of pathogens representative of the species based on the comparison and using the identified subset to generate an antibody. For example, the plurality of pathogens could be grouped so that each group is 97% similar in genomes. Then, from each group, a single pathogen is selected to represent the group in antibody production. Therefore, this small subset of pathogens would be used in antibody production. The subset would represent most, or all, clinically relevant strains of the species. Accordingly, a set of antibodies would be produced that can bind most or all of the clinically relevant strains of a species.

Any method for analyzing pathogen genomes is suitable for use with the provided methods. Such methods would be used to assess the level of similarity and/or dissimilarity of different pathogen isolates. The methods include, without limitation, ribotyping, rep-PCR, pulsed-field gel electrophoresis, optical mapping, microarray-based measurements of single-nucleotide polymorphisms, or any combination of these. These methods may also be used to assess the range of genetic variation across or within pathogen species and for the building of "similarity trees" or "cladograms," in which genomically similar strains are grouped together to form branches or "clades." These groupings can be used to guide selection of the smallest subset of strains that still represent the breadth of genomic variation within the species.

Methods of the present disclosure are suitable for developing both monoclonal and polyclonal antibodies. Monoclonal antibodies refer to antibody molecules of singular epitope specificity originally produced by one B-cell and sharing identical sequence. Polyclonal antibodies, on the other hand, refer to antibody molecules which differ in their epitope binding and complementarity region amino acid sequence but share an overall target specificity. Methods of the present disclosure can be used to generate monoclonal antibodies when, for example, the smallest representative subset is a single strain of bacteria featuring an epitope found on all strains. The provided methods are useful for generating polyclonal antibodies when the smallest representative subset includes a number of strains encompassing a host of epitopes.

Although the present methods are useful for developing antibodies against any type of pathogen, they are particularly suited for developing antibodies against bacteria, which are known to encompass a wide range of strains within a single species. Such bacteria may include gram positive bacteria or gram negative bacteria. Exemplary bacterial species that may be used in conjunction with the provided methods include, but are not limited to E. coli, Listeria, Clostridium, Mycobacterium, Shigella, Borrelia, Campylobacter, Bacillus, Salmonella, Staphylococcus, Enterococcus, Pneumococcus, and Streptococcus.

The provided methods use genotyping to survey the extent of genetic variation within a species. The provided method groups genomically-similar isolates together, such that one isolate from a group of closely related strains may be used to represent the entire species, and uses the groupings to guide selection of a minimal subset of strains representative of the spectrum of genomic variation existing within each species. In one aspect of the disclosure pathogens identified as the minimal subset of strains are then used in the production and development of antibodies.

As encompassed by the disclosure genotyping may require the isolation of nucleic acids from a sample, such as a bacterial pathogen. Genotyping, or the process of determining differences in the genetic make-up (genotype) of a species in the sample, is accomplished by examining the species' DNA sequence using biological assays and comparing it to another similar species' sequence or a reference sequence that may or may not be present in the sample. Traditionally genotyping is the use of DNA sequences to define biological populations by analyzing for genetic similarities and differences. Current methods of genotyping known in the art include restriction fragment length polymorphism identification (RFLPI) of genomic DNA, random amplified polymorphic detection (RAPD) of genomic DNA, amplified fragment length polymorphism detection (AFLPD), polymerase chain reaction (PCR), DNA sequencing, allele specific oligonucleotide (ASO) probes, and hybridization to DNA microarrays or beads. Genotyping applies to a broad range of species, including pathogens, microorganisms, viruses, and bacteria.

For genotyping, nucleic acids from the species need to be isolated. Nucleic acid in a sample can be any nucleic acid, including for example, genomic DNA in a tissue sample, cDNA amplified from a particular target in a laboratory sample, or mixed DNA from multiple organisms. In some embodiments, the sample includes homozygous DNA from a haploid or diploid organism. For example, a sample can include genomic DNA from a patient who is homozygous for a rare recessive allele. In other embodiments, the sample includes heterozygous genetic material from a diploid or polyploidy organism with a somatic mutation such that two related nucleic acids are present in allele frequencies other than 50 or 100%, i.e., 20%, 5%, 1%, 0.1%, or any other allele frequency.

After any of the aforementioned processing steps (e.g., obtaining, isolating, fragmenting, or amplification), nucleic acid can be sequenced to generate a plurality of sequence reads, according to certain embodiments of the invention. See the section entitled "Sequencing" for detailed information.

Nucleic acid sequences obtained from the various bacteria can then be compared in any number of ways, including ribotyping, rep-PCR, optical mapping, and Pulsed field gel electrophoresis. Ribotyping involves the fingerprinting of genomic DNA restriction fragments that contain all or part of the geens for the 16S and 23S ribosomal RNA ("rRNA"). Accordingly, ribotyping can be used to identify and classify bacteria based on differences in rRNA. DNA is extracted from a colony of bacteria and then enzymatically restricted into discrete-sized fragments. The DNA is then transferred to a membrane and probed with a region of the rRNA operon to reveal the pattern of rRNA genes. The pattern is recorded, digitized, and stored in a database. The variations that exist among the pathogens in both the position and intensity of rRNA bands can be used for their classification and identification. In other aspects, gel electrophoresis is conducted with the digested samples, whereupon the fragments are visualized as lines on the gel. These lines form a unique pattern for each species and can be used to identify the origin of the DNA. Databases for Listeria (80 pattern types), Salmonella (97 pattern types), Escherichia (65 pattern types), and Staphylococcus (252 pattern types) have been established. Methods of ribotyping are well-known in the art. In a basic protocol, DNA is isolated from bacterial cell cultures and digested in separate reactions with two different restriction endonucleases. Digested fragments are then separated by mass and charge with gel electrophoresis. The DNA fragments are then transferred from the gel to a Nitran filter and probed with labeled rRNA from the particular species. The bound probes are then visualized according to the label used. Each ribotype (i.e., ribosome-associated fingerprint) is analyzed by assigning an alphanumeric pattern based on the distance between bands. Each unique banding pattern is deemed a ribotype and assigned an alphanumeric pattern. Further detail on such methods can be found, for example, in US 2005/0260293.

Repetitive sequence-based PCR or rep-PCR provides another means for comparing pathogen genomes. Prokaryotic and eukaryotic genomes contain dispersed repetitive sequences separating longer single-copy DNA sequences. Interspersed repetitive sequences are characterized as relatively short (usually <500 bp), non-coding dispersed elements in bacterial genomes. Current data indicates that repetitive DNA comprises a substantial portion of the microbial genomes. Rep-PCR primers complement these repetitive sequences and allow for specific binding, providing reproducible, unique rep-PCR DNA fingerprint patterns.

In a basic protocol, nucleic acid is isolated from microorganisms of interest and allowed to hybridize to rep-PCR binders that bind to the many repetitive sequences interspersed throughout the genome. Multiple fragments of various lengths are then amplified. After amplification, the fragments are separated by electrophoresis according to their mass and charge. Based on the distribution and intensity of the bands, a unique rep-PCR DNA fingerprint profile is created. Additional detail on rep-PCR methods are provided in U.S. Patent Nos. 5,691,136 and 5,523,217.

Optical mapping is a method of whole genome analysis that involves the generation of ordered restriction maps for entire genomes called "optical maps." By mapping the location of restriction enzyme sites along the unknown DNA of an organism, the spectrum of resulting DNA fragments together serve as a unique fingerprint for that sequence. In a basic protocol, genomic DNA is obtained from lysed cells, and randomly fragmented to a produce a library of large genomic molecules for optical mapping. A single DNA molecule is then placed onto a slide under a fluorescent microscope. Restriction enzymes are added to digest the DNA at specific positions. Each DNA molecule is then stained with a fluorescent dye. DNA fragments stained with the dye are visualized by fluorescence microscopy and are sized by measuring the integrated fluorescent intensity. This results in an optical map of the single molecules. The individual optical maps are combined to produce a consensus, genomic, optical map. Microfluidic devices can be used in conjunction with optical mapping to improve efficiency and algorithms can be incorporated to determine the best consensus map. Further detail on optical mapping can be found in U.S. Patent Application Nos. 13/147,056 and 12/257,892.

Pulse field gel electrophoresis (PFGE) has also been used to characterize various pathogens, such as bacteria. PFGE is a form of restriction fragment length polymorphism (RFLP) typing in which the bacterial genome is digested with rare cutting enzymes. These restriction enzymes cut genomic DNA infrequently and therefore generate a smaller number of DNA fragments 910-20 bands). These fragments, which can span a wide range of sizes, are separated using specialized electrophoresis techniques. Differences in the restriction profiles are used to conduct genetic comparisons among isolates. Analysis can be performed by computer, enabling rapid and easy comparison between strains. PFGE electrophoresis techniques are well-known in the art. Further detail on such methods is provided, for example, in U.S. Patent No. 7731828 and U.S. Patent Application No. 10/418,837.

Microarray-based measurements of single-nucleotide polymorphisms can also be used. An array is an orderly arrangement of samples where matching of known and unknown DNA samples is performed using base paring rules. An array experiment makes use of common assay systems such as microplates or standard blotting membranes. The sample spot sizes are typically less than 200 microns in diameter. Thousands of spotted samples known as probes are immobilized onto a solid support. The probes can include DNA, cDNA, or oligonucleotides. These are used to determine the complementary binding of the unknown sequences, therefore allowing parallel analysis for gene expression and discovery. In certain aspects, genomic DNA is used. The genes might differ from each other by as little as a single nucleotide base. This single base difference between two sequences is known as a single nucleotide polymorphism (SNP). SNPs can be used to distinguish between otherwise highly similar strains of bacteria. The use of microarrays to detect and differentiate microorganisms such as bacteria is well-known in the art. Additional detail can be found, for example, in Zhou et al., Microarrays for bacterial detection and microbial community analysis, Current Opinion in Microbiology, 2003, 6: 288-294, and U.S. Patent Application No. 10/418,837.

In an aspect of the disclosure, species that are genetically-similar or closely related are identified and grouped so that a representative species may be used to represent the entire grouping in antibody production. The groupings serve as guides to selection of a minimal subset of strains representative of the spectrum of genomic variation existing within each species. With sequencing, the entire genome of a species (such as bacteria) can be obtained and analyzed for genetic relationships and similarities. For bacteria, pathogens, viruses, etc., the entire genome or specific genes within the genome can be compared for distinguishing species or classes. See for example Syst Appl Microbiol. 2010 Jun;33(4):175-82. doi: 10.1016/j.syapm.2010.03.003. Epub 2010 Apr 20, which is incorporated by reference and discusses revealing phylogenetic coherence by 16S rRNA gene analyses.

It would be appreciated that the percentage of similarities to which to base a determination of grouping can vary depending on the species. For example, a method could be employed that groups pathogens together based upon a 97% similarity determination. Other methods could employ grouping based upon a 92% similarity determination. Other methods could employ grouping based upon a 85% grouping determination.

Historically, bacteria and other microorganisms were classified based upon shape and staining methods. However, advances in sequencing provide for the opportunity to reconcile microbial systematics and genomics. See Syst Appl Microbiol. 2010 Jun;33(4):175-82. doi, 10.1016/j.syapm.2010.03.003. Epub 2010 Apr 20. Classifying or grouping pathogens or bacteria on the basis of genomic similarities allows for prediction of common antigens or surface proteins.

The present disclosure provides a method that incorporates using DNA sequencing data to determine genetic relatedness for the purposes of grouping, not based on shape or staining methods, but on genomic similarities. Classification and analysis based upon genomes or DNA sequencing allows for genetically similar bacteria to be determined. Whole-genome sequences can also be quantified to measure divergence due to all processes, including vertical and horizontal transfer and genome reduction. See IJSEM November 2003 vol. 53 no. 6 1893-1900, which is incorporated by reference.

Once the sequences are compared using any of the above methods, similar sequences may be matched together using classification schemes known in the art, including the preparation of cladograms. A cladogram is a branching, tree-like diagram in which the endpoints of the branches represent specific species of organisms. It is used to illustrate phylogenetic relationships and show points at which various species have diverged from common ancestral forms. Although cladograms have traditionally been constructed largely on the basis of morphological characteristics, DNA and RNA sequencing data can also be used to prepare cladograms. Cladograms are constructed by grouping organisms together based on their shared derived characteristics.

A greatly simplified procedure for generating a cladogram involves the steps of gathering and organizing data, considering the possible cladograms, and from those cladograms, selecting the best cladogram. Cladistic analysis begins by compiling the list of taxa to be organized; the list of characteristics to be compared; and for each taxon, the values of each of the listed characteristics. For example, if analyzing 20 different strains of bacterial within a species, the data might comprise the list of the 20 strains; the list of characteristics might comprise only genome sequence information; and for each of the 20 strains, its particular genome sequence. All the data are then organized into a taxon-character matrix, which is then used to perform the phylogenetic analysis.

The inference of phylogenies from genetic data can incorporate various statistical methods, such as use of a parsimony criterion, maximum likelihood models, Bayesian inference, and the use of genomic retrotransposon markers.

The consideration of potential cladograms is typically performed by computer. A typical cladistics program begins by using heuristic techniques to identify a small number of candidate cladograms. Many cladistics programs then continue the search by repeating the following steps: evaluation of the candidate cladograms by comparing them to the characteristic data; identification of the best candidates that are most consistent with the characteristic data; creation of additional candidates by creating several variants of each of the candidates from the prior step; use of heuretics to create several new candidate cladograms unrelated to the prior candidates; repeating these steps until no further improvement is observed.

In selecting the best cladogram, an algorithm may be used. The algorithm may be implemented by computer. Most algorithms use a metric to determine how consistent a candidate cladogram is with the data. Most cladogram algorithms use the mathematical techniques of optimization and minimization. Algorithms for cladograms can further include least squares, neighbor-joining, parsimony, maximum likelihood, and Bayesian inference. Further detail on the generation of cladograms is provided in U.S. Patent No. 7,043,371. Once the cladograms have been prepared, the cladogram can be used to identify minimally different subsets of bacteria. These minimally different subsets can then be used to generate target specific binding moieties. Using DNA sequencing whole genome sequences can be employed to determine genetic closeness among related bacteria. See for example, Nature. 2009 December 24; 462(7276): 1056-1060, doi: 10.1038/nature08656, which discusses sequencing and analyzing genomes of Bacteria and Archaea for reconstruction of phylogenetic history and the discovery of new protein families and biological properties for prediction functions of known genes. Genetic similarities can be indicative of surface protein or antigenic similarities and provide a method for grouping pathogens by their genomes.

Once the genomic sequence is identified, as discussed above, the critical genetic characteristics of major antigens of the pathogen can be identified, which can be implemented in organizing groups into subgroups in order to find representative species. See for example Korean J Parasitol. 2009 October; 47(Suppl): S51-S58, Published online 2009 October 26. doi: 10.3347/kjp.2009.47.S.S51, wherein the Plasmodium vivax protozoan pathogen genomic sequence is analyzed for identification of major antigens of the pathogen. Therefore, instead of selecting a group based upon whole genomic similarity, groups could also be formed by the genetic similarities within surface protein or antigen genes. The genomic make-up of the groups of pathogens can be employed to identify genes related to surface proteins or antigens common within a group. The surface proteins or antigens associated or common within a group of the pathogens, can be determined by known methods in the art. See for example Genes & Genetic Systems [2004, 79(3):129-137], DOI: 10.1266/ggs.79.129, which discloses the identification of surface protein antigen genes. Surface proteins are likely to interact with the host immune system and are ideal candidates for vaccine development. In this aspect of the disclosure, identifying or grouping species based upon associated antigen genes allows for selection of a representative species or species. In an example, a series of monoclonal antibodies were isolated which reacted with one of two major surface proteins of rhesus rotavirus, and effectively neutralized the rhesus rotavirus. See J. Virol. August 1983 vol. 47 no. 2 267-275, which is incorporated by reference. See the section entitled "Methods of Antibody Production" for further details.

Compositions of the disclosure may be designed to isolate different species of bacteria. These different species may include gram positive bacteria, gram negative bacteria, or a combination of gram positive and gram negative bacteria. Such compositions allow for isolation of essentially all bacteria from a sample.

In still other embodiments, compositions are designed to isolate specific pathogen from a sample. Exemplary bacterial species that may be captured and isolated by methods of the invention include E. coli, Listeria, Clostridium, Mycobacterium, Shigella, Borrelia, Campylobacter, Bacillus, Salmonella, Staphylococcus, Enterococcus, Pneumococcus, Streptococcus, and a combination thereof. These sets can be mixed together to isolate for example, E. coli and Listeria; or E. coli, Listeria, and Clostridium; or Mycobacterium, Campylobacter, Bacillus, Salmonella, and Staphylococcus, etc. Any combination of sets may be used and compositions of the invention will vary depending on the suspected pathogen or pathogens to be isolated.

A suitable nucleic acid probe assay generally includes sample treatment and lysis, hybridization with selected probe(s), hybrid capture, and detection. This method is discussed in the prior sections. Detection of bacteria of interest can also be performed by use of PCR techniques, which have been discussed above. An exemplary embodiment would entail employing genotyping to survey the extent of genetic variation within a species. Once genomes have been identified, the genomes are grouped so that each group member is 97% similar to other group members. Within the grouped genomically-similar isolates, one isolate from each group is selected to represent the entire group. Cadograms could be employed to guide selection of a minimal subset of strains representative of the spectrum of genomic variation within each species. Each representative is then used in antibody production as immunogens to produce a spectrum of antibodies to address pathogens within a species.

Multi-functionalized Magnetic Particles

In another embodiment, the disclosure provides magnetic particles in which each particle is conjugated to at least two sets of antibodies, with each set specific for a different pathogen. Compositions of the disclosure may be introduced to a body fluid sample in order to create a mixture. The mixture is incubated to allow the particles to bind to multiple types of pathogens in the body fluid, and a magnetic field is applied to capture pathogen/magnetic particle complexes on a surface. Optionally, the surface can be washed with a wash solution that reduces particle aggregation, thereby isolating pathogen/magnetic particle complexes. A particular advantage of the present compositions is the ability to capture and isolate different types of pathogens, such as different species of bacteria, directly from blood samples at low concentrations typical of many clinical samples (as low as 1 CFU/ml of bacteria in a blood sample). In certain embodiments, such compositions may be used with the above described cartridges. In other embodiments, such compositions may be used without the need for the above described cartridges.

In certain aspects, the disclosure provides a composition for isolating pathogen from a heterogeneous sample. The composition includes at least one magnetic particle in which the magnetic particle is conjugated to at least two sets of antibodies, with each set of antibodies specific for a different pathogen. The antibodies conjugated to the particles may be either monoclonal or polyclonal antibodies. Compositions of the disclosure may be used with any type of heterogeneous sample. In particular embodiments, the heterogeneous sample is a blood sample.

Since each particle is conjugated with antibodies have different specificities for different pathogens, compositions of the disclosure may be provided such that each set of antibodies on the particle is present at a concentration designed for detection of a specific pathogen in the sample. In certain embodiments, the concentration ratio of one set of antibodies relative to a second set of antibodies is at a ratio of 50:50. In other embodiments, the concentration ratio ranges from 1:99 to 99:1.

To facilitate detection of the different sets of pathogen/magnetic particle complexes the particles may be detectably labeled, as discussed above in the related section.. Any detectable label may be used with compositions of the invention, such as fluorescent labels, radiolabels, enzymatic labels, and others. In particular embodiments, the detectable label is an optically-detectable label, such as a fluorescent label. Exemplary fluorescent labels include Cy3, Cy5, Atto, cyanine, rhodamine, fluorescien, coumarin, BODIPY, alexa, and conjugated multi-dyes.

Compositions of the disclosure may be designed to isolate different species of bacteria from a sample. These different species may include gram positive bacteria, gram negative bacteria, or a combination of gram positive and gram negative bacteria. Exemplary bacterial species that may be captured and isolated by methods of the invention include E. coli, Listeria, Clostridium, Mycobacterium, Shigella, Borrelia, Campylobacter, Bacillus, Salmonella, Staphylococcus, Enterococcus, Pneumococcus, Streptococcus, and a combination thereof.

Compositions of the disclosure are not limited to isolating multiple types of pathogens from a body fluid. Compositions of the invention may be designed to isolate multiple types of other target analytes, such as fungi, protein, a cell, a virus, a nucleic acid, a receptor, a ligand, or any molecule known in the art.

Compositions of the disclosure may use any type of magnetic particle, as discussed above in the related section. In particular embodiments, the target-specific binding moiety is an antibody, such as an antibody that binds a particular bacterium. General methodologies for antibody production are described above in the section entitled, "Methods of Antibody Production." Any antibody or fragment thereof having affinity and specific for the bacteria of interest is within the scope of the disclosure provided herein. Immunomagnetic particles against Salmonella are provided in Vermunt et al. (J. Appl. Bact. 72:112, 1992). Immunomagnetic particles against Staphylococcus aureus are provided in Johne et al. (J. Clin. Microbiol. 27:1631, 1989). Immunomagnetic particles against Listeria are provided in Skjerve et al. (Appl. Env. Microbiol. 56:3478, 1990). Immunomagnetic particles against Escherichia coli are provided in Lund et al. (J. Clin. Microbiol. 29:2259, 1991).

Methods for attaching the target-specific binding moiety to the magnetic particle are known in the art, and are discussed in the preceding sections. Since each particle is conjugated with antibodies having different specificities for different pathogens, compositions of the invention may be provided such that each set of antibodies on the particle is present at a concentration designed for detection of a specific pathogen in the sample. In certain embodiments, the concentration ratio of one set of antibodies relative to a second set of antibodies is 50:50. In other embodiments, the concentration ratio ranges from 1:99 to 99:1. For example, particles may be prepared such that antibody sets that bind gram positive bacteria are added to a preparation mixture at a concentration of $2 \times 10^9$ antibodies per/ml, while antibody sets that bind gram negative bacteria are added to the preparation mixture at a concentration of $4 \times 10^9$ antibodies per/ml. Compositions of the disclosure are not affected by antibody cross-reactivity. However, in certain embodiments, sets are specifically designed such that there is no cross-reactivity between different antibodies and different sets.

Compositions of the disclosure may be designed to isolate different species of bacteria. These different species may include gram positive bacteria, gram negative bacteria, or a combination of gram positive and gram negative bacteria. Such compositions allow for isolation of essentially all bacteria from a sample.

In still other embodiments, compositions are designed to isolate specific pathogen from a sample. Exemplary bacterial species that may be captured and isolated by methods of the invention include E. coli, Listeria, Clostridium, Mycobacterium, Shigella, Borrelia, Campylobacter, Bacillus, Salmonella, Staphylococcus, Enterococcus, Pneumococcus, Streptococcus, and a combination thereof. These sets can be mixed together to isolate for example, E. coli and Listeria; or E. coli, Listeria, and Clostridium; or Mycobacterium, Campylobacter, Bacillus, Salmonella, and Staphylococcus, etc. Any combination of sets may be used and compositions of the invention will vary depending on the suspected pathogen or pathogens to be isolated.

Capture of a wide range of different target microorganisms simultaneously can be achieved by utilizing multiple sets of antibodies, with each set specific to target class, such as pan-Gram-positive antibodies, pan-Gran-negative antibodies or antibodies specific to a subset of organisms of a certain class. Further, expanded reactivity can be achieved by mixing particles of different reactivity. The addition of a high concentration of non-specific particles does not interfere with the capture efficiency of target-specific particles. Similarly, several different particle preparations can be combined to allow for the efficient capture of desired pathogens. In certain embodiments the particles can be utilized at a concentration between $1 \times 10^8$ and $5 \times 10^{10}$ particles/mL.

In certain embodiments the expanded coverage can be provided by mixing antibodies with different specificity before attaching them to magnetic particles. Purified antibodies can be mixed and conjugated to activated magnetic particle using standard methods known in the art.

To facilitate detection of the pathogen/magnetic particle complexes the particles may be detectably labeled. See the previous sections for a discussion of labels. The sample is then mixed with compositions of the invention to generate a mixture that is allowed to incubate such that the compositions of the invention can bind to at least two different types of bacteria in the blood sample. The types of bacteria that will bind compositions of the invention will depend on the design of the composition, i.e., which antibodies are conjugated to the particles. The mixture is allowed to incubate for a sufficient time to allow for the composition to bind to the pathogen in the blood. The process of binding the composition to the pathogen associates a magnetic moment with the pathogen, and thus allows the pathogen to be manipulated through forces generated by magnetic fields upon the attached magnetic moment.

In general, incubation time will depend on the desired degree of binding between the pathogens and the compositions of the invention (e.g., the amount of moment that would be desirably attached to the pathogens), the amount of moment per target, the amount of time of mixing, the type of mixing, the reagents present to promote the binding and the binding chemistry system that is being employed. Incubation time can be anywhere from about 5 seconds to a few days. Exemplary incubation times range from about 10 seconds to about 2 hours. Binding occurs over a wide range of temperatures, generally between 15 °C and 40 °C.

In certain embodiments, a buffer solution, as discussed in prior sections, is added to the sample along with the compositions of the invention. One can also use a static mixer or other device that provides efficient mixing of viscous samples at high flow rates to achieve high binding efficiency while reducing time required for the binding step. In one embodiment, the sample is mixed with binding buffer in ratio of, or about, 1:1, using a mixing interface connector. The diluted sample then flows through a mixing interface connector where it is mixed with target-specific nanoparticles. Additional mixing interface connectors providing mixing of sample and antigen-specific nanoparticles can be attached downstream to improve binding efficiency. The combined flow rate of the labeled sample is selected such that it is compatible with downstream processing.

After binding of the compositions of the disclosure to pathogens in the sample to form complexes comprising the magnetic particle and at least two different types of pathogens, a magnetic field is applied to the mixture to capture the complexes on a surface. Components of the mixture that are not bound to magnetic particles will not be affected by the magnetic field and will remain free in the mixture. As discussed in prior sections, methods and apparatuses for separating target/magnetic particle complexes from other components of a mixture are known in the art. In certain embodiments, the magnetic capture is achieved at high efficiency by utilizing a flow-through capture cell with a number of strong rare earth bar magnets placed perpendicular to the flow of the sample. When using a flow chamber with flow path cross-section 0.5 mm x 20 mm (h x w) and 7 bar NdFeB magnets, the flow rate could be as high as 5 mL/min or more, while achieving capture efficiency close to 100%.

The above described type of magnetic separation produces efficient capture of a target analyte and the removal of a majority of the remaining components of a sample mixture. However, such a process produces a sample that contains a very high percent of magnetic particles that are not bound to target analytes because the magnetic particles are typically added in excess, as well as non-specific target entities. Non-specific target entities may for example be bound at a much lower efficiency, for example 1% of the surface area, while a target of interest might be loaded at 50% or nearly 100% of the available surface area or available antigenic cites. However, even 1 % loading may be sufficient to impart force necessary for trapping in a magnetic gradient flow cell or sample chamber.

For example, in the case of immunomagnetic binding of bacteria or fungi in a blood sample, the sample may include: bound targets at a concentration of about 1/mL or a concentration less than about 106/mL; background particles at a concentration of about 107/ml to about 1010/ml; and non-specific targets at a concentration of about 10/ml to about 105/ml.

The presence of magnetic particles that are not bound to target analytes and non-specific target entities on the surface that includes the target/magnetic particle complexes interferes with the ability to successfully detect the target of interest. The magnetic capture of the resulting mix, and close contact of magnetic particles with each other and bound targets, result in the formation of aggregate that is hard to dispense and which might be resistant or inadequate for subsequent processing or analysis steps. In order to remove magnetic particles that are not bound to target analytes and non-specific target entities, the surface may be washed with a wash solution that reduces particle aggregation, thereby isolating target/magnetic particle complexes from the magnetic particles that are not bound to target analytes and non-specific target entities. The wash solution (discussed in prior sections) minimizes the formation of the aggregates.

Once the target/magnetic particle complexes are isolated, the targets may be analyzed by a multitude of existing technologies, such as miniature NMR, Polymerase Chain Reaction (PCR), mass spectrometry, fluorescent labeling and visualization using microscopic observation, fluorescent in situ hybridization (FISH), growth-based antibiotic sensitivity tests, and variety of other methods that may be conducted with purified target without significant contamination from other sample components. In one embodiment, isolated bacteria are lysed with a chaotropic solution, and DNA is bound to DNA extraction resin. After washing of the resin, the bacterial DNA is eluted and used in quantitative RT-PCR to detect the presence of a specific species, and/or, subclasses of bacteria.

In another embodiment, captured bacteria is removed from the magnetic particles to which they are bound and the processed sample is mixed with fluorescent labeled antibodies specific to the bacteria or fluorescent Gram stain. After incubation, the reaction mixture is filtered through 0.2 $\mu$m to 1.0 $\mu$m filter to capture labeled bacteria while allowing majority of free particles and fluorescent labels to pass through the filter. Bacteria is visualized on the filter using microscopic techniques, e.g. direct microscopic observation, laser scanning or other automated methods of image capture. The presence of bacteria is detected through image analysis. After the positive detection by visual techniques, the bacteria can be further characterized using PCR or genomic methods.

Detection of bacteria of interest can be performed by use of nucleic acid probes following procedures which are known in the art. Suitable procedures for detection of bacteria using nucleic acid probes are described, for example, in Stackebrandt et al. (U.S. 5,089,386), King et al. (WO 90/08841), Foster et al. (WO 92/15883), and Cossart et al. (WO89/06699).

A suitable nucleic acid probe assay generally includes sample treatment and lysis, hybridization with selected probe(s), hybrid capture, and detection. This assay method is discussed in detail in the prior sections. Detection of bacteria of interest can also be performed by use of PCR techniques. A suitable PCR technique is described, for example, in Verhoef et al. (WO 92/08805). Such protocols may be applied directly to the bacteria captured on the magnetic particles. The bacteria are combined with a lysis buffer and collected nucleic acid target molecules are then utilized as the template for the PCR reaction.

For detection of the selected bacteria by use of antibodies, isolated bacteria are contacted with antibodies specific to the bacteria of interest. As noted above, either polyclonal or monoclonal antibodies can be utilized, but in either case have affinity for the particular bacteria to be detected. These antibodies will adhere/bind to material from the specific target bacteria. With respect to labeling of the antibodies, these are labeled either directly or indirectly with labels used in other known immunoassays. Direct labels may include fluorescent, chemiluminescent, bioluminescent, radioactive, metallic, biotin or enzymatic molecules. Methods of combining these labels to antibodies or other macromolecules are well known to those in the art. Examples include the methods of Hijmans, W. et al. (1969), Clin. Exp. Immunol. 4, 457-, for fluorescein isothiocyanate, the method of Goding, J. W. (1976), J. Immunol. Meth. 13, 215-, for tetramethylrhodamine isothiocyanate, and the method of Ingrall, E. (1980), Meth. in Enzymol. 70, 419-439 for enzymes.

These detector antibodies may also be labeled indirectly. In this case the actual detection molecule is attached to a secondary antibody or other molecule with binding affinity for the anti-bacteria cell surface antibody. If a secondary antibody is used it is preferably a general antibody to a class of antibody (IgG and IgM) from the animal species used to raise the anti-bacteria cell surface antibodies. For example, the second antibody may be conjugated to an enzyme, either alkaline phosphatase or to peroxidase. To detect the label, after the bacteria of interest is contacted with the second antibody and washed, the isolated component of the sample is immersed in a solution containing a chromogenic substrate for either alkaline phosphatase or peroxidase. A chromogenic substrate is a compound that can be cleaved by an enzyme to result in the production of some type of detectable signal which only appears when the substrate is cleaved from the base molecule. The chromogenic substrate is colorless, until it reacts with the enzyme, at which time an intensely colored product is made. Thus, material from the bacteria colonies adhered to the membrane sheet will become an intense blue/purple/black color, or brown/red while material from other colonies will remain colorless. Examples of detection molecules include fluorescent substances, such as 4-methylumbelliferyl phosphate, and chromogenic substances, such as 4-nitrophenylphosphate, 3,3',5,5'-tetramethylbenzidine and 2,2'-azino-di-[3-ethelbenz-thiazoliane sulfonate (6)]. In addition to alkaline phosphatase and peroxidase, other useful enzymes include $\beta$-galactosidase, $\beta$-glucuronidase, $\alpha$-glucosidase, $\beta$-glucosidase, $\alpha$-mannosidase, galactose oxidase, glucose oxidase and hexokinase.

In certain embodiments, methods of the invention are useful for direct detection of multiple species of bacteria in blood. Such a process is described here. Sample is collected in sodium heparin tube by venipuncture, acceptable sample volume is about 1 mL to 10 mL. Sample is diluted with binding buffer and superparamagnetic particles, each having multiple sets of target-specific binding moieties specific for different targets are added to the sample. The sample is then incubated on a shaking incubator at 37 oC for about 30 min to 120 min. Alternative mixing methods can also be used. In a particular embodiment, sample is pumped through a static mixer, such that reaction buffer and magnetic particles are added to the sample as the sample is pumped through the mixer. This process allows for efficient integration of all components into a single fluidic part, avoids moving parts and separate incubation vessels and reduces incubation time.

Capture of the labeled targets allows for the removal of blood components and reduction of sample volume from 30 mL to 5 mL. The capture is performed in a variety of magnet/flow configurations. In certain embodiments, methods include capture in a sample tube on a shaking platform or capture in a flow-through device at flow rate of 5 mL/min, resulting in total capture time of 6 min.

After capture, the sample is washed with wash buffer including heparin to remove blood components and free particles. The composition of the wash buffer is optimized to reduce aggregation of free particles, while maintaining the integrity of the particle/target complexes.

The detection method is based on a miniature NMR detector tuned to the magnetic resonance of water. When the sample is magnetically homogenous (no bound targets), the NMR signal from water is clearly detectable and strong. The presence of magnetic material in the detector coil disturbs the magnetic field, resulting in reduction in water signal. One of the primary benefits of this detection method is that there is no magnetic background in biological samples which significantly reduces the requirements for stringency of sample processing. In addition, since the detected signal is generated by water, there is a built-in signal amplification which allows for the detection of a single labeled bacterium.

Methods For Universal Target Capture

Early detection of bacterial infections is the key to preventing the onset of sepsis. Traditional methods of detecting blood-borne infections include lab cultures that require days to complete. Other molecular methods of detecting bacteria require the bacteria to first be captured and the DNA isolated from the captured bacteria. Capturing the bacteria typically requires prior knowledge about the bacteria that is sought to be detected. That information is not always available, particularly when a patient comes to a healthcare facility having an unknown infection. Certain aspects of the invention provide methods for universal target capture.

Methods of the invention involve introducing an agent to a sample that causes an unknown target in the sample to display a universally recognized element. Accordingly, any capture moiety that is able to bind to the element can be used to isolate the target from the sample, regardless of the type of target in the sample. Methods of the invention are particularly useful for isolating unknown microorganisms from a sample by causing the microorganisms to display a certain protein that is recognized by an antibody, so that the antibody can be used to capture the microorganism in the sample. In certain embodiments, such methods may be carried out with the above described cartridges. In other embodiments, such methods are conducted without the need for the above described cartridges.

In a particular embodiment, the target can then be isolated using a binding partner that specifically binds to the binding element; such as an antibody binding to an antigen or streptavidin binding to biotin, and others known in the art. In one embodiment, the binding element is attached to a substrate, enabling the sample to be washed, leaving behind the target to be detected. In a preferred embodiment, the targets are pathogens or environmental hazards.

Methods of the invention involve using a magnetic particle as a substrate attached to a binding partner, thereby forming a target/magnetic particle complex. Methods of the invention can further involve applying a magnetic field to capture the target/magnetic complex on a surface and wash away the sample. The invention further provides devices for extracting the pathogens. Devices of the invention can involve fluidic channels and chambers having macrofluidic dimension, microfluidic dimensions, or both.

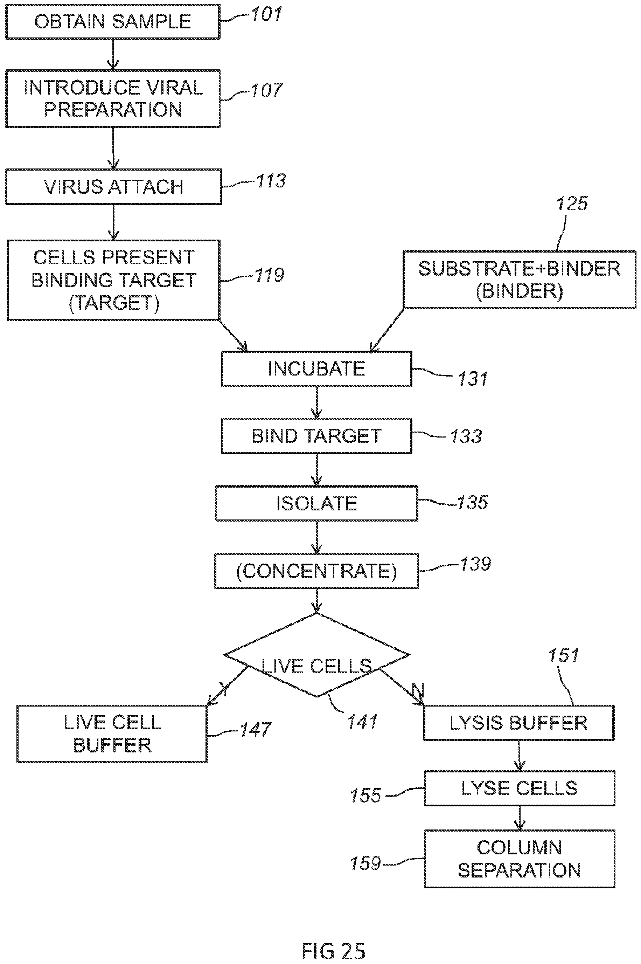

FIG. 25 provides an exemplary diagram of certain methods for isolating different pathogens. A sample that contains two or more different pathogens, or targets, is obtained 101.

In certain aspects, the target pathogen is a bacterium or two or more different bacteria. Methods of devices of the invention can be used to isolate or extract known bacteria, unknown bacteria, or a combination thereof. Both gram positive bacteria, gram negative bacteria, archaea, or eukaryotes can be isolated using the methods disclosed herein. Specific genera of pathogens that may be sought and assayed for using the disclosed methods include Alphaproteobacteria, Bacillus, Betaproteobacteria, Bifidobacterium, Borrelia, Campylobacter, Candida, Citrobacter, Clostridium, Enterobacter, Enterococcus, Escherichia, Flavobacterium, Fusobacterium, Gammaproteobacteria, Klebsiella, Kluyvera, Lactobacillus, Legionella, Leuconostoc, Listeria, Micrococcus, Mycobacterium, Neisseriaceae, Pediococcus, Pneumococcus, Porphyromonas, Prevotella, Propionibacterium, Proteus, Rhodospirillum, Rickettsia, Saccharomyces, Salmonella, Serratia, Shigella, Sphaerotilus, Staphylococcus, Streptococcus, Thermoanaerobacter, Thermoproteus, Vibrio, and Yersinia.

Organisms that can be assayed for with methods and devices of the invention include Acinetobacter calcoaceticus, Aeromonas hydrophilia, Bacillus anthracis, Bacillus subtilis, Candida albicans, Citrobacter freundii, E. coli, Enterobacter aerogenes, Enterobacter cloacae, Enterococcus faecium, Fusobacterium nucleatum, Klebsiella oxytoca, Klebsiella pneumoniae, Klebsiella travesanii, Kluyvera ascorbata, Lactobacillus casei, Lactobacillus cellobiosus, Lactobacillus fermentum, Lactobacillus helveticus, Lactobacillus paracasei, Lactobacillus salivarius, Listeria monocytogenes, Micrococcus luteus, Pediococcus parvulus, Porphyromonas gingivalis, Prevotella intermedia, Propionibacterium freudenreichii, Proteus vulgaris, Pseudomonas aeruginosa, Rhodospirillum rubrum, Rickettsia conorii, Saccharomyces cerevsiae, Salmonella agona, Salmonella enteritidis, Salmonella heidelberg, Salmonella infantis, Salmonella minnesota, Salmonella montevideo, Salmonella ohio, Salmonella typhimurium, Serratia marcescens, Shigella flexneri, Sphaerotilus natans, Staphylococcus aureus, Staphylococcus epidermis, Staphylococcus xylosus, Streptococcus faecalis, Streptococcus pyrogenes, Thermoproteus tenax, Vibrio cholerae, and Yersinia pseudotuberculosis. Any combination of organisms may be the target of methods and devices of the invention and the target of an assay will vary depending on the suspected pathogen or pathogens to be isolated.

Methods of the invention may be conducted on any sample, and exemplary samples are described above. In particular embodiments, the sample is a blood sample. As shown in FIG. 25, once the sample is obtained 101, a viral preparation is introduced 107 into the sample. The viral preparation includes at least one virus that attaches 113 to the pathogens. As a result, the pathogens present 119 a binding element.

Any viral preparation may be used that causes different unknown pathogens to present a binding element. In some embodiments, the viral preparation contains a cocktail of bacteriophage. The cocktail contains different phage viruses that infect different bacteria. In certain embodiments, a broad-host-range phage is used that can infect different bacteria.

Phages are viruses that can be used as a transduction vector to introduce genetic material into the different pathogens and induce expression of, for example, a common antigen. Phage can be used as transduction vectors to introduce exogenous (non-viral) genetic material into the pathogens. See U.S. Pat. 7,148,054 for discussion. During lytic infection with a phage, DNA is packaged into phage heads as phage particles are formed. Promiscuous high-transducing (HT) mutants of P22 which efficiently package DNA with little sequence specificity are known.

As diagrammed in FIG. 25, infection involves attachment 113 of the phage particle to a target cell. When transduction is successful, the exogenous DNA is received in the target cell and expressed 119. For example, RecA-mediated homologous recombination following injection of the donor fragment can result in the inheritance of donor traits.

Any suitable phage or combination of phages can be used in methods of the invention. Examples of transducing phage vectors include bacteriophages P1 and P22 of E. coli and S. typhimurium, respectively. Other phages are suitable for use with methods of the invention. For example, the bacteriophage Mu can be used to insert DNA into a target pathogen genome for expression 119. See Groisman, 1991, In vivo genetic engineering with bacteriophage Mu, Methods in Enzymology 202:180-212. Further, hybrid phages such as Mud-P22 may be used. Mud-P22 is a hybrid combining features of phage Mu and P22. Mud-P22 can be inserted at essentially any desired site on the Salmonella chromosome. See Crain, 2007, Mud-P22, Methods Enzymology 421:249-59. Other exemplary phages (and their targets) include T4 (E. coli), T5 (E. coli), λ phage (E. coli), T7 phage (E. coli), G4 (E. coli), P1 (E. coli), φ6 (Pseudomonas), Thermoproteus tenax virus 1 (Thermoproteus tenax), M13 (E. coli), MS2 (E. coli), Qβ (E. coli), φX174 (E. coli), Φ29 (Bacillus), PZA (Bacillus), Φ15 (Bacillus), BS32 (Bacillus), B103 (Bacillus), M2Y (M2) (Bacillus), Nf (Bacillus), GA-1 (Bacillus), FWLBc1 (Bacillus), FWLBc2 (Bacillus), FWLLm3 (Listeria), B4 (Propionibacterium).

Phages are discussed in Chopin, et al., 2002, J Bact 184(7):2030-2033; Muramatsu et al., 1991, Two generalized transducing phages in Vibrio parahaemolyticus and Vibrio alginolyticus, Microbiol Immunol 35(12): 1073-1084; Regue et al., 1991, A generalized transducing bacteriophage for Serratia marcescens, Res Microbiol 42(1):23-27; Kiesel et al., 1993, Phage Acm1-mediated transduction in the facultatively methanol-utilizing Acetobacter methanolicus MB 58/4, J. Gen Virol 74(9):1741-1745; Zhang, et al., 2012, Food Microbiol 31(1):133-36; U.S. Pat. 7,732,150; U.S. Pub. 2009/0246752; U.S. Pub. 2009/0047254; and U.S. Pub. 2004/0156831.

Exemplary phages are further discussed in Welker, 1988, Transduction in Bacillus stearothermophilus, J. Bacteriol, 176(11):3354-3359; Darzins et al., 1989, Mini-D3112 bacteriophage transposable elements for genetic analysis of Pseudomonas aeruginosa, J. Bacteriol 171(7):3909-3916; Blahova et al., 1994, Transduction or imipenem resistance by the phage F-116 from a nosocomial strain of Pseudomonas aeruginosa isolated in Slovakia, Acta Virol 38(5):247-250; Weiss et al., 1994, Isolation and characterization of a generalized transducing phage for Xanthomonas campestris pv. campestris, J. Bacteriol 176(11): 3354-3359; Schicklmaier et al., 1995, Frequency of generalized transducing phages in natural isolates of the Salmonella typhimurium complex, Appl Environ Microbiol 61(4): 61(4): 1637-1640; Humphrey et al., 1997, Purification and characterization of VSH-1, a generalized transducing bacteriophage of Serpulina hyodysenteriae, J Bacteriol 179(2):323-329; Willi et a., 1997, Transduction of antibiotic resistance markers among Actinobacillus actinomycetemcomitans strains by temperate bacteriophages Aa phi 23, Cell Mol Life Sci 53(11-12):904-910; Nedelmann et al., 1998, Generalized transduction for genetic linkage analysis and transfer of transposon insertions in different Staphylococcus epidermidis strains, Zentiviralalbl Bakteriol 287(1-2):85-92; Int. Pat. Application Pub. WO 2003/035889; U.S. Pat. 8,071,337 and U.S. Pat. 7,951,579.

Any suitable phage virus or viruses, including any of those mentioned herein, can be included in the viral preparation. A viral preparation according to the disclosure is a composition containing a vector or viral material with the ability to interact with one or more different targets. A broad-host-range can be provided by including a cocktail of phages (e.g., two or more of any phage such as those mentioned herein), a phage that infects a range of hosts, or a combination thereof.

Any suitable broad-host-range phage can be used. For example, phage SN-1, SN-2, SN-X, SN-T, BHR1, BHR2, BHR3, BHR4, BHR5, PRD1, KVP40, PY100, PRD1, PVP-SE1, or a combination thereof may be included in a viral preparation. Broad host range phages are discussed in Miller et al., 2003, Complete genome sequence of the broad-host-range vibriophage KVP40: comparative genomics of a T4-related bacteriophage; J Bact 185(17):5220-5233; Beumer, 2005, A broad-host-range, generalized transducing phage SN-T acquires 16S rRNA genes from different genera of bacteria, Appl Env Microb 71(12):8301-8304; Green et al., 1985, Isolation and preliminary characterization of lytic and lysogenic phages with wide host range within the streptomycetes, J. Gen Microbiol 131(9):2459-2465; Jensen et al., 1998, Prevalence of broad-host-range lytic bacteriophages of Sphaerotilus natans, Escherichia coli, and Pseudomonas aeruginosa, Appl Environ Microbiol 64(2):575-580; Bamford et al., 1995, Bacteriophage PRD1: a broad host range dsDNA tectivirus with an internal membrane, Adv Virus Res 45:281-319; Schwudke, et al., 2008, Broad-host-range Yersinia phage PY100: genome sequence, proteome analysis of virions, and DNA packaging strategy, J Bact 190(1):332-342; Olsen et al., 1974, Characteristics of PRD1, a plasmid-dependent broad host range DNA bacteriophage, J Viriol 14(3):689-699; Santos et al., 2011, Genomic and proteomic characterization of the broad host range Salmonella phage PVP-SE1: creation of a new phage genus, J Viriol 85(21):11265-73; Sillankorva et al., Efficacy of a broad host range lytic bacteriophage against E. coli adhered to urothelium, Curr Microbiol 62(4):1128-1132; Evans et al, 2010, Characterization of a broad-host-range flagellum-dependent phage that mediates high-efficiency generalized transduction in, and between, Serratia and Pantoea, Microbiol 156:240-247; Garbe et al., 2010, Characterization of JG024, a Pseudomonas aeruginosa PB1-like broad-host range phage under simulated infection conditions, BMC Microbiol 10:301; Schwarzer et al., 2012, A multivalent adsorption apparatus explains the broad host range of phage phi92: a comprehensive genomic and structural analysis, J Viriol JVI.00801-12; U.S. Pub. 2012/0168372; U.S. Pub. 2012/0128652; and U.S. Pub. 2011/0064699.

A broad-host-range viral preparation can be provided by including more than one phage in the composition. For example, if each included phage is specific for one species, the phage cocktail will have the ability to infect multiple species. Any number of different phages (e.g., 3, 5, tens, hundreds) may be included in a phage cocktail. Those phages may themselves be narrow or broad in host range. Phage cocktails are discussed in Kelly et al., 2011, Development of a broad-host-range phage cocktail for biocontrol, Bioengineered Bugs 2:1, 31-37; Int. Application Pub. WO 02/07742; U.S. Pat. 6,121,036; U.S. Pub. 2009/0047254; and U.S. Pub 2005/0032036.

Other vectors may be used to cause different unknown pathogens to present a common binding element. Suitable vectors include plasmids, phagemids, and bacterial ghosts. In some embodiments, a plasmid is included, optionally enveloped in a lipid layer (e.g., a liposome, micelle, or reverse micelle), bi-layer, or a protein coat. Suitable plasmids are known in the art and include Col E1, RSF1030, clo DF13, R6K, F, R1, and Ent P 307.

In certain embodiments, one or more of the pathogens are targeted using a bacterial ghost. Bacterial ghosts are bacterial cell envelopes devoid of cytoplasmic content. A bacterial ghost can be included that exhibits one or more cell surface moieties that bind to any number of cell-surface targets on one or a range of bacteria. Further, the bacterial ghosts may each include the known common binding element (e.g., common antigen, biotinylated protein, streptavidin, etc.). Bacterial ghosts may be preferred since they can carry multiple surface moieties due to their size and are non-pathogenic themselves due to inactivity and can be stored (e.g., on-chip) for long periods of time. Bacterial ghosts are discussed in Lubitz et al., 2009, Applications of bacterial ghosts in biomedicine, Adv Exp Med Biol 655:159-70; Langemann et al., 2010, The bacterial ghost platform system: production and applications, Bioeng Bugs 1(5):326-36; Tabrizi et al., 2004, Bacterial ghosts-biological particles as delivery systems for antigens, nucleic acids, and drugs, Curr Opp Biotechnol 15:530-537; U.S. Pat. 7,067,639; U.S. Pat. 6,896,887; U.S. Pub. 2012/0040829; and U.S. Pub. 2010/0203082.

Preferably, the viral preparation is introduced 107 with a high concentration of phage or vector so that viruses attach 113 to all target pathogens. Viral preparations can further include additional components to facilitate transduction by, for example, contributing to the competency of the target cells to receive extrinsic genetic material or to inhibit infection or lysis by sources other than the phage vector or vectors used. Preparations may include one or more salt, buffer, chelator, ion, or combination thereof, in any suitable concentration. Infectious, non-transducing phage can be inhibited by ion chelators (e.g., citrate or EGTA). In some embodiments, bacterial nutrients are added to the viral preparation to encourage cell vitality. For example, trypticase soy or a sugar can be included.

As shown in FIG. 25, a binding partner, optionally bound to a substrate, is obtained 125 and introduced into the sample. The sample with the binding partner is incubated 131 so that the binding partner can bind 133 to the binding element on the pathogens. In some embodiments, the virus or vector is used to cause different pathogens to present different binding elements. The pathogens may then be extracted using different binding partners. For example, phages can be used that cause some bacteria to express antigen A, some to express antigen B, and so on. Substrate 219, such as magnetic beads, can then be used that include anti-A, anti-B, etc., antibodies bound to the surface. The different binding partners may be mixed on each substrate, or separated (e.g., some beads specific for antigen A and some for antigen B). This can facilitate downstream sorting (e.g., where magnetic meads have different magnetic strengths or other sortable properties). Once the pathogens are bound to the binding partner, the pathogens can be isolated 135 from the remainder of the sample. Methods are provided to optionally concentrate 139 the isolated pathogens. Isolation and concentration are discussed below.

The isolated pathogens may be handled according to whether isolated live cells or another product is intended 141. For live cells, the pathogens may be introduced 147 into a live cell buffer. Any other suitable detection or isolation may be performed. For example, where a product from the cells is desired, a lysis buffer may be introduced 151 to lyse 155 the cells. The lysate may be purified, extracted, separated, or similar. In some embodiments, DNA is extracted by separation 159 on a column.

Methods of the invention include using a viral preparation to cause the target pathogens to present 119 a binding element. Any suitable method of associating a pathogen with a binding element may be used with the invention. Suitable methods include expression of a cell-surface protein, phage display of a protein, or any other method of exposing a protein. A protein can be a binding element such as antigen or a protein can be further functionalized to present a binding element. For example, a protein can include a target for biotinylation and can be biotinylated such that biotin is a binding element and streptavidin can be used as a binding partner.

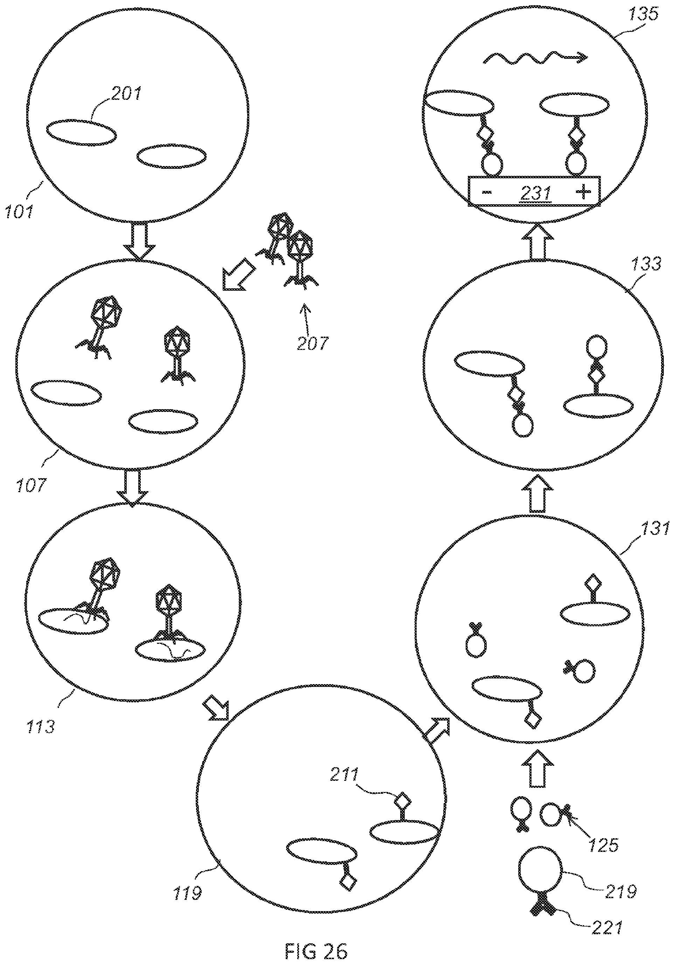

FIG. 26 illustrates the use of a viral preparation 207 to induce the expression of a common antigen 211 according to certain embodiments. As shown in FIG. 26, a sample containing unknown pathogens 201 is obtained 101 and viral preparation 207 is introduced 107. Viruses attach 113 to pathogens 201 and introduce exogenous DNA into those cells.

Phage viruses, such as any suitable one of those discussed herein, can be engineered to contain a gene for a common antigen 211 such as a cell surface protein. The phage or phages from viral preparation 207 inject the gene into pathogens 201. Pathogens 201 then express the gene causing them to present 119 antigen 211 on their cell surface. Engineering a gene into a phage vector is discussed in Green and Sambrook, 2012, Molecular Cloning: A Laboratory Manual 4Ed, Cold Spring Harbor Laboratory Press (Cold Spring Harbor, NY), 2,000 pages and Primrose and Twyman, 2006, Principles of Gene Manipulation and Genomics, 7th Edition, Wiley-Blackwell (Maiden, MA) 672 pages. Induction of antigen expression is discussed in Prisco and Berardinis, 2012, Filamentous bacteriophage Fd as an antigen delivery system in vaccination, Int J. Mol. Sci. 13:5179-5194 and Gahan et al., 2009, Bacterial antigen expression is an important component in inducing an immune response to orally administered Salmonella-delivered DNA vaccines, PLoS One 4(6):e6062.

Antibody 221 may be obtained for use as the binding partner 125. As shown in FIG. 26, antibody 221 is bound to substrate 219. Substrate 219 can be any suitable substrate known in the art. In some embodiments, substrate 219 is the surface of a fluidic channel or other vessel. In some embodiments, substrate 219 is a biomolecule, such as a oligonucleotide, a protein, an aptamer, biotin, or some other such moiety that itself, in turn, has affinity for another material. For example, substrate 219 can be a DNA or RNA sequence, optionally with one or more locked nucleic acid (LNA) bases therein, and can be captured by exposure to a complementary sequence, such as a primer or oligo fixed on a glass slide or within a well in a microtitre plate. In certain embodiments, substrate 219 is a magnetic bead.

With continued reference to FIG. 26, the binding partner (e.g., antibody 221) is introduced into the vessel containing cells 201 expressing cell-surface antigen 211 and incubated 131. Incubation of antibody-bead complexes with cells 201 leads to binding 133 of substrate 219 to cells 201. Cells 201 can then be isolated 135 from the sample. Where substrate 219 is a surface of a vessel or channel, or a surface of beads packed into a column or otherwise held in place, cells 201 can be isolated 135 by using a wash solution to wash away excess sample. Where substrate 219 is a magnetic bead, a magnet 231 can then be used to isolate 135 cells 201 from the remainder of the sample.

Magnetic materials may be bound to target entities and used to separate such entities through the use of magnet fields and gradients. Magnetic materials and separations are known in the art and have been previously described, for example, in Murphy, 2011, Janeway's Immunobiology 8 Ed, Garland Science (New York, NY), 888 pages, the contents of which are incorporated by reference herein. Methods of producing suitable magnetic particles are known in the art, and are described above in the section entitled, "Magnetic Particles." See for example U.S. Pat. 5,597,531; U.S. Pat. 4,230,685; U.S. Pat. 4,677,055; U.S. Pat. 4,695,393; U.S. Pat. 5,695,946; U.S. Pat. 4,018,886; U.S. Pat. 4,267,234; U.S. Pat. 4,452,773; U.S. Pat. 4,554,088; U.S. Pat. 4,659,678; U.S. Pat. 5,186,827; U.S. Pat. 4,795,698.

Each set of magnetic particles 219 has a binding partner 221 that allows for each set to specifically bind 133 the target 201 of interest in the sample. The binding partner may be any molecule known in the art and will depend on the target to be captured and isolated. Exemplary binding partners include nucleic acids, proteins, ligands, antibodies, aptamers, and receptors.

In particular embodiments, the binding partner 221 is an antibody, such as an antibody that binds a particular antigen 211 (see, e.g., FIG. 26). General methodologies for antibody production are described in the above related section. Methods for attaching the binding partner 221 to the magnetic particle 219, including the coating of particles with antibodies, are known in the art, and discussed above in the related section.. In some embodiments, more than one antibody 221 is used to create sets of magnetic particles. Since each set of particles 219 may be conjugated with antibodies 221 having different specificities for one or more antigen 211 coded in the genome of the phage or phages, compositions and concentrations may be optimized for detection of multiple unknown pathogens in the sample. In certain embodiments, all of the sets are provided at the same concentration. Alternatively, the sets are provided at different concentrations. For example, compositions may be designed such that sets that bind gram positive bacteria (e.g., antibody 221 to antigen 211 in genome of phage that targets Gram + cells 201) are added to the sample at a concentration of $2 \times 10^9$ particles per/ml, while sets that bind gram negative bacteria (e.g., antibody to antigen in genome of phage specific to Gram -) are added to the sample at a concentration of $4 \times 10^9$ particles per/ml. Compositions used with methods of the invention are not affected by antibody cross-reactivity. However, in certain embodiments, sets are specifically designed such that there is no cross-reactivity between different antibodies and different sets.

To facilitate downstream detection of pathogens 201, one or more labels may be added to antibody 221, particle 219, or both. For example, a label may be added during preparation of antibody/bead complexes. Any detectable label may be used with compositions of the invention, such as fluorescent labels, radiolabels, enzymatic labels, and others. The detectable label may be directly or indirectly detectable. In certain embodiments, the exact label may be selected based, at least in part, on the particular type of detection method used. Exemplary detection methods include radioactive detection, optical absorbance detection (e.g., UV-visible absorbance detection, optical emission detection, e.g., fluorescence, phosphorescence, chemiluminescence), or Raman scattering. Preferred labels include optically-detectable labels, such as fluorescent labels. Examples of fluorescent labels include without limit acridine and derivatives; BODIPY; Brilliant Yellow; coumarin and derivatives; DABITC; eosin and derivatives; erythrosin and derivatives; isothiocyanate; ethidium; fluorescein and derivatives; FAM; DTAF; QFITC, (XRITC); Malachite Green isothiocyanate; Phenol Red; pyrene and derivatives; Reactive Red 4 (Brilliant Red 3B-A sold under the trademark CIBACRON); rhodamine and derivatives; Texas Red; TAMRA; TRITC; riboflavin; Atto dyes; La Jolta Blue; phthalo cyanine; and naphthalo cyanine. Preferred labels include cyanine-3 and cyanine-5. Labels other than fluorescent labels are contemplated by the invention, including other optically-detectable labels. Methods of linking fluorescent labels to magnetic particles or antibodies are known in the art. Suitable labels and methods of their use are described in U.S. Pub. 2011/0262926, the contents of which are hereby incorporated by reference.

As shown in FIG. 26, the magnetic particles described above are then introduced 125 to the sample in order to bind 133 to the unknown pathogens 201. For example, a blood sample may be mixed with the described magnetic particles to generate a mixture that is allowed to incubate 131 such that the compositions bind to at least one pathogen 201 in the blood sample. The mixture is allowed to incubate 131 for a sufficient time to allow for the composition to bind to the pathogen in the blood. The process of binding the composition to the pathogen associates a magnetic moment with the pathogen, and thus allows the pathogen to be manipulated through forces generated by magnetic fields upon the attached magnetic moment.

In general, time for incubation 131 will depend on the desired degree of binding between the pathogen and the compositions of the invention (e.g., the amount of moment that would be desirably attached to the pathogen), the amount of moment per target, the amount of time of mixing, the type of mixing, the reagents present to promote the binding and the binding chemistry system that is being employed. Incubation time can be anywhere from about 5 seconds to a few days. Exemplary incubation times range from about 10 seconds to about 2 hours. Binding occurs over a wide range of temperatures, generally between about 5° C and about 65° C, e.g., between about 15° C and about 40° C.

In certain embodiments, a buffer solution (discussed above) is added to the sample along with the compositions of the invention. With reference to FIG. 26, after binding 133 of particles 219 to pathogens 201 in the sample to form pathogen/magnetic particle complexes, a magnetic field 231 may be applied to the mixture to capture or isolate 135 the complexes on a surface. Components of the mixture that are not bound to magnetic particles will not be affected by the magnetic field and will remain free in the mixture. Methods and apparatuses for separating 135 target/magnetic particle complexes from other components of a mixture are known in the art, and discussed above. The foregoing illustrative embodiment is described generally in terms of the induction of the expression of a common antigen 211 by one or more different unknown pathogen 211, as shown in FIG. 26. The invention generally provides methods of using a vector or viral preparation 207 to cause unknown pathogens 201 to present a common binding element.

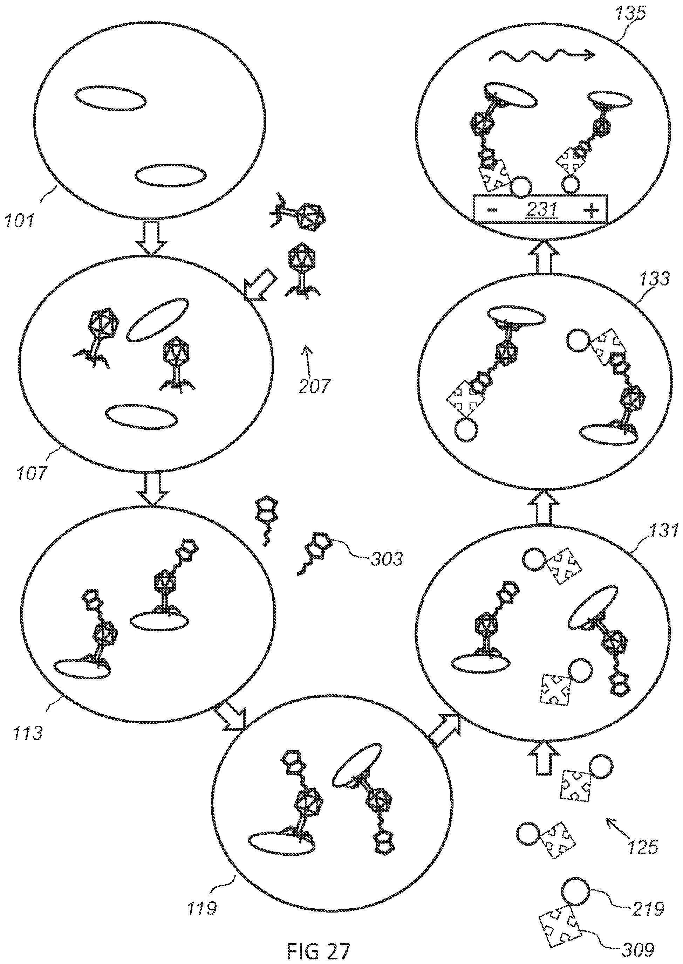

FIG. 27, for example, illustrates an alternative embodiment of a method of using a viral preparation 207 to cause different unknown pathogens to present 119 a common binding element 303. As shown in FIG. 27, viral preparation 207 includes at least one phage.

In certain embodiments, a bacteriophage is used that expresses a biotin binding domain (aka, a biotinylation peptide) on at least one capsid protein. The phage contains the genetic information to display a biotinylation domain and be biotinylated. In some embodiments, the phage displays a biotinylation domain when introduced 107 into the sample. When the phage attach to cells 201, those cells are then biotinylated. In certain embodiments, the phage infects the target cells and progeny virions produced from the infection will have been biotinylated by the target cells' 201 biotin ligase protein (BLP). Biotinylation of phage with biotinylation peptides on their capsids is discussed in Edgar et al., 2006, High-sensitivity bacterial detection using biotin-tagged phage and quantum-dot nanocomplexes, PNAS 103(13):4841-4945 and Gervias et al., 2007, Immobilization of biotinylated bacteriophages on biosensor surfaces, Sensors and Actuators B 125: 615-621.

Methods of labeling targets, e.g., including biotinylation of phage, are discussed in U.S. Pat. 7,517,643; U.S. Pat. 6,342,588; and U.S. Pub. 2011/0086338 In some embodiments, phage capsid is streptavidin or avidin-linked and substrate 219 is biotinylated, as discussed in U.S. Pat. 6,740,492.

With continued reference to FIG. 27, cells 201 present 119 biotin 303 as a common binding element. Binding partner 125 is introduced. Here, binding partner 125 includes streptavidin 309 coated beads 219. Biotinylation of phage is further discussed in Smelyanski and Gershoni, 2011, Site directed biotinylation of filamentous phage structural proteins, Virol J 8:495; U.S. Pat. 5,994,519; and U.S. Pub. 2001/0019820.

Biotinylated target cells 201 are incubated 131 with streptavidin-coated beads 219. The binding partner binds 133 to the target, after which the target 201 can be isolated 135. For example, where beads 219 are magnetic, a magnet 231 can be used to hold cells 201 in a fluidic channel while the sample is washed away. With reference back to FIG. 27, where live cells are intended 139, the sample can be replaced with a maintenance buffer 147. In an alternative embodiment, cells 201 can be lysed 155, optionally using a lysis buffer 151. In some embodiments, a component of the cells 201 is extracted. For example, DNA may optionally be extracted by column separation 159.

While discussed above in the illustrative embodiments involving biotinylated phage, streptavidin-linked phage, or expression of a common antigen after transduction by a phage, methods of the invention include any suitable method or phenomenon to cause different unknown pathogens 201 to exhibit a common binding element through the introduction of a viral preparation 207. In some embodiments, phage display of an antigen is employed.

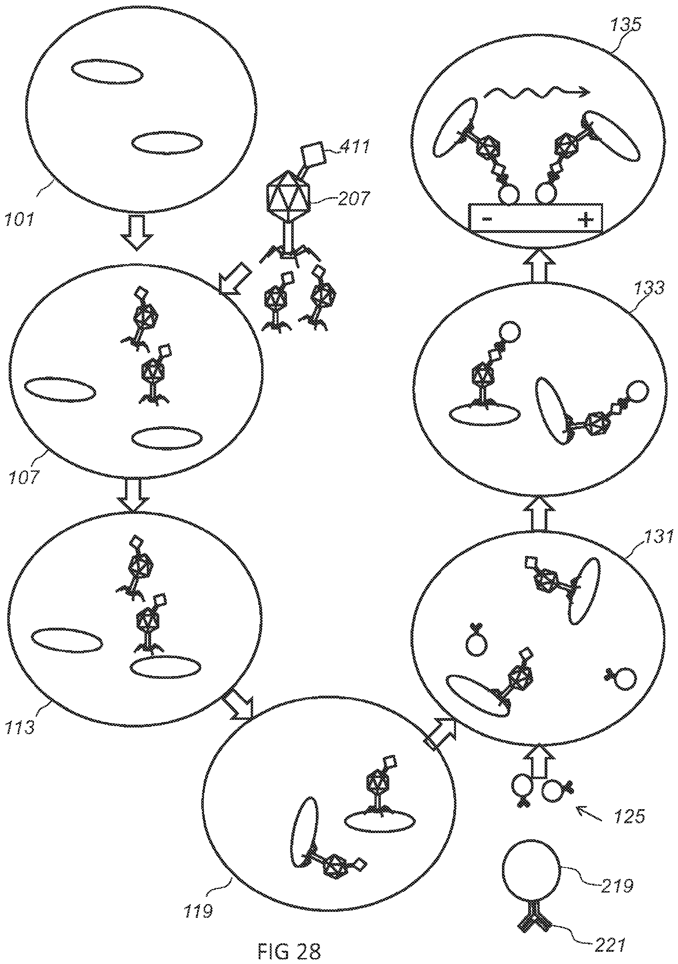

FIG. 28 illustrates phage display of a common antigen by different unknown pathogens 201. In phage display, a gene for antigen 411 is ligated into the phage genome, for example, within the gene encoding one of the coat proteins, or capsid proteins. Multiple cloning sites may be used to ensure in-frame cloning and proper expression of the protein product. The viral preparation 207 including one or more phage engineered for phage display of a common antigen 411 is then introduced 107 into the sample containing unknown pathogens 201. A binding partner is used (e.g., linked to a substrate 219 such as a well in a microtitre plate, a surface of a fluidic channel, beads in a column, or magnetic beads) that binds to antigen 411. After the phage attach 119 to pathogens 201, they display antigen 411. Phage display is discussed in Haq, 2012, Bacteriophages and their implications on future biotechnology, a review, Virol J 9:9; U.S. Pat. 8,227,242 (e.g., phage display without helper phage); U.S. Pat. 8,216,797; U.S. Pat. 7,238,669; U.S. Pat. 6,740,492; U.S. Pub. 2010/0240579 (detection of unknown target by phage display); and U.S. Pub. 2006/0063149.

In some embodiments, phage display antigen 411 when introduced 107 into the sample. In certain embodiments, the genetically-engineered phage is introduced and infects pathogens 201 and generates progeny virions that express antigen 411. Viral preparation 207 may include a bacterial growth medium to allow low concentrations (e.g., single cells) of target to grow and be infected and propagate the phage. Moreover, a combination of the foregoing may occur in that phage may exhibit antigen 411 upon introduction 107 into the sample, and may infect and propagate, generating progeny.

While any suitable substrate 219 may be used, and any suitable binder (e.g., aptamers, co-factors, proteins, etc.), in certain embodiments, magnetic beads linked to antibody 221 are used. This binding partner 125 is introduced into the infected sample and allowed to incubate 131.

FIG. 27 shows that incubation 131 leads to the binding 133 of binding partner 125 to antigen 411 on cells 201. After cells 201 are bound to magnetic beads 219 via antibody 221/antigen 411 interaction, the cells 201 may be isolated 135 from the sample by magnetic separation. Further, additional steps may be performed to optimize the isolation or extraction of cells 201 from the sample. Additional steps optionally include washing with one or more solutions, further concentrating 139 captured cells (e.g., using a magnetic concentrator), introduction of additional buffers such as, for example, cell maintenance buffers or lysis buffers, other suitable processes known in the art, or a combination thereof.

Other methods of using a binding partner to capture a target are operable with methods of the invention. For example, where pathogens 201 are intracellular parasites, a known binding element may be associated with the host cells. A binding partner may be used that would not be able to target an unknown pathogen directly but instead bind to the host (if the host is eukaryotic, instead of a phage viral preparation, a binding partner specific to a eukaryotic protein may be employed). See, e.g., Drancourt et al., 1992, Diagnosis of Mediterranean spotted fever by indirect immunofluorescence of Rickettsia conorii in circulating endothelial cells isolated with monoclonal antibody-coated immunomagnetic beads, J Inf Dis 166:660-3. Optionally, the membrane of the eukaryotic host can be ruptured (e.g., with detergent, a virus or particle, differential osmolality, etc.) and the infectious agent targeted by methods herein. In some embodiments, methods of the invention may be used in combination with, or in sequence with, alternative methodologies that would only operate, for example, where one or more target is known. If a target is known, it may exhibit a known binding element that can be captured with a binding partner. See, e.g., Fu et al., Rapid detection of E. coli O157:H7 by immunomagnetic separation and real-time PCR.

The process of magnetic separation 135 described above (e.g., with respect to FIG. 27) produces efficient capture of different unknown pathogens 201 and the removal of all or majority of the remaining components in the mixture. However, it is still possible that a relatively small amount of non-specific analytes are unintentionally captured along with the target/magnetic particle complexes. It may be desired to remove these non-specific analytes. Accordingly, the surface may be washed with a wash solution that reduces particle aggregation, thereby isolating target/magnetic particle complexes from the non-specific target entities.

Any wash solution that does not disrupt interaction between the binding partner of the magnetic particle and the target may be used. The wash solution should also not disrupt the capture of the target/particle complexes on the intended surface. Wash solutions are detailed in the prior sections. Once the different unknown pathogens 201 have been isolated 135, they may be preserved as living cells or they may be lysed and the lysate, or a component thereof, may be detected, extracted, isolated, quantified, or used.

Where live cells are intended 141, a live cell buffer may be introduced 147, for example, in a step that includes flushing away any wash buffers, other solutions or reagents, or remaining components of the sample.

Once the target/magnetic particles have been captured, the target is then lysed 155. In certain embodiments, lysis of the target occurs without separating the particles from the target prior to the lysis step. Conducting the lysis without the pre-separation step allows more efficacious collection of analytes contained within the target. For instance, if the analyte of interest is a bacterially-derived nucleic acid, some analyte may be lost when bacteria separated from the magnetic particles are inadvertently lost. With the disclosed methods, the bacteria are still bound to the particles and captured on a surface as lysis occurs. Accordingly, there is a concentrated sample to work with during the lysis step. In addition, the disclosed methods allow for recovery of a specific analyte in a relatively small collection volume. This is especially useful when the analyte of interest is a nucleic acid or something that is similarly present in only very small quantities. For example, one could begin with a blood sample of 2 ml and use the described methods to concentrate a desired pathogen from the blood onto an appropriate surface. With the pathogen captured, one could decant the blood, add 0.3 ml of a suitable buffer, and subsequently perform the lysis step. Lysing is described above."

The lysate containing the contents of the lysed target can then be eluted. In certain aspects, the lysate contains nucleic acids of interest associated with a particular bacteria present in the starting sample. The lysate is removed from the magnetic particles and the analytes contained therein can be analyzed. Analytes may include, without limitation, nucleic acids, proteins, organelles, and other components found within the target of interest.

The analyte may be analyzed by a multitude of existing technologies, such as NMR, Polymerase Chain Reaction (PCR), sequencing, mass spectrometry, fluorescent labeling and visualization using microscopic observation, fluorescent in situ hybridization (FISH), growth-based antibiotic sensitivity tests, and variety of other methods that may be conducted with purified target without significant contamination from other sample components. Analysis using NMR is described in U.S. Pub. 2011/0262925. In some embodiments, the different unknown pathogens 201 are analyzed in order to identify genes they express. For example, captured bacteria 201 are lysed without first separating the bacteria from the magnetic particles. The lysate is then eluted from the magnetic particles and DNA contained within the lysate/eluate is bound to DNA extraction resin. After washing of the resin, the bacterial DNA is eluted and used in quantitative RT-PCR to detect the presence of a specific species, and/or, subclasses of bacteria.

Detection of bacteria of interest can be performed by use of nucleic acid probes following procedures which are known in the art. Suitable procedures for detection of bacteria using nucleic acid probes are described in U.S. Pat. 7,943,346; U.S. Pat. 5,620,847; U.S. Pat. 5,569,586; U.S. Pat. 5,541,308; U.S. Pat. 5,401,631; U.S. Pat. 5,089,386; and U.S. Pat. 5,055,394.

A suitable nucleic acid probe assay generally includes sample treatment and lysis, hybridization with selected probe(s), hybrid capture, and detection. This assay is discussed in prior sections. Detection of bacteria of interest can also be performed by use of PCR techniques. A suitable PCR technique is described, for example, in Verhoef et al. (WO 92/08805). Bacterially-derived nucleic acids isolated from the lysate can be used as templates for the PCR reaction. PCR can also include the use of reverse-transcriptase PCR (RT-PCR), in which RNA isolated from the target is reverse transcribed into its DNA complement (i.e., cDNA) using the enzyme reverse transcriptase, and the resulting cDNA is amplified using PCR. RT-PCR is described in detail in U.S. Pub. 2011/0071033.

In certain embodiments, differential gene expression associated with the target can also be identified, or confirmed using a microarray technique. In this method, polynucleotide sequences of interest (including cDNAs and oligonucleotides) are plated, or arrayed, on a microchip substrate. The arrayed sequences are then hybridized with specific DNA probes from cells or tissues of interest. Methods for making microarrays and determining gene product expression (e.g., RNA or protein) are shown in U.S. Pub. 2006/0195269.

In particular aspects, nucleic acids isolated from the target can be sequenced to generate a plurality of sequence reads, thereby identifying a pathogen according to a known genetic sequence. Sequencing may be by any method known in the art. Sequencing is detailed in prior related sections. In certain embodiments, methods of the invention are useful for direct detection of unknown bacteria 201 from blood. Such a process is described here. Sample is collected in sodium heparin tube by venipuncture, acceptable sample volume is about 1 mL to 10 mL. Sample is diluted with binding buffer and superparamagnetic particles having binding partners are added to the sample, followed by incubation on a shaking incubator at 37° C for about 30 min to 120 min. Alternative mixing methods can also be used. In a particular embodiment, sample is pumped through a static mixer, such that reaction buffer and magnetic particles are added to the sample as the sample is pumped through the mixer. This process allows for efficient integration of all components into a single fluidic part, avoids moving parts and separate incubation vessels and reduces incubation time.

Capture of the labeled targets allows for the removal of blood components and reduction of sample volume from 30 mL to 5 mL. The capture is performed in a variety of magnet/flow configurations. In certain embodiments, methods include capture in a fluidic cartridge or other flow-through device at flow rate of 5 mL/min, resulting in total capture time of 6 min.

After capture, the captured target is washed with wash buffer including heparin to remove blood components and free particles. The composition of the wash buffer is optimized to reduce aggregation of free particles, while maintaining the integrity of the particle/target complexes. After the wash step, the still captured targets are sonicated in order to lyse the cells. The bacterial cells are not separated from the magnetic particles prior to sonication. While the appropriate settings for sonication can be determined empirically, a sonicator can be used for a duration of 3 minutes to achieve effective lysis. The resulting lysate can then be eluted and the expression profile of any nucleic acid found in the lysate can be determined.

Devices for conducting the above methods are also provided. Any suitable device or system may be used. In general, the devices comprise an input channel, an output channel, a chamber, a magnetic assembly, and a lysing device. The input channel and output channel are in fluid communication with the chamber and the magnetic assembly is adapted to capture a magnetic particle inputted into the input channel onto a surface of the chamber. The lysing device is adapted to lyse a target bound to the magnetic particle. In one illustrative embodiment, the device is a fluidic cartridge.

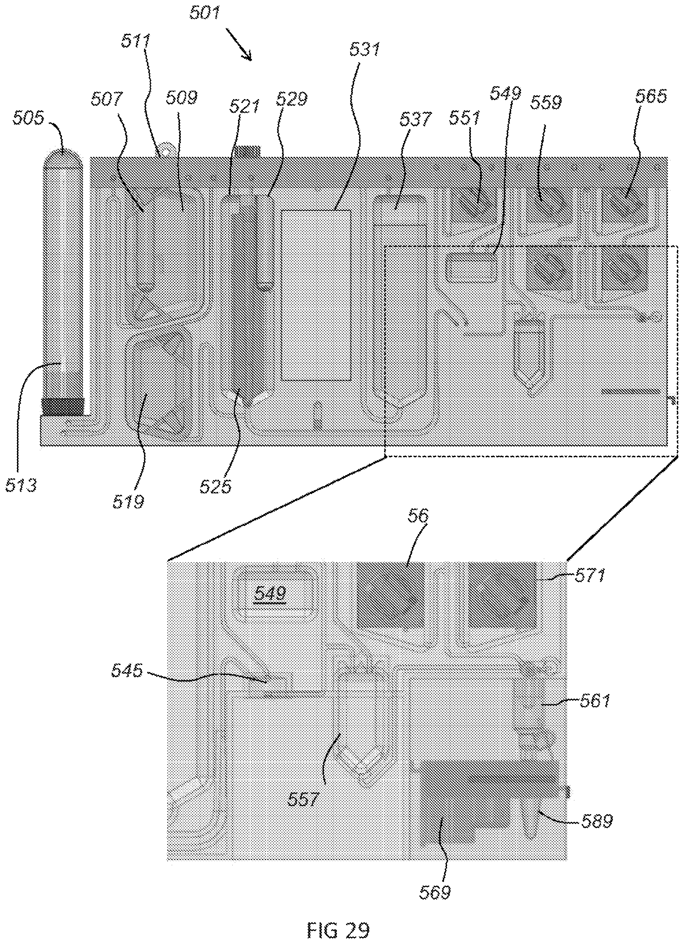

FIG. 29 illustrates a fluidic cartridge 501 according to certain embodiments. Fluidic cartridge 501 is provided to operate with blood collection tube 505 (e.g., a vacutainer). Cartridge 501 includes long needle 513 that penetrates into an interior of tube 505 when tube 505 is inserted thereon. Magnetic beads are stored in magnetic bead ampule 507 shown disposed within bead buffer 509. Pneumatic interface 511 provides pressure to flow sample, solutions, and buffers through channels of cartridge 501 and may optionally be used to crush ampules such as magnetic bead ampule 507 (and plant pathogen ampule 529). When operation is begun and tube 505 containing a sample is inserted, magnetic bead ampule 507 is crushed, introducing beads 219 to bead buffer 509. Beads 219 and buffer 509 may be mixed, for example, using agitation provided by air from pneumatic interface 511, to produce a bead mixture.

Air from pneumatic interface 511 forces blood from tube 505 into mixing chamber 521. The bead mixture is forced through long needle 513 to rinse the inside of tube 505. The bead mixture is then sent to mixing chamber 521 to mix with the blood. This step may be repeated, sending more bead mixture from bead buffer 509, through tube 505, to mixing chamber 521, until tube 505 is evacuated of target and a desired proportion (e.g., 2:1) of bead mixture to sample is present in mixing chamber 521.

Mixing paddle 525 is used to mixing the contents of chamber 521, agitating the blood and beads. Air pressure from pneumatic interface 511 then forces the mixture into magnetic trap 531. Optionally, the mixture can be pushed back, from magnetic trap 531 to mixing chamber 521, and the cycle repeated any number times until mixing chamber 521 is satisfactorily evacuated of target pathogens 201 and the bead-bound target pathogens 201 as well as the other components of the sample are in magnetic trap 431.

Then, magnetic trap 431 is evacuated of the other components of the sample, leaving magnetic particles 219 bound to magnets therein. The waste fluid is pushed back through mixing chamber 521 and discarded. Wash buffer 519 is then forced into magnetic trap 531, filling it. The wash buffer can then be pushed back to mixing chamber 521 to ensure good washing of beads 219. Wash buffer is sent back into magnetic trap 531 and held there.

Magnets can then be moved away from magnetic trap 531, allowing beads 219 to resuspend in wash buffer 519 within trap 531. At this point, target pathogens 201 have been extracted from the original sample and held in wash buffer 519. Wash buffer 519 can then be pushed through magnetic concentrator 545 to waste chamber 549.

Turning now to the inset portion of FIG. 29, magnetic concentrator 545 can be seen to be in fluid communication with magnetic trap 531. As wash buffer 519 flows through concentrator 545, beads 219 are captured in concentrator 545 while the remainder of buffer 519 is passed on to waste chamber 549. Original pathogens 201 are thus concentrated in concentrator 545.

The contents of concentrator 545 may be processed according to whether live cells or an extracted cellular component is intended. If live cells are intended, the magnet is removed from magnetic concentrator 545 and buffer 551 (here, a live cell buffer) is introduced through concentrator 545 and used to flush the cells into output vial 589.

If, for example, extracted DNA is intended, buffer 551 is a lysis buffer and is introduced into magnetic concentrator 545. The magnet is removed from concentrator 545 and a sonicator may be applied to a wall of the chamber of concentrator 545. Optionally, beads may be included for bead-bashing to aid in lysis. The sonicator or other lysis means is activated and the target cells 201 are lysed. In certain embodiments, a probe of a sonicator extends into the chamber, where it delivers vibrations into the liquid medium surrounding the captured complexes. In other embodiments, the sonication transducer is brought in contact with the chamber by way of a structural interface. For example, the structural interface may constitute the floor or the ceiling of the chamber. The sonication transducer vibrates the structural interface such that lysis of the targets captured in the chamber is achieved.

Lysate is then pushed into pre-column mixer 557. DNA binding buffer 559 is added to pre-column mixer 557 (optionally agitated by bubbling air). The contents of pre-column mixer 557 is then forced through the DNA extraction column 561 and the elutant is discarded as waste. DNA extraction may be completed using washes from first column wash 565, second column wash 569, and water 571. Air from pneumatic interface 511 can be forced through DNA extraction column 561 to remove volatile organic compounds. Water 571 can be used to rinse the purified DNA (optionally including the use of a de-binding buffer or a modulator of stringency) into output vial 589.

Coordination of the on-cartridge steps can be supported by an operations device, such as a bench-top electro-mechanical device. The timing of pneumatic injections, the breaking of ampules, and the piercing of reagent reservoirs can be coordinated manually, or by a computer program or mechanical system. Cartridge 501 can include any suitable materials, shape, or dimensions. For example, in some embodiments, fluids are handled in macrofluidic environments up until entered into magnetic concentrator 545 and are handled according to microfluidic principles thereafter. In general, microfluidic may refer to sub-microliters volumes.

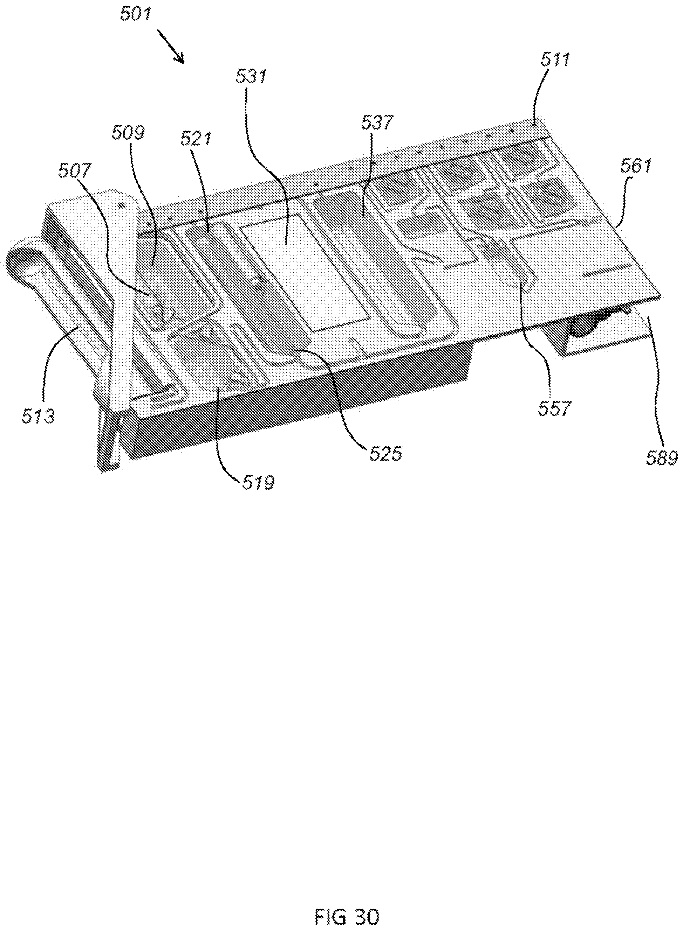

FIG. 30 gives a perspective view of an exemplary cartridge according to certain embodiments. Methods for manufacturing and operating fluidic systems are known and discussed in Fredrickson and Zan, 2004, Macro-to-micro interfaces for microfluidic devices, Lab Chip 4(6):526-33; U.S. Pat. 8,105,783; U.S. Pat. 7,785,869; U.S. Pat. 7,745,207;

U.S. Pat. 7,553,647; U.S. Pub. 2009/0227005; U.S. Pub. 2008/0241000; and U.S. Pub. 2008/0003564

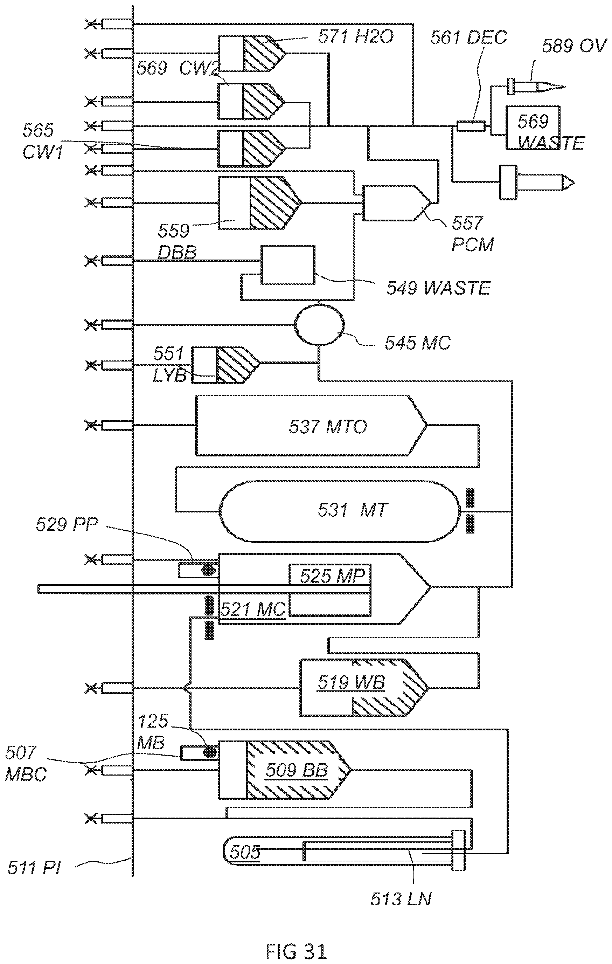

FIG. 31 gives a schematic diagram of elements and steps of the invention as discussed above with respect to FIG. 29.

While discussed above as magnetic beads, substrate 219 for capture of pathogens 201 may be the floor of a chamber such as, for example, trap chamber 531. In other embodiments, the capture surface is the ceiling. The captured pathogens 201 can there be washed to remove non-specific analytes and unbound entities.

In certain embodiments, fluidic cartridge 501 is designed to be disposable. In this instance, once pathogens 201 have been lysed and the eluate collected, cartridge 501 can be discarded. In this manner, each cartridge is intended for a single use, and the potential for cross-contaminating pathogens due to repeated use is eliminated.

The cartridge can be prepared from any material in the art suitable for containing liquids and withstanding the rigors of sonication. In certain aspects, the entire cartridge is made of plastic or an acrylic plastic polymer. In other aspects, the bottom or top cover of the cartridge can be prepared from a film such as the biaxially-oriented polyethylene terephthalate (BoPET) sold under the trademark MYLAR (general purpose file, commercially available by DuPont) while the rest of the cartridge is made of plastic or an acrylic plastic polymer. In certain embodiments, the BoPET film constitutes the aforementioned structural interface in contact with the sonicator transducer.

In certain embodiments, the cartridge is connected to a fluidic device or system configured to flow liquids into and out of the cartridge. The fluidic system can comprise a pump that delivers liquid, air, gas, beads, reagents, electricity or other signals, light, power or a combination thereof to the cartridge. The fluidic system can have a first tubing line connected to the inlet of the input channel, for flowing liquid into the cartridge. The other end of the first tubing line is connected to the pump. A second tubing line for fluid leaving the cartridge is connected to the outlet of the output channel. The other end of the second tubing line may also be connected to the pump or to a container for collecting exiting fluid. The fluidic system facilitates the delivery of the target/magnetic particle complexes into the chamber, as well as any washing of captured complexes. The collection of lysate is also facilitated by the fluidic system.

Devices of the disclosure may also include a detection module. The detection module is a component in which molecules, cells, or other particles are to be detected, identified, measured or interrogated on the basis of at least one predetermined characteristic. The molecules, cells, or other particles can be examined one at a time, and the characteristic is detected or measured optically, for example, by testing for the presence or amount of a label. In some aspects, the detection module is in connection with one or more detection apparatuses. The detection apparatuses can be optical or electrical detectors or combinations thereof. Examples of suitable detection apparatuses include optical waveguides, microscopes, diodes, light stimulating devices (e.g., lasers), photomultiplier tubes, and processors (e.g., computers and software), and combinations thereof, which cooperate to detect a signal representative of a characteristic, marker, probe, label, or reporter, and to determine and direct a measurement or sorting action. However, other detection techniques can be used as well.

Devices of the disclosure may include a computer component. For example, a user interface may be provided with input/output mechanisms (monitor, keyboard, touchscreen, etc.) coupled to a memory and a processor (e.g., a silicone chip and a solid-state or magnetic hard drive). Input/output mechanisms may be included for data, such as a USB port, Ethernet port, or Wi-Fi card or a hardware connection to a detection module, fluidic chip and pneumatic interface, or both.

In certain aspects, the detection module is in fluid connection with the fluidic cartridge. For example, the outlet of the fluidic cartridge may be connected to the detection module by means of a tubing line. In this manner, lysate leaving the cartridge can enter the detection module wherein the contents of the lysate can be detected and analyzed.

Devices For Target Detection And Methods Of Use Thereof

Due to the nature that traditional testing methods are problematic because they are slow and have limited sensitivity, embodiments of the invention provides rapid, sensitive, and accurate tests for chemical and microbial contamination of products and facilities. For example, many tests are unable to detect very low numbers of molecules or organisms and thus require a target to be concentrated or grown in a culture. Culturing microbes on agar plates can require days or even weeks. Molecular tests based on technologies such as polymerase chain reaction or enzyme-linked immunosorbent assays generally require an enrichment step that can require 24 to 48 hours or longer.

Those tests impose large costs on industry as products must stand by unsold and unused while the tests are performed. If a slow-growing bacterium requires a 76-hour enrichment culture, for example, many truckloads' worth of product may have to sit idly in warehouses for those days. Nevertheless, the safety of our food and drug supply requires that accurate and reliable tests be performed.

Methods and devices of the invention are capable of detecting very small amounts of contamination, as low as a single target in a milliliter of sample. Sensitive detection is accomplished by introducing a molecular binding moiety to the sample and allowing it to bind to the target and then separating the binder/target complex from the remainder of the sample. Speed and sensitivity is maximized by the techniques, devices, and reagents employed.

A binding buffer may be used to flush the sample into the separation chamber and is chemically optimized to encourage binding of target while preventing non-specific aggregation of particles in the sample. The binding buffer may be replaced with a wash buffer to further remove non-target materials from the bound target. The entire contaminant capture procedure can be performed rapidly within a fluidic device, allowing the target contaminant to be detected from a very low starting concentration within a short period of time. Since low concentrations of contaminant can be detected very rapidly, methods and device of the invention can be used for bioburden or microbial limit testing in settings throughout the food and drug industries. Since testing is rapid and accurate, food and drugs may be produced and distributed safely, while avoiding high costs associated with prior art tests.

In some aspects, the invention provides a method for detecting a target analyte in a sample. The method includes transferring the sample from a collection tube into a chamber on a device, flushing the collection tube with a liquid that includes a binding moiety, and delivering the liquid comprising the binding moiety into the chamber. The target analyte is isolated from the sample and analyzed.

Isolating the target analyte can employ a binding moiety that is linked to a magnetic particle, such as superparamagnetic particle with a diameter less than about 250 nm. Magnetic separation techniques can be used to wash away the sample. For example, where the binding moiety is an antibody, it may bind specifically to the target analyte, allowing other components of the sample to be washed away. The sample may be analyzed by optical detection, non-optical detection, or other methods. For example, nucleic acid may be extracted from the sample and analyzed by PCR, sequencing, hybridization to probes, or other methods. In some embodiments, the DNA extraction occurs within the device.

The device can include a fluidic chip or cartridge. A pneumatic interface may be included and materials can be transferred through, stored within, or flushed from different channels and chambers of the device by pneumatic pressure. The liquid that includes the binding moiety may include a binding buffer-a buffer that is formulated to enhance binding of the binding moiety to a target analyte and reduce formation of particle aggregates, e.g., a solution comprising a moiety that specifically binds the target. After the target analyte is captured by the binding moiety, it may optionally be washed with a wash solution, for example, to remove non-specifically bound components from the binding moiety.

Methods of the invention can be rapid, with all steps taking less than 24 hours. In some embodiments, the steps take less than about 12 hours, 6 hours, or 3 hours.

In related aspects, the disclosure provides a method for detecting a target analyte by performing the following steps within a fluidic device. A binding buffer, a binding moiety, and a sample containing a target analyte are delivered into a chamber on the device. The sample is incubated until the binding moiety binds to the target analyte. The target analyte is isolated and analyzed. The method can include loading a collection tube holding the sample into or onto the device and using pressure to deliver the sample into the chamber. The binding moiety, the buffer, or both may optionally be flushed through the collection tube, for example, while on the device, and into the chamber to ensure very sensitive detection.

The target analyte, bound to the binding moiety, is retained within a portion of the device. There, it can be washed with the binding buffer, water, a wash solution, or a combination thereof. That portion of the device may be the chamber in which the sample was initially incubated, may be a second chamber, or both. In some embodiments, all of the steps of the method can be performed in less than 12 hours, for example, in less than 4 hours or 2 hours.

Isolation of the target analyte may employ properties of the binding moiety. For example, the binding moiety may be bound to a solid substrate such as a wall of a fluidic channel or chamber, a surface of a material in a column, or particles such as beads. The particles can be magnetic particles such as small magnetic particles that include a paramagnetic material, have a diameter of less than about 250 nm, or both. The binding moiety can be an antibody, a ligand, streptavidin, biotin, or any other suitable binding molecule. In certain embodiments, the target analyte is a microorganism, such as a pathogenic bacterium. An antibody may be used to bind to an antigen on a cell surface of the organism.

In other aspects, the disclosure provides methods for detecting a target analyte that involve introducing a sample containing a target analyte and a plurality of beads each linked to a binding moiety into a chamber on a device and isolating the target analyte from the sample within the chamber. The target analyte is further transferred into a concentrator chamber on the device, concentrated there, and analyzed. The beads may be small magnetic beads (e.g., d < 300 nm). Pressure is used to introduce the sample into the chamber and transfer the target analyte into the concentrator chamber. Pneumatic pressure may be used.

In some embodiments, the beads are suspended in a liquid that includes a binding buffer formulated to enhance binding of the binding moiety to the target analyte, to reduce formation of particle aggregates, or both. Further, non-specifically bound components may be washed from the target, for example, using the binding buffer, using a wash buffer, using water, or a combination thereof. All of the steps may be performed in less than a day, e.g., half a day or a few hours.

In other related aspects, the disclosure provides a device for detecting a target analyte. The device includes an ampoule containing a plurality of beads linked to binding moieties, a reservoir of binding buffer, and a network of fluidic channels configured to transfer a sample, the beads, and the binding buffer into a chamber on the device. The device further includes a mechanism to retain the bead-bound target analyte in the chamber while buffer is removed and replaced by a wash buffer. The device also provides a vessel for collection of the target analyte. In some embodiments, the device is provided with a mechanism to rupture the ampoule. Fluidic channels may be used to conduct sample or reagents within the device. For example, in some embodiments, macrofluidic channels conduct the sample into the chamber, microfluidic channels transfer the target analyte into the collection vessel, or both. The device may also include a magnetic concentrator configured for fluid communication with the chamber.

The present disclosure provides methods and devices for microbial limit and bioburden testing in an industrial, environmental, or biological sample. The invention allows the rapid detection of chemical or microbial contaminants at very low levels in the sample, thus allowing early and accurate detection and identification of the contaminants. Since methods of the invention are able to isolate target analytes at very low levels, methods of the invention reduce or eliminate culturing or enrichment steps that are typically associated with bioburden testing and allow for more rapid analysis of the target. In this manner, methods of the invention provide data that organizations may use to assure the fitness of their products for commercial distribution.

Moreover, methods and devices of the disclosure may be used for environmental quality sampling (e.g., to make pollution remediation recommendations) or national security purposes (e.g., to detect anthrax).

The invention generally relates to conducting an assay on a sample that isolates a target analyte from the sample and allows for analysis of the analyte with minimal (e.g., less than 24 hours of) or no culturing of the pathogen. In certain embodiments, methods of the invention involve obtaining a sample including a target analyte, conducting an assay that isolates the target analyte from the sample, and analyzing the pathogen. In some embodiments, methods include a short culturing or enrichment step, for example, a step that is shorter than a few hours. In particular embodiments, there is no culturing or enriching step and the isolated target analyte is analyzed directly without any culturing enrichment.

Any sample may be collected and assayed using methods and device of the invention. For example, samples can be taken of pharmaceutical or chemical compounds. Samples can be taken from the ambient air or surfaces, for example, of structures, equipment, devices such as medical devices, animals, or people. Biological samples can be taken such as a bodily tissue or fluid.

In some embodiments, the sample is an air sample, for example, as collected with a volumetric sampler that is capable of collecting a sufficient volume of air (e.g., about 100 liters to about 1,000 liters). A volumetric air sampler may include a blower and a filter. Air is drawn into the sampler and through a glass fiber or quartz filter, so that matter collects on the filter surface. The filter can then be immersed in liquid, such as a buffer, a nutrient medium (e.g., trypticase soy) or an alcohol, within, for example, a collection tube. Air sampling may collect particulate matter, microbes, insects, aerosols such as oil mists or fog droplets, exhaled breath, or other constituents of the atmosphere. Suitable air samplers are discussed in U.S. Pat. 8,171,803; U.S. Pat. 7,046,011; U.S. Pat. 6,087,183; U.S. Pat. 5,467,776; and U.S. Pat. 5,201,231.

A sample may be collected from a surface. Surface sampling techniques for bioburden or microbial limit testing are known in the art and any suitable method can be used. For example, in some embodiments, the replicate organism detection and counting (RODAC) method-also known as the contact plate method-is used for surface sampling. The contact plate method involves pressing an over-filled Petri dish against the surface to be tested. The dish can then be cultured or scraped directly into a collection tube. The contact plate method is described in Hall and Hartnett, 1964, Measurement of bacterial contamination on surfaces, Pub Health Report 79:1012-1024; U.S. pat. 3,787,290; U.S. Pat. 3,337,416. In certain embodiments, a sterile swab is unwrapped and a surface is swabbed. The swab is then enclosed in a collection tube, optionally immersed therein in a liquid such as a buffer, a nutrient medium, or an alcohol. Surface sampling techniques are described in U.S. Pat. 7,611,862; U.S. Pat. 5,859,375; U.S. Pat. 5,823,592; and U.S. Pub. 2010/0313685.

In some embodiments, the sample is a bodily fluid, or biological sample, as discussed in prior sections. Methods of the invention may be used to detect any target analyte. The target analyte refers to the substance in the sample that will be captured and isolated by methods of the invention. The target analyte may be inorganic (e.g., a metal, a cyanide or cyanate, a salt, etc.) or organic chemicals, macromolecules (chitin, peptidoglycan, carbohydrates, proteins, nucleic acids, lipids, etc.,), bacteria, fungi, a cell (such as a cancer cell, a white blood cell a virally infected cell, or a fetal cell circulating in maternal circulation), a virus, a nucleic acid (e.g., DNA or RNA), a receptor, a ligand, a hormone, a drug, a chemical substance, or any molecule known in the art. In certain embodiments, the target is a pathogenic bacteria. In other embodiments, the target is a gram positive or gram negative bacteria. Exemplary bacteria that may be captured and isolated by methods of the invention include those of the genera Alphaproteobacteria, Bacillus, Betaproteobacteria, Bifidobacterium, Borrelia, Campylobacter, Candida, Citrobacter, Clostridium, Enterobacter, Enterococcus, Escherichia, Flavobacterium, Fusobacterium, Gammaproteobacteria, Klebsiella, Kluyvera, Lactobacillus, Legionella, Leuconostoc, Listeria, Micrococcus, Mycobacterium, Neisseriaceae, Pediococcus, Pneumococcus, Porphyromonas, Prevotella, Propionibacterium, Proteus, Rhodospirillum, Rickettsia, Saccharomyces, Salmonella, Serratia, Shigella, Sphaerotilus, Staphylococcus, Streptococcus, Thermoanaerobacter, Thermoproteus, Vibrio, and Yersinia, or a combination thereof.

A particular advantage of methods of the invention is for capture and isolation of bacteria and fungi directly from blood samples at low concentrations that are present in many clinical samples (as low as 1 CFU/ml of bacteria in a blood sample).

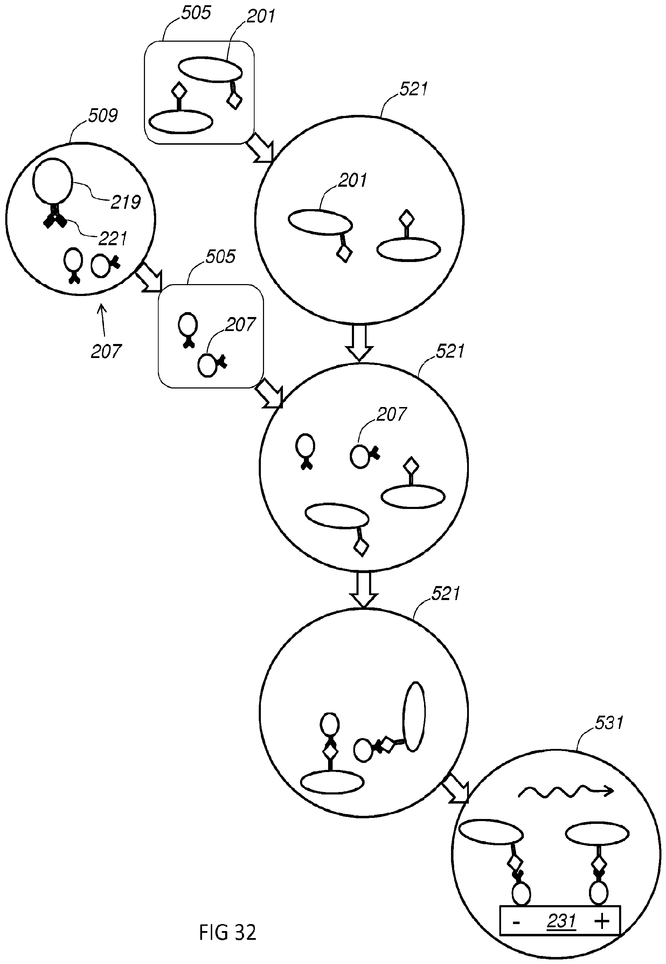

FIG. 32 shows a method for detecting a target analyte 201 in a sample. The sample is transferred from a collection tube 505 into a chamber 521 (e.g., on a fluidic device). Chamber 505 is flushed with a liquid containing a binding buffer 207 (e.g., a solution comprising a moiety that binds to a target), and that liquid is delivered into chamber 521. The binding buffer 207 includes particles 219 used to isolate target analyte 207 from the sample. Isolation can take place within chamber 521, or the bound target analyte 201 may be transferred to another chamber 531 on the device.

In some embodiments, the particles 219 in binding buffer 207 are magnetic particles. The sample is mixed with magnetic particles 219 having a particular magnetic moment and also including a target-specific binding moiety 221 to generate a mixture that is allowed to incubate such that the particles bind to a target analyte 201 in the sample, such as a bacterium in a blood sample. The mixture is allowed to incubate for a sufficient time to allow for the particles to bind to the target analyte. The process of binding magnetic particles 219 to the target analyte 201 associates a magnetic moment with the target analytes 201, and thus allows the target analytes to be manipulated through forces generated by magnetic fields upon the attached magnetic moment.

As will be shown below, transfer of the sample into a chamber 521 and flushing collection tube 505 with binding buffer 207 may take place on a fluidic device or system. Chamber 521 and collection tube 505 may be in fluid communication by one or more channels. Liquids may be transferred among chambers and channels through the use of pressure, such as pressure applied through the use of another liquid (e.g., an immiscible liquid) or pneumatic pressure.

In general, incubation time (e.g., in chamber 521) will depend on the desired degree of binding between the target analyte and the magnetic beads (e.g., the amount of moment that would be desirably attached to the target), the amount of moment per target, the amount of time of mixing, the type of mixing, the reagents present to promote the binding and the binding chemistry system that is being employed. Incubation time can be anywhere from about 5 seconds to a few days. Exemplary incubation times range from a few seconds to about 2 hours. Incubation times can be optimized to allow the entire isolation method to take place within a specific time, e.g., 6 hours, 3 hours, 1 hour, 30 minutes, or 10 minutes, or a few minutes. Binding occurs over a wide range of temperatures, generally between 15° C and 40° C.

In certain aspects, methods of the invention involve introducing magnetic particles 219 including a target-specific binding moiety 221 to a sample in order to create a mixture, incubating the mixture to allow the particles 207 to bind to a target 201, applying a magnetic field to capture target/magnetic particle complexes on a surface, thereby isolating target/magnetic particle complexes. Methods of the invention may further involve incubating the mixture in a binding solution that facilitates binding, washing the mixture in a wash solution that reduces particle aggregation, using a solution that facilitates binding and reduce aggregation, or a combination thereof. Certain fundamental technologies and principles are associated with binding magnetic materials to target entities and subsequently separating by use of magnet fields and gradients. Such fundamental technologies and principles are known in the art and have been previously described, such as those described in Murphy, 2011, Janeway's Immunobiology 8 Ed, Garland Science (New York, NY), 888 pages, the contents of which are incorporated by reference herein. Methods of producing suitable magnetic particles are known in the art, and discussed above. See for example U.S. Pat. 5,597,531; U.S. Pat. 4,230,685; U.S. Pat. 4,677,055; U.S. Pat. 4,695,393; U.S. Pat. 5,695,946; U.S. Pat. 4,018,886; U.S. Pat. 4,267,234; U.S. Pat. 4,452,773; U.S. Pat. 4,554,088; U.S. Pat. 4,659,678; U.S. Pat. 5,186,827; U.S. Pat. 4,795,698.

In particular embodiments, the target-specific binding moiety 221 is an antibody, such as an antibody that binds a particular bacterium. General methodologies for antibody production are described in the section entitled, "Methods of Antibody Production." Methods for attaching the target-specific binding moiety 221 to the magnetic particle 219, including the coating of particles with antibodies, are known in the art. See for example Kontermann, 2010, Antibody Engineering Volume 1 2Ed, Springer-Verlag (Berlin Heidelberg) 800 pages; Harlow, 1988, Antibodies: A Laboratory Manual, Cold Spring Harbor Laboratory Press (Cold Spring Harbor, NY) 726 pages; and Stanley, 2002, Essentials in Immunology and Serology, Delmar Cengage (Independence, KY) 560 pages; U.S. Pat. 7,699,979.

Such methodology can easily be modified by one of skill in the art to bind other types of target-specific binding moieties 221 to magnetic particles 219. In addition, certain types of magnetic particles coated with a functional moiety are commercially available from Sigma-Aldrich (St. Louis, Mo.).

In certain embodiments, a buffer solution, as discussed above, 207 is added to the sample along with the magnetic beads 219. An alternative approach to achieve high binding efficiency while reducing time required for the binding step is to use static mixer, or other mixing devices that provide efficient mixing of viscous samples at high flow rates, such as at or around 5 mL/min. In one embodiment, the sample is mixed with binding buffer in ratio of, or about, 1:1, using a mixing interface connector. The diluted sample then flows through a mixing interface connector where it is mixed with target-specific nanoparticles. Additional mixing interface connectors providing mixing of sample and antigen-specific nanoparticles can be attached downstream to improve binding efficiency. The combined flow rate of the labeled sample is selected such that it is compatible with downstream processing.

After binding of the magnetic particles 219 to the target analyte 201 in the mixture to form target/magnetic particle complexes, a magnetic field is applied to the mixture to capture the complexes on a surface (e.g., using magnet 231 as shown in FIG. 1). Components of the mixture that are not bound to magnetic particles will not be affected by the magnetic field and will remain free in the mixture. Methods and apparatuses for separating target/magnetic particle complexes from other components of a mixture are known in the art. For example, a steel mesh may be coupled to a magnet, a linear channel or channels may be configured with adjacent magnets, or quadrapole magnets with annular flow may be used. Other methods and apparatuses for separating target/magnetic particle complexes from other components of a mixture are shown in Rao et al. (U.S. Pat. No. 6,551,843), Liberti et al. (U.S. Pat. No. 5,622,831), Hatch et al. (U.S. Pat. No. 6,514,415), Benjamin et al. (U.S. Pat. No. 5,695,946), Liberti et al. (U.S. Pat. No. 5,186,827), Wang et al. (U.S. Pat. No. 5,541,072), Liberti et al. (U.S. Pat. No. 5,466,574), and Terstappen et al. (U.S. Pat. No. 6,623,983).

With continued reference to FIG. 32, the magnetic capture is achieved at high efficiency by utilizing a flow-through capture cell 531 with a number of strong rare earth bar magnets 231 placed perpendicular to the flow of the sample. When using a flow chamber with flow path cross-section 0.5 mm $\times$ 20 mm (h$\times$w) and 7 bar NdFeB magnets, the flow rate could be as high as 5 mL/min or more, while achieving capture efficiency close to 100%.

The above described type of magnetic separation produces efficient capture of a target analyte and the removal of a majority of the remaining components of a sample mixture. However, such a process may produce a sample that contains a percent of magnetic particles 219 that are not bound to target analytes 201, as well as non-specific target entities. Non-specific target entities may for example be bound at a much lower efficiency, for example 1% of the surface area, while a target of interest might be loaded at 50% or nearly 100% of the available surface area or available antigenic cites. However, even 1% loading may be sufficient to impart force necessary for trapping in a magnetic gradient flow cell or sample chamber.

The presence of magnetic particles 219 that are not bound to target analytes 201 and non-specific target entities on the surface that includes the target/magnetic particle complexes may interfere with the ability to successfully detect the target of interest. The magnetic capture of the resulting mix, and close contact of magnetic particles 219 with each other and bound targets 201, result in the formation of aggregate that is hard to dispense and which might be resistant or inadequate for subsequent processing or analysis steps. In order to remove magnetic particles that are not bound to target analytes and non-specific target entities, methods of the invention may further involve washing the surface with a wash solution that reduces particle aggregation, thereby isolating target/magnetic particle complexes from the magnetic particles that are not bound to target analytes and non-specific target entities. The wash solution minimizes the formation of the aggregates. Methods of the invention may use any wash solution, discussed above, that imparts a net negative charge to the magnetic particle that is not sufficient to disrupt interaction between the target-specific moiety 221 of the magnetic particle 219 and the target analyte 201.

Since methods of the invention are able to isolate pathogens at very low levels, methods of the invention reduce or eliminate the culturing step that is typically associated with pathogen analysis and allow for more rapid analysis of the isolated pathogen. Reduced culturing refers to culturing for at most about 24 hours, for example, for at most about 23 hours, for at most about 22 hours for at most about 21 hours, for at most about 20 hours, for at most about 15 hours, for at most about 10 hours, for at most about 9 hours, for at most about 8 hours, for at most about 7 hours, for at most about 6 hours, for at most about 5 hours, for at most about 4 hours, for at most about 3 hours, for at most about 2 hours, or for at most about 1 hr. In particular embodiments, culturing is for less than 1 hr., for example, about 45 minutes, about 30 minutes, about 15 minutes, about 10 minutes, or for less than 10 minutes. In particular embodiments, culturing is completely eliminated and the isolated bacteria is analyzed directly without any culturing, i.e., culturing is eliminated.

The target may be analyzed by a multitude of existing technologies, such as nuclear magnetic resonance (NMR), miniature NMR, Polymerase Chain Reaction (PCR), mass spectrometry, fluorescent labeling and visualization using microscopic observation, fluorescent in situ hybridization (FISH), growth-based antibiotic sensitivity tests, and variety of other methods that may be conducted with purified target without significant contamination from other sample components. Analysis using NMR is described in U.S. Pub. 2011/0262925, herein incorporated by reference in its entirety. In one embodiment, isolated cells are lysed with a chaotropic solution, and DNA is bound to DNA extraction resin. After washing of the resin, the DNA may be eluted and used in quantitative RT-PCR to detect the presence or an identity of the cells, such as detecting genera, species, or subclasses of bacteria.

In another embodiment, captured bacteria is removed from the magnetic particles 219 to which they are bound and the processed sample is mixed with a detectable label such as fluorescent labeled antibodies specific to the bacteria or fluorescent Gram stain. After incubation, the reaction mixture is filtered through 0.2 $\mu$m to 1.0 $\mu$m filter to capture labeled bacteria while allowing majority of free beads and fluorescent labels to pass through the filter. Bacteria is visualized on the filter using microscopic techniques, e.g. direct microscopic observation, laser scanning or other automated methods of image capture. The presence of bacteria is detected through image analysis. After the positive detection by visual techniques, the bacteria can be further characterized using PCR or genomic methods.

Detection of bacteria of interest can be performed by use of nucleic acid probes following procedures which are known in the art. Suitable procedures for detection of bacteria using nucleic acid probes are described, for example, in Stackebrandt et al. (U.S. Pat. No. 5,089,386), King et al. (WO 90/08841), Foster et al. (WO 92/15883), and Cossart et al. (WO 89/06699).

A suitable nucleic acid probe assay generally includes sample treatment and lysis, hybridization with selected probe(s), hybrid capture, and detection. This assay is discussed in the prior related sections. Detection of bacteria of interest can also be performed by use of PCR techniques. A suitable PCR technique is described, for example, in Verhoef et al. (WO 92/08805). Such protocols may be applied directly to the bacteria captured on the magnetic beads. The bacteria is combined with a lysis buffer and collected nucleic acid target molecules are then utilized as the template for the PCR reaction.

For detection of the selected bacteria by use of antibodies, isolated bacteria are contacted with antibodies specific to the bacteria of interest. As noted above, either polyclonal or monoclonal antibodies can be utilized, but in either case have affinity for the particular bacteria to be detected. These antibodies, will adhere/bind to material from the specific target bacteria. With respect to labeling of the antibodies, these are labeled either directly or indirectly with labels used in other known immunoassays. Direct labels may include fluorescent, chemiluminescent, bioluminescent, radioactive, metallic, biotin or enzymatic molecules. Methods of combining these labels to antibodies or other macromolecules are well known to those in the art. Examples include the methods of Hijmans et al., 1969, An immunofluorescence procedure for the detection of intracellular immunoglobulins, Clin Exp Immunol 4:457-472, for fluorescein isothiocyanate, the method of Goding, 1976, Conjugation of antibodies with fluorochromes: modifications to the standard methods, J Immunol Meth 13:215-226, for tetramethylrhodamine isothiocyanate, and the method of Engvall, 1980, Enzyme immunoassay ELISA and EMIT, J Clin Micro 70:419-439, for enzymes.

These detector antibodies 221 may also be labeled indirectly. In this case the actual detection molecule is attached to a secondary antibody or other molecule with binding affinity for the anti-bacteria cell surface antibody. If a secondary antibody is used it is preferably a general antibody to a class of antibody (IgG and IgM) from the animal species used to raise the anti-bacteria cell surface antibodies. For example, the second antibody may be conjugated to an enzyme, either alkaline phosphatase or to peroxidase. To detect the label, after the bacteria of interest is contacted with the second antibody and washed, the isolated component of the sample is immersed in a solution containing a chromogenic substrate for either alkaline phosphatase or peroxidase.

A chromogenic substrate is a compound that can be cleaved by an enzyme to result in the production of some type of detectable signal which only appears when the substrate is cleaved from the base molecule. The chromogenic substrate is colorless, until it reacts with the enzyme, at which time an intensely colored product is made. Thus, material from the target (e.g., bacterial cells) will become an intense blue/purple/black color, or brown/red. Examples of detection molecules include fluorescent substances, such as 4-methylumbelliferyl phosphate, and chromogenic substances, such as 4-nitrophenylphosphate, 3,3',5,5'-tetramethylbenzidine and 2,2'-azino-di-[3-ethelbenz-thiazoliane sulfonate]. In addition to alkaline phosphatase and peroxidase, other useful enzymes include β-galactosidase, β-glucuronidase, α-glucosidase, β-glucosidase, α-mannosidase, galactose oxidase, glucose oxidase and hexokinase.

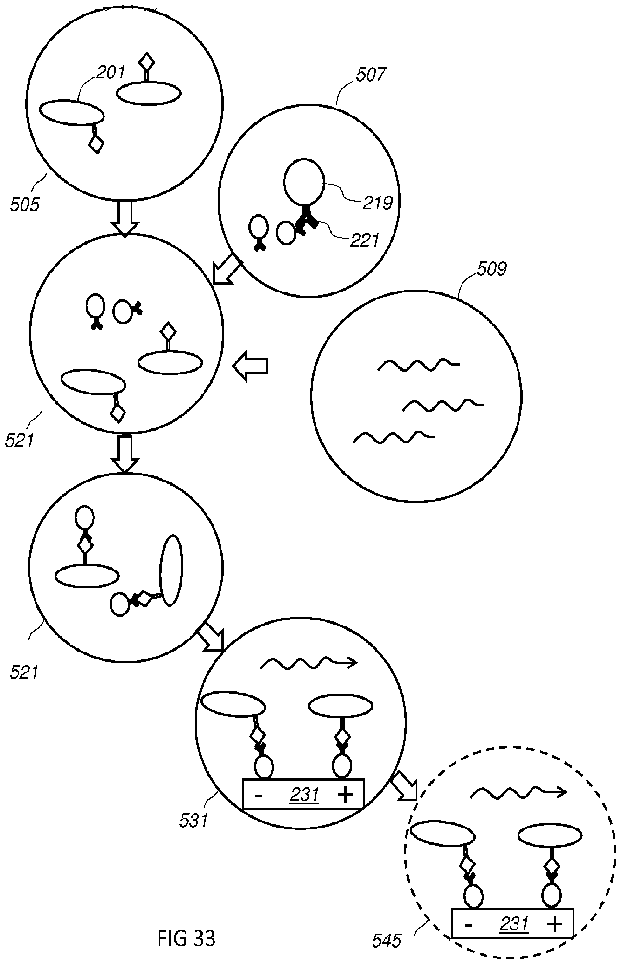

FIG. 33 illustrates methods of detecting a target analyte 201 using a fluidic device according to certain embodiments. A sample is delivered, for example from a collection tube 505, into a chamber 521. A binding buffer is delivered from reservoir 509 to chamber 521 and particles 219 with binding moiety 221 are delivered (for example, from ampoule 507) to chamber 521. Reservoir 509 becomes pressurized by pneumatic passages, or tubes, in pneumatic interface 511. The binding buffer may be formulated to enhance binding of the binding moiety to the target analyte, reduce formation of particle aggregates, or both. The sample is incubated in chamber 521 until binding moiety 221 binds target analyte 221. Note that in certain embodiments, the bead buffer mixture is optionally flowed through tube 505 and binding also occurs there.

Target analyte 201 is then isolated from a remainder of the sample. In some embodiments, the bead-bound target analyte 201 is transferred into magnetic trap 531, and is isolated using magnet 231 (e.g., using magnetic isolation techniques as described above). Moreover, the binding buffer, a wash buffer, or both, can be used-serially or in combination-to wash away components of the sample. In certain embodiments, bead-bound target analyte 201 is transferred to magnetic concentrator 545 where the target is further concentrated (e.g., washed by a wash buffer and concentrated to a desired concentration). In some embodiments, concentrating target analyte 201 involves flushing concentrator chamber 545 with the wash buffer. In certain alternative embodiments, a single chamber is used, and a wash solution is flushed through the chamber after the incubation with the buffer solution. Target analyte 201 can then be analyzed, for example, by replacing the wash buffer with a cell maintenance solution to keep cells alive or with a lysis buffer to extract a component of the analyte.

In certain embodiments, methods of the invention are useful for direct detection of bacteria from blood. Such a process is described here. Sample is collected in sodium heparin tube by venipuncture, acceptable sample volume is about 1 mL to 10 mL. Sample is diluted with binding buffer and superparamagnetic particles having target-specific binding moieties are added to the sample, followed by incubation on a shaking incubator at 37° C for about 30 min to 120 min. Alternative mixing methods can also be used. In a particular embodiment, sample is pumped through a static mixer, such that reaction buffer and magnetic beads are added to the sample as the sample is pumped through the mixer. This process allows for efficient integration of all components into a single fluidic part, avoids moving parts and separate incubation vessels and reduces incubation time.

Capture of the labeled targets allows for the removal of blood components and reduction of sample volume from 30 mL to 5 mL. The capture is performed in a variety of magnet/flow configurations. In certain embodiments, methods include capture in a sample tube on a shaking platform or capture in a flow-through device at flow rate of 5 mL/min, resulting in total capture time of 6 min.

After capture, the sample is washed with wash buffer including heparin to remove blood components and free beads. The composition of the wash buffer is optimized to reduce aggregation of free beads, while maintaining the integrity of the bead/target complexes.

In some embodiments, the detection method is based on a miniature NMR detector tuned to the magnetic resonance of water. When the sample is magnetically homogenous (no bound targets), the NMR signal from water is clearly detectable and strong. The presence of magnetic material in the detector coil disturbs the magnetic field, resulting in reduction in water signal. One of the primary benefits of this detection method is that there is no magnetic background in biological samples which significantly reduces the requirements for stringency of sample processing. In addition, since the detected signal is generated by water, there is a built-in signal amplification which allows for the detection of a single labeled bacterium. This method provides for isolation and detection of as low as or even lower than 1 CFU/ml of bacteria in a blood sample.

In certain aspects, the disclosure provides fluidic devices and systems for the analysis of target analytes. Any suitable device or system may be used. In general, the devices comprise an input channel, an output channel, a chamber, a magnetic assembly, and a lysing device. The input channel and output channel are in fluid communication with the chamber and the magnetic assembly is adapted to capture a magnetic particle inputted into the input channel onto a surface of the chamber. The lysing device is adapted to lyse a target bound to the magnetic particle. In one illustrative embodiment, the device is a fluidic cartridge.

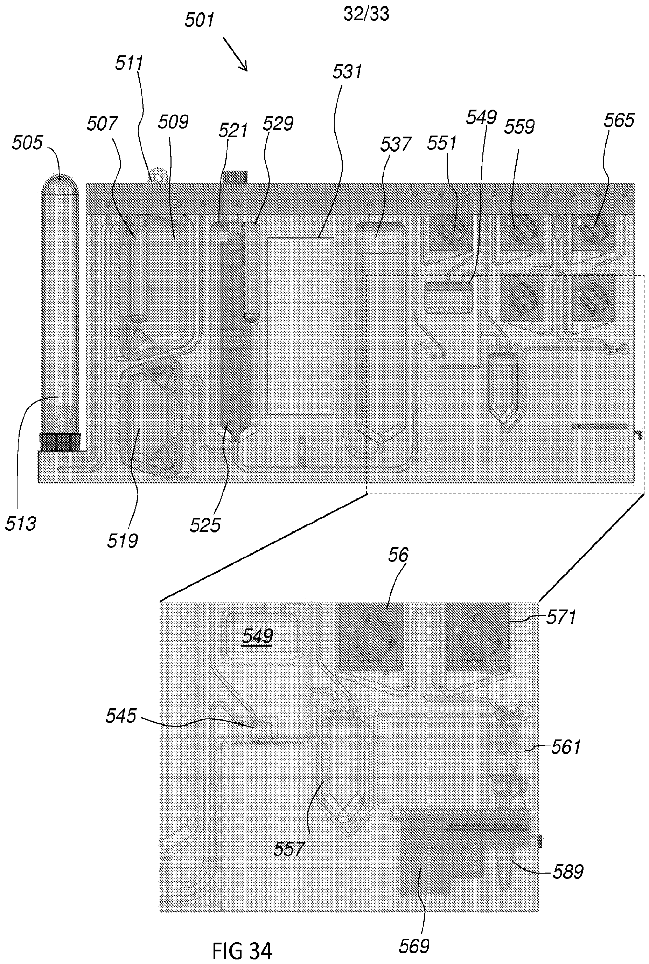

FIG. 34 illustrates a fluidic cartridge 501 according to certain embodiments. Fluidic cartridge 501 is provided to operate with sample collection tube 505 (e.g., a vacutainer). Cartridge 501 includes long needle 513, extending from a receiving member of catridge 501. Needle 513 defines a hollow member to penetrate into an interior of tube 505 when tube 505 is inserted thereon. Magnetic beads 219 may be stored in magnetic bead ampule 507 shown disposed within bead buffer 509. Pneumatic interface 511 provides pressure to flow sample, solutions, and buffers through channels of cartridge 501 and may optionally be used to rupture ampoules such as magnetic bead ampule 507 (and plant pathogen ampoule 529). When operation is begun and tube 505 containing a sample is inserted, magnetic bead ampule 507 is crushed, introducing beads 219 to bead buffer 509. Beads 219 and buffer 509 may be mixed using agitation provided by air from pneumatic interface 511, to produce a bead mixture.

Pressure from pneumatic interface 511 forces sample from tube 505 into mixing chamber 521. The bead mixture is forced through long needle 513 to rinse the inside of tube 505. The bead mixture is then sent to mixing chamber 521 to mix with the sample. This step may be repeated, sending more bead mixture from bead buffer 509, through tube 505, to mixing chamber 521, until tube 505 is evacuated of target and a desired proportion (e.g., 2:1) of bead mixture to sample is present in mixing chamber 521.

Mixing paddle 525 is used to mix the contents of chamber 521, agitating the sample and beads. Air pressure from pneumatic interface 511 then forces the mixture into magnetic trap 531. Optionally, the mixture can be pushed back, from magnetic trap 531 to mixing chamber 521, and the cycle repeated any number times until mixing chamber 521 is satisfactorily evacuated of target pathogens 201 and the bead-bound target pathogens 201 as well as the other components of the sample are in magnetic trap 431.

Then, magnetic trap 431 is evacuated of the other components of the sample, leaving magnetic particles 219 bound to magnets therein. The waste fluid is pushed back through mixing chamber 521 and discarded. Wash buffer 519 is then forced into magnetic trap 531, filling it. The wash buffer can then be pushed back to mixing chamber 521 to ensure good washing of beads 219. Wash buffer is sent back into magnetic trap 531 and held there.

Magnets can then be moved away from magnetic trap 531, allowing beads 219 to resuspend in wash buffer 519 within trap 531. At this point, target pathogens 201 have been extracted from the original sample and held in wash buffer 519. Wash buffer 519 can then be pushed through magnetic concentrator 545 to waste chamber 549.

Turning now to the inset portion of FIG. 34, magnetic concentrator 545 can be seen to be in fluid communication with magnetic trap 531. As wash buffer 519 flows through concentrator 545, beads 219 are captured in concentrator 545 while the remainder of buffer 519 is passed on to waste chamber 549. Original target analytes 201 are thus concentrated in concentrator 545.

The contents of concentrator 545 may be processed according to a desired output. Where target analyte 201 includes cells (e.g., a microbial pathogen) whether live cells or an extracted cellular component is intended can be used to determine a following step. If live cells are intended, the magnet is removed from magnetic concentrator 545 and buffer 551 (here, a live cell buffer) is introduced through concentrator 545 and used to flush the cells into output vial 589.

If, for example, extracted DNA is intended, buffer 551 is a lysis buffer and is introduced into magnetic concentrator 545. The magnet is removed from concentrator 545 and a sonicator may be applied to a wall of the chamber of concentrator 545. Optionally, beads may be included for bead-bashing to aid in lysis. The sonicator or other lysis means is activated and the target cells 201 are lysed. In certain embodiments, a probe of a sonicator extends into the chamber, where it delivers vibrations into the liquid medium surrounding the captured complexes. In other embodiments, the sonication transducer is brought in contact with the chamber by way of a structural interface. For example, the structural interface may constitute the floor or the ceiling of the chamber. The sonication transducer vibrates the structural interface such that lysis of the targets captured in the chamber is achieved.

Lysate is then pushed into pre-column mixer 557. DNA binding buffer 559 is added to pre-column mixer 557 (optionally agitated by bubbling air). The contents of pre-column mixer 557 is then forced through the DNA extraction column 561 and the elutant is discarded as waste. DNA extraction may be completed using washes from first column wash 565, second column wash 569, and water 571. Air from pneumatic interface 511 can be forced through DNA extraction column 561 to remove volatile organic compounds. Water 571 can be used to rinse the purified DNA (optionally including the use of a de-binding buffer or a modulator of stringency) into output vial 589.

Coordination of the on-cartridge steps can be supported by an operations device, such as a bench-top electro-mechanical device. The timing of pneumatic injections, the breaking of ampoules, and the piercing of reagent reservoirs can be coordinated manually, or by a computer program or mechanical system. Cartridge 501 can include any suitable materials, shape, or dimensions. For example, in some embodiments, fluids are handled in macrofluidic (e.g., fluidic) environments up until entered into magnetic concentrator 545 and are handled according to microfluidic principles thereafter. In general, microfluidic may refer to sub-microliter volumes. Macrofluidic, or fluidic, can refer to fluidic volumes that are not microfluidic.

Generally, microfluidics relates to small sample volumes and small channel pathways. For example, microfluidic volumes are normally below 1 mL, or on the microliter (μL) scale or smaller, for example, nL (nanoliters) and pL (picoliters). As used herein, microfluidic volumes relate to volumes less than 1 mL. In addition, microfluidics relates to small channel pathways on the micrometer scale. As used herein, microfluidic channels within systems of the invention refer to channels that have channel heights and/or widths equal to or less than 500 μm. See "Microfluidics and Nanofluidics: Theory and Selected Applications," Kleinstruer, C., John Wiley & Sons, 2013, which is incorporated by reference. The channel height or width is defined as the height or width of the path that the sample volume must pass through within the cartridge. Comparatively, macrofluidics volumes relate to volumes greater than the microliter (μL) scale, for example, sample volumes on the milliliter (mL) scale. As used herein, macrofluidic volumes are volumes of 1 mL or greater. Macrofluidic channels within systems of the invention are channels having channel heights and/or widths of greater than 500 μm.

Other macrofluidic components are chambers, reservoirs, traps, mixers, etc. Such macrofluidic components are dimensioned to hold 1 mL or more of fluid. For example, the individual volume can range without limitation from about 10 to about 50 mL. Other microfluidic components are chambers, reservoirs, traps, mixers, etc. Such microfluidic components are dimensioned to hold less than 1 mL of fluid. For example, the individual volumes can range without limitation from about 1 μL to about 500 μL.

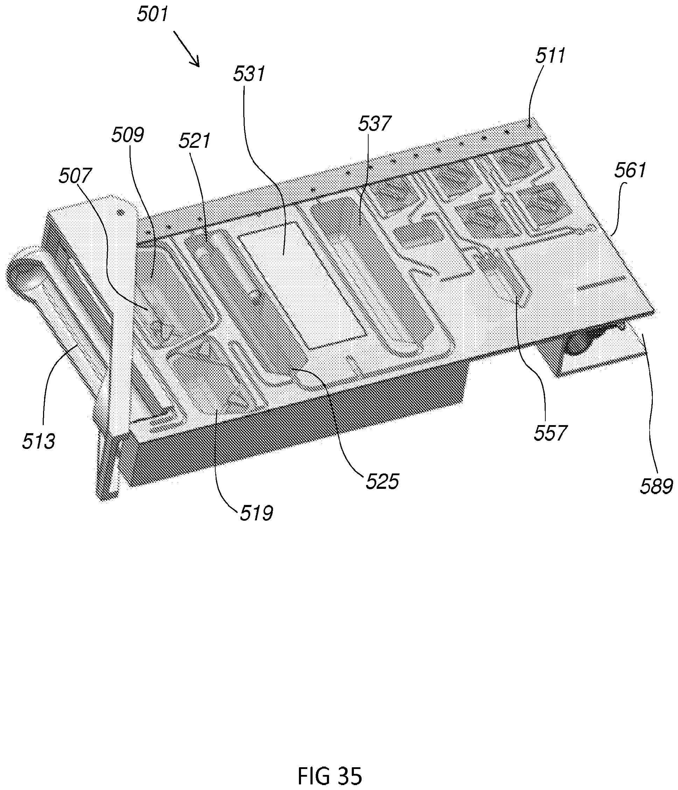

FIG. 35 gives a perspective view of an exemplary cartridge 501 according to certain embodiments. Methods for manufacturing and operating fluidic systems are known and discussed in Fredrickson and Zan, 2004, Macro-to-micro interfaces for microfluidic devices, Lab Chip 4(6):526-33; U.S. Pat. 8,105,783; U.S. Pat. 7,785,869; U.S. Pat. 7,745,207; U.S. Pat. 7,553,647; U.S. Pub. 2009/0227005; U.S. Pub. 2008/0241000; and U.S. Pub. 2008/0003564.

FIG. 31 gives a schematic diagram of elements and steps of the invention as discussed above with respect to FIG. 34.

While discussed above as magnetic beads, substrate 219 for capture of pathogens 201 may be the floor of a chamber such as, for example, trap chamber 531. In other embodiments, the capture surface is the ceiling. The captured pathogens 201 can there be washed to remove non-specific analytes and unbound entities.

In certain embodiments, fluidic cartridge 501 is designed to be disposable. In this instance, once pathogens 201 have been lysed and the eluate collected, cartridge 501 can be discarded. In this manner, each cartridge is intended for a single use, and the potential for cross-contaminating pathogens due to repeated use is eliminated.

The cartridge can be prepared from any material in the art suitable for containing liquids and withstanding the rigors of sonication. In certain aspects, the entire cartridge is made of plastic or an acrylic plastic polymer. In other aspects, the bottom or top cover of the cartridge can be prepared from a film such as the biaxially-oriented polyethylene terephthalate (BoPET) sold under the trademark MYLAR (general purpose file, commercially available by DuPont) while the rest of the cartridge is made of plastic or an acrylic plastic polymer. In certain embodiments, the BoPET film constitutes the aforementioned structural interface in contact with the sonicator transducer.

In certain embodiments, the cartridge is connected to a fluidic device or system configured to flow liquids into and out of the cartridge. The fluidic system can comprise a pump that delivers liquid, air, gas, beads, reagents, electricity or other signals, light, power or a combination thereof to the cartridge. The fluidic system can have a first tubing line connected to the inlet of the input channel, for flowing fluid (e.g., gas or liquid) into the cartridge. The other end of the first tubing line is connected to the pump. A second tubing line for fluid leaving the cartridge is connected to the outlet of the output channel. The other end of the second tubing line may also be connected to the pump or to a container for collecting exiting fluid. The fluidic system facilitates the delivery of the target/magnetic particle complexes into the chamber, as well as any washing of captured complexes. The collection of lysate is also facilitated by the fluidic system. In some embodiments, all steps are performed on a single cartridge 501. For example, sample collection tube 505 may be loaded into cartridge 501 and all of the described steps may be performed "on-chip" including delivering cells or an extracted material into an output vial. In certain embodiments, all of the on-chip steps are substantially automated.

Devices of the disclosure may also include a detection module. The detection module is a component in which molecules, cells, or other particles are to be detected, identified, measured or interrogated on the basis of at least one predetermined characteristic. The molecules, cells, or other particles can be examined one at a time, and the characteristic is detected or measured optically, for example, by testing for the presence or amount of a label. In some aspects, the detection module is in connection with one or more detection apparatuses. The detection apparatuses can be optical or electrical detectors or combinations thereof. Examples of suitable detection apparatuses include optical waveguides, microscopes, diodes, light stimulating devices (e.g., lasers), photomultiplier tubes, optically transmissive cuvettes, fluidic chambers with electrodes to sense impedance changes, and processors (e.g., computers and software), and combinations thereof, which cooperate to detect a signal representative of a characteristic, marker, probe, label, or reporter, and to determine and direct the measurement or sorting action at the sorting module. However, other detection techniques can be used as well. In some embodiments, the target analyte is detected by a change in electrical conductivity or impendence in a fluidic medium. Measuring impedance or resistance for analyte detection is discussed in U.S. Pat. 6,990,849; U.S. Pub. 2011/0136102; U.S. Pub. 2011/0020459; and U.S. Pub. 2007/0238112.

Devices of the invention may include a computer component. For example, a user interface may be provided with input/output mechanisms (monitor, keyboard, touchscreen, etc.) coupled to a memory and a processor (e.g., a silicone chip and a solid-state or magnetic hard drive). Input/output mechanisms may be included for data, such as a USB port, Ethernet port, or Wi-Fi card or a hardware connection to a detection module, fluidic chip and pneumatic interface, or both.

In certain aspects, the detection module is in fluid connection with the fluidic cartridge. For example, the outlet of the fluidic cartridge may be connected to the detection module by means of a tubing line. In this manner, lysate leaving the cartridge can enter the detection module wherein the contents of the lysate can be detected and analyzed.

Combination of Embodiments

As will be appreciated by one skilled in the art, individual features of the invention may be used separately or in any combination. Particularly, it is contemplated that one or more features of the individually described above embodiments may be combined into a single embodiment.

Equivalents

Various modifications of the invention and many further embodiments thereof, in addition to those shown and described herein, will become apparent to those skilled in the art from the full contents of this document, including references to the scientific and patent literature cited herein. The subject matter herein contains important information, exemplification and guidance that can be adapted to the practice of this invention.

**Claims**

1. A method for isolating a pathogen from a sample, the method comprising providing a vessel containing a sample that comprises a pathogen;
   removably coupling the vessel to a cartridge via a cartridge interface that is configured to provide communication between the vessel and the cartridge;
   flowing a plurality of magnetic particles from the cartridge into the vessel to thereby form a mixture in the vessel that comprises the sample and the magnetic particles, wherein a plurality of the particles are conjugated to a capture moiety specific to the pathogen;
   flowing the mixture from the vessel into the cartridge;
   flowing a fluid from the cartridge into the vessel to flush any residual mixture from the vessel into the cartridge;
   incubating the mixture in the cartridge to allow the particles to bind to the pathogen, thereby forming pathogen/magnetic particle complexes;
   applying a magnetic field to capture pathogen/magnetic particle complexes on a surface of a first magnetic trap;
   washing the captured pathogen/magnetic particle complexes with a wash solution, thereby isolating the pathogen/magnetic particle complexes;
   removing the magnetic field;

re-introducing the wash solution, thereby re-suspending the pathogen/magnetic particle complexes;

flowing the resuspended pathogen/magnetic particle complexes from the first magnetic trap through a channel and into a second magnetic trap; and

engaging the second magnetic trap, thereby re-capturing the pathogen/magnetic particle complexes;

wherein the first magnetic trap is configured to capture the pathogen/magnetic particle complexes from a macro-scale volume of fluid, and the second magnetic trap is configured to concentrate substantially all of the pathogen/magnetic particle complexes into a micro-scale volume of fluid.

2. The method according to claim 1, further comprising lysing the captured pathogen in a region selected from the group consisting of the first magnetic trap, the second magnetic trap, and a combination thereof.

3. The method according to claim 2, further comprising separating nucleic acid from the lysate; and collecting the separated nucleic acid into a vial.

4. The method according to claim 3, further comprising analyzing the nucleic acid to thereby identify the pathogen.

5. The method according to claim 4, wherein analyzing comprises conducting a sequencing reaction.

6. The method according to claim 4, wherein analyzing comprises conducting an amplification reaction and wherein the vial comprises at least one detectably labeled nucleic acid probe specific for the pathogen.

7. The method according to claim 1, wherein the sample comprises a plurality of pathogen and the plurality of magnetic particles are composed of different sets, wherein members of the different sets are conjugated to different capture moieties that are specific for the different pathogen.

8. The method according to claim 7, further comprising conducting a multiplex reaction to analyze nucleic acid from the plurality of isolated pathogen and thereby identify the plurality of pathogen.

9. The method according to claim 1, wherein the capture moiety is a type selected from the group consisting of antibodies, lectins, bacteriophages, antimicrobial agents, oligonucleotides, and combinations thereof.

10. The method according to claim 1, wherein the pathogen is selected from the group consisting of fungi, bacteria or both.

11. The method according to claim 10, wherein the fungi are selected from the group consisting of the Candida genus, the Aspergillus genus, the Cryptococcus genus, and a combination thereof.

12. The method according to claim 10, wherein the bacteria are selected from the group consisting of the Staphylococcus genus, the Enterobacteriaceae genus, the Acinetobacter genus, the Strenotrophomonas genus, the Pseudomanos genus, the Neisseria genus, the Clostridium genus, and the Enterococcus genus.

**Patentansprüche**

1. Verfahren zum Isolieren eines Pathogens aus einer Probe, wobei das Verfahren umfasst: Bereitstellen eines Gefäßes, das eine Probe mit einem Pathogen enthält;

entfernbares Koppeln des Gefäßes an eine Kartusche über eine Kartuschenschnittstelle, die konfiguriert ist, um eine Kommunikation zwischen dem Gefäß und der Kartusche bereitzustellen;

Fließen mehrerer Magnetpartikel aus der Kartusche in das Gefäß, um dadurch ein Gemisch in dem Gefäß zu bilden, das die Probe und die Magnetpartikel umfasst, wobei mehrere Partikel an eine für das Pathogen spezifische Einfangeinheit konjugiert sind;

Fließen des Gemischs aus dem Gefäß in die Kartusche;

Fließen einer Flüssigkeit aus der Kartusche in das Gefäß, um jeglichen Gemischrest aus dem Gefäß in die Kartusche zu spülen;

Inkubieren des Gemischs in der Kartusche, damit sich die Partikel an das Pathogen binden können, wodurch Pathogen/Magnetpartikel-Komplexe gebildet werden;

Anlegen eines Magnetfeldes zum Einfangen von Pathogen/Magnetpartikel-Komplexen auf einer Oberfläche einer ersten Magnetfalle;

Waschen der eingefangenen Pathogen/Magnetpartikel-Komplexe mit einer Waschlösung, wodurch die Pathogen/Magnetpartikel-Komplexe isoliert werden;

Entfernen des Magnetfeldes;

erneutes Einführen der Waschlösung, wodurch die Pathogen/Magnetpartikel-Komplexe resuspendiert werden;

Fließen der resuspendierten Pathogen/Magnetpartikel-Komplexe von der ersten Magnetfalle durch einen Kanal in eine zweite Magnetfalle; und

Eingreifen in die zweite Magnetfalle, wodurch die Pathogen/Magnetpartikel-Komplexe wieder eingefangen werden; wobei die erste Magnetfalle konfiguriert ist, um die Pathogen/Magnetpartikel-Komplexe aus einem Flüssigkeitsvolumen im Makromaßstab einzufangen, und die zweite Magnetfalle konfiguriert ist, um im Wesentlichen alle Pathogen/Magnetpartikel-Komplexe in einem Flüssigkeitsvolumen im Mikromaßstab zu konzentrieren.

2. Verfahren nach Anspruch 1, das weiterhin das eingefangene Pathogen in einem Bereich, der aus der Gruppe bestehend aus der ersten Magnetfalle, der zweiten Magnetfalle und einer Kombination davon ausgewählt ist, lysiert.

3. Verfahren nach Anspruch 2, das weiterhin das Trennen von Nukleinsäure aus dem Lysat umfasst; und Sammeln der abgetrennten Nukleinsäure in einem Fläschchen.

4. Verfahren nach Anspruch 3, das weiterhin das Analysieren der Nukleinsäure umfasst, um dadurch das Pathogen zu identifizieren.

5. Verfahren nach Anspruch 4, wobei das Analysieren das Durchführen einer Sequenzierungsreaktion umfasst.

6. Verfahren nach Anspruch 4, wobei das Analysieren das Durchführen einer Amplifikationsreaktion umfasst und wobei das Fläschchen mindestens eine für das Pathogen spezifische, nachweisbar markierte Nukleinsäuresonde umfasst.

7. Verfahren nach Anspruch 1, wobei die Probe mehrere Pathogene umfasst und die magnetischen Partikel aus verschiedenen Sätzen zusammengesetzt sind, wobei die Elemente der verschiedenen Sätze an verschiedene Einfangeinheiten konjugiert werden, die für die verschiedenen Pathogene spezifisch sind.

8. Verfahren nach Anspruch 7, das weiterhin das Durchführen einer Multiplex-Reaktion umfasst, um Nukleinsäure aus den mehreren isolierten Pathogenen zu analysieren und dadurch die mehreren Pathogene zu identifizieren.

9. Verfahren nach Anspruch 1, wobei die Einfangeinheit von dem Typ ist, der aus der Gruppe bestehend aus Antikörpern, Lectinen, Bakteriophagen, antimikrobiellen Mitteln, Oligonukleotiden und Kombinationen davon ausgewählt ist.

10. Verfahren nach Anspruch 1, wobei das Pathogen aus der Gruppe von Pilzen, Bakterien oder beiden ausgewählt ist.

11. Verfahren nach Anspruch 10, wobei die Pilze aus der Gruppe ausgewählt sind, die aus der Gattung Candida, der Gattung Aspergillus, der Gattung Cryptococcus und einer Kombination davon besteht.

12. Verfahren nach Anspruch 10, wobei die Bakterien aus der Gruppe ausgewählt sind, die aus der Gattung Staphylococcus, der Gattung Acinetobacter, der Gattung Enterobacteriaceae, der Gattung Strenotrophomonas, der Gattung Pseudomonas, der Gattung Neisseria, der Gattung Clostridium und der Gattung Enterococcus besteht.

**Revendications**

1. Procédé d'isolation d'un agent pathogène à partir d'un échantillon, le procédé comprenant la fourniture d'un récipient contenant un échantillon qui comprend un agent pathogène ;

l'accouplement amovible du récipient à une cartouche par le biais d'une interface de cartouche qui est conçue pour assurer la communication entre le récipient et la cartouche ;

la circulation d'une pluralité de particules magnétiques de la cartouche dans le récipient pour former ainsi un mélange dans le récipient qui comprend l'échantillon et les particules magnétiques, dans lequel une pluralité des particules sont conjuguées à une fraction de capture spécifique à l'agent pathogène ;

la circulation du mélange du récipient dans la cartouche ;

la circulation d'un fluide à partir de la cartouche dans le récipient pour évacuer tout mélange résiduel du récipient dans la cartouche ;

l'incubation du mélange dans la cartouche pour permettre aux particules de se lier à l'agent pathogène, formant ainsi des complexes de particules magnétiques/agent pathogène ;

**EP 2 935 613 B1**

l'application d'un champ magnétique pour capturer des complexes de particules magnétiques/agent pathogène sur une surface d'un premier piège magnétique;

le lavage des complexes de particules magnétiques/agent pathogène capturés à l'aide d'une solution de lavage, isolant ainsi les complexes de particules magnétiques/agent pathogène;

la suppression du champ magnétique;

la réintroduction de la solution de lavage, remettant ainsi en suspension les complexes de particules magnétiques/agent pathogène;

la circulation des complexes de particules magnétiques/agent pathogène remis en suspension à partir du premier piège magnétique à travers un canal et dans un second piège magnétique; et

la mise en prise du second piège magnétique, capturant ainsi de nouveau les complexes de particules magnétiques/agent pathogène;

dans lequel le premier piège magnétique est conçu pour capturer les complexes de particules magnétiques/agent pathogène à partir d'un volume de fluide à macro-échelle, et le second piège magnétique est conçu pour concentrer sensiblement tous les complexes de particules magnétiques/agent pathogène dans un volume de fluide à micro-échelle.

2. Procédé selon la revendication 1, comprenant en outre la lyse de l'agent pathogène capturé dans une région choisie dans le groupe constitué du premier piège magnétique, du second piège magnétique et d'une combinaison de ceux-ci.

3. Procédé selon la revendication 2, comprenant en outre la séparation de l'acide nucléique du lysat; et la collecte de l'acide nucléique séparé dans un flacon.

4. Procédé selon la revendication 3, comprenant en outre l'analyse de l'acide nucléique pour ainsi identifier l'agent pathogène.

5. Procédé selon la revendication 4, dans lequel l'analyse comprend la réalisation d'une réaction de séquençage.

6. Procédé selon la revendication 4, dans lequel l'analyse comprend la réalisation d'une réaction d'amplification et dans lequel le flacon comprend au moins une sonde d'acide nucléique marquée de manière détectable et spécifique à l'agent pathogène.

7. Procédé selon la revendication 1, dans lequel l'échantillon comprend une pluralité d'agents pathogènes et la pluralité de particules magnétiques sont composés de différents ensembles, dans lesquels les membres des différents ensembles sont conjugués à différentes fractions de capture qui sont spécifiques aux différents agents pathogènes.

8. Procédé selon la revendication 7, comprenant en outre la réalisation d'une réaction multiplexe pour analyser l'acide nucléique de la pluralité de pathogènes isolés et ainsi identifier la pluralité de pathogènes.

9. Procédé selon la revendication 1, dans lequel la fraction de capture est un type choisi dans le groupe constitué d'anticorps, de lectines, de bactériophages, d'agents antimicrobiens, d'oligonucléotides et de leurs combinaisons.

10. Procédé selon la revendication 1, dans lequel l'agent pathogène est choisi dans le groupe constitué de champignons, de bactéries ou des deux.

11. Procédé selon la revendication 10, dans lequel les champignons sont choisis dans le groupe constitué du genre Candida, du genre Aspergillus, du genre Cryptococcus et d'une combinaison de ceux-ci.

12. Procédé selon la revendication 10, dans lequel les bactéries sont choisies dans le groupe constitué du genre Staphylococcus, du genre Enterobacteriaceae, du genre Acinetobacter, du genre Strenotrophomonas, du genre Pseudomanos, du genre Neisseria, du genre Clostridium et du genre Enterococcus.

1100 — Insert Vacutainer in Cartridge & Insert Cartridge into PCS

1110 — Add Blood + Beads + Buffer to Mixing Chamber

1120 — Incubate & Agitate

1130 — Capture Bead-Target (B*T)

1140 — Wash and Elute Target

1150 — Remove Target for analysis

1160 — Wash, Lyse & Extract DNA

1170 — Bind DNA to Column & Wash

1180 — Elute DNA with $H_2O$

1190 — Remove Purified DNA for analysis

Pathogen Capture System

FIG 1

74

FIG 2

FIG 3

FIG 4

FIG 5

FIG 6

FIG 7

EP 2 935 613 B1

FIG 8

FIG 9

FIG 10

FIG 11

FIG 12

FIG 13

FIG 14

FIG 15

FIG 16

FIG 17

EP 2 935 613 B1

**FIG 18**

**FIG 19**

FIG 21

FIG 20

FIG 22

FIG 23

Blood sample with suspected pathogen

Blood mixed with antibody-coated magnetic beads

Beads bind to antigens on bacteria

Beads and bacteria captured in magnetic trap and washed to remove blood

100m ID tube

Beads and bacteria eluted in small volume and passed through NMR detector

Multiple magnetic beads attached to bacterium produce strong signal in NMR detector – single cell detection of infection

FIG 24A

FIG 24B

OBTAIN SAMPLE — *101*

INTRODUCE VIRAL PREPARATION — *107*

VIRUS ATTACH — *113*

CELLS PRESENT BINDING TARGET (TARGET) — *119*

SUBSTRATE+BINDER (BINDER) — *125*

INCUBATE — *131*

BIND TARGET — *133*

ISOLATE — *135*

(CONCENTRATE) — *139*

LIVE CELLS — *141*

LIVE CELL BUFFER — *147*

LYSIS BUFFER — *151*

LYSE CELLS — *155*

COLUMN SEPARATION — *159*

FIG 25

FIG 26

FIG 27

FIG 28

FIG 29

FIG 30

FIG 31

FIG 32

FIG 33

FIG 34

FIG 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61739647
- US 61739577
- US 61739644
- US 61739612
- US 61739511
- US 61739616
- US 61739567
- US 61739575
- US 61739618
- US 20110263833 A
- US 20110262925 A
- US 20110262932 A
- US 20110262933 A
- US 20110262926 A
- US 20110262927 A
- US 3970518 A
- US 4230685 A, Senyi
- US 4677055 A, Dodin
- US 4695393 A, Whitehead
- US 5695946 A, Benjamin
- US 4018886 A, Giaever
- US 4267234 A, Rembaum
- US 4452773 A, Molday
- US 4554088 A, Whitehead
- US 4659678 A, Forrest
- US 5186827 A, Liberti
- US 4795698 A, Own
- WO 9102811 A, Liberti
- US 5004699 A
- US 5270163 A, Gold
- US 4851330 A
- US 8703009 W, Hogan
- US 091506
- US 20110300609 A
- US 5089386 A, Stackebrandt
- WO 9008841 A, King
- WO 9215883 A, Foster
- WO 8906699 A, Cossart
- WO 9208805 A, Verhoef
- US 20130059762 A
- US 20050026144 A
- US 4942124 A
- US 5149625 A, G.M. Church and S. Kieffer-Higgins
- US 6551843 B, Rao
- US 5622831 A, Liberti
- US 6514415 B, Hatch
- US 5541072 A, Wang
- US 5466574 A, Liberti
- US 6623983 B, Terstappen
- US 20060195269 A, Yeatman
- US 4943531 A
- US 5405776 A
- US 5618703 A
- US 6925389 B
- US 6989100 B
- US 6890763 B
- US 7300770 B
- US 855147
- US 20110262893 A
- WO 906699 A, Cossart
- US 7169560 B, Lapidus
- US 20090191565 A, Lapidus
- US 6818395 B, Quake
- US 7282337 B, Harris
- US 20020164629 A, Quake
- US 20090026082 A
- US 20090127589 A
- US 20100035252 A
- US 20100137143 A
- US 20100188073 A
- US 20100197507 A
- US 20100282617 A
- US 20100300559 A
- US 20100300895 A
- US 20100301398 A
- US 20100304982 A
- US 20090026082
- US 20050260293 A
- US 5691136 A
- US 5523217 A
- US 147056
- US 12257892 B
- US 7731828 B
- US 418837
- US 418837 A
- US 7043371 B
- US 7148054 B
- US 7732150 B
- US 20090246752 A
- US 20090047254 A

- US 20040156831 A
- WO 2003035889 A
- US 8071337 B
- US 7951579 B
- US 20120168372 A
- US 20120128652 A
- US 20110064699 A
- WO 0207742 A
- US 6121036 A
- US 20050032036 A
- US 7067639 B
- US 6896887 B
- US 20120040829 A
- US 20100203082 A
- US 5597531 A
- US 7517643 B
- US 6342588 B
- US 20110086338 A
- US 6740492 B
- US 5994519 A
- US 20010019820 A
- US 8227242 B
- US 8216797 B
- US 7238669 B
- US 20100240579 A
- US 20060063149 A
- US 7943346 B
- US 5620847 A
- US 5569586 A
- US 5541308 A
- US 5401631 A
- US 5055394 A
- US 20110071033 A
- US 8105783 B
- US 7785869 B
- US 7745207 B
- US 7553647 B
- US 20090227005 A
- US 20080241000 A
- US 20080003564 A
- US 8171803 B
- US 7046011 B
- US 6087183 A
- US 5467776 A
- US 5201231 A
- US 3787290 A
- US 3337416 A
- US 7611862 B
- US 5859375 A
- US 5823592 A
- US 20100313685 A
- US 7699979 B
- US 6990849 B
- US 20110136102 A
- US 20110020459 A
- US 20070238112 A

**Non-patent literature cited in the description**

- **HARLOW et al.** Antibodies. Cold Spring Harbor Laboratory, 1988, 93-117
- **INAI et al.** *Histochemistry,* May 1993, vol. 99 (5), 335-362
- **MULDER et al.** *Hum. Immunol.,* 1993, vol. 36 (3), 186-192
- **HARADA et al.** *J. Oral Pathol. Med.,* 1993, vol. 22 (4), 145-152
- **STAUBER et al.** *J. Immunol. Methods,* 1993, vol. 161 (2), 157-168
- **VENKATESWARAN et al.** *Hybridoma,* 1992, vol. 11 (6), 729-739
- **VERMUNT et al.** *J. Appl. Bact.,* 1992, vol. 72, 112
- **JOHNE et al.** *J. Clin. Microbiol.,* 1989, vol. 27, 1631
- **SKJERVE et al.** *Appl. Env. Microbiol.,* 1990, vol. 56, 3478
- **LUND et al.** *J. Clin. Microbiol.,* 1991, vol. 29, 2259
- **STODDART, R. W. ; B. M. HERBERTSON.** The use of fluorescein-labelled lectins in the detection and identification of fungi pathogenic for man: a preliminary study. *Journal of medical microbiology,* 1978, vol. 11.3, 315-324
- **SAMBROOK et al.** MOLECULAR CLONING: A LABORATORY MANUAL. Cold Spring Harbor Laboratory Press, 1989
- *Nature Methods,* 2005, vol. 2, 151-152
- **SINGH ; AMIT et al.** Bacteriophage based probes for pathogen detection. *Analyst,* 2012, vol. 137.15, 3405-3421
- **DOVER ; JASON E. et al.** Recent advances in peptide probe-based biosensors for detection of infectious agents. *Journal of microbiological methods,* 2009, vol. 78.1, 10-19
- **HARLOW et al.** Antibodies. Cold Spring Harbor Laboratory, 1988
- **HUNTER et al.** *Immunoassays for Clinical Chemistry,* 1983, 147-162
- **STANLEY ; DELMAR.** *Essentials in Immunology and Serology,* 2002, 152-153
- **KLEINSTRUER, C.** Microfluidics and Nanofluidics: Theory and Selected Applications. John Wiley & Sons, 2013
- **ELNIFRO ; ELFATH M. et al.** Multiplex PCR: optimization and application in diagnostic virology. *Clinical Microbiology Reviews,* 2000, vol. 13.4, 559-570

- **CARROLL, N. M. ; E. E. JAEGER et al.** Detection of and discrimination between gram-positive and gram-negative bacteria in intraocular samples by using nested PCR. *J Clin Microbiol,* 2000, vol. 38 (5), 1753-1757
- **KLASCHIK, S. ; L. E. LEHMANN et al.** Real-time PCR for detection and differentiation of gram-positive and gram-negative bacteria. *J Clin Microbiol,* 2002, vol. 40 (11), 4304-4307
- **HIJMANS, W. et al.** *Clin. Exp. Immunol.,* 1969, vol. 4, 457
- **GODING, J. W.** *J. Immunol. Meth.,* 1976, vol. 13, 215
- **INGRALL, E.** *Meth. in Enzymol.,* 1980, vol. 70, 419-439
- **JANEWAY.** Immunobiology. Garland Science Publishing
- **PARKER ; BARNES.** *Methods in Molecular Biology,* 1999, vol. 106, 247-283
- **HOD.** *Biotechniques,* 1992, vol. 13, 852-854
- **WEIS et al.** *Trends in Genetics,* 1992, vol. 8, 263-264
- **AUER et al.** *Nucleic Acids Res.,* 1996, vol. 24, 5021-5025
- **LEVY D D ; TEEBOR G W.** *Nuc. Acids Res.,* 1991, vol. 19 (12), 3337-3343
- **WARREN R A.** *Annu. Rev. Microbiol.,* 1980, vol. 34, 137-158
- **LEVY D D ; TEEBOR G W.** *Nuc. Acids Res.,* 1991, vol. 19 (12), 3337-3343
- **KLICKSTEIN et al.** Conversion of mRNA into Double-Stranded cDNA. Current Protocols. *Molecular Biology,* 2001, vol. 29, 5.5.1-5.5.14
- **HELD et al.** *Genome Research,* 1996, vol. 6, 986-994
- **DING ; CANTOR.** *Proc. Natl. Acad. Sci. USA,* 2003, vol. 100, 3059-3064
- **LIANG ; PARDEE.** *Science,* 1992, vol. 257, 967-971
- **KAWAMOTO et al.** *Genome Res.,* 1999, vol. 12, 1305-1312
- **OLIPHANT et al.** Discovery of Markers for Disease. *Supplement to Biotechniques,* June 2002
- **FERGUSON et al.** *Analytical Chemistry,* 2000, vol. 72, 5618
- **YANG et al.** *Genome Res.,* 2001, vol. 11, 1888-1898
- **FUKUMURA et al.** *Nucl. Acids. Res.,* 2003, vol. 31 (16), e94
- **SCHENA et al.** *Proc. Natl. Acad. Sci. USA,* 1996, vol. 93 (2), 106-149
- **HARLOW ; LANE.** ANTIBODIES: A LABORATORY MANUAL. Cold Spring Harbor, 1988
- **VELCULESCU et al.** *Science,* 1995, vol. 270, 484-487
- **VELCULESCU et al.** *Cell,* 1997, vol. 88, 243-51
- **BRENNER et al.** *Nature Biotechnology,* 2000, vol. 18, 630-634
- Current Protocols in Molecular Biology. John Wiley & Sons, Inc, 1994, vol. 1-4
- Molecular Cloning: A Laboratory Manual. Cold Spring Harbor Laboratory Press, 1989, vol. 1-3
- **RADEMAKER et al.** *IJSEM,* March 2000, vol. 50 (2), 665-677
- *J. Clin. Microbiol.,* December 2003, vol. 41 (12), 5456-5465
- *Appl. Environ. Microbiol.,* January 2002, vol. 68 (1), 245-253
- **WANG.** *PLOS,* 17 November 2003
- *J. Clin. Microbiol.,* January 1995, vol. 33 (1), 248-250
- *PNAS,* 01 July 1984, vol. 81 (13), 4154-4158
- *PNAS,* 23 May 2006, vol. 103 (21), 8107-8112
- *Lancet,* 24 May 2003, vol. 361 (9371), 1779-1785
- *J. Clin. Microbiol.,* June 1999, vol. 37 (6), 1661-1669
- **WOLK et al.** *Eur. J. Immunol.,* vol. 36, 1309-1323
- *Molecular Microbiology,* vol. 44, 9-19
- *J Virol Methods.,* December 2009, vol. 162 (1-2), 194-202
- *Methods Mol Biol.,* 2009, vol. 508, 63-74
- *PLOS,* 08 November 2012
- *PLOS,* 16 June 2011
- *BioTechniques,* March 2005, vol. 38, 451-458
- *Genetics,* 01 November 1992, vol. 132 (3), 665-673
- **BÜSSOW ; KONRAD et al.** *Nucleic Acids Research,* 1998, vol. 26.21, 5007-5008
- **ASHOK KUMAR ; AKSHAY SRIVASTAVA.** Cell separation using cryogel-based affinity chromatography. *Nature protocols,* 2010, vol. 5, 1737-1747
- **BREWSTER J.D.** Isolation and concentration of Salmonellae with an immunoaffinity column. *J Microbiol Methods,* October 2003, vol. 55 (1), 287-93
- **WANG et al.** Open-Tubular Capillary Cell Affinity Chromatography: Single and Tandem Blood Cell Separation. *Anal. Chem.,* 2008, vol. 80, 2118-2124
- **ZENG, H. ; SUN, S.** Syntheses, Properties, and Potential Applications of Multicomponent Magnetic Nanoparticles. *Adv. Funct. Mater.,* 2008, vol. 18, 391-400
- *J. Mater. Chem.,* 13 February 2004, vol. 14, 1336-1341
- **HUNTER et al.** Immunoassays for Clinical Chemistry. 1983, 147-162
- **STANLEY.** *Essentials in Immunology and Serology,* 2002, 152-153
- **HARRIS T. D. et al.** *Science,* 2008, vol. 320, 106-109
- **BRASLAVSKY et al.** *PNAS (USA),* 2003, vol. 100, 3960-3964

- **MARGULIES, M et al.** *Nature,* 2005, vol. 437, 376-380
- **SONI G V ; MELLER A.** *Clin Chem,* 2007, vol. 53, 1996-2001
- **MOUDRIANAKIS E. N. ; BEER M.** *Proc Natl Acad Sci USA.,* March 1965, vol. 53, 564-71
- **ZHOU et al.** Microarrays for bacterial detection and microbial community analysis. *Current Opinion in Microbiology,* 2003, vol. 6, 288-294
- *Syst Appl Microbiol.,* June 2010, vol. 33 (4), 175-82
- *IJSEM,* November 2003, vol. 53 (6), 1893-1900
- *Nature,* 24 December 2009, vol. 462 (7276), 1056-1060
- *Korean J Parasitol.,* 26 October 2009, vol. 47, 51-58
- **GENES ; GENETIC.** *Systems,* 2004, vol. 79 (3), 129-137
- *J. Virol.,* August 1983, vol. 47 (2), 267-275
- **GROISMAN.** In vivo genetic engineering with bacteriophage Mu. *Methods in Enzymology,* 1991, vol. 202, 180-212
- **CRAIN.** *Mud-P22, Methods Enzymology,* 2007, vol. 421, 249-59
- **CHOPIN et al.** *J Bact,* 2002, vol. 184 (7), 2030-2033
- **MURAMATSU et al.** Two generalized transducing phages in Vibrio parahaemolyticus and Vibrio alginolyticus. *Microbiol Immunol,* 1991, vol. 35 (12), 1073-1084
- **REGUE et al.** A generalized transducing bacteriophage for Serratia marcescens. *Res Microbiol,* 1991, vol. 42 (1), 23-27
- **KIESEL et al.** Phage Acm1-mediated transduction in the facultatively methanol-utilizing Acetobacter methanolicus MB 58/4. *J. Gen Virol,* 1993, vol. 74 (9), 1741-1745
- **ZHANG et al.** *Food Microbiol,* 2012, vol. 31 (1), 133-36
- **WELKER.** Transduction in Bacillus stearothermophilus. *J. Bacteriol,* 1988, vol. 176 (11), 3354-3359
- **DARZINS et al.** Mini-D3112 bacteriophage transposable elements for genetic analysis of Pseudomonas aeruginosa. *J. Bacteriol,* 1989, vol. 171 (7), 3909-3916
- **BLAHOVA et al.** Transduction or imipenem resistance by the phage F-116 from a nosocomial strain of Pseudomonas aeruginosa isolated in Slovakia. *Acta Virol,* 1994, vol. 38 (5), 247-250
- **WEISS et al.** Isolation and characterization of a generalized transducing phage for Xanthomonas campestris pv. campestris. *J. Bacteriol,* 1994, vol. 176 (11), 3354-3359
- **SCHICKLMAIER et al.** Frequency of generalized transducing phages in natural isolates of the Salmonella typhimurium complex. *Appl Environ Microbiol,* 1995, vol. 61 (4), 1637-1640
- **HUMPHREY et al.** Purification and characterization of VSH-1, a generalized transducing bacteriophage of Serpulina hyodysenteriae. *J Bacteriol,* 1997, vol. 179 (2), 323-329
- **WILLI.** Transduction of antibiotic resistance markers among Actinobacillus actinomycetemcomitans strains by temperate bacteriophages Aa phi 23. *Cell Mol Life Sci,* 1997, vol. 53 (11-12), 904-910
- **NEDELMANN et al.** Generalized transduction for genetic linkage analysis and transfer of transposon insertions in different Staphylococcus epidermidis strains. *Zentiviralalbl Bakteriol,* 1998, vol. 287 (1-2), 85-92
- **MILLER et al.** Complete genome sequence of the broad-host-range vibriophage KVP40: comparative genomics of a T4-related bacteriophage. *J Bact,* 2003, vol. 185 (17), 5220-5233
- **BEUMER.** A broad-host-range, generalized transducing phage SN-T acquires 16S rRNA genes from different genera of bacteria. *Appl Env Microb,* 2005, vol. 71 (12), 8301-8304
- **GREEN et al.** Isolation and preliminary characterization of lytic and lysogenic phages with wide host range within the streptomycetes. *J. Gen Microbiol,* 1985, vol. 131 (9), 2459-2465
- **JENSEN et al.** Prevalence of broad-host-range lytic bacteriophages of Sphaerotilus natans, Escherichia coli, and Pseudomonas aeruginosa. *Appl Environ Microbiol,* 1998, vol. 64 (2), 575-580
- **BAMFORD et al.** Bacteriophage PRD1: a broad host range dsDNA tectivirus with an internal membrane. *Adv Virus Res,* 1995, vol. 45, 281-319
- **SCHWUDKE et al.** Broad-host-range Yersinia phage PY100: genome sequence, proteome analysis of virions, and DNA packaging strategy. *J Bact,* 2008, vol. 190 (1), 332-342
- **OLSEN et al.** Characteristics of PRD1, a plasmid-dependent broad host range DNA bacteriophage. *J Viriol,* 1974, vol. 14 (3), 689-699
- **SANTOS et al.** Genomic and proteomic characterization of the broad host range Salmonella phage PVP-SE1: creation of a new phage genus. *J Viriol,* 2011, vol. 85 (21), 11265-73
- **SILLANKORVA et al.** Efficacy of a broad host range lytic bacteriophage against E. coli adhered to urothelium. *Curr Microbiol,* vol. 62 (4), 1128-1132
- **EVANS et al.** Characterization of a broad-host-range flagellum-dependent phage that mediates high-efficiency generalized transduction in, and between. *Serratia and Pantoea, Microbiol,* 2010, vol. 156, 240-247

- **GARBE et al.** Characterization of JG024, a Pseudomonas aeruginosa PB1-like broad-host range phage under simulated infection conditions. *BMC Microbiol,* 2010, vol. 10, 301
- **SCHWARZER et al.** A multivalent adsorption apparatus explains the broad host range of phage phi92: a comprehensive genomic and structural analysis. *J Viriol JVI.00801-12,* 2012
- **KELLY et al.** Development of a broad-host-range phage cocktail for biocontrol. *Bioengineered Bugs,* 2011, vol. 2 (1), 31-37
- **LUBITZ et al.** Applications of bacterial ghosts in biomedicine. *Adv Exp Med Biol,* 2009, vol. 655, 159-70
- **LANGEMANN et al.** The bacterial ghost platform system: production and applications. *Bioeng Bugs,* 2010, vol. 1 (5), 326-36
- **TABRIZI et al.** Bacterial ghosts-biological particles as delivery systems for antigens. *nucleic acids, and drugs, Curr Opp Biotechnol,* 2004, vol. 15, 530-537
- **GREEN ; SAMBROOK.** Molecular Cloning: A Laboratory Manual. Cold Spring Harbor Laboratory Press, 2012, 2, , 000
- **PRIMROSE ; TWYMAN.** Principles of Gene Manipulation and Genomics. Wiley-Blackwell, 2006, 672
- **PRISCO ; BERARDINIS.** Filamentous bacteriophage Fd as an antigen delivery system in vaccination. *Int J. Mol. Sci.,* 2012, vol. 13, 5179-5194
- **GAHAN et al.** Bacterial antigen expression is an important component in inducing an immune response to orally administered Salmonella-delivered DNA vaccines. *PLoS One,* 2009, vol. 4 (6), e6062
- **MURPHY.** Janeway's Immunobiology. Garland Science, 2011, 888
- **EDGAR et al.** High-sensitivity bacterial detection using biotin-tagged phage and quantum-dot nanocomplexes. *PNAS,* 2006, vol. 103 (13), 4841-4945
- **GERVIAS et al.** Immobilization of biotinylated bacteriophages on biosensor surfaces. *Sensors and Actuators B,* 2007, vol. 125, 615-621
- **SMELYANSKI ; GERSHONI.** Site directed biotinylation of filamentous phage structural proteins. *Virol J,* 2011, vol. 8, 495
- **HAQ.** Bacteriophages and their implications on future biotechnology, a review. *Virol J,* 2012, vol. 9, 9
- **DRANCOURT et al.** Diagnosis of Mediterranean spotted fever by indirect immunofluorescence of Rickettsia conorii in circulating endothelial cells isolated with monoclonal antibody-coated immunomagnetic beads. *J Inf Dis,* 1992, vol. 166, 660-3
- **FU et al.** *Rapid detection of E. coli O157:H7*
- **FREDRICKSON ; ZAN.** Macro-to-micro interfaces for microfluidic devices. *Lab Chip,* 2004, vol. 4 (6), 526-33
- **HALL ; HARTNETT.** Measurement of bacterial contamination on surfaces. *Pub Health Report,* 1964, vol. 79, 1012-1024
- **MURPHY.** Janeway's Immunobiology 8 Ed. Garland Science, 2011, 888
- **KONTERMANN.** Antibody Engineering. Springer-Verlag, 2010, vol. 1, 800
- **HARLOW.** Antibodies: A Laboratory Manual. Cold Spring Harbor Laboratory Press, 1988, 726
- **STANLEY.** Essentials in Immunology and Serology. *Delmar Cengage (Independence, KY),* 2002, 560
- **HIJMANS et al.** An immunofluorescence procedure for the detection of intracellular immunoglobulins. *Clin Exp Immunol,* 1969, vol. 4, 457-472
- **GODING.** Conjugation of antibodies with fluorochromes: modifications to the standard methods. *J Immunol Meth,* 1976, vol. 13, 215-226
- **ENGVALL.** Enzyme immunoassay ELISA and EMIT. *J Clin Micro,* 1980, vol. 70, 419-439